# EUROPEAN PATENT APPLICATION

(11) **EP 4 455 131 A1**
(43) Date of publication of application: **30.10.2024**
(21) Application number: 22910167.0
(22) Date of filing: 23.12.2022
(51) Int. Cl.: C07D 401/10, A61K 31/395, A61P 35/00

(54) **AROMATIC VINYL COMPOUND, METAL COMPLEX THEREOF, AND PREPARATION METHOD THEREFOR AND USE THEREOF**

(30) Priority: 24.12.2021 WO PCT/CN2021/141330; 16.12.2022 CN 202211627117
(71) Applicant: Shanghai Maxinovel Pharmaceuticals Co., Ltd., Shanghai 201203 (CN)
(72) Inventor: WANG, Yuguang, Shanghai 201203 (CN); CAI, Senlin, Shanghai 201203 (CN); HE, Min, Shanghai 201203 (CN); FENG, Zhenhua, Shanghai 201203 (CN); WU, Xinliang, Shanghai 201203 (CN); ZHAO, Qiang, Shanghai 201203 (CN)
(74) Representative: Spencer, Michael David
(86) International application number: PCT/CN2022/141252
(87) International publication number: WO 2023/116856

(57) **Abstract**

Provided are an aromatic vinyl compound, a metal complex thereof, and a preparation method therefor and the use thereof. The aromatic vinyl compound and the metal complex thereof have an inhibitory activity on the binding of PD-1/PD-L1, and therefore the aromatic vinyl compound and the metal complex thereof can be used for treating related diseases such as tumors. In addition, the obtained metal complex can also be used as an imaging agent.

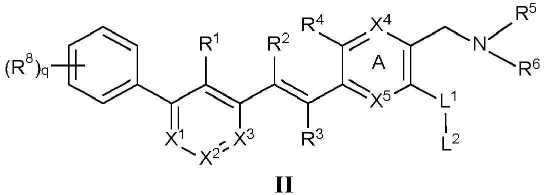

## Description

The present application claims the priority of PCT patent application PCT/CN2021/141330 with the filing date of December 24, 2021, and the present application claims the priority of Chinese patent application CN202211627117.3 with the filing date of December 16, 2022. The contents of the above patent applications are incorporated herein by reference in their entireties.

### TECHNICAL FIELD

The present disclosure relates to an aromatic vinyl compound, metal complex thereof, and preparation method therefor and use thereof.

### BACKGROUND

Nuclear medicine which is mainly based on radionuclides and their labeled compounds is an important branch of modern medicine and applies nuclear technology to the research, diagnosis and treatment of diseases. Radiopharmaceuticals refer to a type of special drugs containing radionuclides for medical diagnosis and treatment, and they are radionuclide-labeled compounds or biological agents used for medical diagnosis or treatment in the body and are further divided into diagnostic radiopharmaceuticals and therapeutic radiopharmaceuticals. These two types of drugs include radioactive non-metal drugs and radioactive metal drugs. The former includes radioactive non-metal nuclide drugs represented by ¹³¹I, ¹⁸F, and so on, while the latter includes radioactive metal nuclide drugs represented by ⁶⁸Ga, ¹⁷⁷Lu, ¹⁸⁶Re and so on.

PD-1 (programmed death 1) is an important immunosuppressive molecule. It is a member of CD28 superfamily and was originally cloned from the apoptotic mouse T-cell hybridoma 2B4.11. Immunomodulation targeting PD-1 is of great importance to anti-tumor, anti-infection, anti-autoimmune diseases and survival of organ transplantation. Its ligand PD-L1 can also be served as a target, and the corresponding antibodies and small molecular drugs can also inhibit the binding of PD-1 to PD-L1, thus serving to activate the immune response to destroy tumor cells.

Aromatic vinyl derived compounds containing radioactive metal elements have not been successfully marketed as small molecule nucleophiles targeting PD-L1 in the current state of the art, and there have been no technical reports of such compounds being used in PET tumor imaging and in tumor therapy.

### CONTENT OF THE PRESENT INVENTION

The purpose of the present disclosure is to provide a novel aromatic vinyl compound, metal complex thereof, and preparation method therefor and use thereof.

The present disclosure provides a compound represented by general formula II or a metal complex thereof, or a tautomer or stereoisomer of any one of described above (namely, the compound represented by general formula II or the metal complex thereof), or a pharmaceutically acceptable salt of any one of described above (namely, the compound represented by general formula II or the metal complex thereof, the tautomer thereof, or the stereoisomer thereof), or a solvate of any one of described above (namely, the compound represented by general formula II or the metal complex thereof, the tautomer thereof, the stereoisomer thereof, or the pharmaceutically acceptable salt thereof): 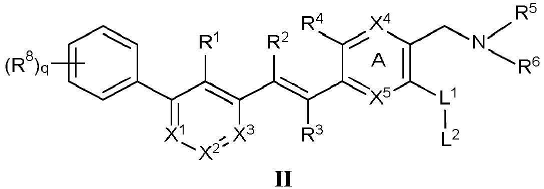
X¹, X², X³, X⁴, and X⁵ are each independently CH or N;
R¹ is hydrogen, halogen, cyano, C₁-C₄ alkyl, or C₁-C₄ alkyl substituted by one or more R^{a};
R² and R³ are each independently hydrogen or halogen;
R⁴ is hydrogen, halogen, C₁-C₄ alkyl, or C₁-C₄ alkyl substituted by one or more R^{b};
R⁵ and R⁶ are each independently hydrogen, deuterium, C₁-C₄ alkyl, or C₁-C₄ alkyl substituted by one or more R^{c}; or, R⁵, R⁶ together with the nitrogen atom to which they are attached form a 5- to 7-membered heterocyclic ring, or a 5- to 7-membered heterocyclic ring substituted by one or more R^{c}; in the 5- to 7-membered heterocyclic ring, the number of heteroatoms is 1, 2, 3, or 4, and each heteroatom is independently selected from N, O, and S;
each R^{a}, each R^{b} and each R^{c} are independently deuterium, halogen, hydroxyl, amino, C₁-C₄ alkyl, C₁-C₄ alkyl-O-, -COOH, or -C(O)OR^{g};
each R⁸ is independently hydrogen, deuterium, halogen, hydroxyl, amino, C₁-C₄ alkyl, C₁-C₄ alkyl-S-, C₁-C₄ alkyl-O-, -C(O) NH2, -C(O)OC₁₋₄ alkyl, -OR^{8a}, -NHR^{8a}, -NR^{8a}R^{8b}, -NH-C(O)-R^{8d}, C₁-C₄ alkyl substituted by one or more R^{8c}, C₁-C₄ alkyl-S- substituted by one or more R^{8c}, or C₁-C₄ alkyl-O- substituted by one or more R^{8c};
or, two adjacent R⁸ together with the carbon atoms on the benzene ring to which they are attached form a 5- to 7-membered carbocyclic ring, a 5- to 7-membered heterocyclic ring, a 5- to 7-membered carbocyclic ring substituted by one or more C₁₋₄ alkyl, or a 5- to 7-membered heterocyclic ring substituted by one or more C₁₋₄ alkyl; in the 5- to 7-membered heterocyclic ring, the number of heteroatoms is 1, 2, 3, or 4, and each heteroatom is independently selected from N and O;
each R^{8a}, R^{8b}, and each R^{8c} are independently C₁-C₄ alkyl-S-, halogen, C₁-C₄ alkyl, C₁-C₄ alkyl-O-, -COOH, -(C₁-C₄ alkylene)-COOH, -C(O)OC₁-C₄ alkyl, -C(O)NH₂, - C(O)NHC₁-C₄ alkyl, 5- to 7-membered heterocyclic ring, or -NR^{8e}R^{8f}; in the 5- to 7-membered heterocyclic ring, the species of heteroatoms are independently selected from one or more of N, O, and S, and the number of heteroatoms is independently 1, 2, or 3;
R^{8e} and R^{8f} are each independently hydrogen, halogen, C₁-C₄ alkyl, or C₁-C₄ alkyl substituted by one or more R^{8g};
each R^{8g} is independently halogen, C₁-C₄ alkyl, hydroxyl, -NR^{8h}R^{8k}, C₁-C₄ alkyl-O-, -COOH, -(C₁-C₄ alkylene)-COOH, -C(O)OC₁-C₄ alkyl, -C(O)NH₂, or -C(O)NHC₁-C₄ alkyl;
R^{8h} and R^{8k} are each independently hydrogen or C₁-C₄ alkyl;
each R^{8d} is independently C₆-C₁₀ aryl substituted by one or more R^{8d-1}, or 5- to 10-membered heteroaryl substituted by one or more R^{8d-2}; in the 5- to 10-membered heteroaryl, the species of heteroatoms are independently selected from one or more of N, O, and S, and the number of heteroatoms is independently 1, 2, or 3;
each R^{8d-1} and R^{8d-2} are independently C₁-C₄ alkoxy or C₁-C₄ alkyl substituted by one or more R^{8d-1-1};
each R^{8d-1-1} is independently 5- to 7-membered heterocyclic ring substituted by one carboxyl; in the 5- to 7-membered heterocyclic ring, the species of heteroatoms are independently selected from one or more of N, O, and S, and the number of heteroatoms is independently 1, 2, or 3;
q is 0, 1, 2, or 3;
L¹ is
   (i) a single bond or -(CH₂)ₙ-;
   (ii) -(CH₂)ₘ-, in which 1, 2, 3, 4, or 5 non-adjacent CH₂ are independently replaced by -Y₁-, and each Y₁ is independently -C(O)-, -C(O)O-, -O-, -NH-, -C(O)NH-, or -NHC(O)NH-; or
   (iii) -(CH₂)ₚ-, in which one CH₂ is replaced by -Y₂-, and the other 0, 1, 2, 3, or 4 non-adjacent CH₂ are independently replaced by -Y₃-; each Y₃ is independently -C(O)-, -C(O)O-, -O-, -NH-, -C(O)NH-, or -NHC(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
L¹ is unsubstituted or 1, 2, or 3 H contained in L¹ are each independently substituted by R⁷;
n, m, and p are each independently 1, 2, 3, 4, 5, 6, 7, 8, 9, 10, 11, 12, 13, or 14;
each R⁷ is independently C₁-C₄ alkyl or -L³-R⁹;
L³ is
   (i) -(CH₂)ⱼ-; or
   (ii) -(CH₂)ₖ-, in which 1, 2, 3, or 4 non-adjacent CH₂ are independently replaced by -Y₄-, and each Y₄ is independently -C(O)-, -C(O)O-, -O-, -NH-, -C(O)NH-, or -NHC(O)NH-;
L³ is unsubstituted or 1, 2, or 3 H contained in L³ are each independently substituted by R¹⁰;
j and k are each independently 1, 2, 3, 4, 5, 6, 7, 8, 9, 10, 11, 12, 13, or 14;
R⁹ is hydrogen, C₆-C₁₀ aryl, or C₆-C₁₀ aryl substituted by one or more R^{d};
each R¹⁰ is independently C₁-C₄ alkyl;
each R^{d} is independently C₁-C₄ alkyl or C₁-C₄ alkyl substituted by one or more R^{e};
each R^{e} is independently hydroxyl, amino, C₁-C₄ alkyl, C₁-C₄ alkyl-O-, -COOH, or - C(O)OR^{h};
R^{g} and R^{h} are each independently C₁-C₄ alkyl or halogenated C₁-C₄ alkyl;
L² is metal chelating group;
the metal complex is a complex chelated by the compound represented by general formula II with a metal atom or ion.
In some embodiments, q is 0, 1, or 2;
each R⁸ is independently C₁-C₄ alkyl, -NH-C(O)-R^{8d}, or C₁-C₄ alkyl-O- substituted by one or more R^{8c};
each R^{8c} is independently 5- to 7-membered heterocyclic ring; in the 5- to 7-membered heterocyclic ring, the species of heteroatoms are independently selected from one or more of N, O, and S, and the number of heteroatoms is independently 1, 2, or 3;
each R^{8d} is independently C₆-C₁₀ aryl substituted by one or more R^{8d-1}, or 5- to 10-membered heteroaryl substituted by one or more R^{8d-2}; in the 5- to 10-membered heteroaryl, the species of heteroatoms are independently selected from one or more of N, O, and S, and the number of heteroatoms is independently 1, 2, or 3;
each R^{8d-1} and R^{8d-2} are independently C₁-C₄ alkoxy or C₁-C₄ alkyl substituted by one or more R^{8d-1-1};
each R^{8d-1-1} is independently 5- to 7-membered heterocyclic ring substituted by one carboxyl; in the 5- to 7-membered heterocyclic ring, the species of heteroatoms are independently selected from one or more of N, O, and S, and the number of heteroatoms is independently 1, 2, or 3.

In some embodiments, q is 0.

In some embodiments, R¹ is cyano or C₁-C₄ alkyl; X¹, X², and X³ are each independently CH or N.

In some embodiments, R¹ is cyano or C₁-C₄ alkyl; X¹ and X² are CH, and X³ is N.

In some embodiments, R² and R³ are hydrogen.

In some embodiments, R⁴ is C₁-C₄ alkyl or C₁-C₄ alkyl substituted by one or more R^{b}; each R^{b} is independently halogen; X⁴ and X⁵ are each independently CH or N.

In some embodiments, R⁴ is C₁-C₄ alkyl or C₁-C₄ alkyl substituted by one or more R^{b}; each R^{b} is independently halogen; X⁴ and X⁵ are each independently CH.

In some embodiments, R⁵, R⁶ together with the nitrogen atom to which they are attached form a 5- to 7-membered heterocyclic ring substituted by one or more R^{c}; in the 5- to 7-membered heterocyclic ring, the number of heteroatoms is 1, 2, 3, or 4, and each heteroatom is independently selected from N, O, and S;

R^{c} is -COOH.

In some embodiments, L¹ is
(ii) -(CH₂)ₘ-, in which 1, 2, 3, 4, or 5 non-adjacent CH₂ are independently replaced by -Y₁-, and each Y₁ is independently -C(O)O-, -O-, or -C(O)NH-; or
(iii) -(CH₂)ₚ-, in which one CH₂ is replaced by -Y₂-, and the other 1, 2, 3, or 4 non-adjacent CH₂ are independently replaced by -Y₃-; each Y₃ is independently -C(O)-, -O-, or C(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
   L¹ is unsubstituted or one H contained in L¹ is each independently substituted by R⁷;
   m and p are each independently 5, 6, 7, 8, 9, 10, or 11;
   R⁷ is -L³-R⁹;
   L³ is
(ii) -(CH₂)ₖ-, in which 1, 2, 3, or 4 non-adjacent CH₂ are independently replaced by - Y₄-, and each Y₄ is -C(O)NH-;
   L³ is unsubstituted;
   k is 7, 8, or 9;
   R⁹ is C₆-C₁₀ aryl substituted by one or more R^{d}; each R^{d} is independently C₁-C₄ alkyl.

In some embodiments, the metal complex is a complex chelated by the compound represented by general formula II with the metal ion.

In some embodiments, X¹, X², X³, X⁴, and X⁵ are each independently CH or N;
R¹ is cyano or C₁-C₄ alkyl;
R² and R³ are hydrogen;
R⁴ is C₁-C₄ alkyl or C₁-C₄ alkyl substituted by one or more R^{b}; each R^{b} is independently halogen;
R⁵, R⁶ together with the nitrogen atom to which they are attached form a 5- to 7-membered heterocyclic ring substituted by one or more R^{c}; in the 5- to 7-membered heterocyclic ring, the number of heteroatoms is 1, 2, 3, or 4, and each heteroatom is independently selected from N, O, and S;
R^{c} is -COOH;
q is 0, 1, or 2;
each R⁸ is independently C₁-C₄ alkyl, -NH-C(O)-R^{8d}, or C₁-C₄ alkyl-O- substituted by one or more R^{8c};
each R^{8c} is independently 5- to 7-membered heterocyclic ring; in the 5- to 7-membered heterocyclic ring, the species of heteroatoms are independently selected from one or more of N, O, and S, and the number of heteroatoms is independently 1, 2, or 3;
each R^{8d} is independently C₆-C₁₀ aryl substituted by one or more R^{8d-1}, or 5- to 10-membered heteroaryl substituted by one or more R^{8d-2}; in the 5- to 10-membered heteroaryl, the species of heteroatoms are independently selected from one or more of N, O, and S, and the number of heteroatoms is independently 1, 2, or 3;
each R^{8d-1} and R^{8d-2} are independently C₁-C₄ alkoxy or C₁-C₄ alkyl substituted by one or more R^{8d-1-1};
each R^{8d-1-1} is independently 5- to 7-membered heterocyclic ring substituted by one carboxyl; in the 5- to 7-membered heterocyclic ring, the species of heteroatoms are independently selected from one or more of N, O, and S, and the number of heteroatoms is independently 1, 2, or 3;
L¹ is
   (ii) -(CH₂)ₘ-, in which 1, 2, 3, 4, or 5 non-adjacent CH₂ are independently replaced by -Y₁-, and each Y₁ is independently -C(O)O-, -O-, or -C(O)NH-; or
   (iii) -(CH₂)ₚ-, in which one CH₂ is replaced by -Y₂-, and the other 1, 2, 3, or 4 non-adjacent CH₂ are independently replaced by -Y₃-; each Y₃ is independently -C(O)-, -O-, or C(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
L¹ is unsubstituted or one H contained in L¹ is each independently substituted by R⁷;
m and p are each independently 5, 6, 7, 8, 9, 10, or 11;
R⁷ is -L³-R⁹;
L³ is
   (ii) -(CH₂)ₖ-, in which 1, 2, 3, or 4 non-adjacent CH₂ are independently replaced by - Y₄-, and each Y₄ is -C(O)NH-;
L³ is unsubstituted;
k is 7, 8, or 9;
R⁹ is C₆-C₁₀ aryl substituted by one or more R^{d}; each R^{d} is independently C₁-C₄ alkyl;
L² is metal chelating group;
the metal complex is a complex chelated by the compound represented by general formula II with a metal atom or ion.

In some embodiments, X¹, X², X⁴, and X⁵ are CH;
X³ is CH or N;
R¹ is cyano or C₁-C₄ alkyl;
R² and R³ are hydrogen;
R⁴ is C₁-C₄ alkyl or C₁-C₄ alkyl substituted by one or more R^{b}; each R^{b} is independently halogen;
R⁵, R⁶ together with the nitrogen atom to which they are attached form a 5- to 7-membered heterocyclic ring substituted by one or more R^{c}; in the 5- to 7-membered heterocyclic ring, the number of heteroatoms is 1, 2, 3, or 4, and each heteroatom is independently selected from N, O, and S;
R^{c} is -COOH;
q is 0;
L¹ is
   (ii) -(CH₂)ₘ-, in which 1, 2, 3, 4, or 5 non-adjacent CH₂ are independently replaced by -Y₁-, and each Y₁ is independently -C(O)O-, -O-, or -C(O)NH-; or
   (iii) -(CH₂)ₚ-, in which one CH₂ is replaced by -Y₂-, and the other 1, 2, 3, or 4 non-adjacent CH₂ are independently replaced by -Y₃-; each Y₃ is independently -C(O)-, -O-, or C(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
L¹ is unsubstituted or one H contained in L¹ is each independently substituted by R⁷;
m and p are each independently 5, 6, 7, 8, 9, 10, or 11;
R⁷ is -L³-R⁹;
L³ is
   (ii) -(CH₂)ₖ-, in which 1, 2, 3, or 4 non-adjacent CH₂ are independently replaced by - Y₄-, and each Y₄ is -C(O)NH-;
L³ is unsubstituted;
k is 7, 8, or 9;
R⁹ is C₆-C₁₀ aryl substituted by one or more R^{d}; each R^{d} is independently C₁-C₄ alkyl;
L² is metal chelating group;
the metal complex is a complex chelated by the compound represented by general formula II with the metal ion.

In some embodiments, the stereoisomer is chiral isomer.

In some embodiments, in R¹, the C₁-C₄ alkyl is methyl or ethyl.

In some embodiments, in R⁴, the C₁-C₄ alkyl is methyl or ethyl.

In some embodiments, in R⁴, the C₁-C₄ alkyl in the C₁-C₄ alkyl substituted by one or more R^{b} is methyl or ethyl.

In some embodiments, in R^{b}, the halogen is fluorine or chlorine.

In some embodiments, in R⁴, the C₁-C₄ alkyl substituted by one or more R^{b} is trifluoromethyl.

In some embodiments, in the 5- to 7-membered heterocyclic ring substituted by one or more R^{c}, which is formed by R⁵, R⁶ together with the nitrogen atom they are attached, the 5- to 7-membered heterocyclic ring is a 6-membered saturated monocyclic heterocyclic ring.

In some embodiments, in the 5- to 7-membered heterocyclic ring substituted by one or more R^{c}, which is formed by R⁵, R⁶ together with the nitrogen atom they are attached, the number of heteroatoms is 1.

In some embodiments, in the 5- to 7-membered heterocyclic ring substituted by one or more R^{c}, which is formed by R⁵, R⁶ together with the nitrogen atom they are attached, the heteroatom is N.

In some embodiments, in the 5- to 7-membered heterocyclic ring substituted by one or more R^{c}, which is formed by R⁵, R⁶ together with the nitrogen atom they are attached, the 5- to 7-membered heterocyclic ring is piperidine ring.

In some embodiments, the 5- to 7-membered heterocyclic ring substituted by one or more R^{c}, which is formed by R⁵, R⁶ together with the nitrogen atom they are attached is

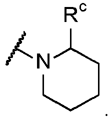

In some embodiments, the 5- to 7-membered heterocyclic ring substituted by one or more R^{c}, which is formed by R⁵, R⁶ together with the nitrogen atom they are attached is 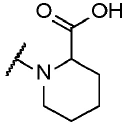 (for example, 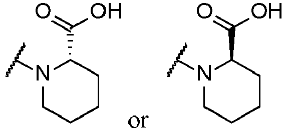 ).

In some embodiments, the 5- to 7-membered heterocyclic ring substituted by one or more R^{c}, which is formed by R⁵, R⁶ together with the nitrogen atom to which they are attached is

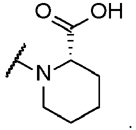

In some embodiments, L¹ is connected to ring A through -Y₁-, and Y₁ connected to ring A is -O-.

In some embodiments, L¹ is connected to L² via -CH₂-.

In some embodiments, L¹ is -O(CH₂)ₙ₂-, -O(CH₂)ₙ₂O(CH₂)ₘ₂-, - O(CH₂)ₙ₂O(CH₂)ₘ₂O(CH₂)ₘ₃-, -O(CH₂)ₙ₂OC(O)(CH₂)ₘ₂-, -O(CH₂)ₙ₂NHC(O)(CH₂)ₘ₂-, - O(CH₂)ₙ₂NHC(O)-(CH₂)ₙ₃-NHC(O)(CH₂)ₘ₃-, -O(CH₂)ₙ₂-O(CH₂)ₙ₃-NHC(O)-(CH₂)ₙ₄NHC(O)-(CH₂)ₘ₃-, -O(CH₂)ₙ₂NHC(O)-Y₂-(CH₂)ₙ₃NHC(O)-(CH₂)ₘ₃-, -O(CH₂)ₙ₂-Y₂-C(O)-(CH₂)ₘ₃-, -O(CH₂)ₙ₂NHC(O)-Y₂-C(O)-(CH₂)ₘ₃-, or -O(CH₂)ₙ₂NHC(O)-Y₂-(CH₂)ₙ₃NHC(O)-(CH₂)ₙ₄NHC(O)-(CH₂)ₘ₃-; the right end of L¹ is connected to L²;
each n2, each n3, each n4, each m2 and each m3 are independently 1, 2, 3, 4, 5, or 6;
L¹ is unsubstituted or one H contained in L¹ is substituted by R⁷;

In some embodiments, m2 is 1 or 2.

In some embodiments, m3 is 1 or 2.

In some embodiments, m2 is 2 and m3 is 2.

In some embodiments, m2 is 1.

In some embodiments, m3 is 1.

In some embodiments, when Y₂ is 5- to 7-membered carbocyclic ring, the 5- to 7-membered carbocyclic ring is 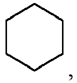 for example, 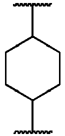 (for example, 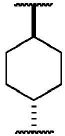 ).

In some embodiments, when Y₂ is 5- to 7-membered heterocyclic ring, the 5- to 7-membered heterocyclic ring is 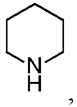 for example,

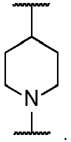

In some embodiments, R⁹ is phenyl or phenyl substituted by one or more R^{d}.

In some embodiments, in R^{d}, the C₁-C₄ alkyl is methyl or ethyl.

In some embodiments, R⁷ is

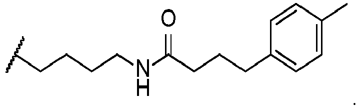

In some embodiments, L² is R¹¹ or -L⁴-(CH₂)ₛ-R¹¹;
s is 1, 2, or 3;
L⁴ is 5- to 7-membered carbocyclic ring (for example, benzene ring, for example, 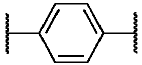 ) or 5- to 7-membered heterocyclic ring, in the 5- to 7-membered heterocyclic ring, the number of heteroatoms is 1, 2, 3, or 4, and each heteroatom is independently selected from N, O, and S;
R¹¹ is 8- to 20- membered (for example 8-, 9-, 10-, 11-, 12-, 13-, 14-, 15-, or 16-membered) saturated monocyclic or bridged carbocyclic ring; 3, 4, 5, or 6 non-adjacent CH₂ of the monocyclic or bridged carbocyclic ring are independently replaced by -Y₅-, and each Y₅ is independently -O-,-NH-, or -N (R^{11a})-;
each R^{11a} is independently C₁-C₄ alkyl or C₁-C₄ alkyl substituted by one or more - COOH (for example, -(C₁-C₄ alkylene)-COOH, for example, -CH₂-COOH).

In some embodiments, when L² is -L⁴-(CH₂)ₛ-R¹¹, the -L⁴-(CH₂)ₛ-R¹¹ is

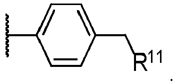

In some embodiments, each Y₅ is independently -NH- or -N(R^{11a})-.

In some embodiments, each R^{11a} is independently C₁-C₄ alkyl substituted by one or more -COOH.

In some embodiments, L² is R¹¹, and R¹¹ is defined as described herein.

In some embodiments, L² is 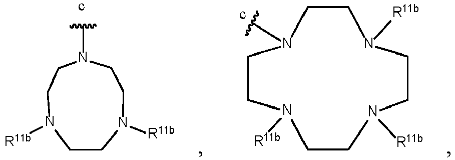 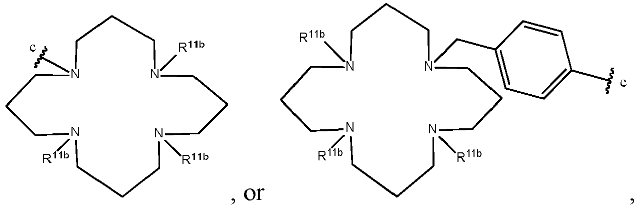 and each R^{11b} is independently H or R^{11a}, and R^{11a} is defined as described herein.

In some embodiments, in each structure of L² above, at least 1, 2, or 3 R^{11b} are R^{11a}, and R^{11a} is defined as described herein. In some embodiments, L² is 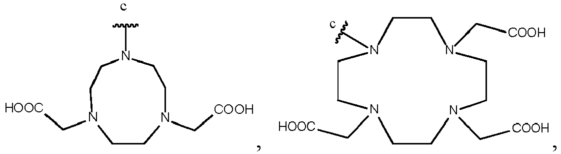 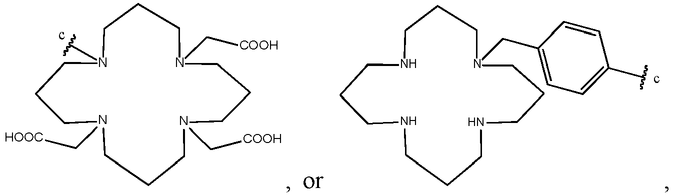 wherein the c-terminal is connected to L¹.

In some embodiments, L² is 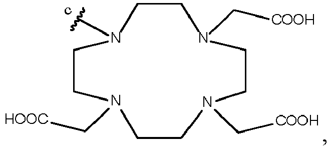 wherein the c-terminal is connected to L¹.

In some embodiments, the metal complex has a structure represented by the following general formula I: 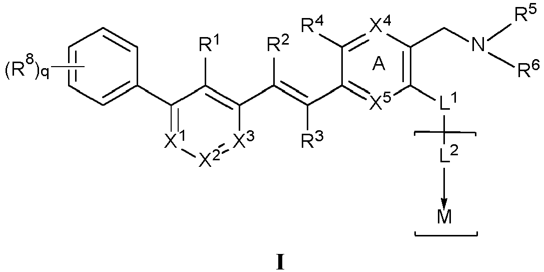 wherein, M is a metal atom or ion, and other variables are defined as in general formula II.

In some embodiments, M is the metal ion.

In some embodiments, the metal ion is as defined in the remaining section.

In some embodiments, the metal complex has the following structure: 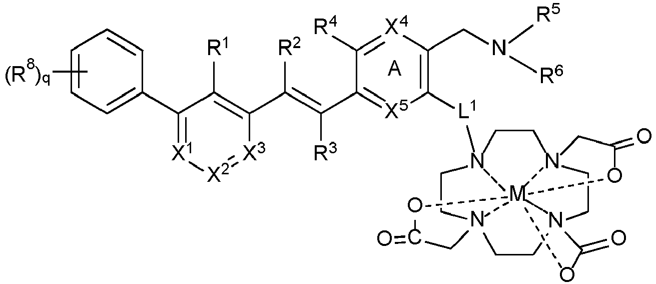 each variable is defined as described herein.

In some embodiments, in the metal complex, the molar ratio of the compound represented by general formula II to the metal atom or ion is 1: 1.

In some embodiments, the metal ion is ion of the following metals: Al, Cu, Ga, Y, Zr, Tc, In, Lu, Re, At, Bi, or Tl.

In some embodiments, the metal ion is ion of the following metals: ²⁷Al, ⁶³Cu, ⁶⁴Cu, ⁶⁸Ga, ⁷⁰Ga, ⁸⁹Y, ⁹⁰Y, ⁸⁹Zr, ⁹¹Zr, ^{99m}Tc, ¹¹¹In, ¹¹³In, ¹⁷⁵Lu, ¹⁷⁷Lu, ¹⁸⁶Re, ¹⁸⁸Re, ²¹¹At, ²¹²Bi, ²¹³Bi, ²⁰¹Tl, or ²⁰³Tl.

In some embodiments, the valence state of the metal ion is monovalent, divalent, trivalent, or tetravalent.

In some embodiments, the valence state of the metal ion is trivalent.

In some embodiments, the metal ion is radioactive metal ion or non-radioactive metal ion.

In some embodiments, the metal ion may further bind to non-metal nuclide, for example, F. The non-metal nuclide may be radioactive non-metal nuclide or non-radioactive non-metal nuclide. The radioactive non-metal nuclide can be ¹⁸F.

In some embodiments, the metal ion may not bind to non-metal nuclide.

In some embodiments, the metal ion may be radioactive as a whole (for example, if the metal ion is combined with non-metal nuclide, then the non-metal nuclide is radioactive, which is also radioactive as a whole).

In some embodiments, the metal ion is [AlF]²⁺, [GaCl]²⁺, [LuCl]²⁺, Ga³⁺, or Lu³⁺.

In some embodiments, the metal ion is [Al¹⁸F]²⁺, [⁶⁸GaCl]²⁺, [¹⁷⁷LuCl]²⁺, ⁶⁸Ga³⁺, or ¹⁷⁷Lu³⁺.

In some embodiments, 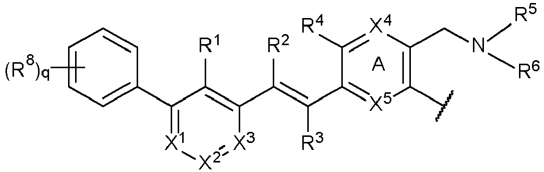 is

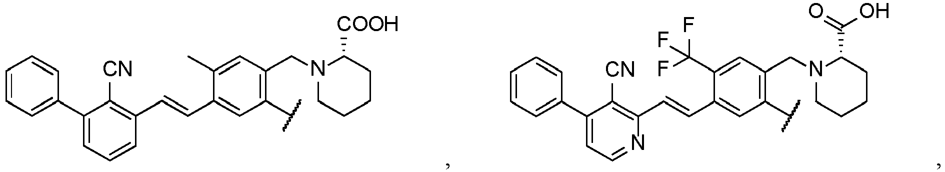

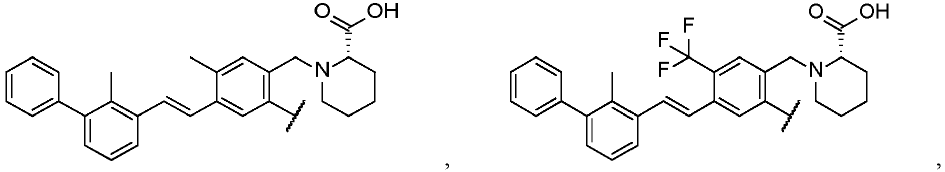

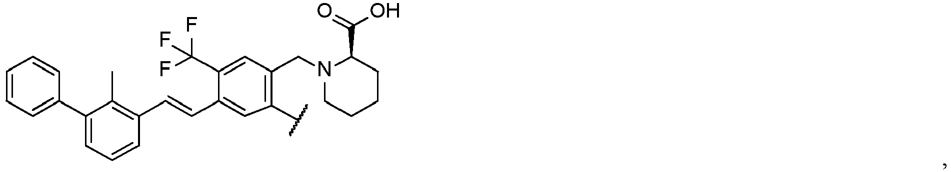

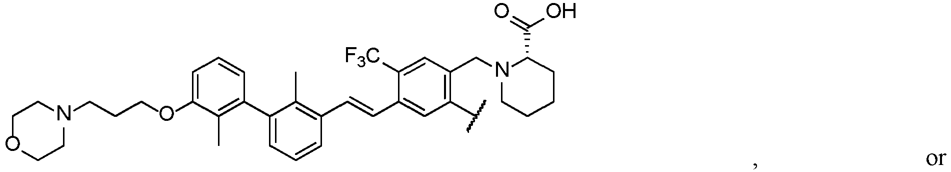

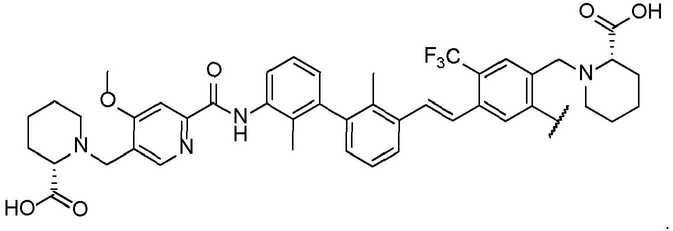

In some embodiments, L¹ is 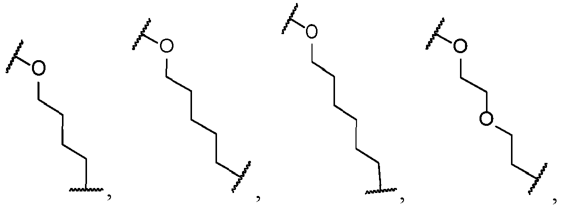 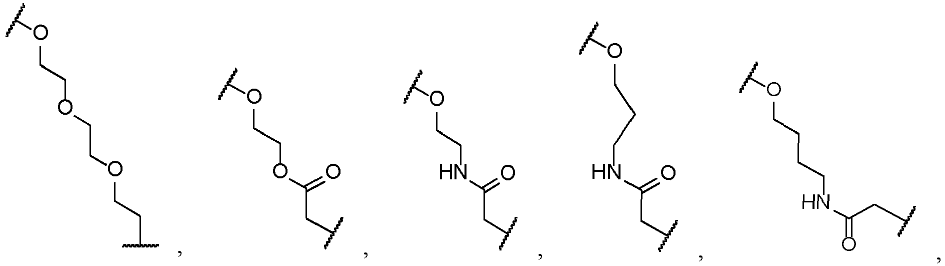 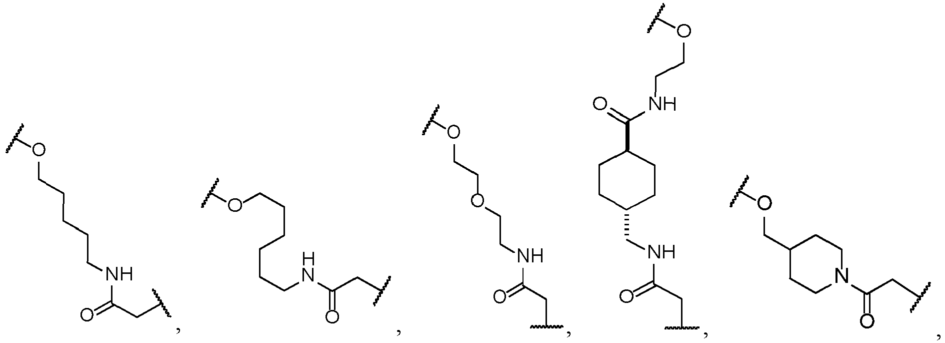 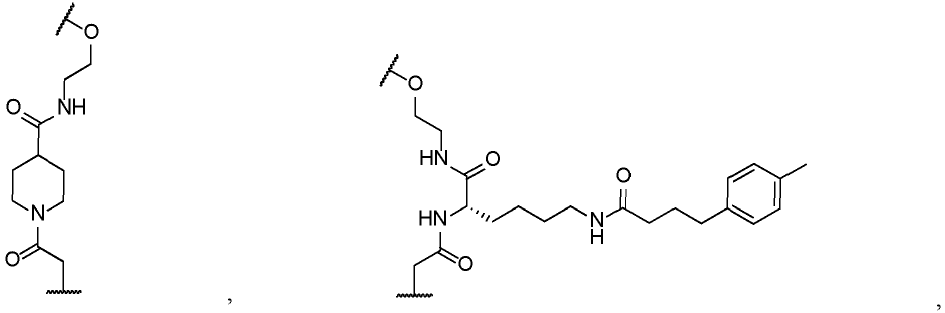 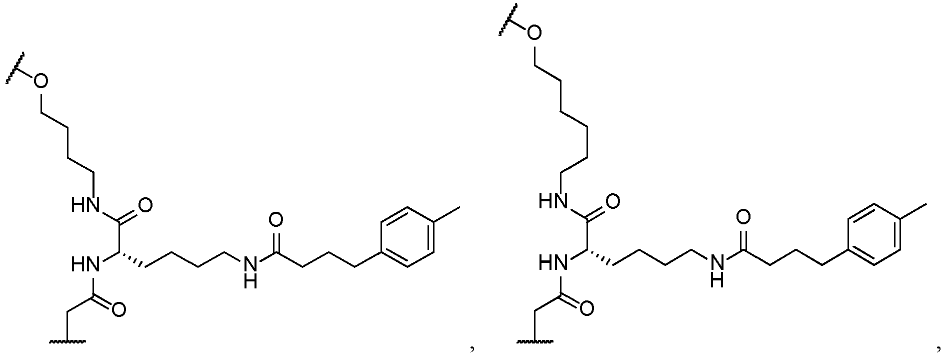 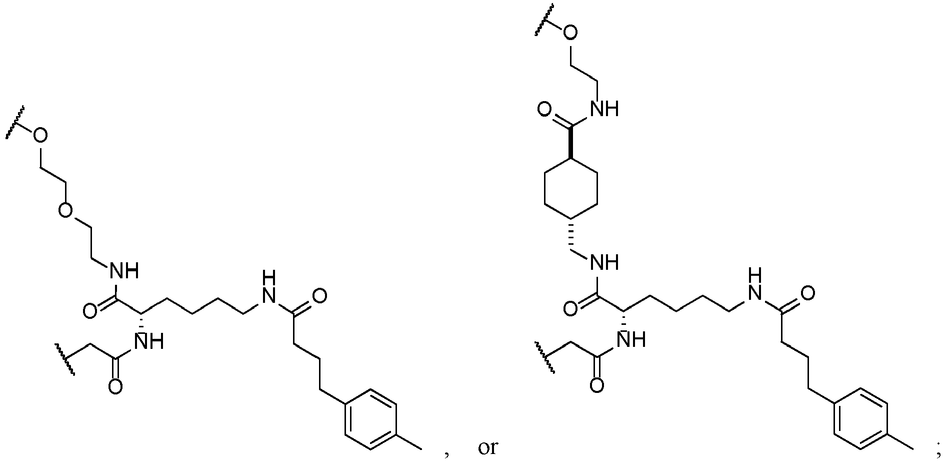 wherein the upper end is connected to ring A, and the lower end is connected to L².

In some embodiments, L² is

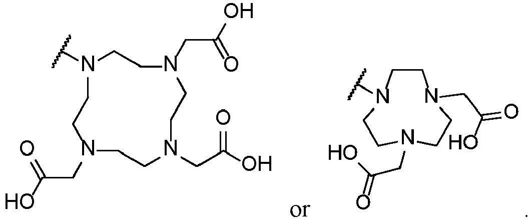

In some embodiments, the L² and the metal ion form any one of the following groups:

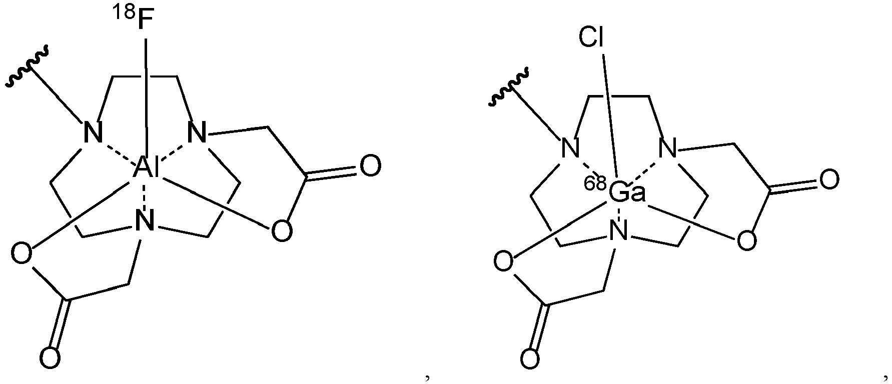

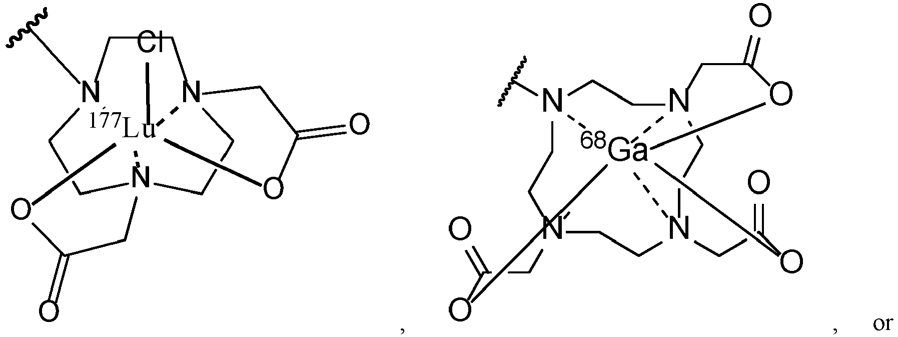

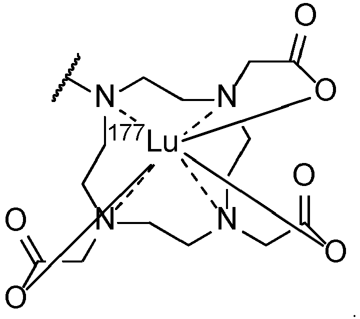

The present disclosure provides a compound represented by general formula II or a metal complex thereof, or a tautomer or stereoisomer of any one of described above (namely, the compound represented by general formula II or the metal complex thereof), or a pharmaceutically acceptable salt of any one of described above (namely, the compound represented by general formula II or the metal complex thereof, the tautomer thereof, or the stereoisomer thereof), or a solvate of any one of described above (namely, the compound represented by general formula II or the metal complex thereof, the tautomer thereof, the stereoisomer thereof, or the pharmaceutically acceptable salt thereof): 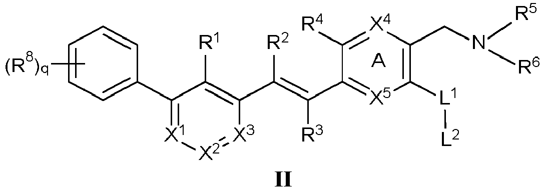
X¹, X², X³, X⁴, and X⁵ are each independently CH or N;
R¹ is hydrogen, halogen, cyano, C₁-C₄ alkyl, or C₁-C₄ alkyl substituted by one or more R^{a};
R² and R³ are each independently hydrogen or halogen;
R⁴ is hydrogen, halogen, C₁-C₄ alkyl, or C₁-C₄ alkyl substituted by one or more R^{b};
R⁵ and R⁶ are each independently hydrogen, deuterium, C₁-C₄ alkyl, or C₁-C₄ alkyl substituted by one or more R^{c}; or, R⁵, R⁶ together with the nitrogen atom to which they are attached form a 5- to 7-membered heterocyclic ring, or a 5- to 7-membered heterocyclic ring substituted by one or more R^{c}; in the 5- to 7-membered heterocyclic ring, the number of the heteroatoms is 1, 2, 3, or 4, and each heteroatom is independently selected from N, O, and S;
each R^{a}, each R^{b} and each R^{c} are independently deuterium, halogen, hydroxyl, amino, C₁-C₄ alkyl, C₁-C₄ alkyl-O-, -COOH, or -C(O)OR^{g};
each R⁸ is independently hydrogen, deuterium, halogen, hydroxyl, amino, C₁-C₄ alkyl, C1-C4 alkyl-S-, C1-C4 alkyl-O-, -C(O) NH2, -C(O)OC₁₋₄ alkyl, -OR^{8a}, -NHR^{8a}, -NR^{8a}R^{8b}, C1-C₄ alkyl substituted by one or more R^{8c}, C₁-C₄ alkyl-S- substituted by one or more R^{8c}, or C₁-C₄ alkyl-O- substituted by one or more R^{8c};
or, two adjacent R⁸ together with the carbon atoms on the benzene ring to which they are attached form a 5- to 7-membered carbocyclic ring, a 5- to 7-membered heterocyclic ring, a 5- to 7-membered carbocyclic ring substituted by one or more C₁₋₄ alkyl, or a 5- to 7-membered heterocyclic ring substituted by one or more C₁₋₄ alkyl; in the 5- to 7-membered heterocyclic ring, the number of heteroatoms is 1, 2, 3, or 4, and each heteroatom is independently selected from N and O;
each R^{8a}, R^{8b}, and each R^{8c} are independently C₁-C₄ alkyl-S-, halogen, C₁-C₄ alkyl, C₁-C₄ alkyl-O-, -COOH, -(C₁-C₄ alkylene)-COOH, -C(O)OC₁-C₄ alkyl, -C(O)NH₂, - C(O)NHC₁-C₄ alkyl, or -NR^{8e}R^{8f};
R^{8e} and R^{8f} are each independently hydrogen, halogen, C₁-C₄ alkyl, or C₁-C₄ alkyl substituted by one or more R^{8g};
each R^{8g} is independently halogen, C₁-C₄ alkyl, hydroxyl, -NR^{8h}R^{8k}, C₁-C₄ alkyl-O-, -COOH, -(C₁-C₄ alkylene)-COOH, -C(O)OC₁-C₄ alkyl, -C(O)NH₂, or -C(O)NHC₁-C₄ alkyl;
R^{8h} and R^{8k} are each independently hydrogen or C₁-C₄ alkyl;
q is 0, 1, 2, or 3;
L¹ is
   (i) a single bond or -(CH₂)ₙ-;
   (ii) -(CH₂)ₘ-, in which 1, 2, 3, 4, or 5 non-adjacent CH₂ are independently replaced by -Y₁-, and each Y₁ is independently -C(O)-, -C(O)O-, -O-, -NH-, -C(O)NH-, or -NHC(O)NH-; or
   (iii) -(CH₂)ₚ-, in which one CH₂ is replaced by -Y₂-, and the other 0, 1, 2, 3, or 4 non-adjacent CH₂ are independently replaced by -Y₃-; each Y₃ is independently -C(O)-, -C(O)O-, -O-, -NH-, -C(O)NH-, or -NHC(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
L¹ is unsubstituted or 1, 2, or 3 H contained in L¹ are each independently substituted by R⁷;
n, m, and p are each independently 1, 2, 3, 4, 5, 6, 7, 8, 9, 10, 11, 12, 13, or 14;
each R⁷ is independently C₁-C₄ alkyl or -L³-R⁹;
L³ is
   (i) -(CH₂)ⱼ-; or
   (ii) -(CH₂)ₖ-, in which 1, 2, 3, or 4 non-adjacent CH₂ are independently replaced by -Y₄-, and each Y₄ is independently -C(O)-, -C(O)O-, -O-, -NH-, -C(O)NH-, or -NHC(O)NH-;
L³ is unsubstituted or 1, 2, or 3 H contained in L³ are each independently substituted by R¹⁰;
j and k are each independently 1, 2, 3, 4, 5, 6, 7, 8, 9, 10, 11, 12, 13, or 14;
R⁹ is hydrogen, C₆-C₁₀ aryl, or C₆-C₁₀ aryl substituted by one or more R^{d};
each R¹⁰ is independently C₁-C₄ alkyl;
each R^{d} is independently C₁-C₄ alkyl or C₁-C₄ alkyl substituted by one or more R^{e};
each R^{e} is independently hydroxyl, amino, C₁-C₄ alkyl, C₁-C₄ alkyl-O-, -COOH, or - C(O)OR^{h};
R^{g} and R^{h} are each independently C₁-C₄ alkyl or halogenated C₁-C₄ alkyl;
L² is metal chelating group;
the metal complex is a complex chelated by the compound represented by general formula II with a metal atom or ion.

In some embodiments, the metal complex is a complex chelated by the compound represented by general formula II with the metal ion.

In some embodiments, in the metal complex, the molar ratio of the compound represented by general formula II to the metal atom or ion is 1:1.

In some embodiments, the metal complex has the structure represented by the following general formula I: 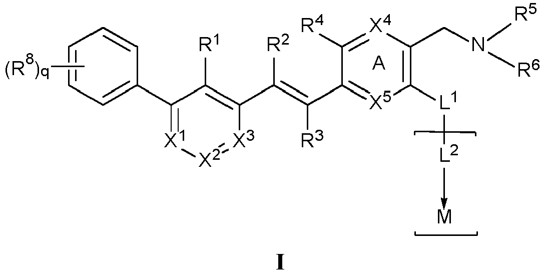 wherein, M is a metal atom or ion, and other variables are defined as in general formula II.

In some embodiments, M is the metal ion.

In some embodiments, the metal is Cu, Ga, Y, Zr, Tc, In, Lu, Re, At, Bi, Tl, or their radioactive or non-radioactive isotopes thereof.

In some embodiments, the metal is Ga, Lu, or their radioactive or non-radioactive isotopes thereof.

In some embodiments, the metal is ⁶³Cu, ⁶⁴Cu, ⁶⁸Ga, ⁷⁰Ga, ⁸⁹Y, ⁹⁰Y, ⁸⁹Zr, ⁹¹Zr, ^{99m}Tc, ¹¹¹In, ¹¹³In, ¹⁷⁵Lu, ¹⁷⁷Lu, ¹⁸⁶Re, ¹⁸⁸Re, ²¹¹At, ²¹²Bi, ²¹³Bi, ²⁰¹Tl, or ²⁰³Tl.

In some embodiments, the valence state of the metal ion can be any valence state of the metal, for example, monovalent, divalent, trivalent, or tetravalent. In some embodiments, the valence state of the metal ion is trivalent.

In some embodiments, the metal ion is Ga³⁺ and Lu³⁺.

In some embodiments, X¹ is CH.

In some embodiments, X² is CH.

In some embodiments, X³ is CH.

In some embodiments, X⁴ is CH.

In some embodiments, X⁵ is CH.

In some embodiments, X¹, X², X³, X⁴, and X⁵ are CH.

In some embodiments, 1, 2, 3, or 4 of X¹, X², X³, X⁴, and X⁵ are N.

In some embodiments, q is 0.

In some embodiments, R¹ is cyano or C₁-C₄ alkyl.

In some embodiments, R¹ is cyano or methyl.

In some embodiments, R² is hydrogen.

In some embodiments, R³ is hydrogen.

In some embodiments, R⁴ is C₁-C₄ alkyl or C₁-C₄ alkyl substituted by one or more R^{b}.

In some embodiments, each R^{b} is independently halogen, for example, fluorine.

In some embodiments, R⁴ is methyl or trifluoromethyl.

In some embodiments, R⁵, R⁶ together with the nitrogen atom to which they are attached form a 5- to 7-membered heterocyclic ring or a 5- to 7-membered heterocyclic ring substituted by one or more R^{c}.

In some embodiments, when R⁵, R⁶ together with the nitrogen atom to which they are attached form a 5- to 7-membered heterocyclic ring or a 5- to 7-membered heterocyclic ring substituted by one or more R^{c}, the heteroatom of the 5- to 7-membered heterocyclic ring is one N.

In some embodiments, when R⁵, R⁶ together with the nitrogen atom to which they are attached form a 5- to 7-membered heterocyclic ring or a 5- to 7-membered heterocyclic ring substituted by one or more R^{c}, the 5- to 7-membered heterocyclic ring is saturated 5- to 7-membered heterocyclic ring, for example, piperidine.

In some embodiments, R⁵, R⁶ together with the nitrogen atom to which they are attached form

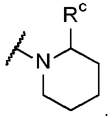

In some embodiments, each R^{c} is independently -COOH.

In some embodiments, R⁵, R⁶ together with the nitrogen atom to which they are attached form 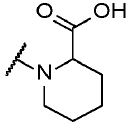 (for example, 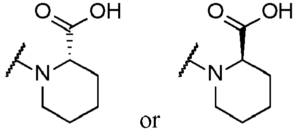 ).

In some embodiments, R⁵, R⁶ together with the nitrogen atom to which they are attached form

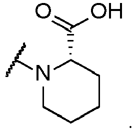

In some embodiments, L¹ is connected to ring A through -Y₁-, and Y₁ connected to ring A is -O-.

In some embodiments, L¹ is connected to L² via -CH₂-.

In some embodiments, n, m, and p are each independently 5, 6, 7, 8, 9, 10, 11, or 12.

In some embodiments, L¹ satisfies the definition of at least one of the foregoing embodiments, and L¹ is a single bond, -(CH₂)ₙ₁NH(CH₂)ₘ₁-, -(CH₂)ₙ₁O(CH₂)ₘ₁-, - (CH₂)ₙ₁NHC(O)NH(CH₂)ₘ₁-, -O(CH₂)ₙ₁NH-, -O(CH₂)ₙ₁O-, -O(CH₂)ₙ₁O(CH₂)ₘ₁O-, - NH(CH₂)ₙ₁NH(CH₂)ₘ₁NH-, -NH(CH₂)ₙ₁O(CH₂)ₘ₁O-, -NH(CH₂)ₙ₁NH(CH₂)ₘ₁O-, - O(CH₂)mNH(CH₂)ₘ₁O-, -O(CH₂)ₙ₁O(CH₂)ₘ₁NH-, -O(CH₂)ₙ₁NH(CH₂)ₘ₁NH-, - NH(CH₂)ₙ₁O(CH₂)ₘ₁NH-, -O-Y₂-(CH₂)ₙ₁NH-, -O(CH₂)ₙ₂OC(O)(CH₂)ₘ₂-, - O(CH₂)ₙ₂O(CH₂)ₘ₂-, -O(CH₂)ₙ₂NHC(O)(CH₂)ₘ₂-, -O(CH₂)ₙ₂NHC(O)-(CH₂)ₙ₃-NHC(O)(CH₂)ₘ₃-, -O-(CH₂)ₙ₂-Y₂-C(O)-(CH₂)ₘ₂-, -O(CH₂)ₙ₂NHC(O)-Y₂-(CH₂)ₙ₃NHC(O)-(CH₂)ₘ₃-, -O(CH₂)ₙ₂NHC(O)-Y₂-C(O)-(CH₂)ₘ₃-, -O(CH₂)ₙ₂-Y₂-C(O)-(CH₂)ₘ₃-, - O(CH₂)ₙ₂NHC(O)-Y₂-(CH₂)ₙ₃NHC(O)-(CH₂)ₙ₄NHC(O)-(CH₂)ₘ₃, -O(CH₂)ₙ₂NHC(O)-(CH₂)ₙ₃NHC(O)-(CH₂)ₘ₃-, or -O(CH₂)ₙ₂-O(CH₂)ₙ₃-NHC(O)-(CH₂)ₙ₄NHC(O)-(CH₂)ₘ₃-, and each n1, each n2, each n3, n4, each m1, each m2, and each m3 are each independently 1, 2, 3, 4, 5, or 6; L¹ is unsubstituted or 1, 2, or 3 H contained in L¹ are each independently substituted by R⁷.

In some embodiments, m2 is 1.

In some embodiments, m3 is 1.

In some embodiments, when Y₂ is 5- to 7-membered carbocyclic ring, the 5- to 7-membered carbocyclic ring is 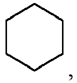 for example, 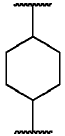 (for example, 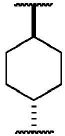 ).

In some embodiments, when Y₂ is 5- to 7-membered heterocyclic ring, the 5- to 7-membered heterocyclic ring is 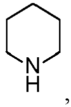 for example,

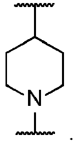

In some embodiments, L¹ is unsubstituted or one H contained in L¹ is substituted by R⁷.

In some embodiments, each R⁷ is independently -L³-R⁹.

In some embodiments, each Y₄ is independently -C(O)NH-.

In some embodiments, L³ is -(CH₂)ₖ-, in which one CH₂ is replaced by -Y₄-, and Y₄ is -C(O)-, -C(O)O-, -O-, -NH -, -C(O)NH-, or -NHC(O)NH-.

In some embodiments, each Y₄ is independently -C(O)NH-.

In some embodiments, L³ is unsubstituted.

In some embodiments, j and k are each independently 7, 8, or 9.

In some embodiments, R⁹ is C₆-C₁₀ aryl or C₆-C₁₀ aryl substituted by one or more R^{d}.

In some embodiments, R⁹ is phenyl or phenyl substituted by one or more R^{d}.

In some embodiments, each R^{d} is independently C₁-C₄ alkyl.

In some embodiments, R⁷ is

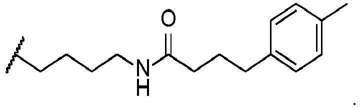

In some embodiments, L¹ is unsubstituted.

In some embodiments, L¹ is 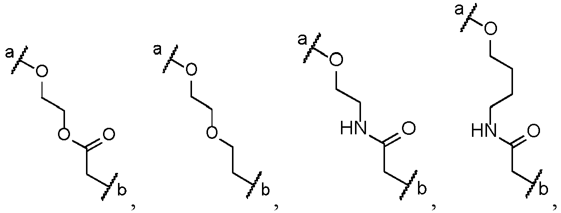 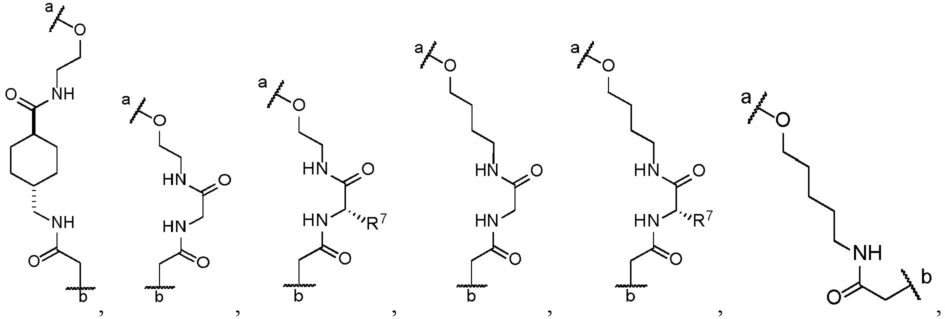 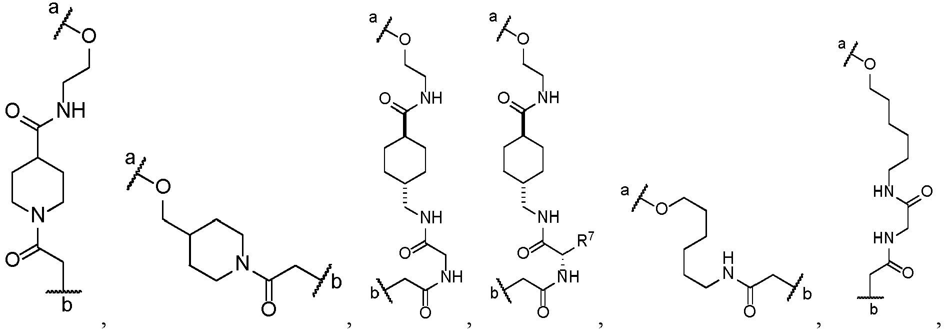 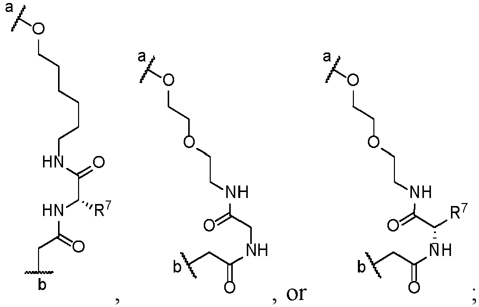 wherein the a-terminal is connected to ring A, and the b-terminal is connected to L².

In some embodiments, L¹ is 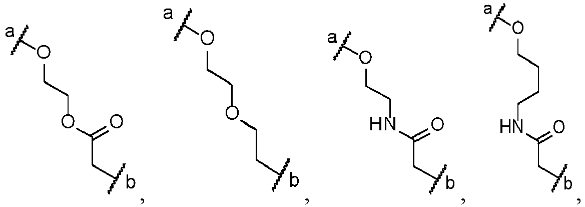 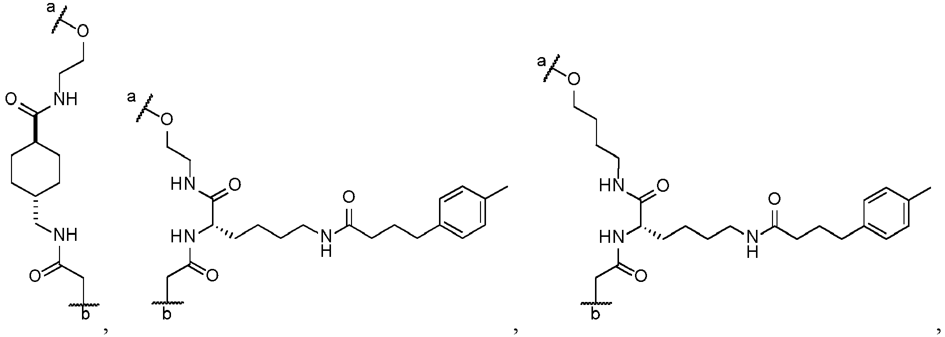 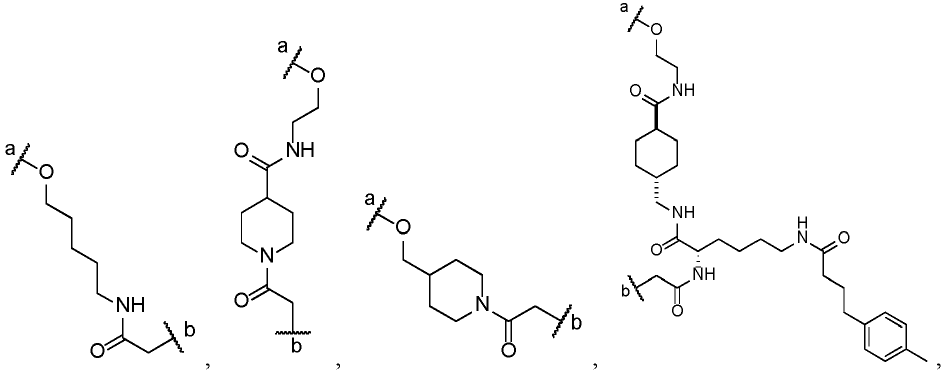 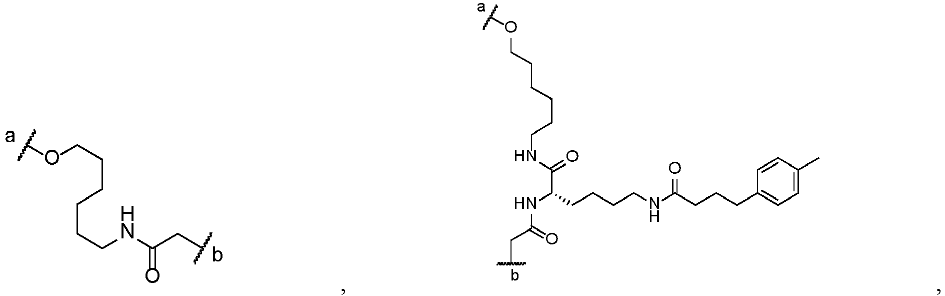 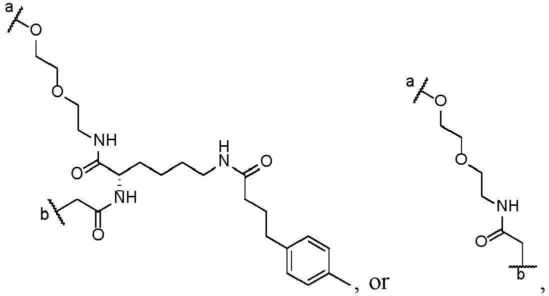 wherein the a-terminal is connected to ring A, and the b-terminal is connected to L². Preferably, L¹ is 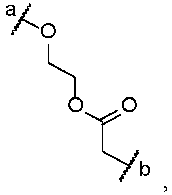 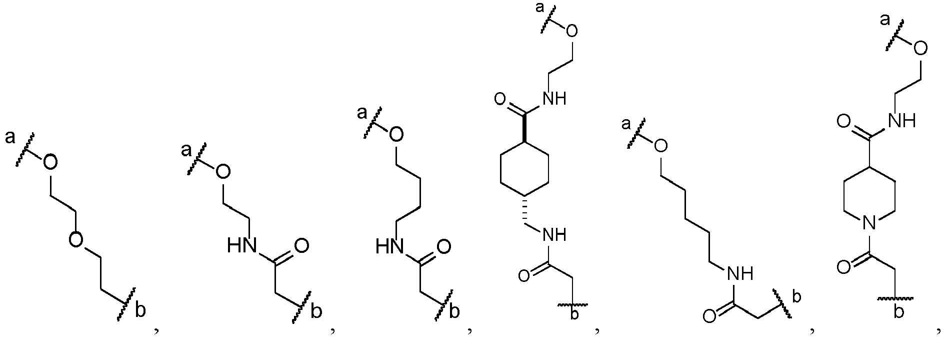 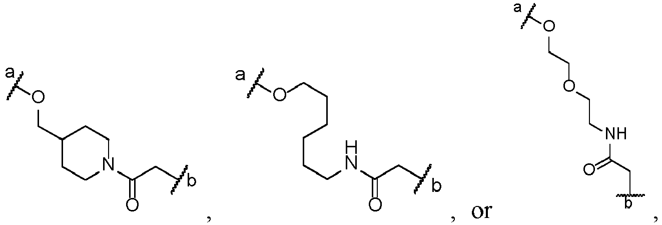 wherein the a-terminal is connected to ring A, and the b-terminal is connected to L².

In some embodiments, L² is R¹¹ or -L⁴-(CH₂)ₛ-R¹¹;
s is 1, 2, or 3;
L⁴ is 5- to 7-membered carbocyclic ring (for example, benzene ring, for example, 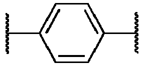 ) or 5- to 7-membered heterocyclic ring, in the 5- to 7-membered heterocyclic ring, the number of heteroatoms is 1, 2, 3, or 4, and each heteroatom is independently selected from N, O, and S;
R¹¹ is 8- to 20- membered (for example 8-, 9-, 10-, 11-, 12-, 13-, 14-, 15-, or 16-membered) saturated monocyclic or bridged carbocyclic ring; 3, 4, 5, or 6 non-adjacent CH₂ of the monocyclic or bridged carbocyclic ring are independently replaced by -Y₅-, and each Y₅ is independently -O-, -NH-, or-N (R^{11a})-;
each R^{11a} is independently C₁-C₄ alkyl or C₁-C₄ alkyl substituted by one or more - COOH (for example, -(C₁-C₄ alkylene)-COOH, for example, -CH₂-COOH).

In some embodiments, when L² is -L⁴-(CH₂)ₛ-R¹¹, the -L⁴-(CH₂)ₛ-R¹¹ is

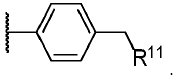

In some embodiments, each Y₅ is independently -NH- or -N(R^{11a})-.

In some embodiments, each R^{11a} is independently C₁-C₄ alkyl substituted by one or more -COOH.

In some embodiments, L² is R¹¹, and R¹¹ is defined as described herein.

In some embodiments, L² is 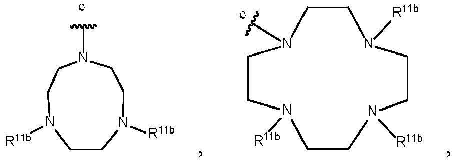 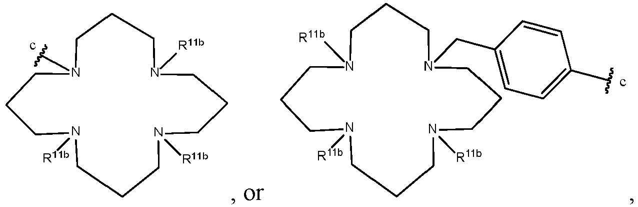 and each R^{11b} is independently H or R^{11a}, and R^{11a} is defined as described herein. In some embodiments, in each structure of L² above, at least 1, 2, or 3 R^{11b} are R^{11a}, and R^{11a} is defined as described herein.

In some embodiments, L² is 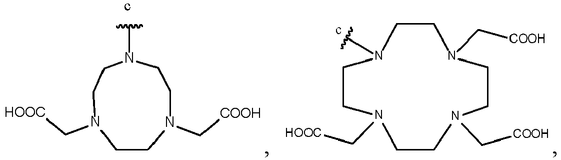 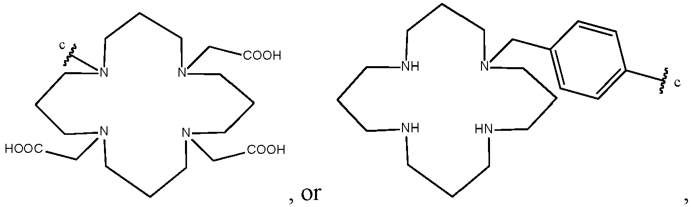 wherein the c-terminal is connected to L¹.

In some embodiments, L² is 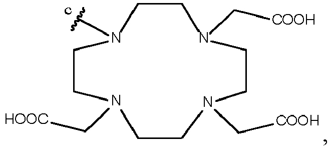 wherein the c-terminal is connected to L¹.

In some embodiments, the metal complex has the following structure: 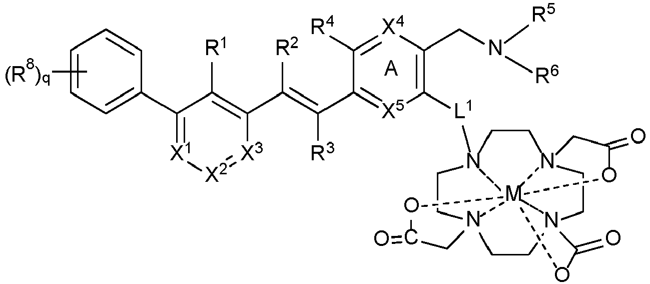 each variable is defined as described herein.

In some embodiments, the compound represented by general formula II is any one of the following structures:

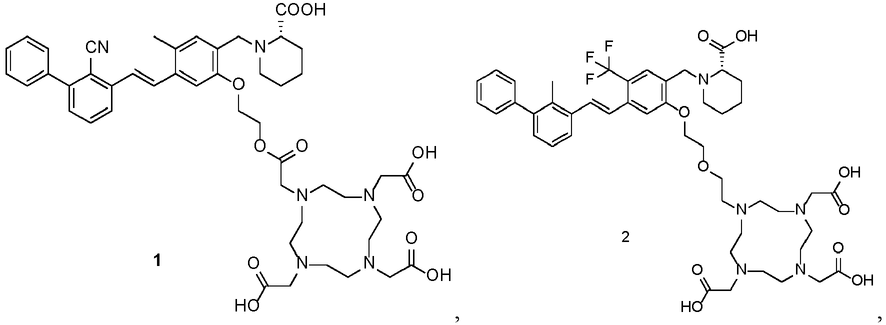

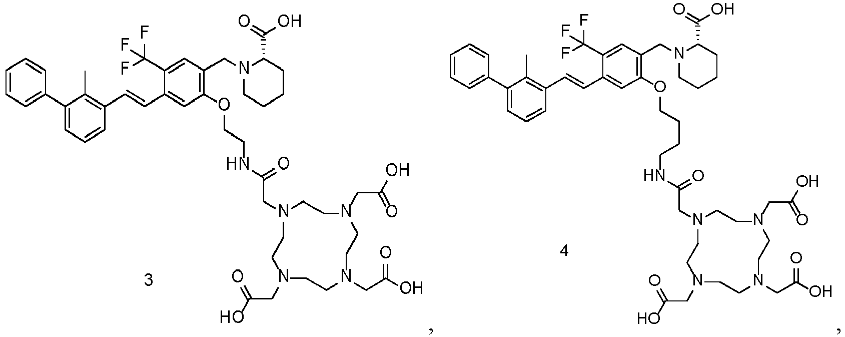

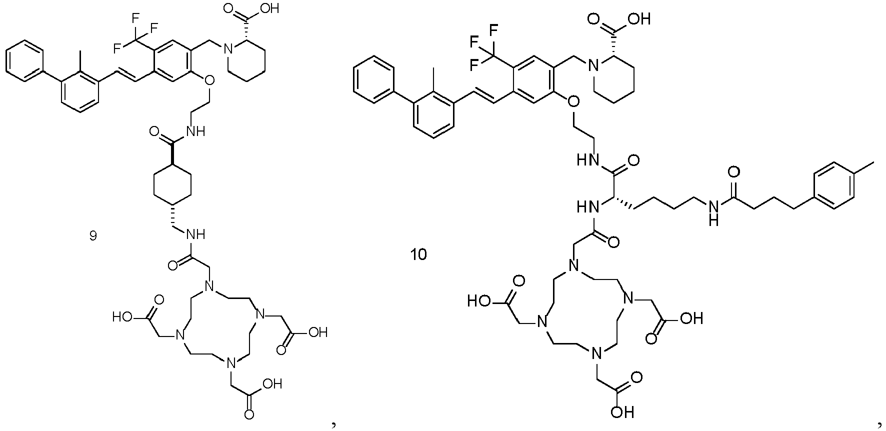

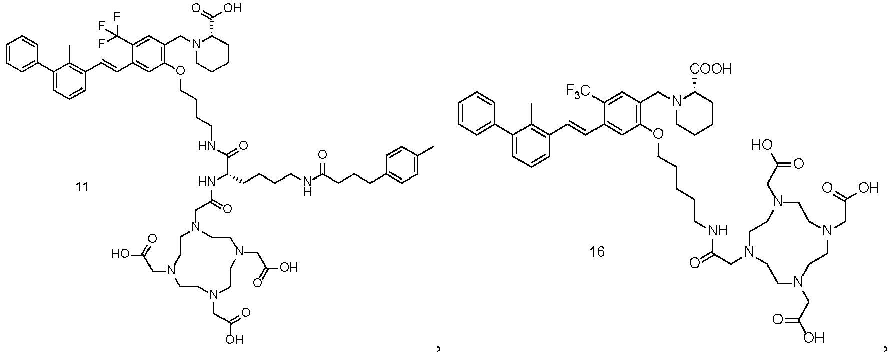

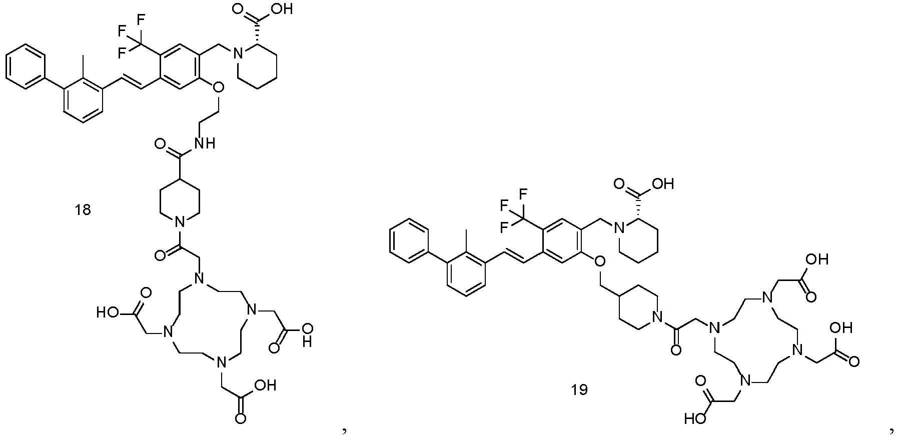

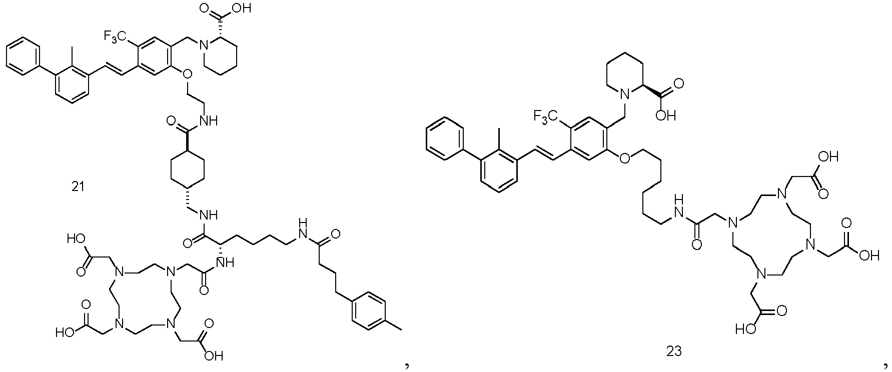

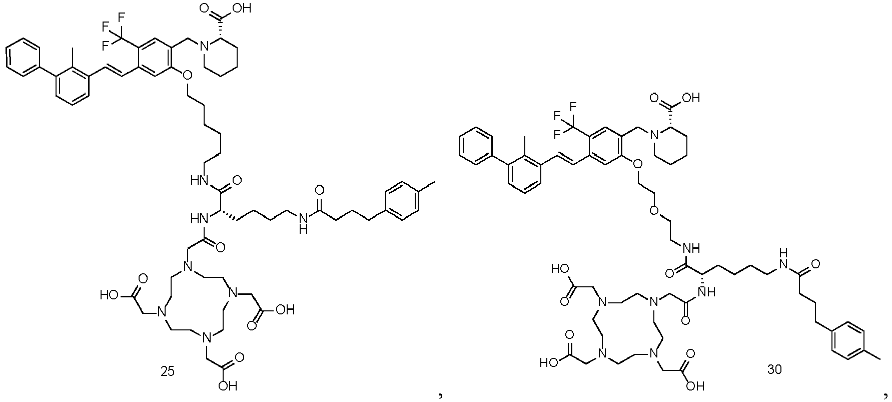

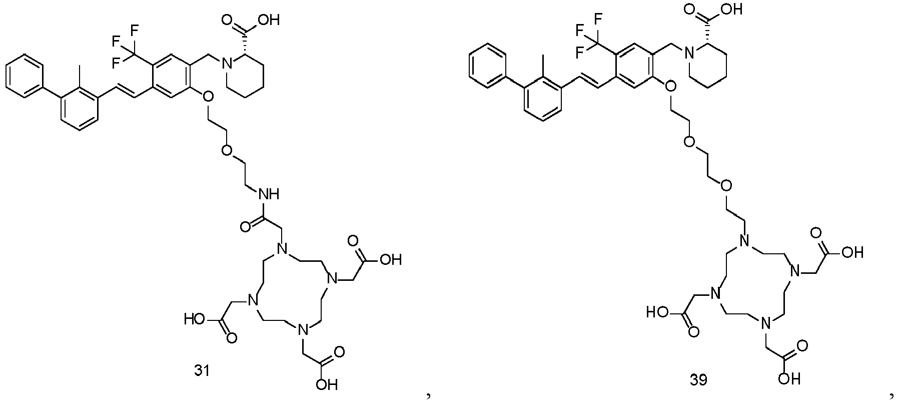

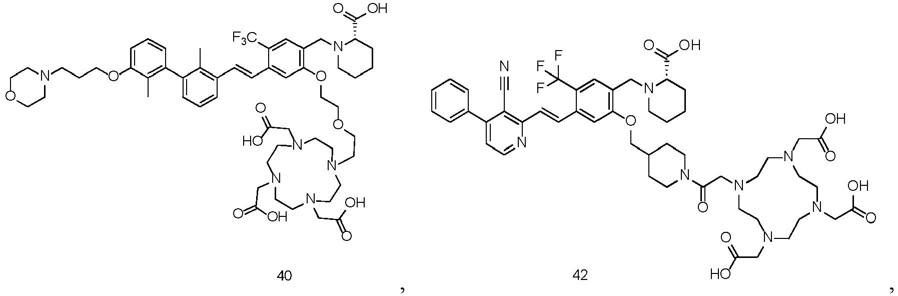

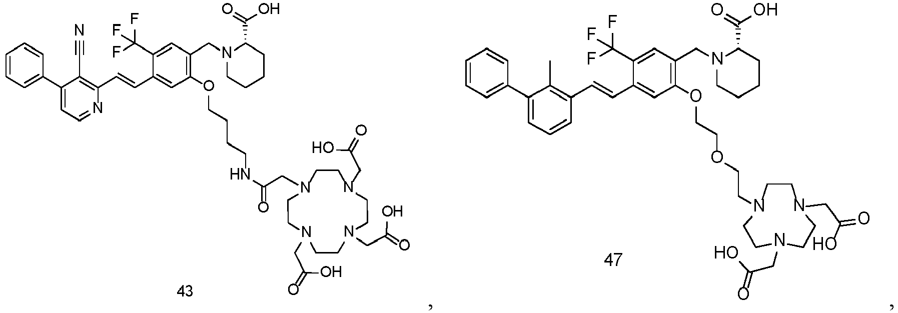

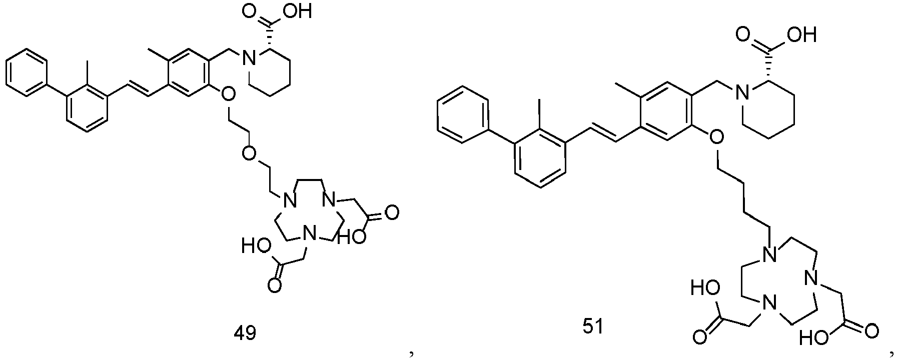

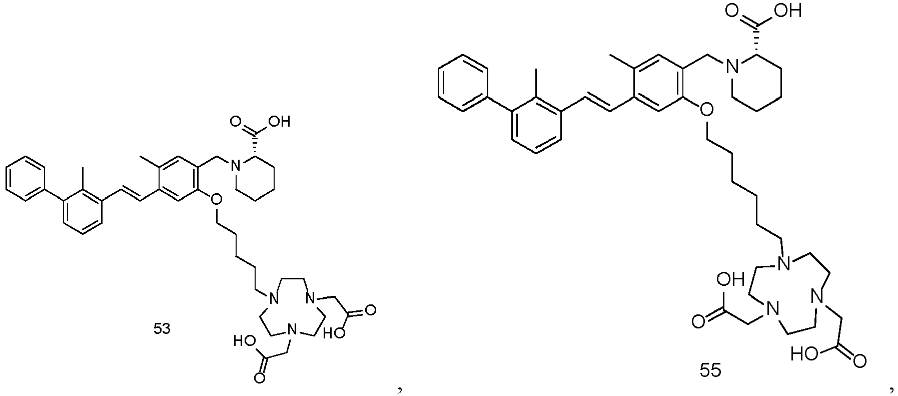

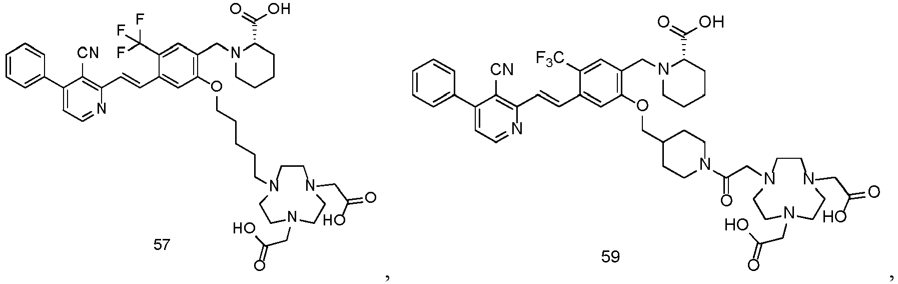

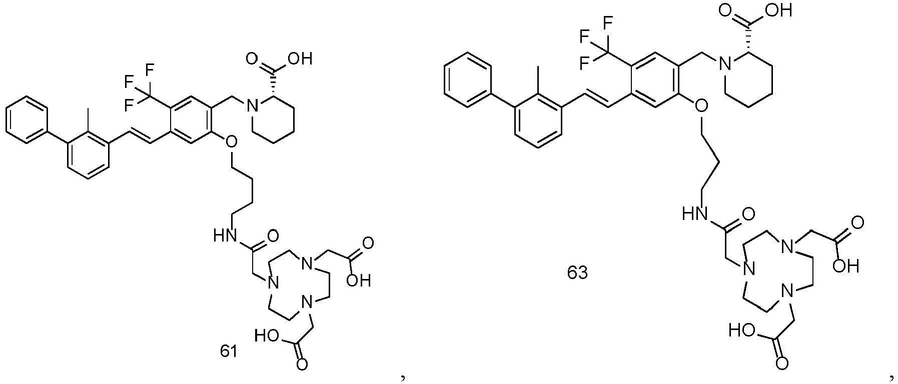

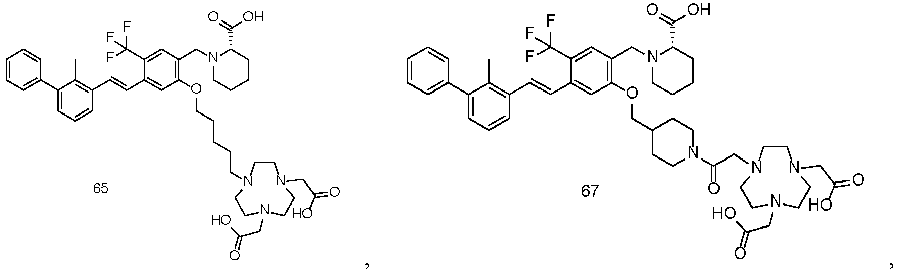

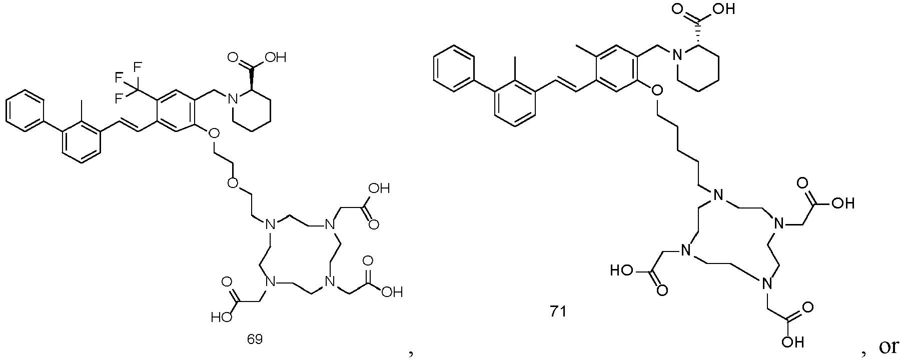

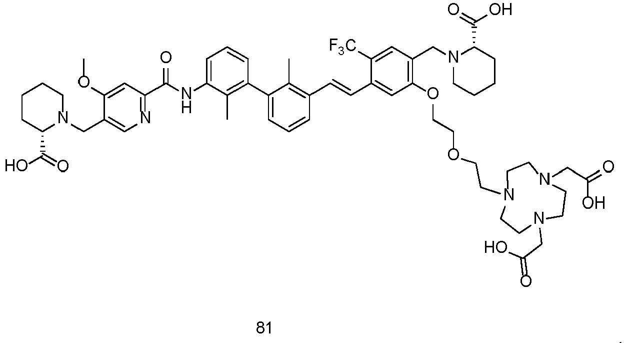

In some embodiments, the metal complex is a complex formed by any one of the following compounds with ¹⁷⁷Lu³⁺: 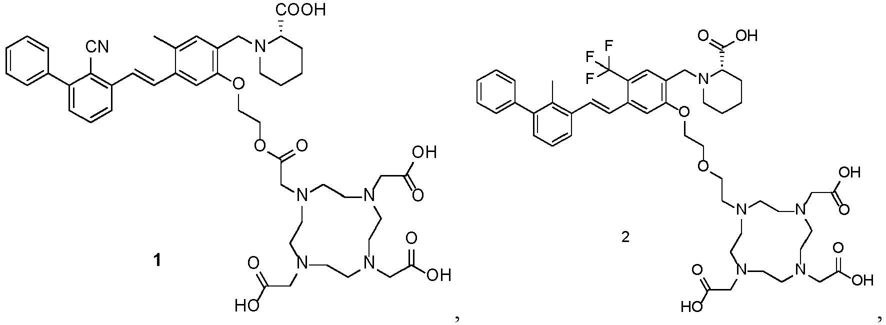 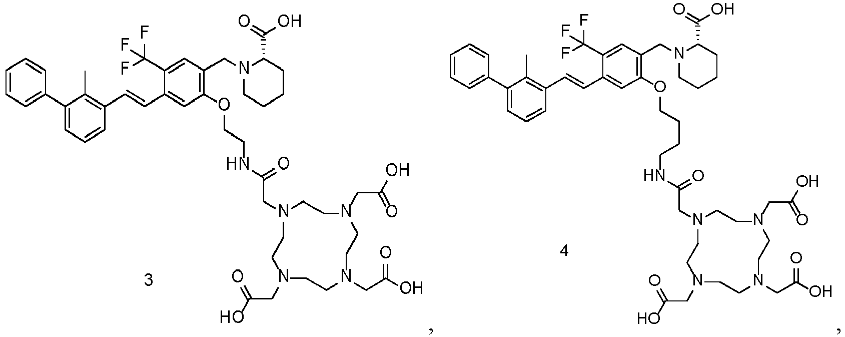 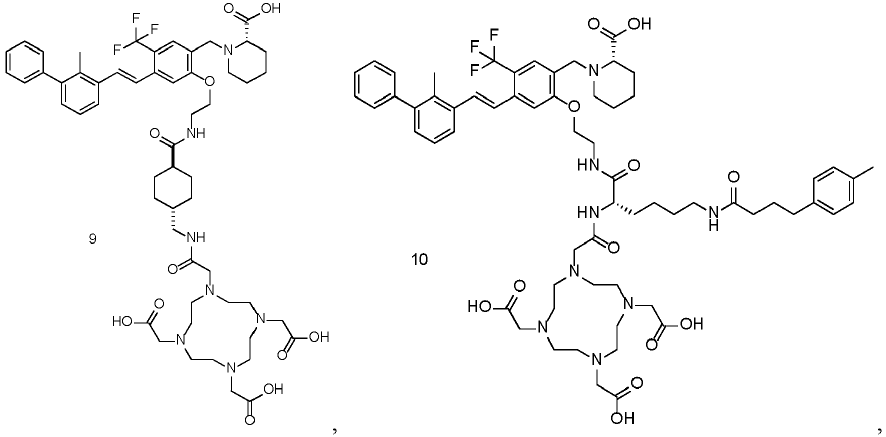 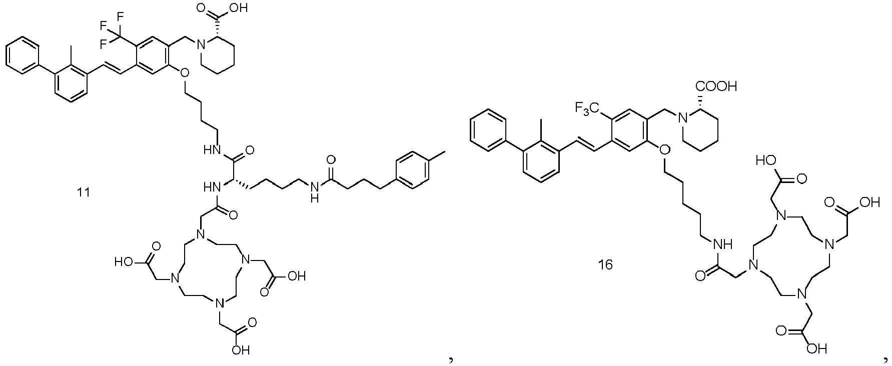 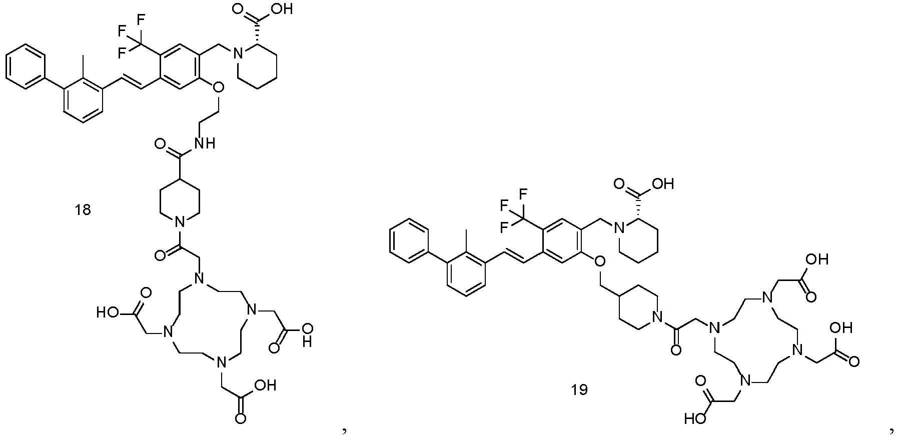 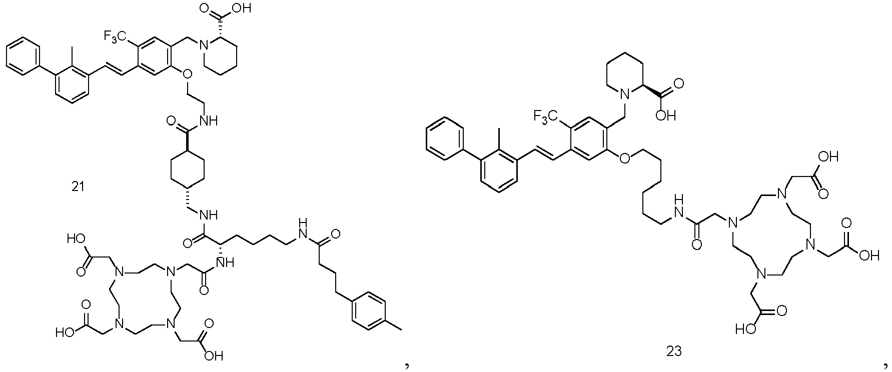 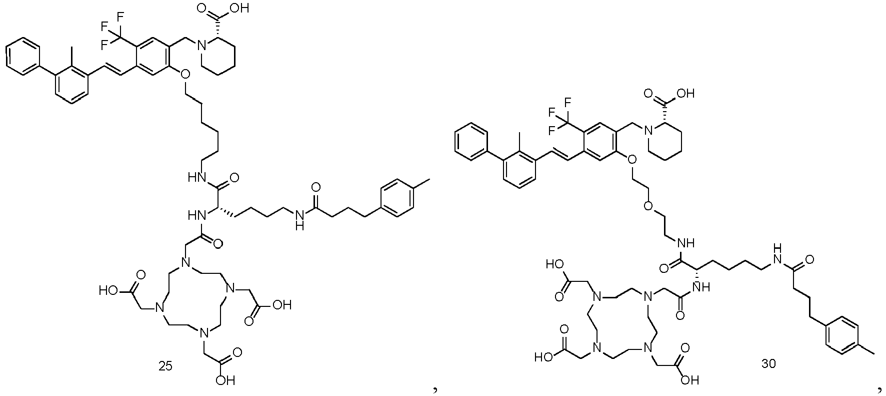 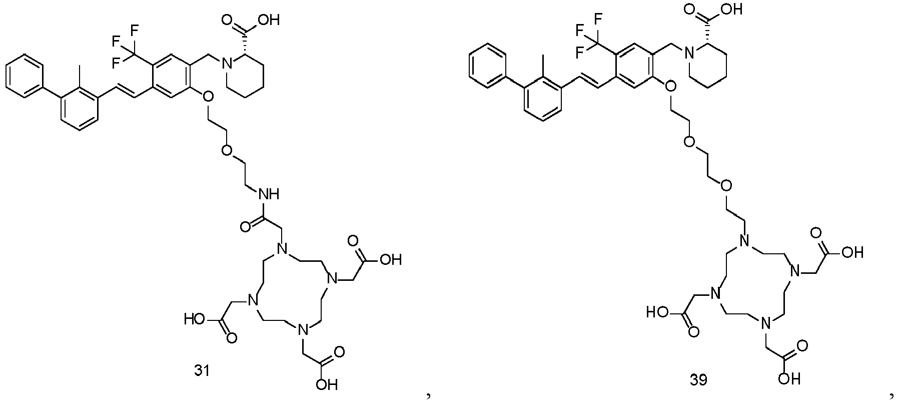 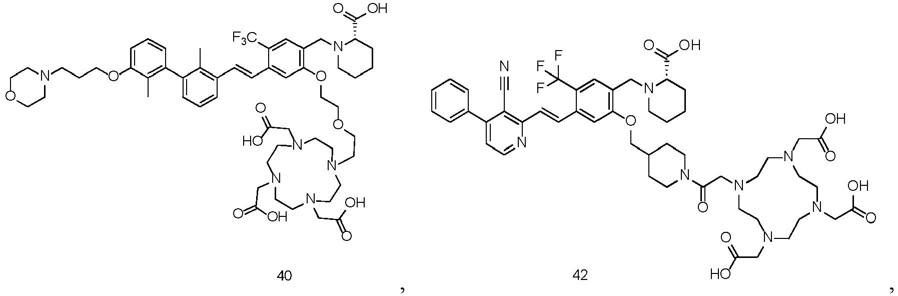 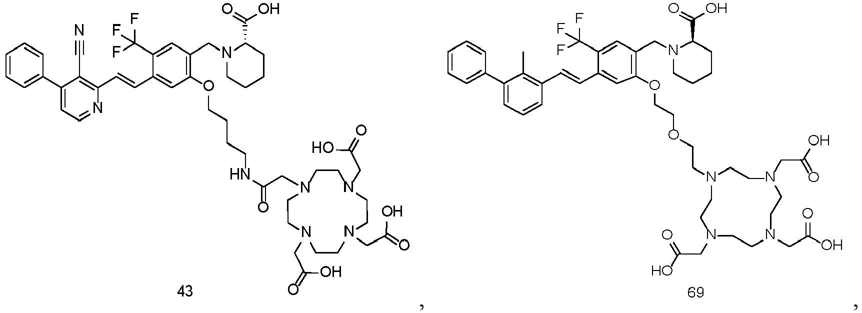 or

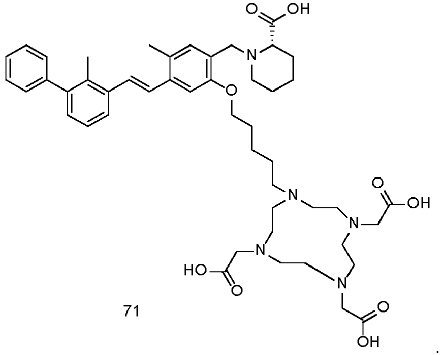

In some embodiments, the metal complex is a complex formed by any one of the following compounds with ⁶⁸Ga³⁺:

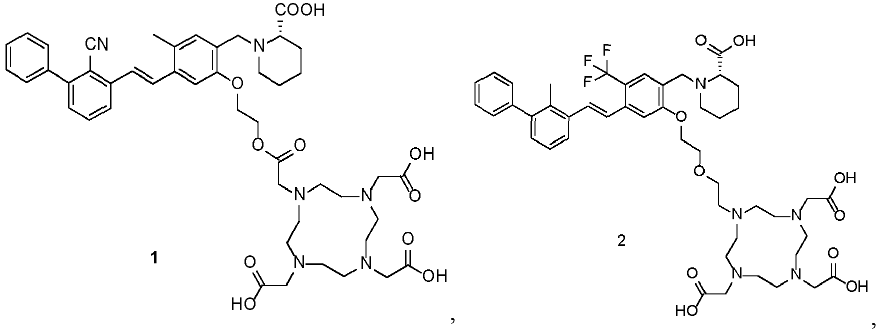

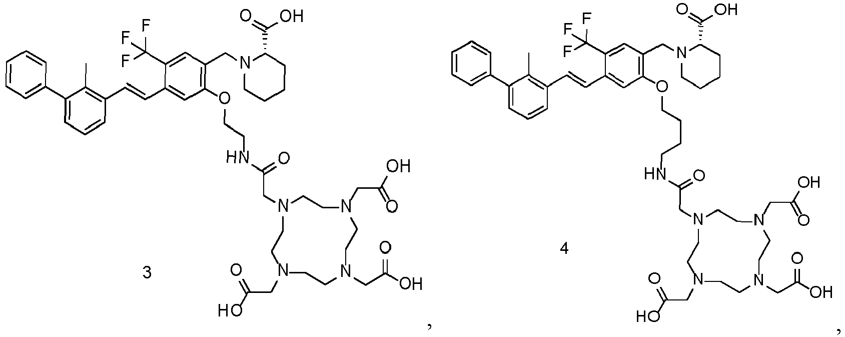

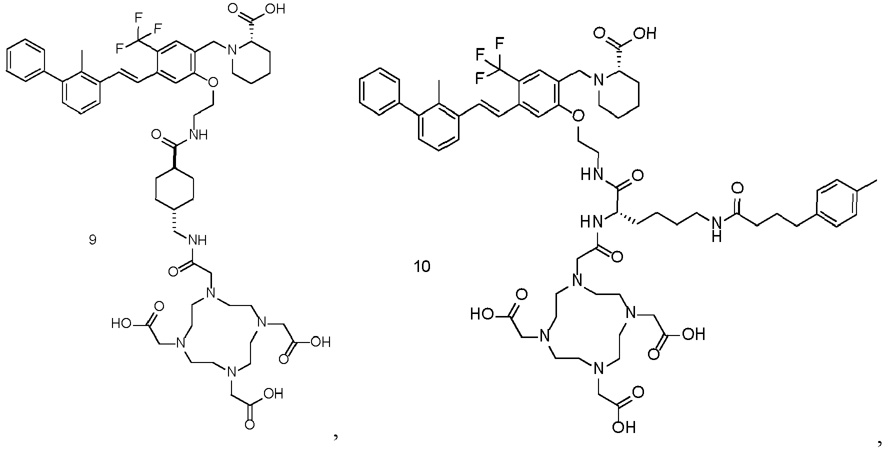

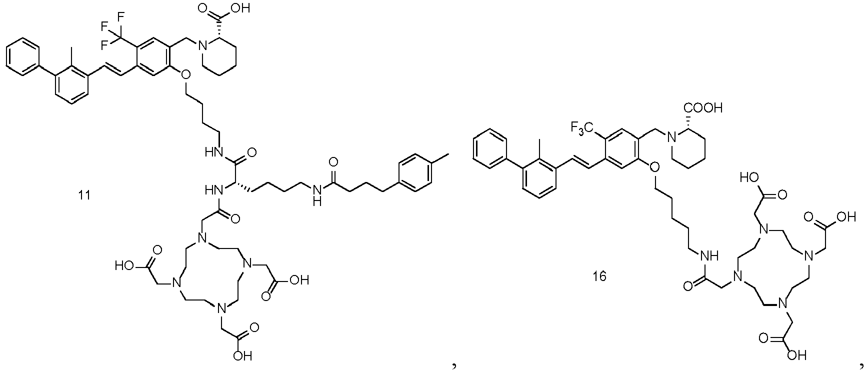

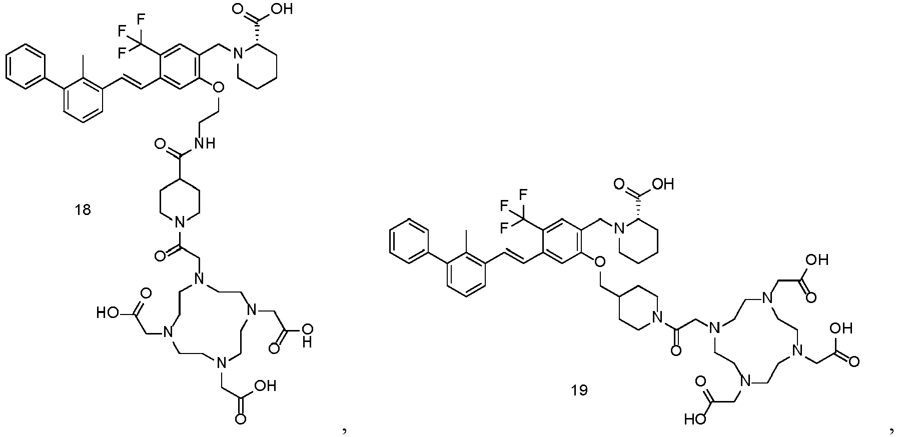

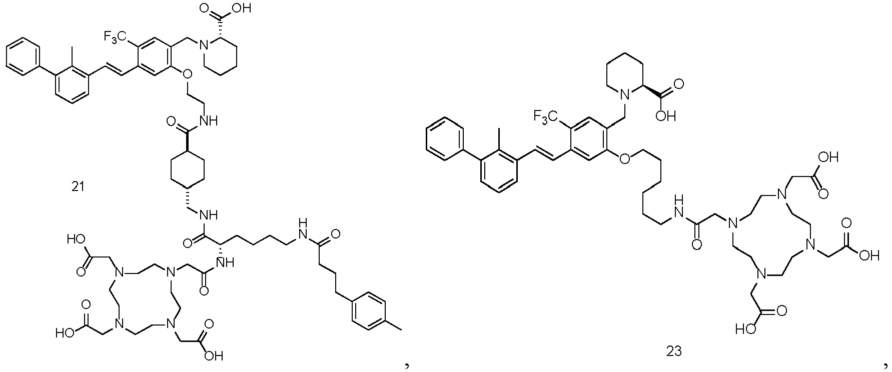

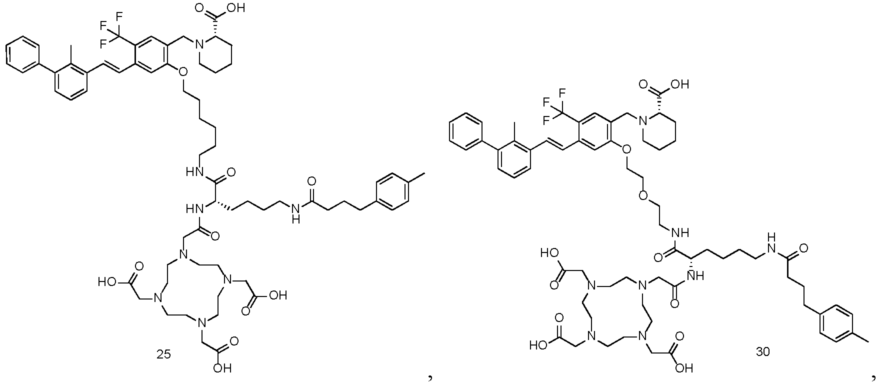

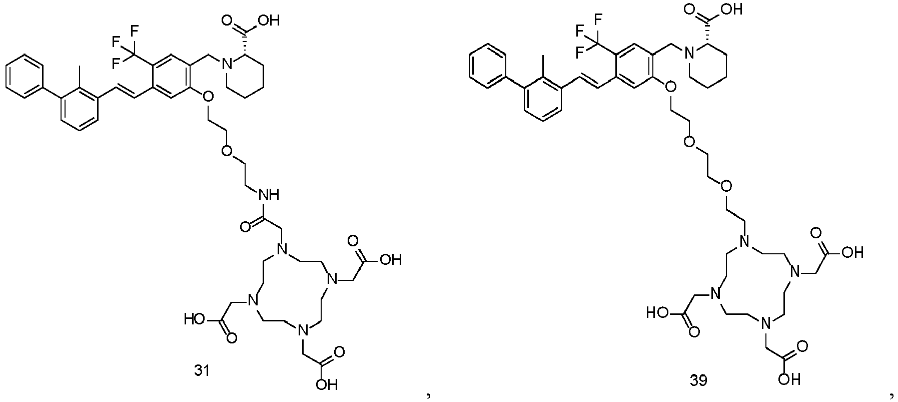

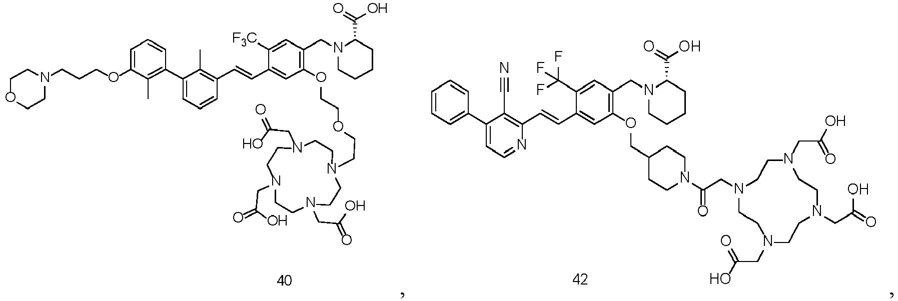

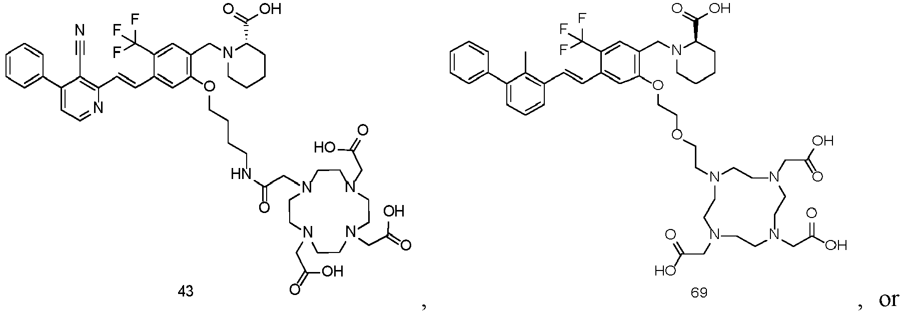

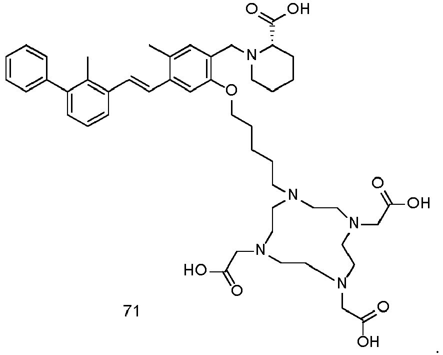

In some embodiments, the metal complex is a complex formed by any one of the following compounds with [Al¹⁸F]²⁺: 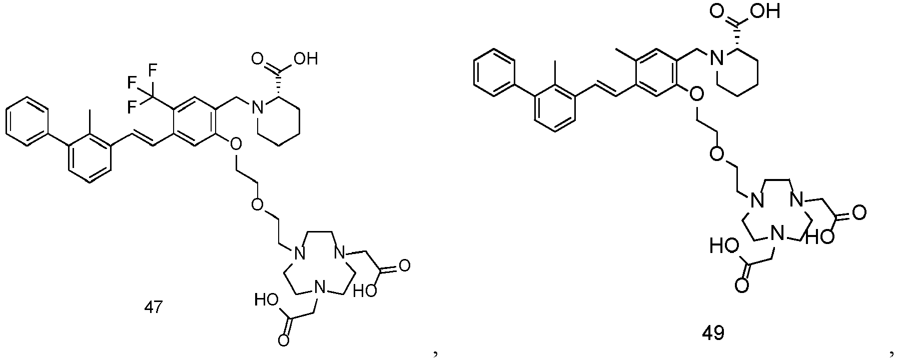 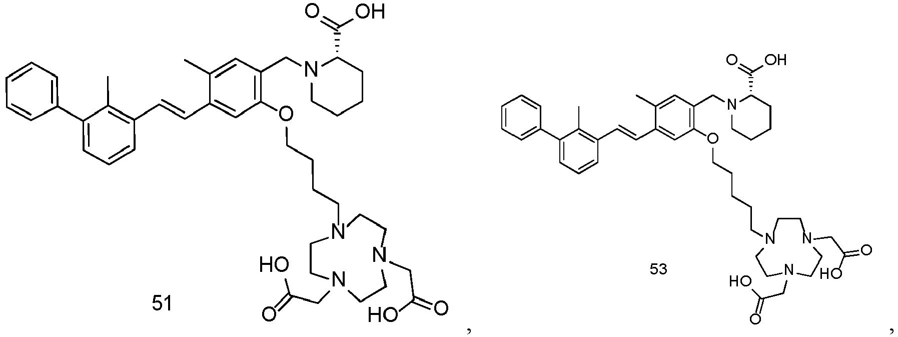 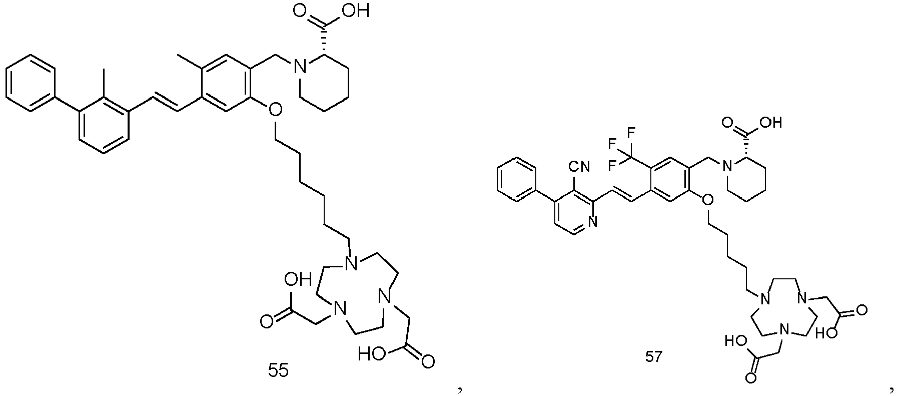 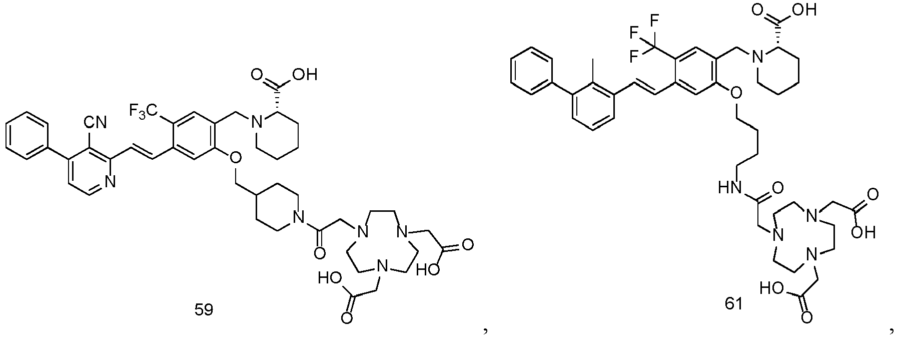 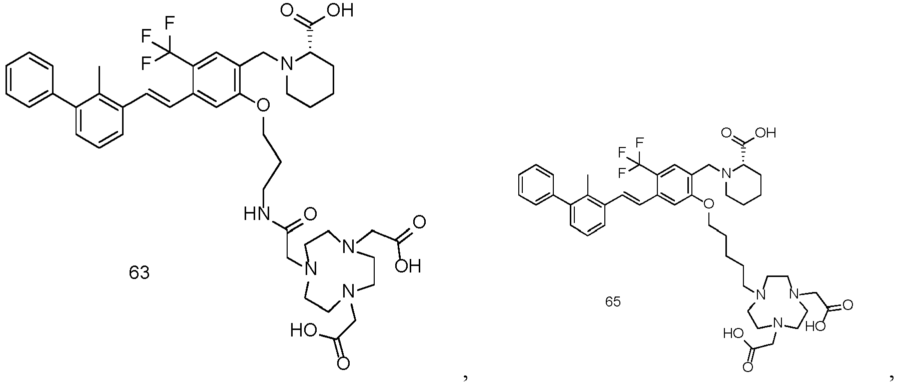 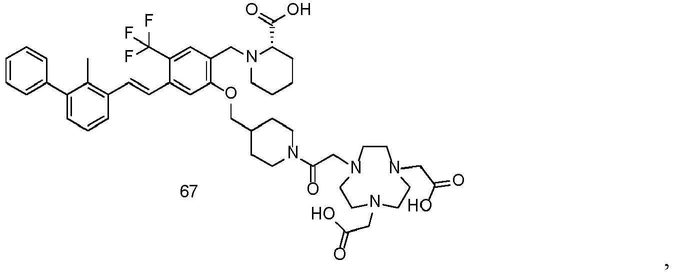 or

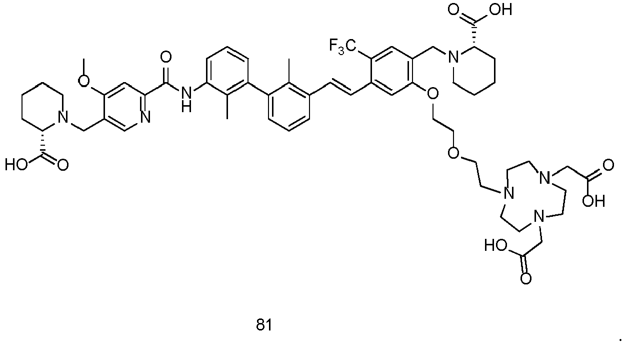

In some embodiments, the metal complex is a complex formed by any one of the following compounds with [⁶⁸GaCl]²⁺: 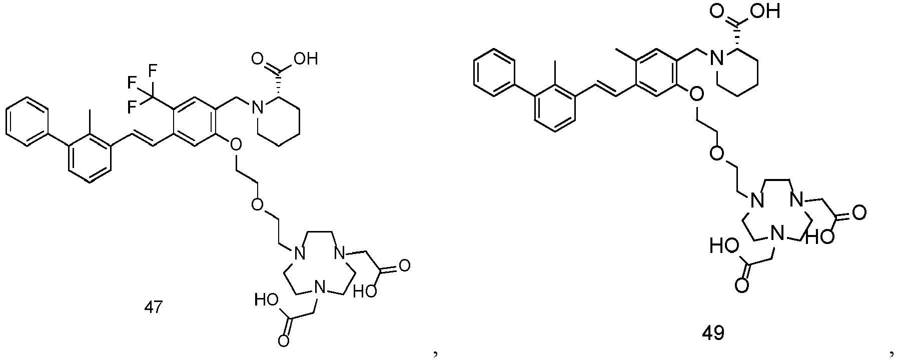 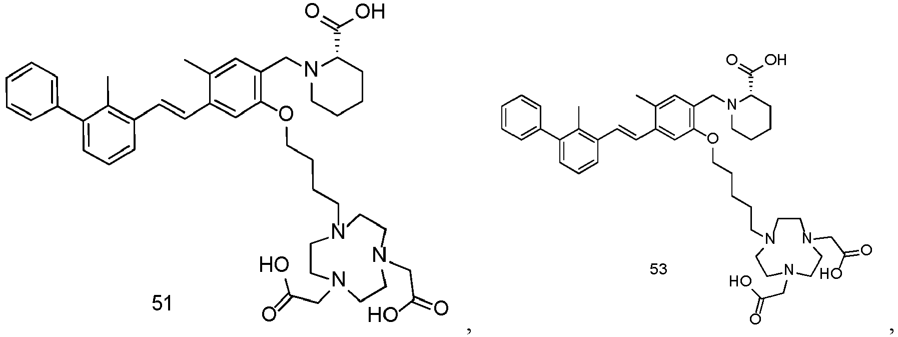 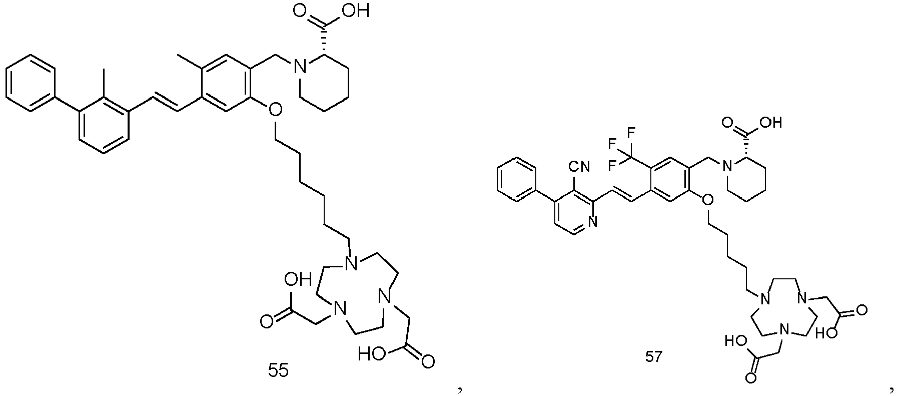 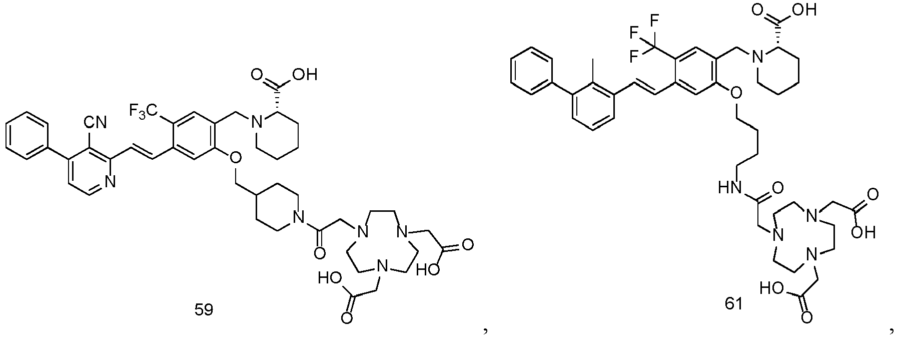 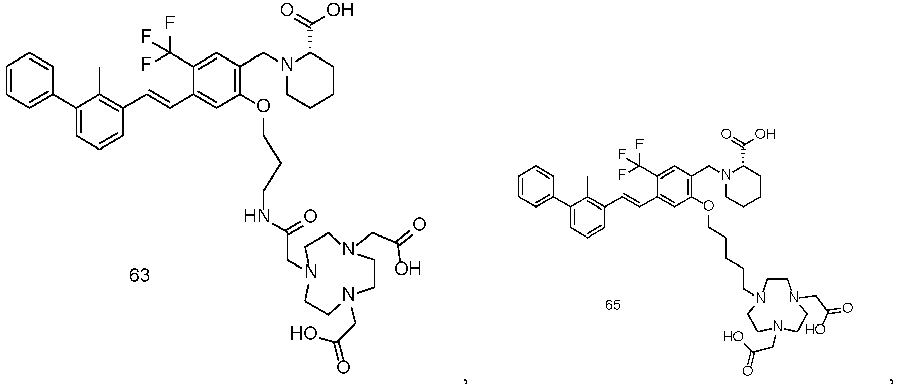 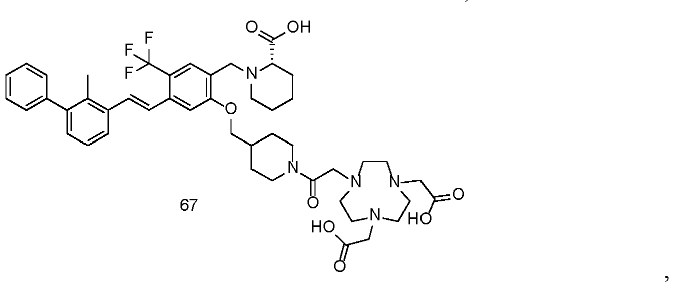 or

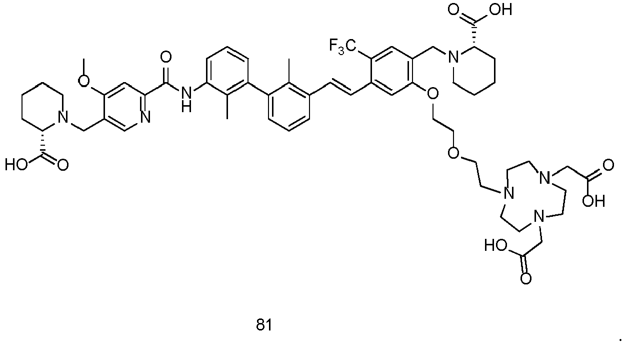

In some embodiments, the metal complex is a complex formed by any one of the following compounds with [¹⁷⁷LuCl]²⁺: 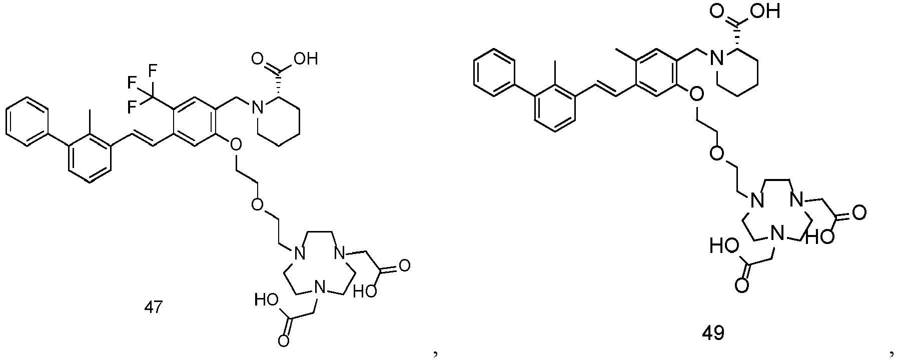 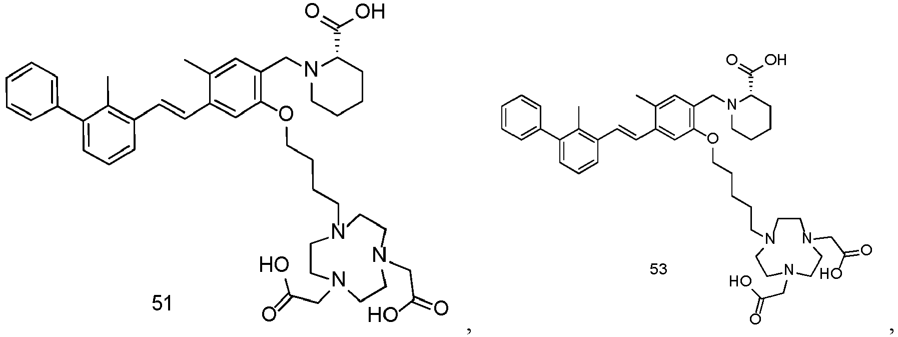 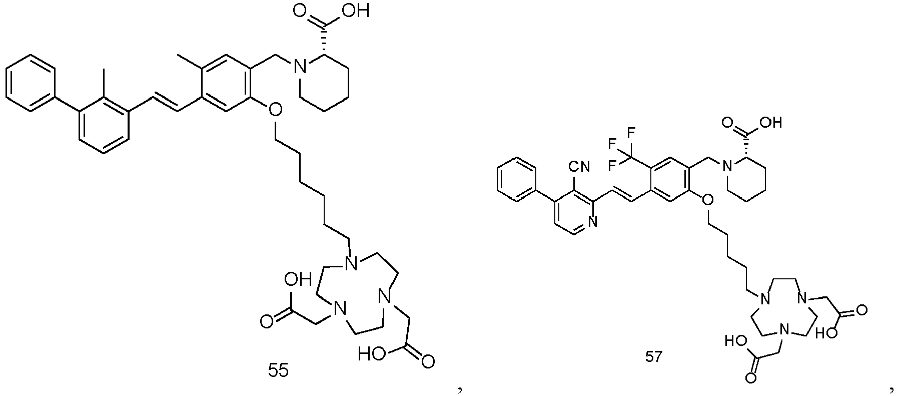 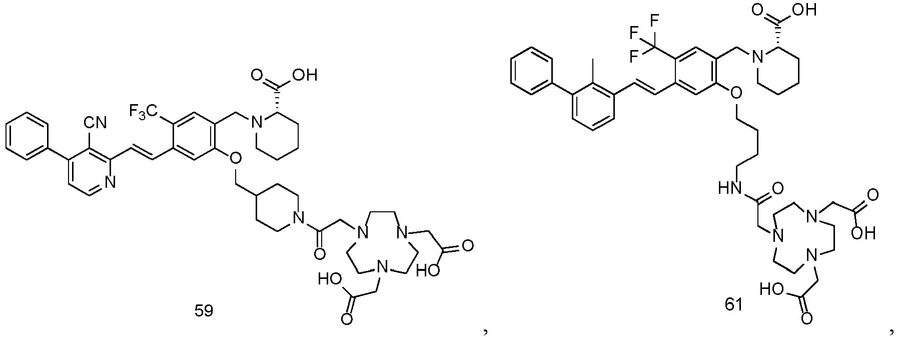 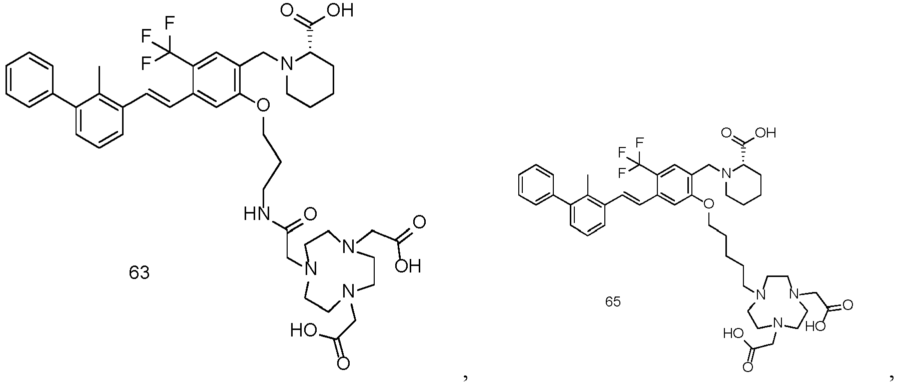 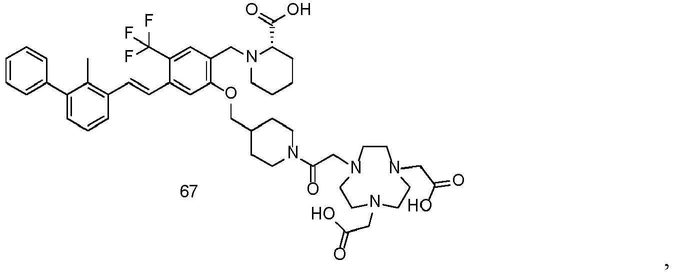 or

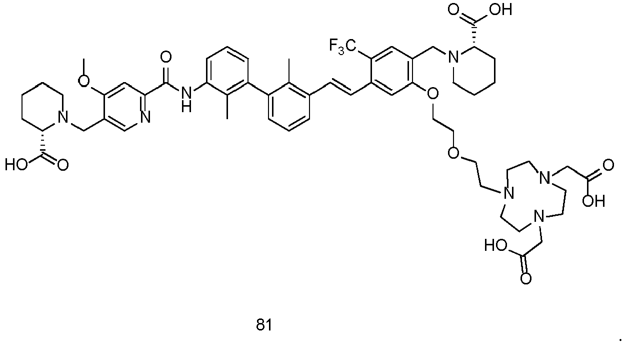

In some embodiments, the metal complex has any one of the following structures: 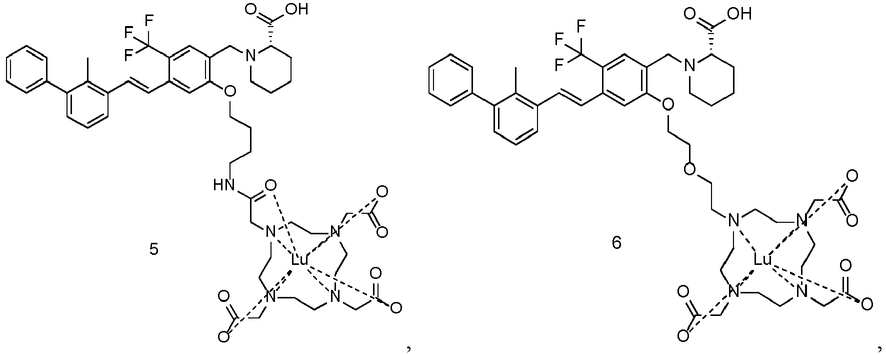 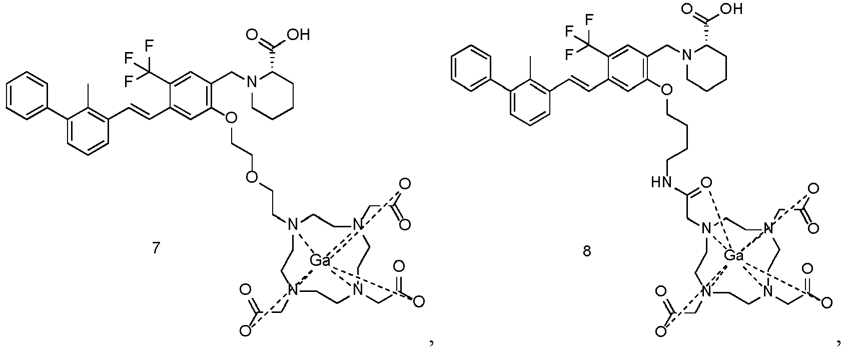 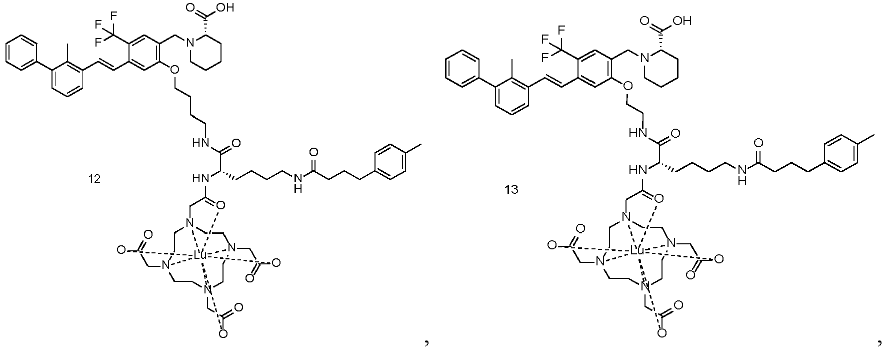 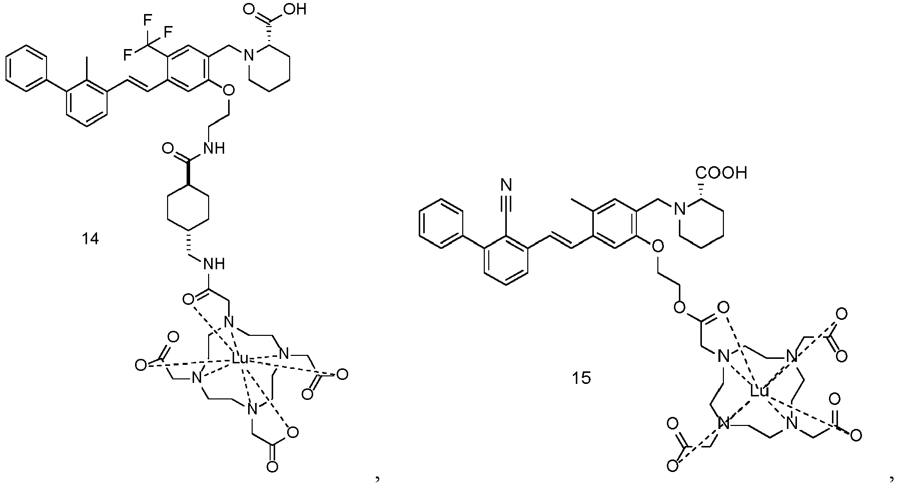 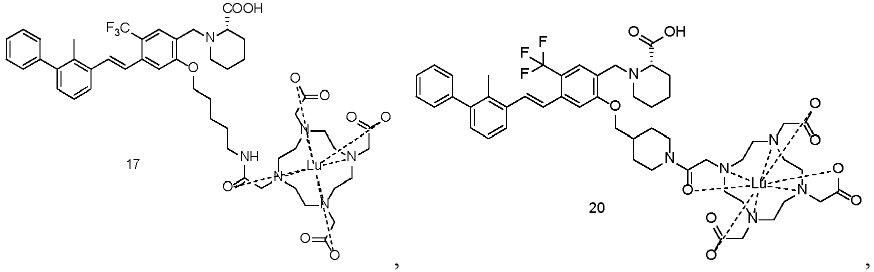 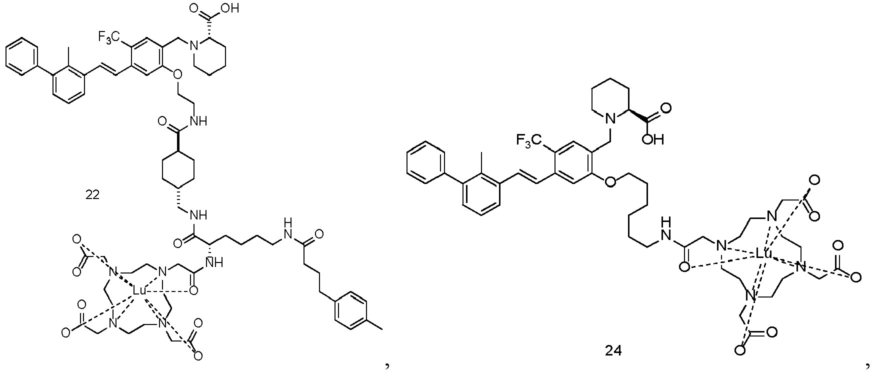 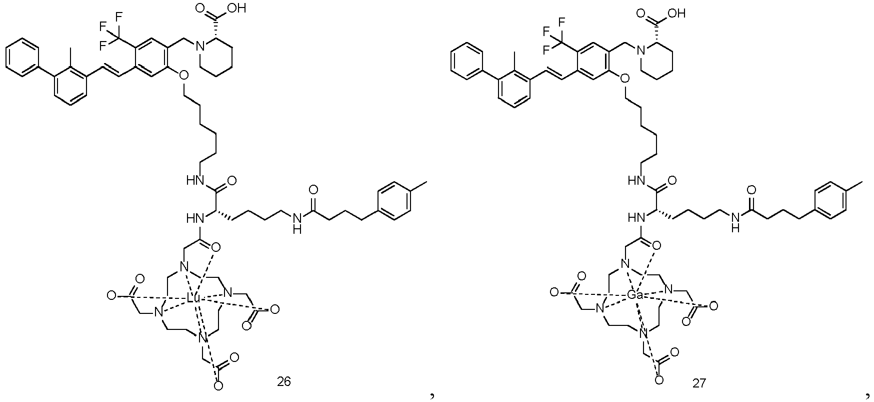 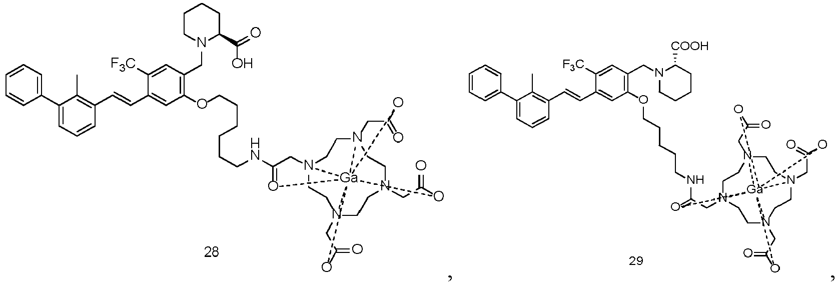 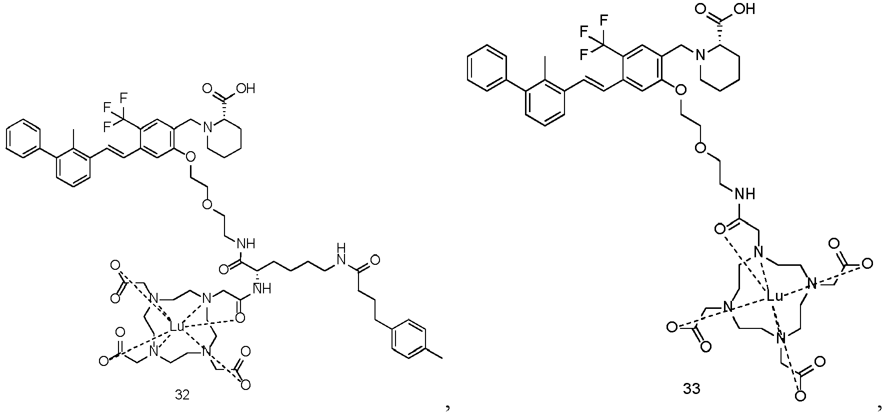 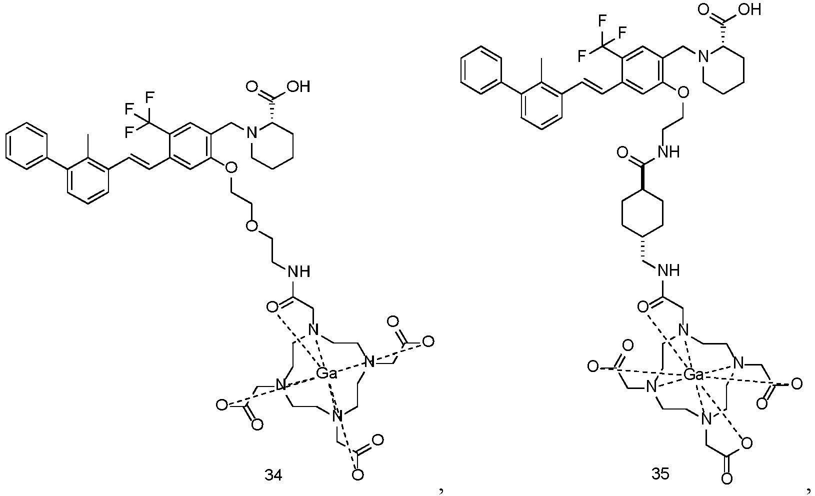 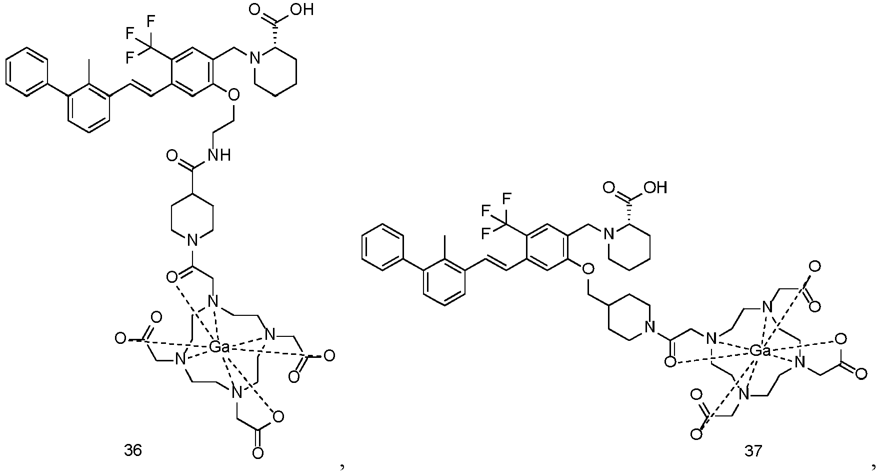 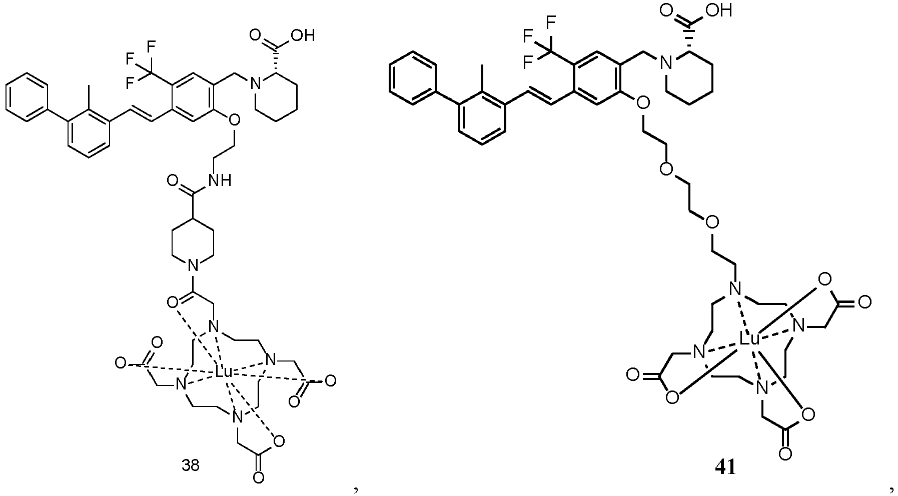 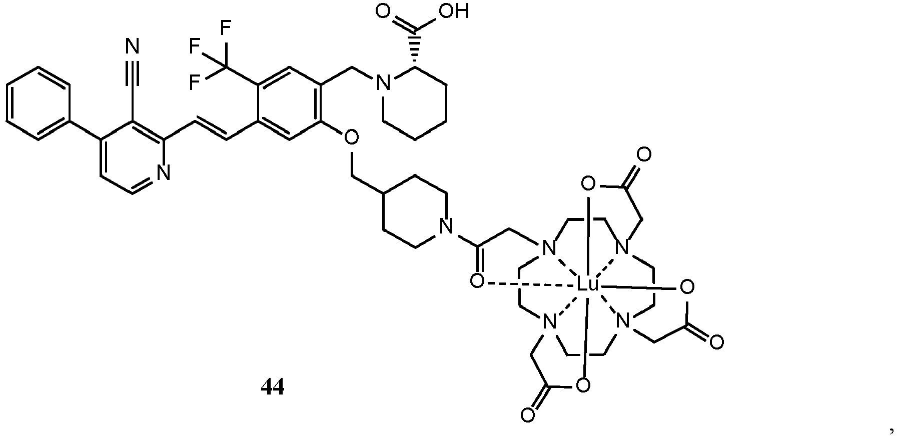 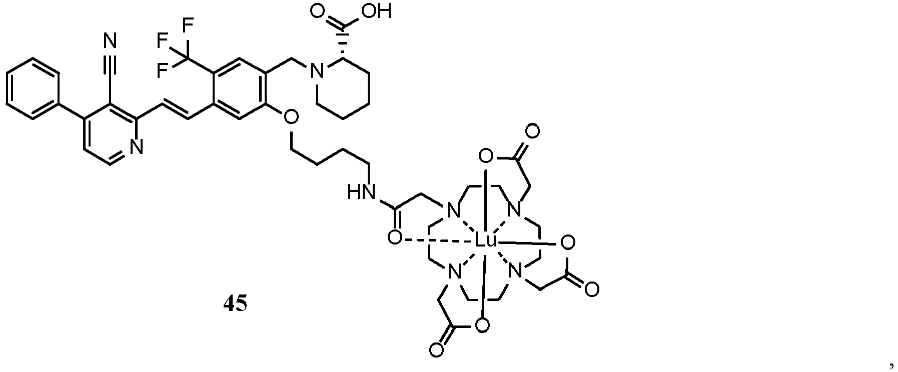 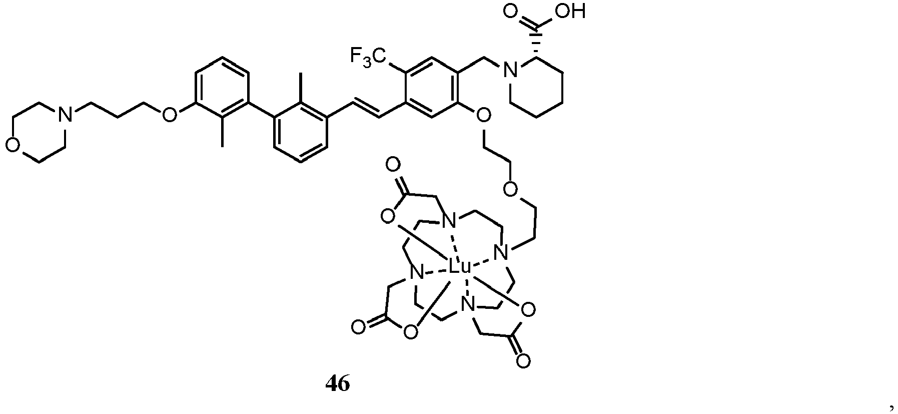 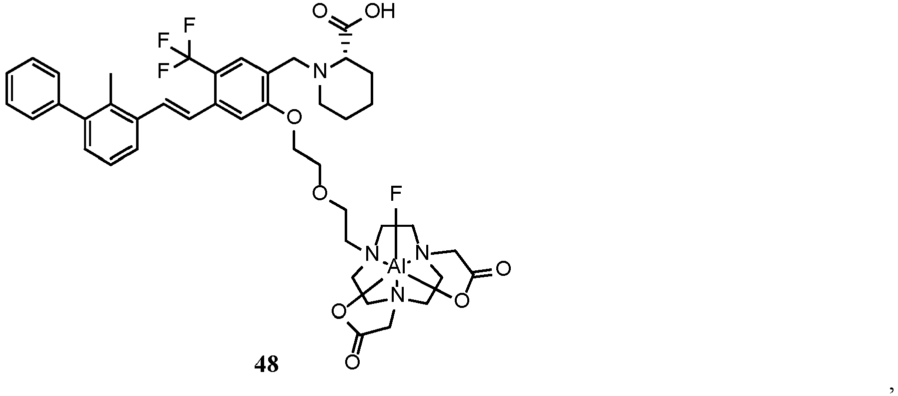 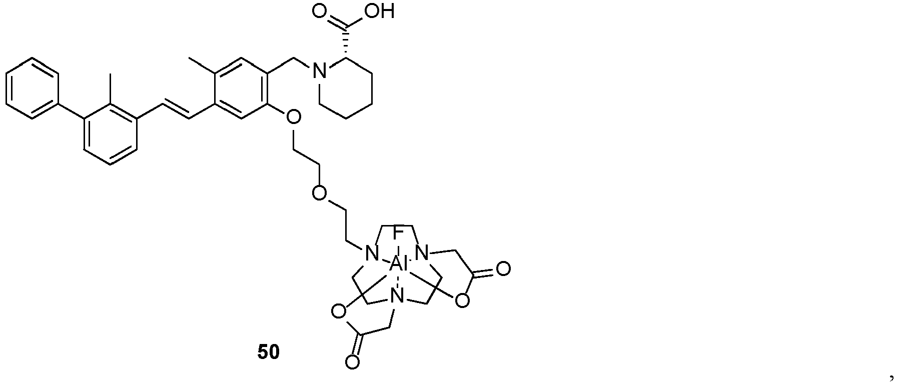 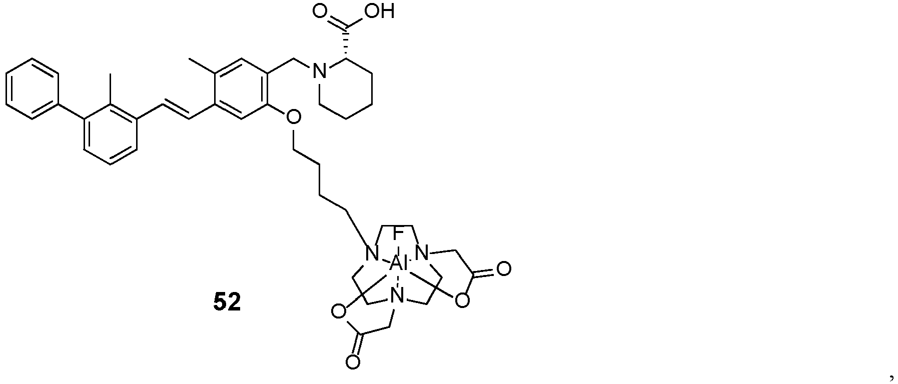 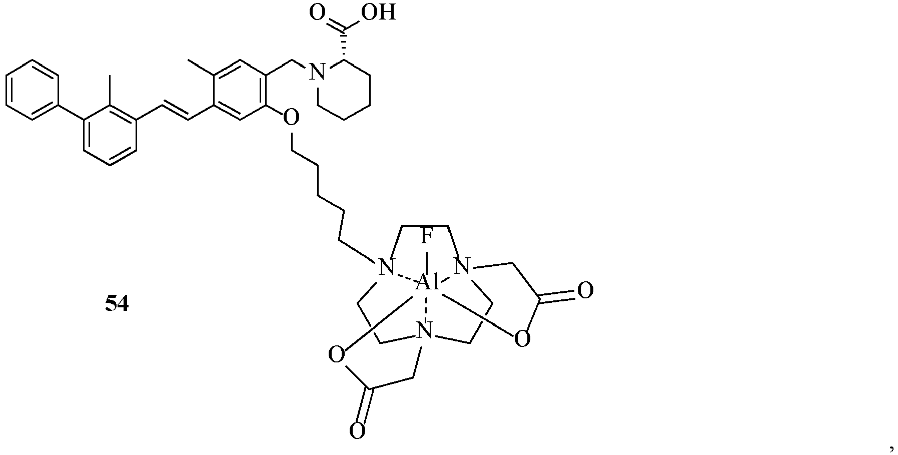 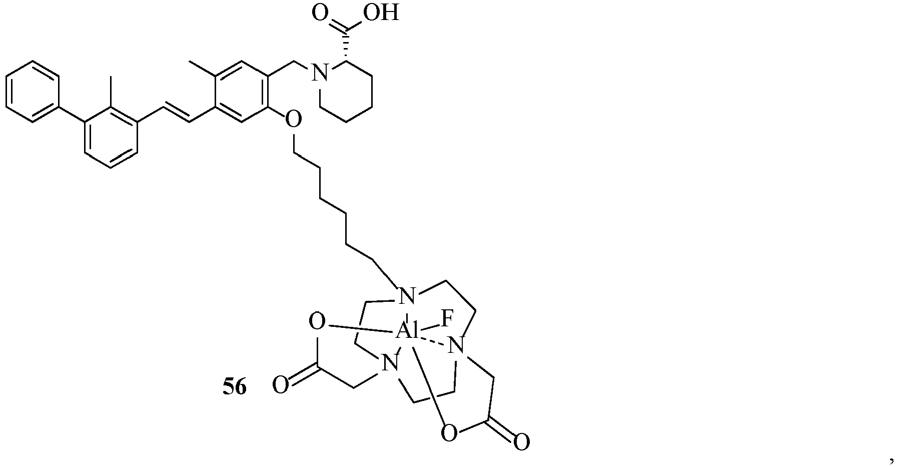 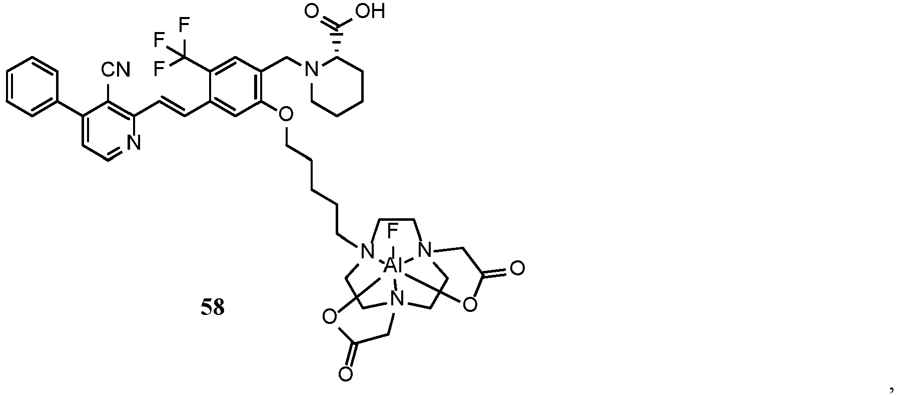 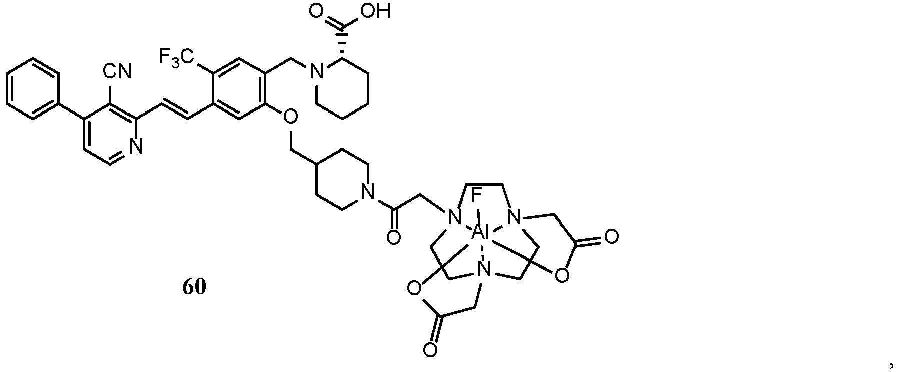 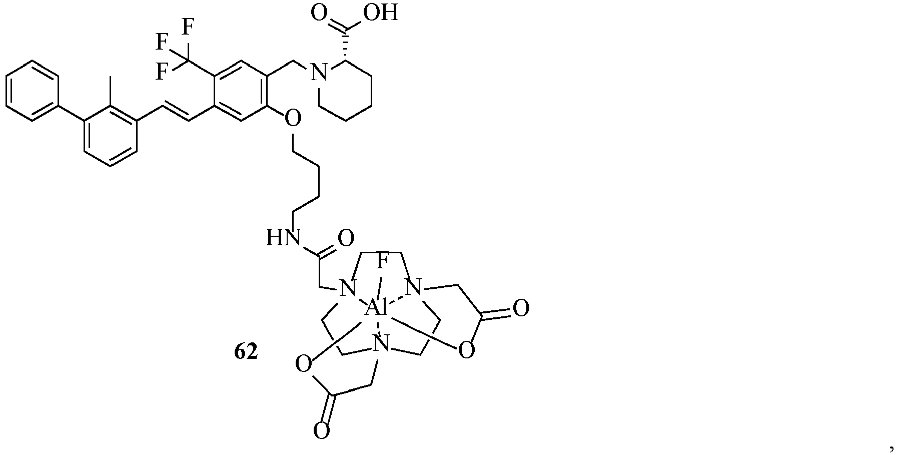 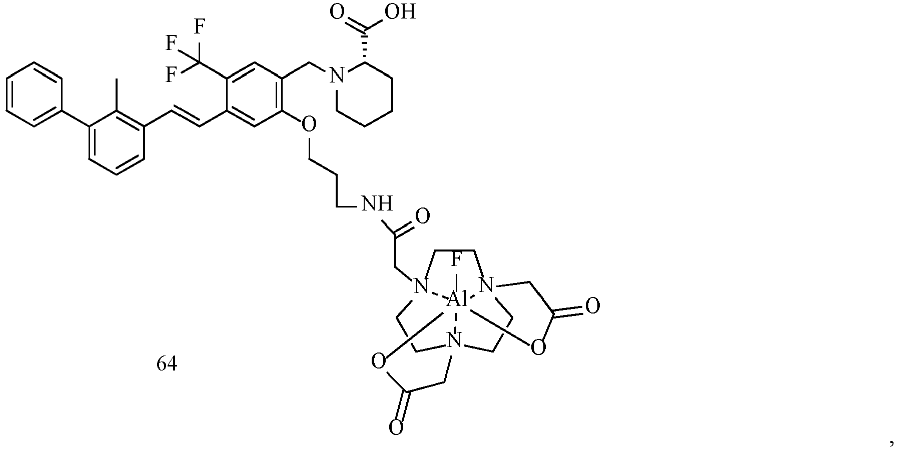 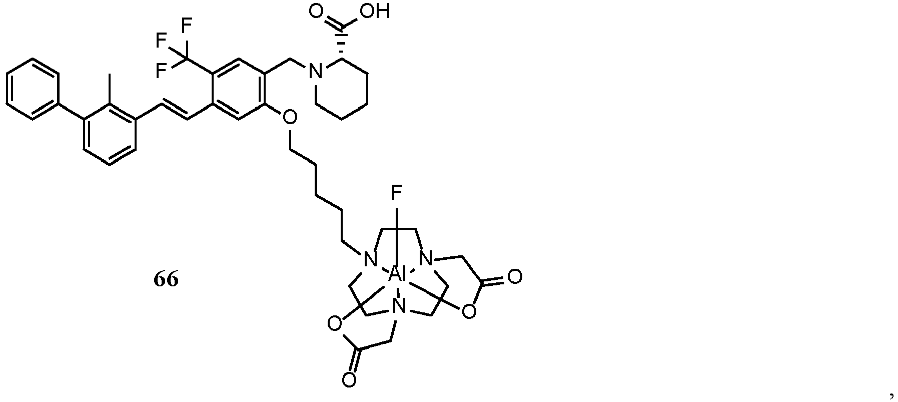 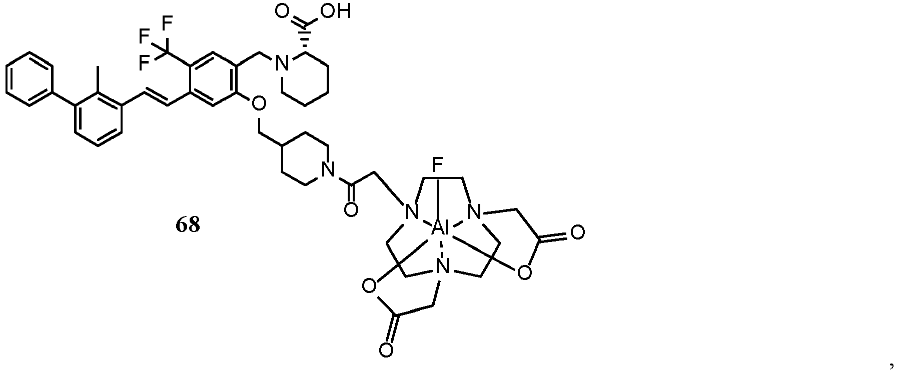 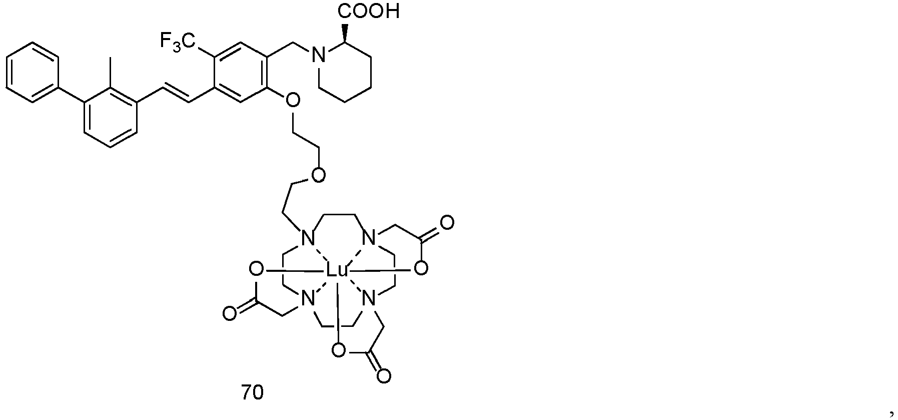 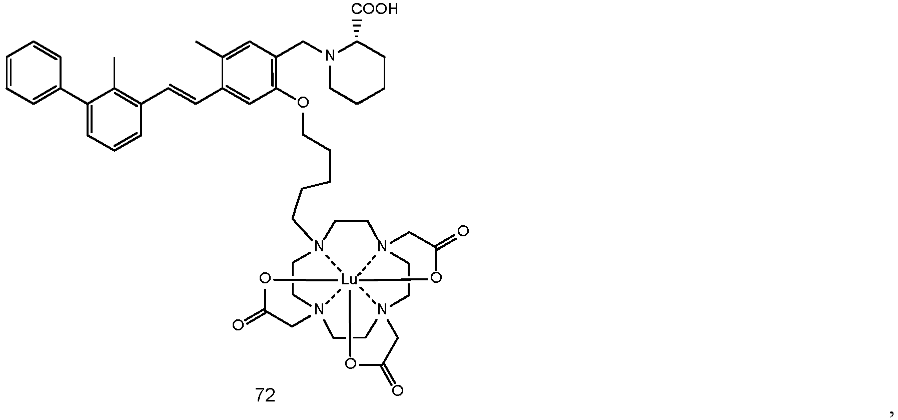 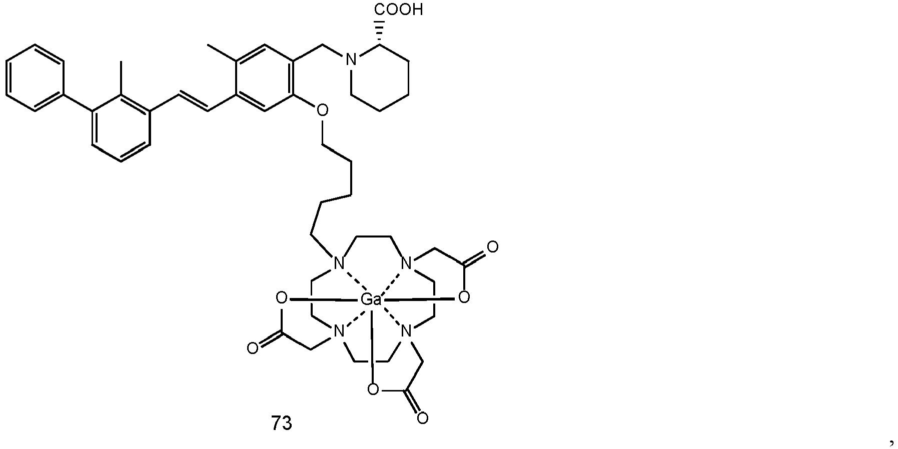 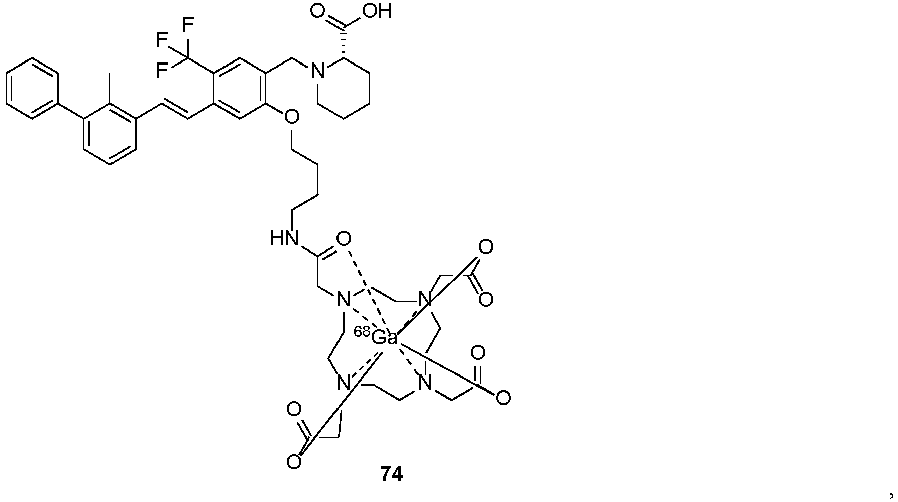 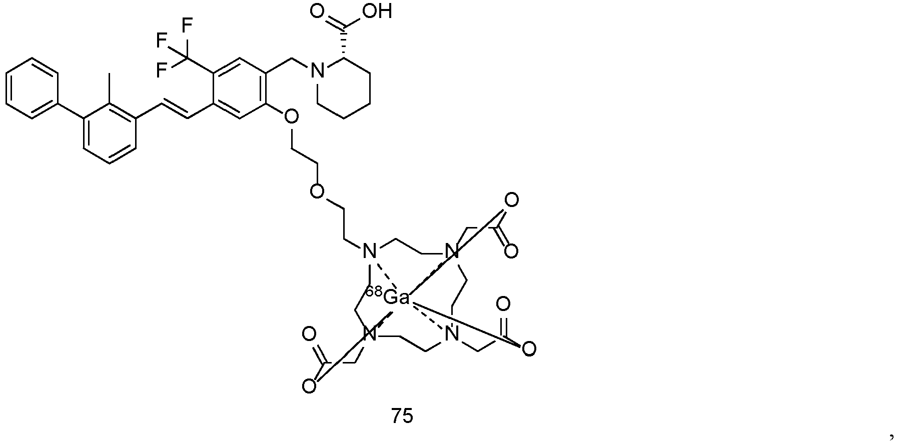 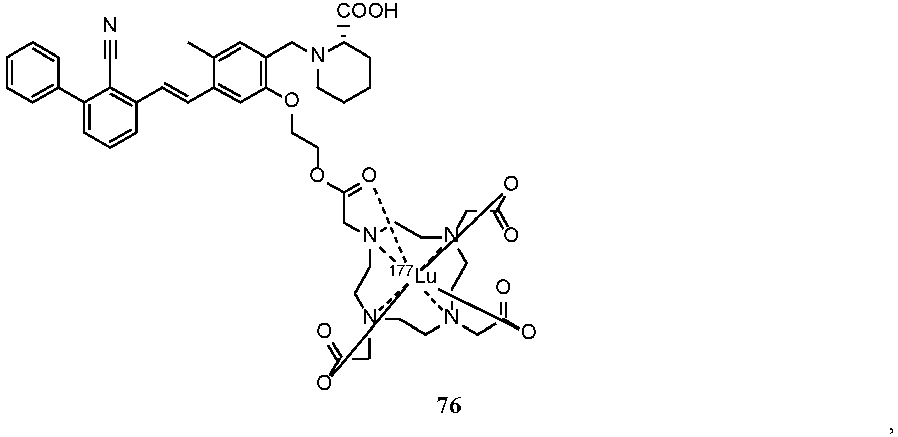 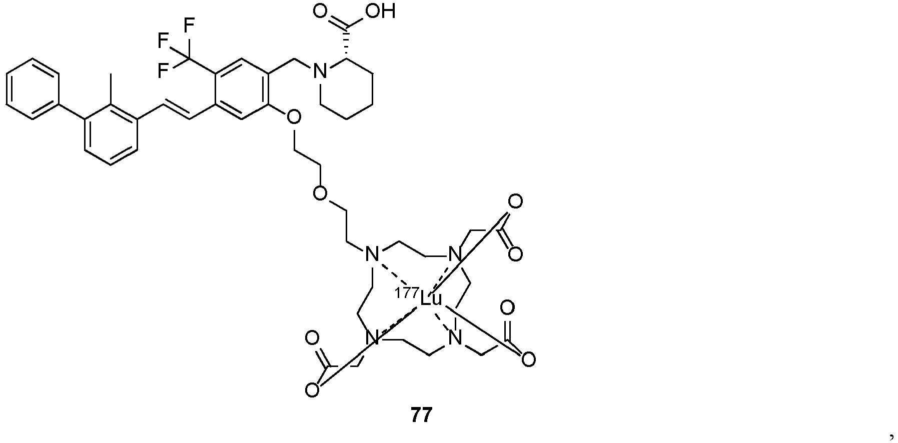 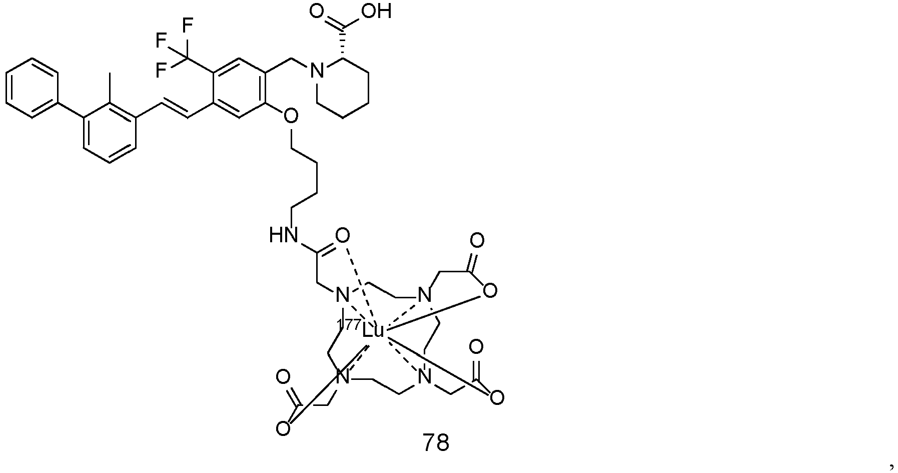 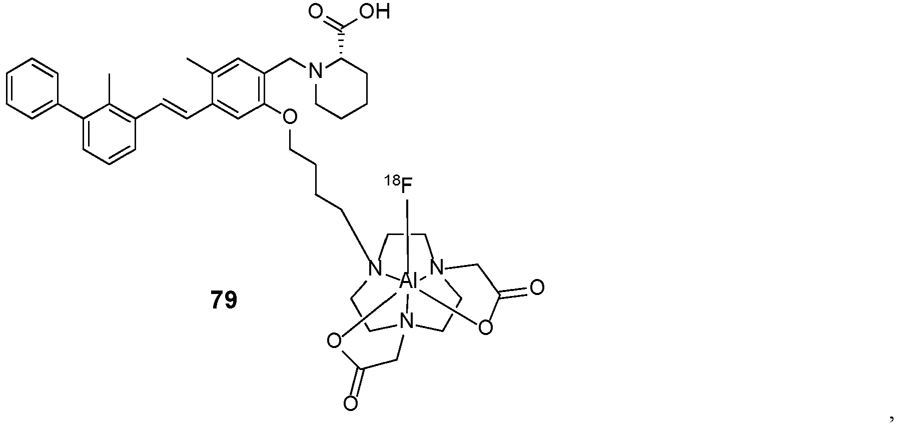 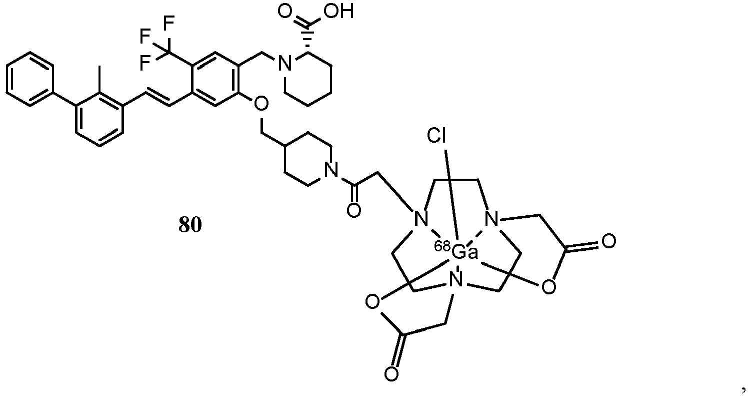 or

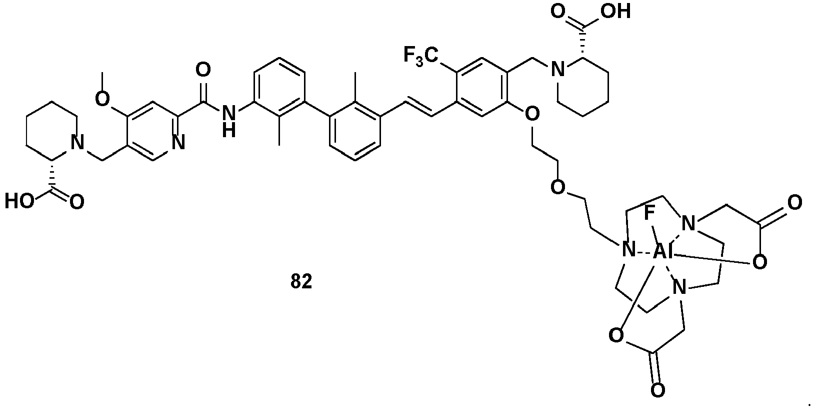

The present disclosure also provides a method for preparing the compound represented by the general formula I as described above, which comprises the following steps: in a solvent (for example water, or a mixed solvent of water and methanol), reacting the compound represented by the general formula II with M metal halide ( for example, chloride, another example, [AlF]²⁺) in the presence or absence of a buffer (for example, sodium acetate-acetic acid), to obtain the compound represented by the general formula I; 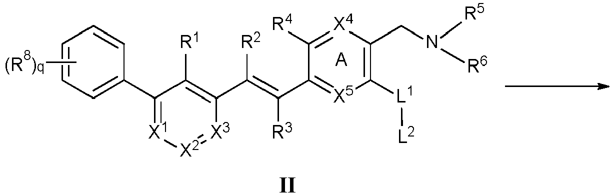 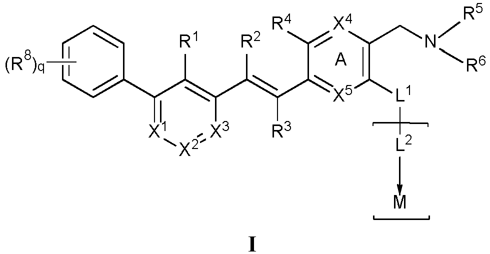 each variable is defined as described herein.

The present disclosure also provides a method for preparing the compound represented by the general formula II as described above, which comprises the following steps: in a solvent (for example, dichloromethane), removing the Boc protective group of the compound represented by the general formula III in the presence of acid (for example, trifluoroacetic acid), to obtain the compound represented by the general formula II; 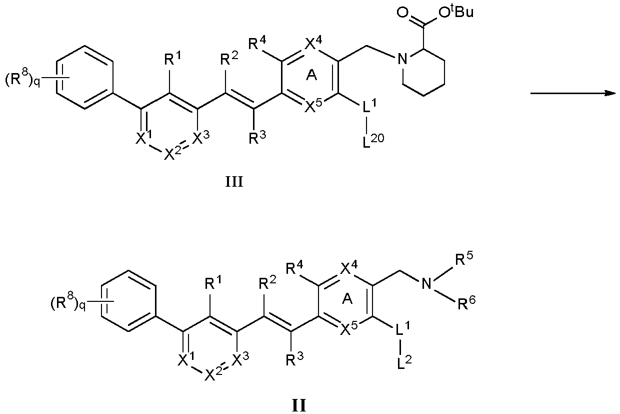
in formula II, L² is R¹¹ or -L⁴-(CH₂)ₛ-R¹¹;
in formula III, L²⁰ is R¹¹⁰ or -L⁴-(CH₂)ₛ-R¹¹⁰;
s is 1, 2, or 3;
L⁴ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
R¹¹ is 8- to 20- membered (for example 8-, 9-, 10-, 11-, 12-, 13-, 14-, 15-, or 16-membered) saturated monocyclic or bridged carbocyclic ring; 3, 4, 5, or 6 non-adjacent CH₂ of the monocyclic or bridged carbocyclic ring are independently replaced by -Y₅-, and each Y₅ is independently -O-, -NH-, or-N (R^{11a})-;
R¹¹⁰ is 8- to 20-membered (for example 8-, 9-, 10-, 11-, 12-, 13-, 14-, 15-, or 16-membered) saturated monocyclic or bridged carbocyclic ring, and 3, 4, 5, or 6 non-adjacent CH₂ of the monocyclic or bridged carbocyclic ring are independently replaced by -Y₆-, and each Y₆ is independently -O-, -NH-, or-N (R^{12a})-;
each R^{11a} is independently C₁-C₄ alkyl or C₁-C₄ alkyl substituted by one or more - COOH (for example, -(C₁-C₄ alkylene)-COOH, for example, -CH₂-COOH);
each R^{12a} is independently C₁-C₄ alkyl or C₁-C₄ alkyl substituted by one or more - COO^{t}Bu (for example, -(C₁-C₄ alkylene)-COO^{t}Bu, for example, -CH₂-COO^{t}Bu);
in formula II and formula III, R⁵, R⁶ together with the nitrogen atom to which they are attached form 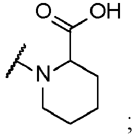
other variables are defined as described herein.

The present disclosure also provides a compound represented by general formula III: 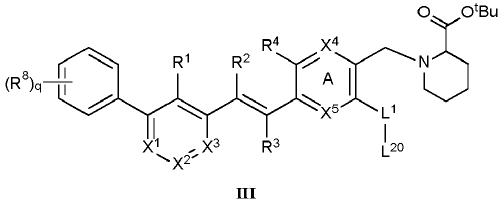 each variable is defined as described herein.

The present disclosure also provides a compound represented by any one of the following:
compound 2-b, 3-b, 4-b, 9-b, 10-b, 11-b, 16-b, 18-b, 19-b, 21-b, 23-b, 25-b, 30 -b, 30-c, 39-b, 40-b, 42-b, 43-b, 49-b, 51-b, 53-b, 55-b, 57-b, 63-b, 16-c , 81-b, 1-a, 2-a, 3-a, 4-a, 9-a, 10-a, 11-a, 16-a, 18-a, 19-a, 21-a, 23 -a, 25-a, 30-a, 31-a, 39-a, 40-a, 42-a, 43-a, 47-a, 49-a, 51-a, 53-a, 55-a , 57-a, 59-a, 61-a, 63-a, 65-a, 67-a, 69-a, 71-a, or 81-a.

The present disclosure also provides a pharmaceutical composition comprising the compound represented by the general formula II or the metal complex thereof, or the tautomer or stereoisomer of any one of described above, or the pharmaceutically acceptable salts of any one of described above, or the solvate of any one of described above, and at least one pharmaceutical excipient.

The present disclosure also provides a use of the compound represented by the general formula II or the metal complex thereof, or the tautomer or stereoisomer of any one of described above, or the pharmaceutically acceptable salts of any one of described above, or the solvate of any one of described above in the manufacture of a medicament for the treatment of tumor.

In some embodiments, the metal complex is a complex chelated by the compound represented by general formula II with radioactive metal ion; the radioactive metal ion is radioactive metal ion for treatment.

In some embodiments, in the metal complex chelated by the compound represented by general formula II with radioactive metal ion, the metal ion is [¹⁷⁷LuCl]²⁺ or ¹⁷⁷Lu³⁺.

The present disclosure also provides a use of the metal complex as described above, or the tautomer or stereoisomer of any one of described above, or the pharmaceutically acceptable salts of any one of described above, or the solvate of any one of described above in the manufacture of a medicament for the radiodiagnosis of tumor.

In some embodiments, the metal complex is a complex chelated by the compound represented by general formula II with radioactive metal ion; the radioactive metal ion is radioactive metal ion for diagnosis.

In some embodiments, in the metal complex chelated by the compound represented by general formula II with radioactive metal ion, the metal ion is [Al¹⁸F]²⁺, [⁶⁸GaCl]²⁺, or ⁶⁸Ga³⁺.

The present disclosure also provides a method for treating tumor, which comprises administering to a tumor patient a therapeutically effective amount of the compound represented by the general formula II or the metal complex thereof, or the tautomer or stereoisomer of any one of described above, or the pharmaceutically acceptable salts of any one of described above, or the solvate of any one of described above.

In some embodiments, the metal complex is a complex chelated by the compound represented by general formula II with radioactive metal ion; the radioactive metal ion is radioactive metal ion for treatment.

In some embodiments, in the metal complex, the metal ion is [¹⁷⁷LuCl]²⁺ or ¹⁷⁷Lu³⁺.

In some embodiments, the tumor is lung cancer, gastric cancer, colorectal cancer, cervical cancer, ovarian cancer, prostate cancer, breast cancer, pancreatic cancer, liver cancer, bladder cancer, renal cancer, bone cancer, skin cancer, melanoma, glioma, glioblastoma, leukemia, or lymphoma.

The present disclosure also provides a method for diagnosing tumor, which comprises administering to a tumor patient a therapeutically effective amount of the metal complex as described above, or the tautomer or stereoisomer of any one of described above, or the pharmaceutically acceptable salts of any one of described above, or the solvate of any one of described above.

In some embodiments, the metal complex is a complex chelated by the compound represented by general formula II with radioactive metal ion; the radioactive metal ion is radioactive metal ion for diagnosis.

In some embodiments, in the metal complex chelated by the compound represented by general formula II with radioactive metal ion, the metal ion is [Al¹⁸F]²⁺, [⁶⁸GaCl]²⁺, or ⁶⁸Ga³⁺.

In some embodiments, the tumor is lung cancer, gastric cancer, colorectal cancer, cervical cancer, ovarian cancer, prostate cancer, breast cancer, pancreatic cancer, liver cancer, bladder cancer, renal cancer, bone cancer, skin cancer, melanoma, glioma, glioblastoma, leukemia, or lymphoma.

### Definition and Description

Unless otherwise indicated, the following terms and phrases used herein are intend to have the following meanings. A particular term or phrase should not be considered uncertain or unclear in the absence of a specific definition, but should be understood in its ordinary meaning. When a trade name appears herein, it is intended to refer to its corresponding commodity or its active ingredient.

In the present disclosure, the term "metal chelating group" refers to a group that forms a complex with a metal atom or ion. The metal chelating group may be any metal chelating group known in the art for complexing pharmaceutically useful metal atoms or ions.

In the present disclosure, the term "substitution" or "substituent" means that a hydrogen atom in a group is replaced by a specified group. When the position of substitution is not specified, substitution may be at any position, but only the formation of a stable or chemically viable chemical is allowed. An example is as follows: structure 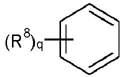 indicates that the hydrogen atoms on the benzene ring are substituted by q R⁸, and each R⁸ is the same or different when there are multiple R⁸.

When any variable (for example R) occurs more than once in the composition or structure of a compound, its definition in each case is independent. Thus, for example, if a group is substituted by 0-2 R, then the group may optionally be substituted by up to two R, and R in each case have independent options. In addition, the combination of substituent and/or variant thereof is allowed only if such a combination will result in a stable compound.

When the connecting group listed in the present disclosure does not specify its connecting direction, its connecting direction can be arbitrary and comprises both left to right connection and from right to left connection. An example is as follows, the linking group L in -A-L-B is -C-D-, and when the connection direction of L is not specified, -A-L-B comprises -A-C-D-B and -A-D-C-B.

When one of the variables is selected from a single bond, it means that the two groups connected to it are directly connected, for example, when L in A-L-Z represents a single bond, it means that the structure is actually A-Z.

In the present disclosure, the term "alkyl" refers to a saturated linear or branched chain monovalent hydrocarbyl. C₁-C₄ alkyl refers to an alkyl with 1-4 carbon atoms, specifically methyl, ethyl, n-propyl, isopropyl, n-butyl, isobutyl, sec-butyl, or tert-butyl.

In the present disclosure, the term "alkylene" refers to a saturated linear or branched chain divalent hydrocarbyl. C₁-C₄ alkylene refers to an alkylene with 1-4 carbon atoms, specifically methylene, ethylene (for example, -CH₂CH₂-, -CH(CH₃)-), propylene (for example, -CH₂CH₂CH₂-, -C(CH₃)₂-, -CH₂CH(CH₃)-), butylene (for example, -CH₂CH₂CH₂CH₂-, - CH(CH₃)CH(CH₃)-, -CH₂CH(CH₃)CH₂-).

In the present disclosure, halogen refers to F, Cl, Br, or I.

In the present disclosure, the term "halogenated alkyl" refers to a group formed by the substitution of one or more hydrogen atoms in an alkyl by halogen, wherein the alkyl is as defined above, and each halogen is independently F, Cl, Br, or I. Halogenated C₁-C₄ alkyl refers to C₁-C₄ alkyl substituted by one or more halogens, for example, fluorinated C₁-C₄ alkyl, wherein C₁-C₄ alkyl is as defined above. Examples of halogenated alkyl include, but are not limited to trifluoromethyl, pentafluoroethyl, 1-fluoro-2-chloroethyl.

In the present disclosure, the term "carbocyclic ring" refers to a saturated, partially unsaturated, or aromatic monocyclic or polycyclic (for example, paracyclic, spirocyclic, or bridged ring) cyclic group formed by carbon atoms. In the saturated carbocyclic ring, each carbon atom on the ring is saturated, and examples of saturated carbocyclic rings include, but are not limited to 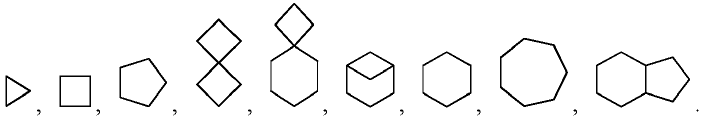 In the aromatic carbocyclic ring, each ring is aromatic, and examples of aromatic carbocyclic rings include, but are not limited to 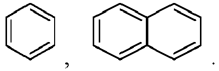 In the partially unsaturated carbocyclic ring, at least one carbon atom on the ring is saturated and at least one carbon atom is unsaturated or aromatic, and examples of partially unsaturated carbocyclic rings include, but are not limited to 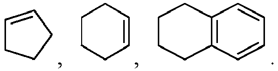 The 5- to 7-membered carbocyclic ring may specifically be a 5, 6, or 7-membered carbocyclic ring. In some embodiments, the 5- to 7-membered carbocyclic ring may specifically be 5, 6, or 7-membered saturated carbocyclic ring. In some embodiments, the 5- to 7-membered carbocyclic ring may specifically be 5, 6, or 7-membered saturated monocyclic carbocyclic ring, including 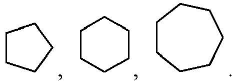 In some embodiments, the 5- to 7-membered carbocyclic ring may specifically be benzene ring.

In the present disclosure, the term "heterocyclic ring" refers to a saturated, partially unsaturated, or aromatic monocyclic or polycyclic (for example paracyclic, spirocyclic or bridged ring) cyclic group formed by carbon atoms and at least one heteroatom, wherein the heteroatoms are independently selected from N, O, and S. In the saturated heterocyclic ring, the carbon atoms and heteroatoms on the ring are all saturated, and examples of saturated heterocyclic rings include, but are not limited to 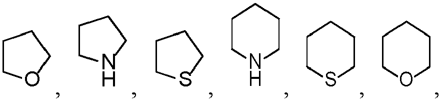 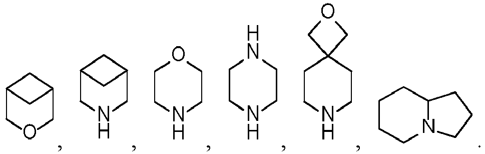 In the aromatic heterocyclic ring, each ring is aromatic, and examples of the aromatic heterocyclic rings include, but are not limited to 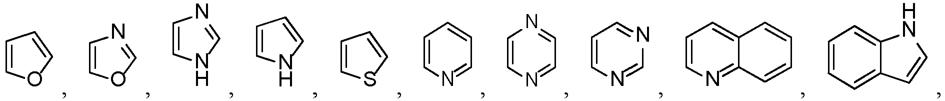 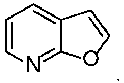 In the partially unsaturated heterocyclic ring, at least one atom on the ring is saturated and at least one atom is unsaturated or aromatic, and examples of partially unsaturated heterocyclic rings include, but are not limited to 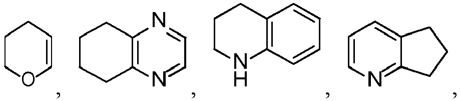 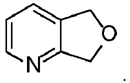 The 5- to 7-membered heterocyclic ring may specifically be 5, 6, or 7-membered heterocyclic ring. In some embodiments, the 5- to 7-membered heterocyclic ring may specifically be 5, 6, or 7-membered saturated heterocyclic ring. In some embodiments, the 5-to 7-membered heterocyclic ring may specifically be 5, 6, or 7-membered saturated monocyclic heterocyclic ring, including 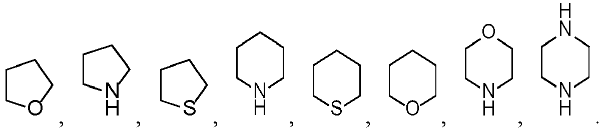 In some embodiments, the 5- to 7-membered carbocyclic ring may specifically be a benzene ring.

In the present disclosure, the term "aryl" refers to an aromatic carbocyclic group, wherein each ring is aromatic. The C₆-C₁₀ aryl may specifically be phenyl or naphthyl. In some embodiments, the C₆-C₁₀ aryl may specifically be phenyl.

In the present disclosure, the term "stereoisomer" includes enantiomer, diastereomer, geometrical isomer. They may be defined as (R)-/(S)-, or (D-/(L)-, or (R, R)-/(R, S)-/(S, S)-according to absolute stereochemistry for amino acids. The present disclosure includes racemic, enantioenriched and optionally pure forms thereof. These isomers can be synthesized using chiral raw materials, prepared by chiral resolution, or can be resolved using conventional techniques such as, but not limited to, high performance liquid chromatography (HPLC) using chiral columns.

In the chemical structure, the wedge-shaped solid bond ( 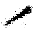 ) and the wedge-shaped dashed bond ( 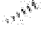 ) are used to represent absolute configuration of a stereocenter, and the straight solid bond ( 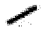 ) and straight dashed bond ( 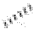 ) are used to represent the relative configuration of a stereocenter. The bond " 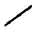 " does not specify a configuration, that is, if configurational isomerism exists in the chemical structure, the bond " 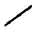 " can be " 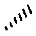 " or " 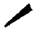 ", or contain both " 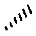 " and " 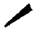 " configurations (for example, the ratio of " 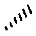 " and " 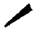 " is 1: 1).

In the present disclosure, the term "pharmaceutically acceptable" refers to a substance (for example, a carrier or diluent) that do not affect the biological activity or properties of the compound of the present disclosure and are relatively non-toxic, namely, the substance can be administered to an individual without causing undesirable biological reaction or interaction in an undesirable manner with any component contained in the composition.

In the present disclosure, the term "pharmaceutically acceptable salt" means a salt formed from a suitable non-toxic organic acid, inorganic acid, organic base, or inorganic base with a compound, which retains the biological activity of the compound. The organic acid may be a variety of conventional organic acids capable of forming salts in the art, preferably one or more of methanesulfonic acid, p-toluenesulfonic acid, maleic acid, fumaric acid, citric acid, tartaric acid, malic acid, lactic acid, formic acid, acetic acid, propionic acid, trifluoroacetic acid, oxalic acid, butanedioic acid, benzoic acid, hydroxyethylsulfonic acid, naphthalenesulfonic acid, and salicylic acid. The inorganic acid may be a variety of conventional inorganic acids capable of forming salts in the art, preferably one or more of hydrochloric acid, sulfuric acid, and phosphoric acid. The organic base may be a variety of conventional organic bases capable of forming salts in the art, preferably one or more of pyridines, imidazoles, pyrazines, indoles, purines, tertiary amines, and anilines. The tertiary amine organic base is preferably triethylamine and/or *N*, *N-*diisopropylethylamine. The aniline organic base is preferably *N*, N dimethylaniline. The pyridine organic base is preferably one or more of pyridine, methylpyridine, 4-dimethylaminopyridine, and 2-methyl-5-ethylpyridine. The inorganic base may be a variety of conventional inorganic bases capable of forming salts in the art, preferably one or more of alkali metal hydride, alkali metal hydroxide, alkali metal alkoxide, potassium carbonate, sodium carbonate, lithium carbonate, cesium carbonate, potassium bicarbonate, and sodium bicarbonate. The alkali metal hydride is preferably sodium hydride and/or potassium hydride. The alkali metal hydroxide is preferably one or more of sodium hydroxide, potassium hydroxide, and lithium hydroxide. The alkali metal alkoxide is preferably one or more of sodium methoxide, sodium ethoxide, potassium tert-butoxide, and sodium tert-butoxide.

In the present disclosure, the term "solvate" refers to a substance formed by a compound or a salt thereof with a suitable solvent. The solvent is preferably water or an organic solvent.

In the present disclosure, the term "therapeutically effective amount" refers to a sufficient amount of medicament or agent that is non-toxic but can achieve the desired effect. The determination of the effective amount varies from person to person, depending on the age and general condition of the recipient, as well as on the specific active substance. The appropriate effective amount in individual cases can be determined by those skilled in the art based on routine experiments.

In the present disclosure, the term "patient" includes any animal, preferably a mammal, and more preferably a human.

In the present disclosure, the term "one or more" may be 1, 2, 3, 4, 5, or 6.

On the basis of not violating common sense in the art, the above preferred conditions can be combined arbitrarily to obtain preferred examples of the present disclosure.

The reagent and raw material used in the present disclosure are all commercially available.

The positive progress effect of the present disclosure lies in that the present disclosure provides a novel aromatic vinyl compound, metal complex thereof, and preparation method therefor and use thereof. The aromatic vinyl compounds and metal complexes thereof of the present disclosure have inhibitory activity on PD-1/PD-L1 binding, and thus can be used to treat related diseases such as tumor. In addition, the metal complexes of the present disclosure can also be used as imaging agents.

### DESCRIPTION OF DRAWINGS

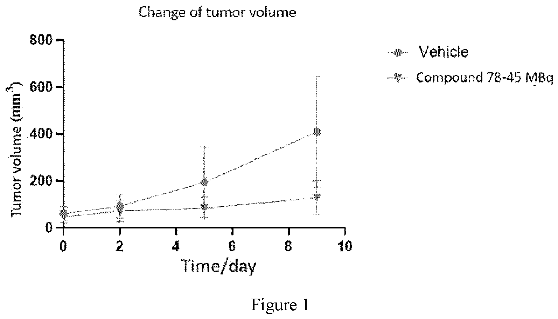

Figure 1 is a graph of the effect of compound 78 on tumor inhibition.

### DETAILED DESCRIPTION OF THE PREFERRED EMBODIMENT

The present disclosure is further illustrated below by means of examples, but the present disclosure is not limited to the scope of the examples. The experimental methods for which specific conditions are not indicated in the following examples are selected according to conventional methods and conditions, or according to the product instructions.

### Main experimental instruments:

Radioactivity meter (CRC-55tR type); electronic balance (YP30002); germanium-gallium generator (20mCi); vortex mixer (MX-F); HPLC (1200); TLC (Scan-RAM); gamma radioimmunoassay counter (GC-2016); UV spectrophotometer (T6 New Century);

### Embodiment 1

### (S,E)-2,2',2"-(10-(2-(2-(2-((2-carboxypiperidin-1-yl)methyl)-5-(2-(2-cyano-[1,1'-biphenyl]-3-yl)vinyl)-4-methylphenoxy)ethoxy)-2-oxoethyl)-1,4,7,10-tetraazacyclododecan-1,4,7-triyl)triacetic acid (compound 1)

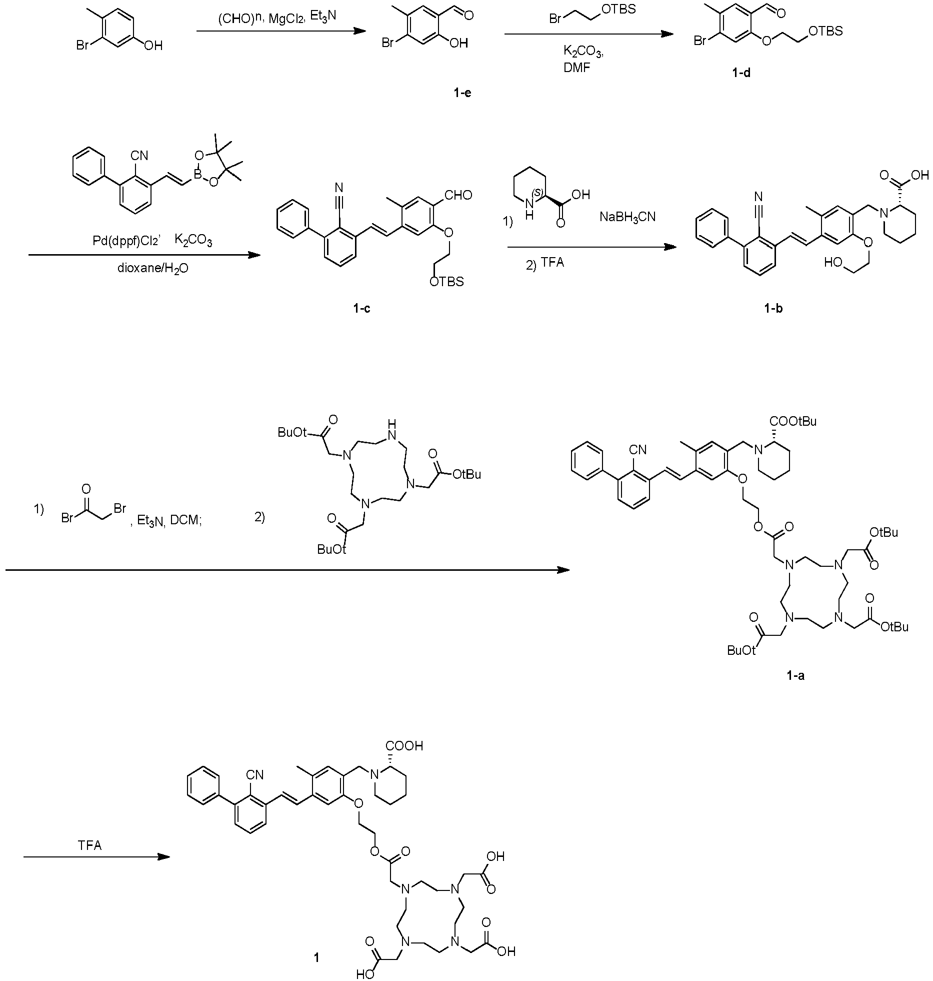

### Synthesis of compound 1-e

3-Bromo-4-methylphenol (3.74 g, 20 mmol), paraformaldehyde (4.41 g, 152 mmol), magnesium chloride (2.86 g, 30 mmol), and triethylamine (7.56 g, 75 mmol) were dissolved in acetonitrile (150 mL), and the reaction solution was heated and stirred at 80°C for 4 hours. The reaction solution was cooled to room temperature, diluted with water (500 mL), and the pH was adjusted to 2-3 with 1 M hydrochloric acid. The mixture was extracted with ethyl acetate (500 mL×2). The organic phases were combined, washed with saturated brine (200 mL×1), and the organic phases were concentrated under reduced pressure. The obtained residue was separated and purified by silica gel column chromatography (petroleum ether: ethyl acetate = 50:1) to obtain white solid **1-e** (2.45 g, yield: 57%).

### Synthesis of compound 1-d

**1-e** (645 mg, 3.0 mmol), (2-bromoethoxy) (tert-butyl) dimethylsilane (1.08 g, 4.5 mmol), and potassium carbonate (829 mg, 6.0 mmol) were dissolved in *N, 'N-*dimethylformamide (5 mL), and the reaction solution was heated at 60°C for 16 hours. The reaction solution was cooled to room temperature and diluted with water (50 mL). The obtained mixture was extracted with ethyl acetate (50 mL×2). The organic phase was washed sequentially with water (50 mL×2) and saturated brine (20 mL×2), dried over anhydrous sodium sulfate, and the organic phase was concentrated under reduced pressure. The obtained residue was separated and purified by silica gel column chromatography (petroleum ether: ethyl acetate = 20:1) to obtain white solid **1-d** (767 mg, yield: 68%).

### Synthesis of compound 1-c

Compound **1-d** (373 mg, 1.0 mmol), (E)-3-(2-(4,4,5,5-tetramethyl-1,3,2-dioxyboran-2-yl)vinyl)-[1,1'-biphenyl]-2-carbonitrile (397 mg, 1.2 mmol), [1,1'-bis(diphenylphosphino)ferrocene]dichloropalladium (73 mg, 0.1 mmol) and potassium carbonate (276 mg, 2.0 mmol) were dissolved in 1,4-dioxane (5 mL) and water (0.5 mL), and the reaction solution was heated and stirred at 90 °C for 16 hours. The reaction solution was cooled to room temperature, and the reaction solution was diluted with saturated brine (20 mL). The resulting mixture was extracted with dichloromethane (30 mL×3). The organic phases were combined, dried over anhydrous sodium sulfate, filtered, and concentrated under reduced pressure. The obtained residue was separated and purified by silica gel column chromatography (petroleum ether: ethyl acetate = 5:1) to obtain yellow solid 1-c (355 mg, yield: 71%).

LC-MS (ESI): m/z = 498.5(M+H)⁺.

### Synthesis of compound 1-b

Compound **1-c** (75 mg, 0.15 mmol) and (*S*)-piperidine-2-carboxylic acid (39 mg, 0.30 mmol) were dissolved in methanol (1 mL) and tetrahydrofuran (1 mL), and sodium cyanoborohydride (38 mg, 0.60 mmol) was added. The reaction solution was stirred at 60°C for 1 hour. The reaction solution was cooled to room temperature and concentrated under reduced pressure, and tetrahydrofuran (1 mL), water (0.2 mL), and trifluoroacetic acid (0.5 mL) were added to the reaction solution, and the reaction solution was stirred at room temperature for 1 hour. The reaction solution was concentrated under reduced pressure, and the residue was purified by pre-HPLC to obtain white solid **1-b** (48.3 mg, yield: 53%).

LC-MS (ESI): m/z = 497.5 (M+H)⁺;

¹H-NMR (400 MHz, DMSO) δ: 8.05 (d, *J* = 8.0 Hz, 1H), 7.79 (t, *J* = 7.9 Hz, 1H), 7.67 (d, *J* = 16.0 Hz, 1H), 7.63-7.47 (m, 6H), 7.40 (d, *J* = 16.0 Hz, 1H), 7.27 (s, 2H), 7.07 (s, 1H), 4.13-3.90 (m, 5H), 3.75 (t, *J* = 4.6 Hz, 2H), 3.28-3.21 (m, 1H), 3.08-3.00 (m, 1H), 2.38 (s, 3H), 1.97-1.86 (m, 1H), 1.78-1.66 (m, 1H), 1.63-1.48 (m, 3H), 1.45-1.31 (m, 1H).

### Synthesis of compound 1-a

Compound **1-b** (40 mg, 0.08 mmol) and triethylamine (65 mg, 0.64 mmol) were dissolved in dichloromethane (1 mL). The reaction solution was cooled to 0°C, and 2-bromoacetyl bromide (19 mg, dissolved in 1 mL of dichloromethane) and tert-butyl 2,2',2"-(1,4,7,10-tetraazacyclododecan-1,4,7-triyl) triacetate (206 mg, 0.40 mmol) were added to the reaction solution sequentially. The reaction solution was raised to room temperature and stirred at room temperature for 5 hours. The reaction solution was concentrated under reduced pressure and the obtained residue was separated by reversed-phase preparative chromatography (column xBridge C18, 19 * 150 mm * 5 um; mobile phase: water (0.1% trifluoroacetic acid), methanol; gradient: 65%-80% (initial mobile phase was 35% water/65% methanol, and ending mobile phase was 20% water/80% methanol, wherein % refers to volume percentage); 13 min; flow rate 15 mL/min) to obtain white solid **1-a** (22 mg, yield: 26%).

LC-MS (ESI): m/z = 1051.87(M+H)⁺.

### Synthesis of compound 1

Compound **1-a** (22 mg, 0.021 mmol) was dissolved in dichloromethane (1 mL), and trifluoroacetic acid (2 mL) was added, and the reaction solution was stirred at room temperature for 16 hours. The reaction solution was concentrated under reduced pressure and the obtained residue was separated by reversed-phase preparative chromatography (column xBridge C18, 19 * 150 mm * 5 um; mobile phase: water (0.1% trifluoroacetic acid), methanol; gradient: 65%-80% (initial mobile phase was 35% water/65% methanol, and ending mobile phase was 20% water/80% methanol, wherein % refers to volume percentage); 13 min; flow rate 15 mL/min) to obtain white solid **1** (7 mg, yield: 38%). LC-MS (ESI): m/z = 883.8(M+H)⁺;

¹H-NMR (400 MHz, DMSO) δ: 8.05 (d, *J* = 8.2 Hz, 1H), 7.79 (t, *J* = 7.9 Hz, 1H), 7.66 (d, *J* = 16.1 Hz,1H), 7.62-7.47 (m, 6H), 7.39 (d, *J* = 16.0 Hz, 1H), 7.28 (d, *J* = 6.1 Hz, 2H), 4.56-4.41 (m, 1H), 4.39-4.21 (m, 3H), 4.08-3.96 (m, 1H), 3.82-3.40 (m, 15H), 3.11-2.73 (m, 16H), 2.38 (s, 3H), 2.36-2.28 (m,1H), 1.94-1.62 (m, 3H), 1.59-1.33 (m, 3H).

### Embodiment 2

### (2S)-1-({4-[(1E)-2-(2-Methyl-3-phenylphenyl)vinyl]-5-(trifluoromethyl)-2-{[2-({2-({2-[4,7,10-tris(carboxymethyl)-1,4,7,10-tetraazacyclododecan-1-yl]ethyl}oxy)ethyl]oxy}phenyl}methyl)hexahydropyridine-2-carboxylic acid (compound 2)

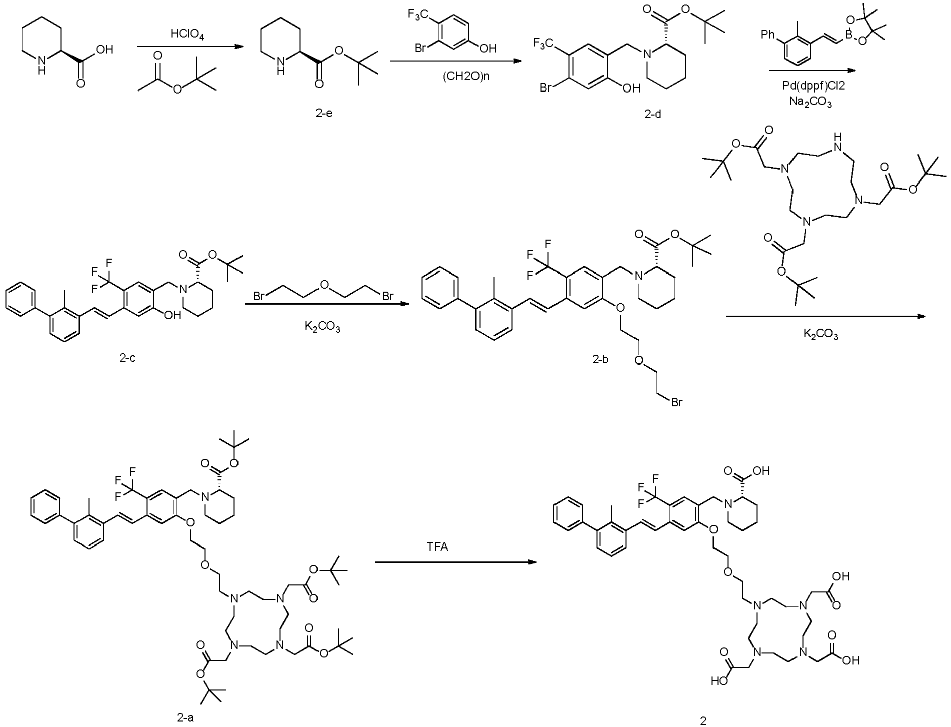

### Synthesis of compound 2-e

(25)-Hexahydropyridine-2-carboxylic acid (20 g, 154.847 mmol) was dissolved in 2-methylpropan-2-yl acetate (313.727 mL, 2322.701 mmol), and perchloric acid (18.630 mL, 309.693 mmol) was added. The reaction solution was stirred at room temperature overnight. The reaction solution was concentrated under reduced pressure, and the obtained residue was diluted with ethyl acetate (50 mL), and the pH was adjusted to 9-10 with saturated sodium carbonate solution, and the mixture was washed sequentially with water (50 mL × 2) and saturated brine (50 mL × 1). The organic phase was dried and concentrated under reduced pressure to obtain **2-e** (23.24g, yield: 81.00%).

### Synthesis of compound 2-d

3-Bromo-4-(trifluoromethyl) phenol (10.96 g, 45.475 mmol) was dissolved in toluene (200 mL), **2-e** (12.64 g, 68.213 mmol) and paraformaldehyde (2.73 g, 90.951 mmol) were added to the reaction solution, and the reaction was heated and stirred at 110°C for 6 hours. The reaction solution was cooled to room temperature, and the reaction solution was diluted with ethyl acetate (50 mL), and washed sequentially with water (50 mL×2) and saturated brine (50 mL×1). The organic phase was dried, and concentrated under reduced pressure to obtain **2-d** (15.26 g, yield: 76.57%).

### Synthesis of compound 2-c

4,4,5,5-Tetramethyl-2-[(1*E*)-2-(2-methyl-3-phenylphenyl)vinyl]-1,3,2-dioxyborane (9.00 g, 28.092 mmol) was dissolved in dioxane (160 mL) and water (4 mL), and compound **2-d** (10.26 g, 23.410 mmol), [1,1'-bis(diphenylphosphino)ferrocene] dichloropalladium (2.02 g, 2.341 mmol), and sodium carbonate (6.20 g, 58.524 mmol) were added to the reaction solution, and the reaction solution was heated and stirred at 80°C for 12 hours. The reaction solution was cooled to room temperature, and the reaction solution was diluted with ethyl acetate (50 mL), and washed sequentially with water (50 mL×2) and saturated brine (50 mL×1). The organic phase was dried and concentrated to obtain **2-c** (10.78 g, yield: 83.50%).

### Synthesis of compound 2-b

Compound **2-c** (551.65 mg, 1.0 mmol) and 1-bromo-2-[(2-bromoethyl)oxy]ethane (695.73 mg, 3.000 mmol) were dissolved in *N*, *N*-dimethylformamide (5 mL), and potassium carbonate (276.42 mg, 2.000 mmol) was added, and the reaction solution was stirred for at 70 °C for 3 hours. The reaction solution was cooled to room temperature, and the reaction solution was diluted with ethyl acetate (50 mL), washed sequentially with water (20 mL×2) and saturated brine (20 mL×2). The organic phase was separated, dried over anhydrous sodium sulfate, filtered, and concentrated under reduced pressure, and the residue was separated by column chromatography (petroleum ether: ethyl acetate=5:1) to obtain product **2-b** (548 mg, yield: 77.99%).

### Synthesis of compound 2-a

Compound **2-b** (400 mg, 0.569 mmol) and tert-butyl 2,2',2"-(1,4,7,10-tetraazacyclododecan-1,4,7-triyl) triacetate (292.87 mg, 0.569 mmol) were dissolved in acetonitrile (4 mL), and potassium carbonate (78.68 mg, 0.569 mmol) was added. The reaction solution was stirred at 60°C for 16 hours. The reaction solution was cooled to room temperature, concentrated under reduced pressure, and separated by silica gel column chromatography (acetic acid in methanol: dichloromethane = 1: 10) to obtain **2-a** (450 mg, yield: 69.56%).

### Synthesis of compound 2

Compound **2-a** was dissolved in dichloromethane (4 mL), and trifluoroacetic acid (4 mL, 0.510 mmol) was added. The reaction solution was stirred at room temperature for 16 hours. The reaction solution was concentrated under reduced pressure, and the residue was purified by Prep-HPLC to obtain **2** (153 mg, yield: 42.37%).

¹H-NMR(400MHz, MeOD) δ: 7.89 (s, 1H), 7.66 (d, *J* = 15.8Hz, 1H), 7.59 (s, 1H), 7.54 (d, *J* = 7.5Hz, 1H), 7.46-7.39 (m, 2H), 7.38-7.24 (m, 5H), 7.18 (d, *J* = 6.9Hz, 1H), 4.61 (d, *J* = 13.0Hz, 1H), 4.50-4.37 (m, 3H), 4.00-3.83 (m, 4H), 3.77-3.56 (m, 7H), 3.53-3.26 (m, 9H), 3.25-2.98 (m, 11H), 2.35-2.29 (m, 3H), 2.25 (d, *J* = 15.2Hz, 1H), 2.02-1.51 (m, 5H).

### Embodiment 3

### (2S)-1-({4-[(1E)-2-(2-Methyl-3-phenylphenyl)vinyl]-5-(trifluoromethyl)-2-{[2-({2-[4,7,10-tris(carboxymethyl)-1,4,7,10-tetraazacyclododecan-1-yl]acetyl}amino)ethyl]oxy}phenyl} methyl)hexahydropyridine-2-carboxylic acid (compound 3)

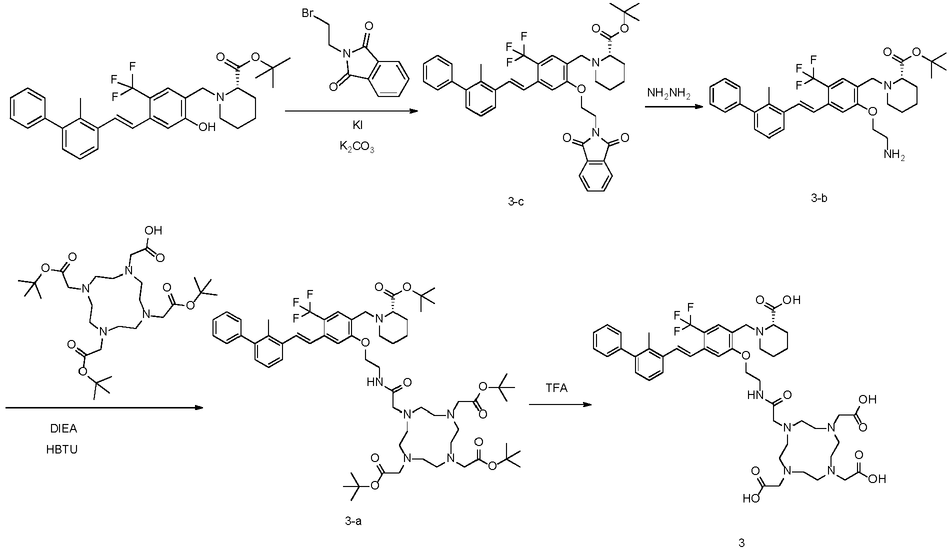

### Synthesis of compound 3-c

Compound **2-c** (22.53 mg, 0.05 mmol), 2-(2-bromoethyl) isoindole-1,3-dione (508.16 mg, 2.000 mmol) and potassium carbonate (276.40 mg, 2.000 mmol) were dissolved in *N*, *N-*dimethylformamide (5 mL). The reaction solution was heated and stirred at 60°C for 16 hours, and the reaction solution was cooled to room temperature, and the reaction solution was directly separated by C18 reversed-phase column chromatography to obtain a mixture of product and raw material **3-c** (450 mg), which was directly put into the next step of reaction.

### Synthesis of compound 3-b

Compound **3-c** (450 mg, 0.174 mmol) and hydrazine hydrate (10.89 mg, 0.174 mmol) were dissolved in methanol (5 mL). The reaction solution was heated and stirred at 65 °C for 2 hours. The reaction solution was cooled to room temperature, and the reaction solution was directly separated by prep-HPLC to obtain product **3-b** (35 mg, yield: 33.82%).

### Synthesis of compound 3-a

Compound **3-b** (35 mg, 0.059 mmol), (10-{ 1-[(methylpropan-2-yl)oxy]-1-oxoethyl-2-yl}-4,7-bis{2-[(2-methylpropan-2-yl)oxy]-2-oxoethyl}-1,4,7,10-tetraazacyclododecan-1-yl)acetic acid (40.45 mg, 0.071 mmol) and HBTU reagent (O-(IH-benzotriazol-1-yl)-*N,N,N',N'*-tetramethylisourea phosphorus hexafluoride, 33.48 mg, 0.088 mmol) was dissolved in *N'N*-dimethylformamide (1 mL), and *N, N*-diisopropylethylamine ( 0.019 mL, 0.118 mmol) was added. The reaction solution was stirred at room temperature for 1 hour. The reaction solution was directly separated by reversed-phase preparative chromatography to obtain **3-a** (76 mg, 0.051 mmol, 86.36%).

### Synthesis of compound 3

Compound **3-a** (76 mg, 0.051 mmol) was dissolved in dichloromethane (1.0 mL), and trifluoroacetic acid (1.0 mL) was added. The reaction solution was stirred at room temperature for 16 hours. The reaction solution was concentrated under reduced pressure and the residue was separated by reversed-phase preparative chromatography (column xBridge C18, 19 * 150 mm * 5 um; mobile phase: water (0.1% trifluoroacetic acid), methanol; gradient: 65%-80% (initial mobile phase was 35% water/65% methanol, and ending mobile phase was 20% water/80% methanol, wherein % refers to volume percentage); 13 min; flow rate 15 mL/min)to obtain 3 (37 mg, yield: 57.26%). LC-MS (ESI): m/z = 925.72(M+H)⁺,

¹H-NMR (400 MHz, D₂O) δ: 7.45 (s, 1H), 7.27-6.18 (m, 11H), 4.3-2.25 (m, 32H), 2.22-0.60 (m, 10H).

### Embodiment 4

### (2S)-1-({4-[(1E)-2-(2-Methyl-3-phenylphenyl)vinyl]-5-(trifluoromethyl)-2-{[4-({2-[4,7,10-tris(carboxymethyl)-1,4,7,10-tetraazacyclododecan-1-yl]acetyl}amino)butyl]oxy}phenyl} methyl)hexahydropyridine-2-carboxylic acid (compound 4)

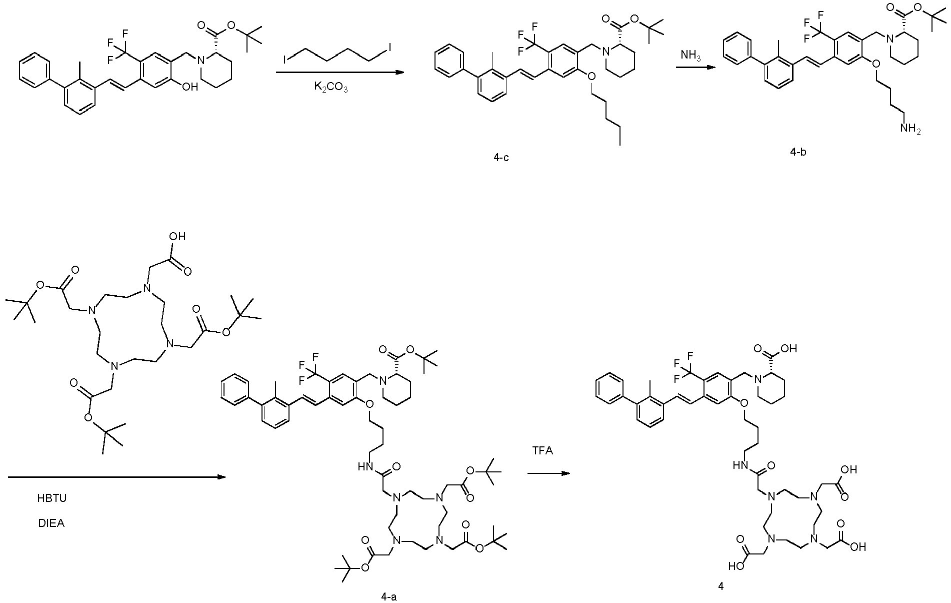

### Synthesis of compound 4-c

Compound **2-c** (275.82 mg, 0.5 mmol) and 1,4-diiodobutane (619.84 mg, 2.000 mmol) were dissolved in *N, N*-dimethylformamide (2 mL), and potassium carbonate (138.21 mg, 1.00 mmol) was added, and the reaction solution was stirred at room temperature for 1 hour. The reaction solution was diluted with ethyl acetate (50 mL), and washed sequentially with water (20 mL×2) and saturated brine (20 mL×1). The organic phase was separated, dried over anhydrous sodium sulfate, filtered, and concentrated under reduced pressure, and the residue was separated by column chromatography (petroleum ether: ethyl acetate=20: 1) to obtain product **4-c** (310 mg, yield: 84.51%).

### Synthesis of compound 4-b

Compound **4-c** (410 mg, 0.559 mmol) was dissolved in ammonia-methanol solution (7 M, 11.975 mL), and the mixture was sealed in a microwave tube, and the reaction solution was stirred at 50°C for 16 hours. The reaction solution was cooled to room temperature, concentrated under reduced pressure, and the residue was dissolved in dichloromethane (20 mL) and methanol (2 mL), and washed with 10% potassium carbonate solution (5 mL). The organic phase was separated, dried over anhydrous sodium sulfate, and filtered to obtain yellow oil **4-b** (280 mg, yield: 80.45%), which was used directly in the next step without purification.

### Synthesis of compound 4-a

Compound **4-b** (180 mg, 0.289 mmol), 2-(4,7,10-tris(2-(tert-butoxy)-2-oxoethyl)-1,4,7,10-tetraazacyclododecan-1-yl) acetate (198.65 mg, 0.347 mmol), HBTU reagent (O-(IH-benzotriazol-1-yl)-*N,N,N',N*'-tetramethylisourea phosphorus hexafluoride, 164.42 mg, 0.434 mmol) was dissolved in *N, N-*dimethylformamide (2 mL). *N, N-*Diisopropylethylamine (0.096 mL, 0.578 mmol) was added. The reaction solution was stirred at room temperature for 0.5 hour, and directly separated by reversed-phase preparative chromatography (column xBridge C18, 19 * 150 mm * 5 um; mobile phase: water (0.1% trifluoroacetic acid), methanol; gradient: 65%-80% (initial mobile phase was 35% water/65% methanol, and ending mobile phase was 20% water /80% methanol, wherein % refers to volume percentage);13 min; flow rate 15 mL/min) to obtain 4-a (268 mg, 0.176 mmol, yield: 61.03%). LC-MS (ESI): m/z = 1178.18(M+H)⁺.

### Synthesis of compound 4

Compound **4-a** (268 mg, 0.176 mmol) was dissolved in dichloromethane (2 mL), and trifluoroacetic acid (2 mL) was added. The reaction solution was stirred at room temperature for 16 hours. The reaction solution was concentrated under reduced pressure, and the residue was separated by reversed-phase preparative chromatography (column xBridge C18, 19 * 150 mm * 5 um; mobile phase: water (0.1% trifluoroacetic acid), methanol; gradient: 65%-80% (initial mobile phase was 35% water/65% methanol, and ending mobile phase was 20% water/80% methanol, wherein % refers to volume percentage); 13 min; flow rate 15 mL/min)to obtain **4** (156 mg, yield: 68.44%). LC-MS (ESI): m/z = 953.64(M+H)⁺.

¹H-NMR (400 MHz, D₂O) δ: 7.48 (s, 1H), 7.19-6.19 (m, 11H), 4.41-2.32 (m, 33H), 2.19-0.83 (m, 13H).

### Embodiment 5

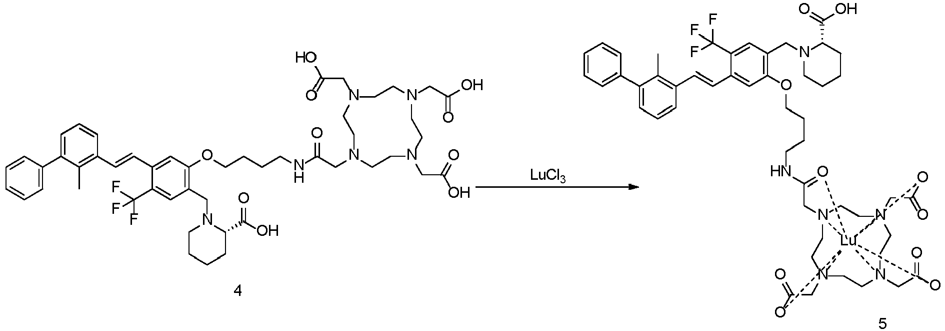

Compound **4** (56 mg, 0.043 mmol) and lutetium trichloride (60.82 mg, 0.216 mmol) were dissolved in sodium acetate-acetic acid buffer (pH=4.5) (2.0 mL), and the reaction solution was stirred at 90°C for 0.5 hour. The reaction solution was cooled to room temperature and purified by pre-HPLC to obtain **5** (33 mg, yield: 52.03%). LC-MS (ESI): m/z = 1125.95(M+H)⁺.

### Embodiment 6

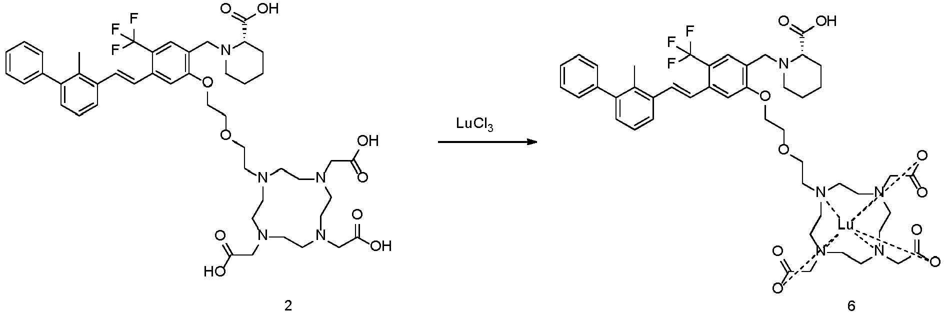

Compound **2** (182.40 mg, 0.2 mmol) and lutetium trichloride (56.26 mg, 0.200 mmol) were dissolved in sodium acetate-acetic acid buffer (pH=4.5) (2.0 mL), and the reaction solution was stirred at room temperature for 1 hour. The reaction solution was purified by pre-HPLC to obtain **6** (80 mg, 0.074 mmol, 36.90%).

LC-MS (ESI): m/z = 1084.68(M+H)⁺;

¹H-NMR (400 MHz, MeOD) δ: 7.85 (s, 1H), 7.62 (d, *J* = 16.0 Hz, 1H), 7.58-7.46 (m, 2H), 7.46-7.38 (m, 2H), 7.38-7.21 (m, 5H), 7.17 (d, *J* = 7.4 Hz, 1H), 4.66-1.40 (m, 44H).

### Embodiment 7

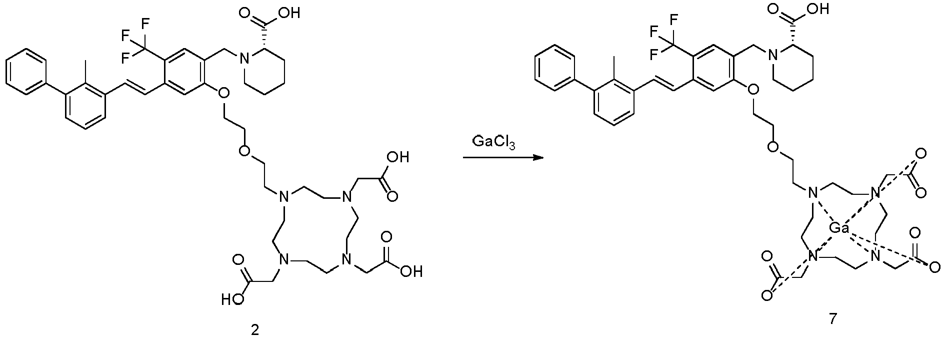

Compound **2** and gallium trichloride (3.52 mg, 0.020 mmol) were dissolved in water (0.2 mL), and the reaction solution was stirred at 90°C for 5 minutes. The reaction solution was purified directly by prep-HPLC (column XT C18, 19 * 150 mm * 5 um; mobile phase: water (0.1% FA), acetonitrile; gradient: 25%-95% (initial mobile phase was 75% water : 25% acetonitrile, and ending mobile phase was 5% water/95% acetonitrile, wherein % refers to volume percentage); 10 min; flow rate 15 mL/min) to obtain white solid **7** (9.7 mg, yield: 99.08%).

LC-MS (ESI): m/z = 978.61 (M+H)⁺.

¹H-NMR (400 MHz, MeOD) δ: 7.88 (s, 1H), 7.63 (d, *J* = 15.9 Hz, 1H), 7.58-7.51 (m, 2H), 7.47-7.39 (m, 2H), 7.38-7.24 (m, 5H), 7.18 (d, J = 7.5 Hz, 1H), 4.62 (d, *J* = 13.0 Hz, 1H), 4.50-4.42 (m, 2H), 4.40 (d, *J* = 13.1 Hz, 1H), 4.06-3.86 (m, 6H), 3.85-3.45 (m, 10H), 3.44-3.32 (m, 9H), 3.26-3.10 (m, 5H), 3.09-2.97 (m, 1H), 2.30 (s, 3H), 2.28-2.17 (m, 1H), 2.05-1.68 (m, 4H), 1.66-1.52 (m, 1H).

### Embodiment 8

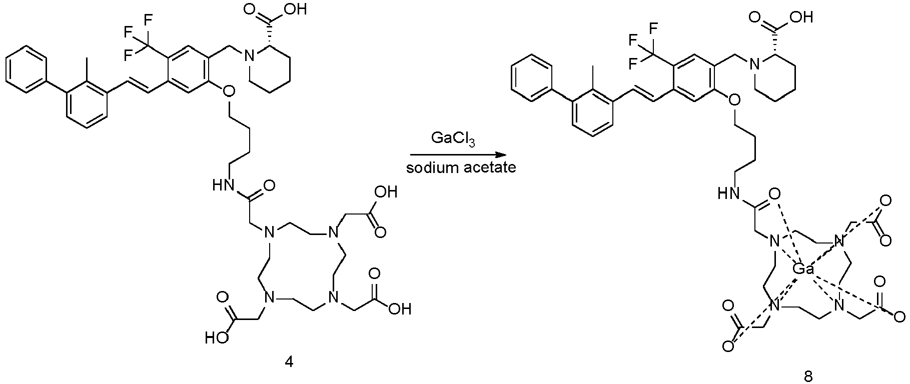

Compound **4** (12.95 mg, 0.010 mmol), gallium trichloride (3.52 mg, 0.020 mmol) and sodium acetate (0.150 mL, 0.030 mmol) were dissolved in water (0.5 mL), and the reaction solution was stirred at 90°C for 10 minutes. The reaction solution was cooled to room temperature, and the reaction solution was concentrated under reduced pressure, and the residue was separated by silica gel column chromatography to obtain **8** (8.76 mg, yield: 85.90%). LC-MS (ESI): m/z =1019.65 (M+H)⁺;

¹H-NMR (400 MHz, MeOD) δ: 7.91 (s, 1H), 7.62 (d, *J* = 15.9 Hz, 1H), 7.57-7.49 (m, 2H), 7.46-7.38 (m, 2H), 7.38-7.23 (m, 5H), 7.18 (d, *J* = 6.8 Hz, 1H), 4.52 (dd, *J* = 34.7, 12.7 Hz, 2H), 4.37-4.22 (m, 2H), 3.89-3.46 (m, 13H), 3.45-2.96 (m, 16H), 2.30 (s, 3H), 2.28-2.19 (m, 1H), 2.05-1.90 (m, 3H), 1.89-1.67 (m, 5H), 1.66-1.50 (m, 1H).

### Embodiment 9

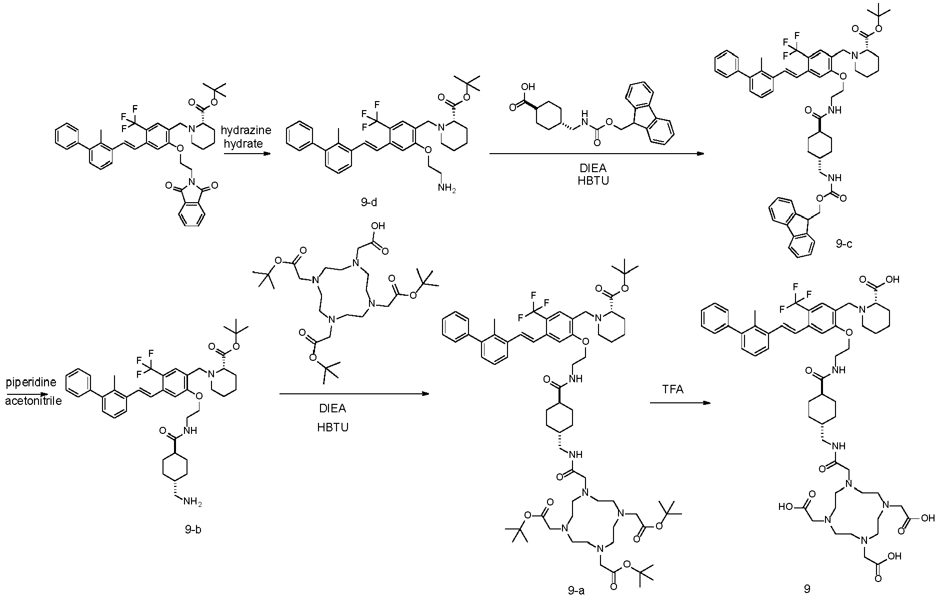

### Synthesis of compound 9-d

2-Methylpropan-2-yl (2*S*)-1-[(2-{[2-(1,3-dioxo-2,3-dihydro-1*H*-isoindole-2-yl)ethyl]oxy}-4-[(1*E*)-2-(2-methyl-3-phenylphenyl)vinyl]-5-(trifluoromethyl)phenyl)methyl]hexahydropyridine-2-carboxylate (610 mg, 0.842 mmol) and hydrazine hydrate (0.511 mL, 8.416 mmol) were dissolved in methanol (6 mL). The reaction solution was stirred at 60°C for 2 hours. The reaction solution was cooled to room temperature, and concentrated under reduced pressure, and the residue was washed with dichloromethane (6 mL), filtered, and the filtrate was concentrated under reduced pressure to obtain **9-d** (498 mg, yield: 99.50%). LC-MS (ESI): m/z = 595.53(M+H)⁺.

### Synthesis of compound 9-c

*N, N-*Diisopropylethylamine (0.020 mL, 0.120 mmol), compound **9-d** (59.47 mg, 0.1 mmol), (1r,4r)-4-(((((9*H*-fluoren-9-yl)methoxy)carbonyl)amino)methyl)cyclohexane-1-carboxylic acid (37.95 mg, 0.100 mmol), and benzotriazole-*N,N,N',N'*-tetramethyluronium hexafluorophosphate (45.51 mg, 0.120 mmol) were dissolved in *N*, *N-*dimethylformamide (0.5 mL), and the reaction solution was stirred at room temperature for 30 minutes. The reaction solution was diluted with ethyl acetate (20 mL), and washed sequentially with water (20 mL×2) and saturated brine (10 mL×1). The organic phase was dried over anhydrous sodium sulfate, filtered, and concentrated under reduced pressure, and the residue was purified by silica gel column chromatography (petroleum ether: ethyl acetate: ethanol = 20:10:1) to obtain **9-c** (72 mg, yield: 75.30%).

### Synthesis of compound 9-b

Piperidine (0.021 mL, 0.226 mmol) and compound **9-c** (72 mg, 0.075 mmol) were dissolved in acetonitrile (1 mL). The reaction solution was stirred at room temperature for 5 hours. The reaction solution was concentrated under reduced pressure, and the residue was separated by silica gel column chromatography (dichloromethane: methanol: ammonia methanol = 400:100:1) to obtain **9-b** (49 mg, yield: 88.66%).

### Synthesis of compound 9-a

*N, N-*Diisopropylethylamine (0.017 mL, 0.100 mmol), compound **9-b** (49 mg, 0.067 mmol), (10-{1-[(2-methylpropan-2-yl)oxy]-1-oxoethyl-2-yl}-4,7-bis{2-[(2-methylpropan-2-yl)oxy]-2-oxoethyll-1,4,7,10-tetraazacyclododecan-1-yl)acetic acid (42.06 mg, 0.073 mmol), and benzotriazole-*N,N,N',N'*-tetramethyluronium hexafluorophosphate (32.92 mg, 0.087 mmol) were dissolved in *N, N-*dimethylformamide (0.5 mL), and the reaction solution was stirred at room temperature for 1 hour, and the residue was separated by Prep-HPLC to obtain **9-a** (72 mg, yield: 83.39%).

### Synthesis of compound 9

Trifluoroacetic acid (2.0 mL) and compound **9-a** (72 mg, 0.056 mmol) were dissolved in dichloromethane (2.0 mL), and the reaction solution was stirred at room temperature for 16 hours. The reaction solution was concentrated under reduced pressure, and the residue was separated by Prep-HPLC to obtain **9** (59.46 mg, yield: 67.27%). LC-MS (ESI): m/z =1065.21 (M+H)⁺.

### Embodiment 10

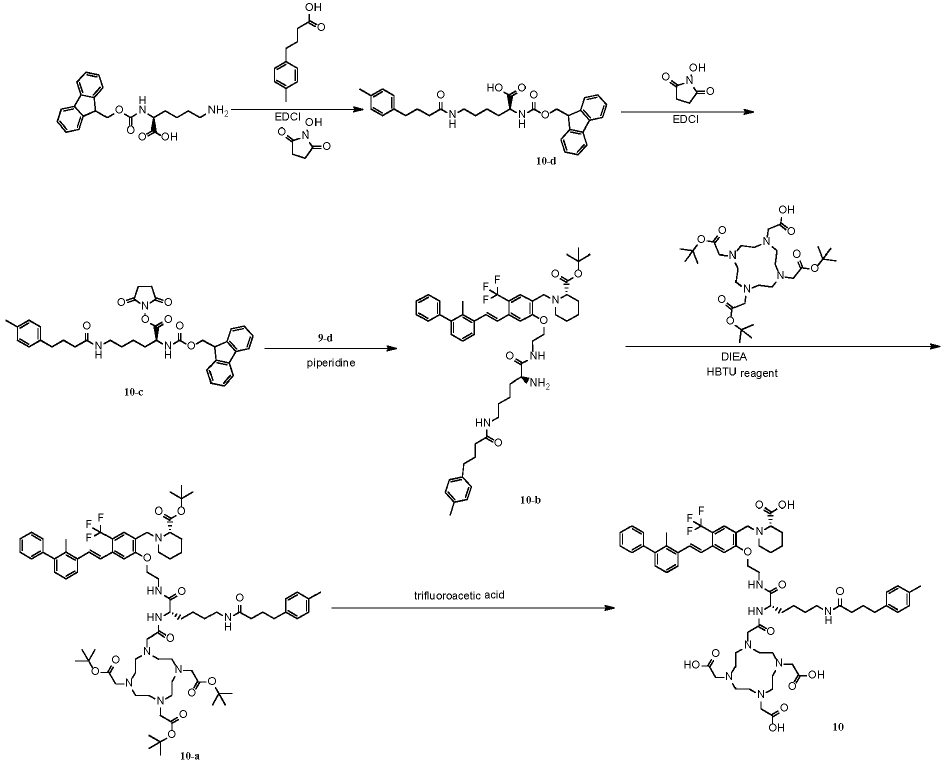

### Synthesis of compound 10-d

1-(3-Dimethylaminopropyl)-3-ethylcarbodiimide hydrochloride (191.70 mg, 1.000 mmol), 4-(4-methylphenyl) butyric acid (178.23 mg, 1.00 mmol) and 1-hydroxytetrahydro-1*H*-pyrrole-2,5-dione (115.09 mg, 1.000 mmol) were dissolved in dimethyl sulfoxide (2 mL). The reaction was stirred at room temperature for 48 hours. (2*S*)-6-Amino-2-({[(9*H*-fluoren-9-ylmethyl)oxy]carbonyl}amino)hexanoic acid (368.43 mg, 1.000 mmol) was added, and the reaction solution was continuously stirred at room temperature for 1 hour. The reaction solution was directly separated by Prep-HPLC to obtain **10-d** (400 mg, yield: 75.66%).

### Synthesis of compound 10-c

1-(3-Dimethylaminopropyl)-3-ethylcarbodiimide hydrochloride (188.56 mg, 0.984 mmol), compound **10-d** and 1-hydroxytetrahydro-1*H*-pyrrole-2,5-dione (95.79 mg, 0.832 mmol) were dissolved in dimethyl sulfoxide (2 mL). The reaction solution was stirred at room temperature for 2 hours. The reaction solution was directly separated by Prep-HPLC to obtain **10-c** (400 mg, yield: 84.49%).

### Synthesis of compound 10-b

Compound **9-d** (59.47 mg, 0.1 mmol) and compound **10-c** (75.09 mg, 0.120 mmol) were dissolved in acetonitrile (1 mL). The reaction solution was stirred at room temperature for 1 hour. Piperidine (0.037 mL, 0.400 mmol) was added, and the reaction solution was continuously stirred at room temperature for 2 hours. The reaction solution was concentrated under reduced pressure, and the residue was separated by silica gel column chromatography (petroleum ether: ethyl acetate = 5:1, 6 CV, then dichloromethane: methanol = 10:1, 8 CV, CV is the column volume) to obtain **10-b** (78 mg, yield: 88.32%).

### Synthesis of compound 10-a

*N, N-*Diisopropylethylamine (0.029 mL, 0.177 mmol), compound **10-b** (78 mg, 0.088 mmol), (10-{1-[(2-methylpropan-2-yl)oxy]-1-oxoethyl-2-yl}-4,7-bis{2-[(2-methylpropan-2-yl)oxy]-2-oxoethyll-1,4,7,10-tetraazacyclododecan-1-yl)acetic acid (55.65 mg, 0.097 mmol), and benzotriazole-*N,N,N',N'*-tetramethyluronium hexafluorophosphate (43.54 mg, 0.115 mmol) were dissolved in *N, N-*dimethylformamide (1.0 mL), and the reaction solution was stirred at room temperature for 1 hour, and directly separated by Prep-HPLC to obtain **10-a** (108 mg, yield: 85.04%).

### Synthesis of compound 10

Trifluoroacetic acid (2.0 mL) and compound **10-a** (72 mg, 0.056 mmol) were dissolved in dichloromethane (2.0 mL), and the reaction solution was stirred at room temperature for 16 hours. The reaction solution was concentrated under reduced pressure, and the residue was separated by Prep-HPLC to obtain **10** (50 mg, yield: 54.86%). LC-MS (ESI): m/z =1214.47 (M+H)⁺.

### Embodiment 11

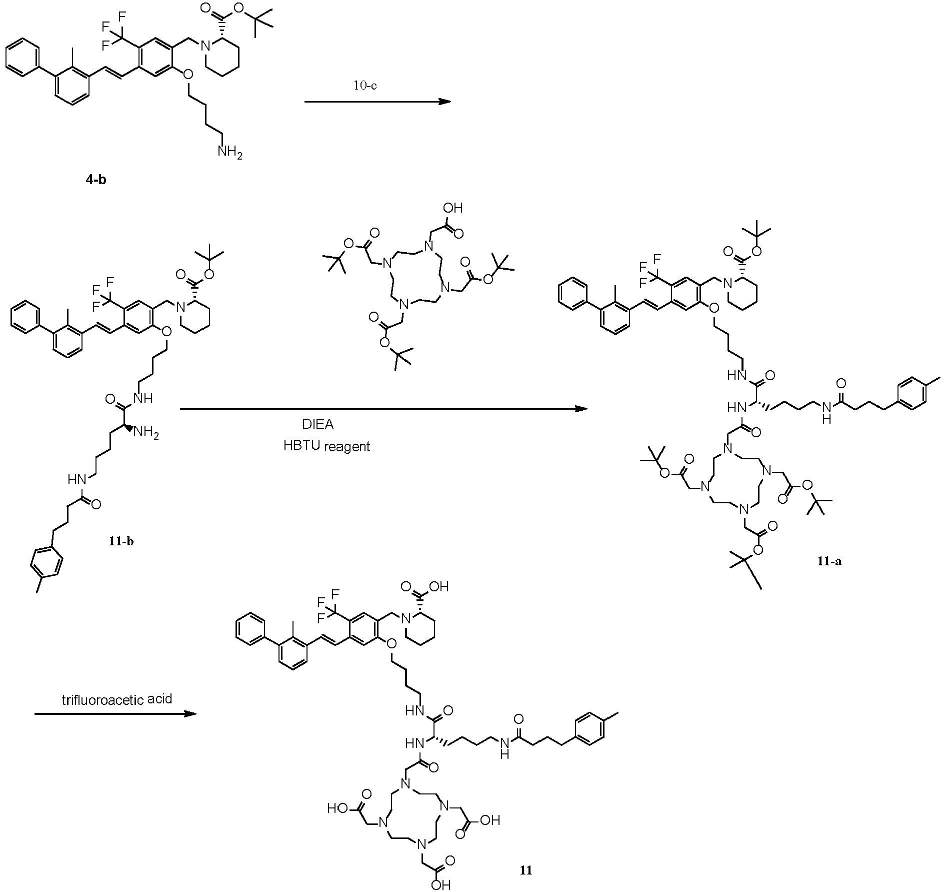

### Synthesis of compound 11-b

Compound **4-b** (124.55 mg, 0.20 mmol) and compound **10-c** (150.17 mg, 0.240 mmol) were dissolved in acetonitrile (5.0 mL). The reaction solution was stirred at room temperature for 1 hour, and dichloromethane (2.0 mL) was added and the reaction solution was stirred for 1 hour. Piperidine (0.073 mL, 0.800 mmol) was added, and the reaction solution was continuously stirred at room temperature for 2 hours. The reaction solution was concentrated under reduced pressure, and the residue was separated by silica gel column chromatography (petroleum ether: ethyl acetate = 5:1) to obtain **11-b** (177 mg, yield: 97.13%).

### Synthesis of compound 11-a

*N, N-*Diisopropylethylamine (0.064 mL, 0.389 mmol), 11-b (78 mg, 0.088 mmol), (10-{1-[(2-methylpropan-2-yl)oxy]-1-oxoethyl-2-yl}-4,7-bis{2-[(2-methylpropan-2-yl)oxy]-2-oxoethyl}-1,4,7,10-tetraazacyclododecan-1-yl)acetic acid (122.39 mg, 0.214 mmol) and benzotriazole-*N,N,N',N'*-tetramethyluronium hexafluorophosphate (95.77 mg, 0.253 mmol) were dissolved in *N, N-*dimethylformamide (2.0 mL), and the reaction solution was stirred at room temperature for 1 hour. The reaction solution was concentrated under reduced pressure, and the residue was separated by silica gel column chromatography (ethyl acetate, 6 CV, then dichloromethane : methanol=10:1, 8 CV, CV is column volume) to obtain **11-a** (284 mg, yield: 99.73%).

### Synthesis of compound 11

Compound **11-a** (284 mg, 0.194 mmol) was dissolved in dichloromethane (3 mL), and the reaction solution was cooled to 0°C, and trifluoroacetic acid (3 mL) was added. The reaction solution was stirred at room temperature for 16 hours. The reaction solution was concentrated under reduced pressure, and the residue was purified by reversed-phase preparative chromatography to obtain **11** (150 mg, 0.121 mmol, 62.37%).

¹H-NMR (400 MHz, MeOD) δ: 8.00 (s, 0H), 7.80 (s, 1H), 7.52 (d, *J* = 15.8 Hz, 1H), 7.47-7.40 (m, 2H), 7.36-7.29 (m, 2H), 7.28-7.15 (m, 5H), 7.08 (d, *J* = 7.0 Hz, 1H), 6.94 (s, 4H), 4.48 (d, *J* = 12.7 Hz, 1H), 4.33 (d, *J* = 12.9 Hz, 1H), 4.23-4.13 (m, 3H), 3.75-3.59 (m, 5H), 3.56-3.26 (m, 11H), 3.10-2.87 (m, 11H), 2.50-2.41 (m, 2H), 2.20 (s, 3H), 2.16 (s, 3H), 2.11-2.05 (m, 2H), 1.96-1.16 (m, 22H).

### Embodiment 12

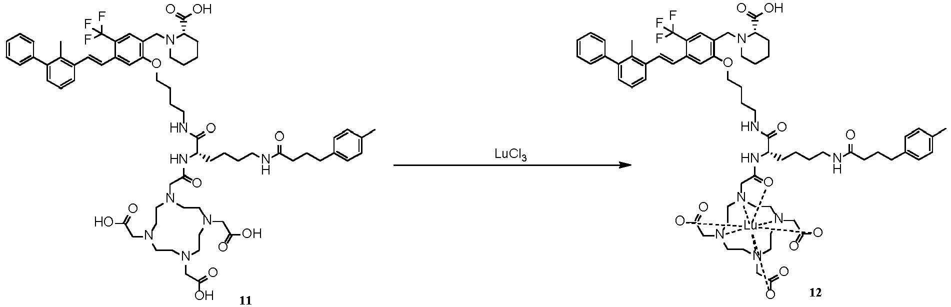

### Synthesis of compound 12

Compound **11** (30 mg, 0.024 mmol) and lutetium trichloride (13.60 mg, 0.048 mmol) were dissolved in methanol (1.0 mL) and sodium acetate-acetic acid buffer (pH=4.5) (0.5 mL). The reaction solution was stirred at room temperature for 20 minutes, then stirred at 90°C for 5 minutes, and the reaction solution was cooled to room temperature. The reaction solution was directly separated by reversed-phase Pre-HPLC to obtain **12** (14 mg, yield: 48.08%). LC-MS (ESI): m/z =1414.3 (M+H)⁺.

### Embodiment 13

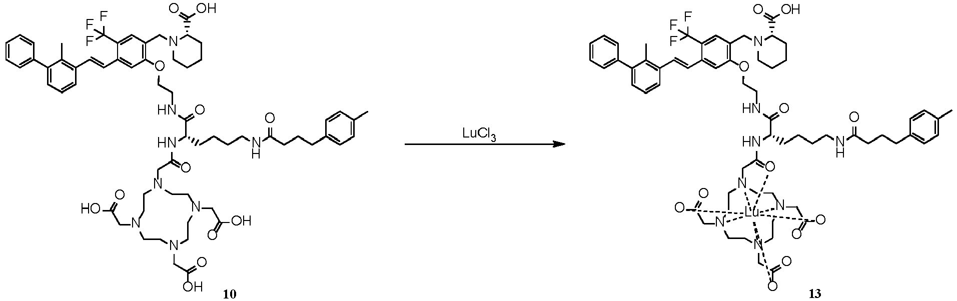

### Synthesis of compound 13

Compound **10** (12.13 mg, 0.01 mmol) and lutetium trichloride (5.63 mg, 0.020 mmol) were dissolved in sodium acetate-acetic acid buffer (pH=4.5) (1.0 mL). The reaction solution was stirred at room temperature for 20 minutes, then stirred at 90°C for 5 minutes, and the reaction solution was cooled to room temperature. The reaction solution was directly separated by reversed-phase Pre-HPLC to obtain **13** (10 mg, yield: 72.20%). LC-MS (ESI): m/z =1386.7 (M+H)⁺.

### Embodiment 14

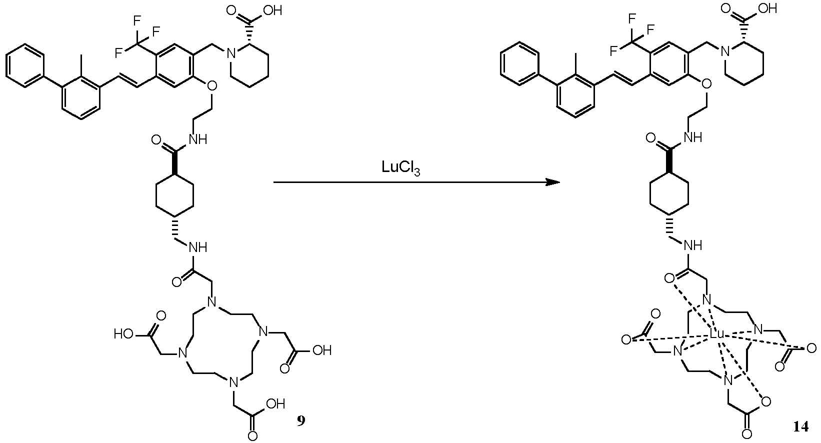

### Synthesis of compound 14

Compound **9** (10.64 mg, 0.01 mmol) and lutetium trichloride (5.63 mg, 0.020 mmol) were dissolved in sodium acetate-acetic acid buffer (pH=4.5) (1.0 mL). The reaction solution was stirred at room temperature for 20 minutes, then the reaction solution was stirred at 90°C for 5 minutes, and the reaction solution was cooled to room temperature. The reaction solution was directly separated by reversed-phase Pre-HPLC to obtain **14** (6 mg, yield: 48.54%). LC-MS (ESI): m/z =1237.3 (M+H)⁺.

### Embodiment 15

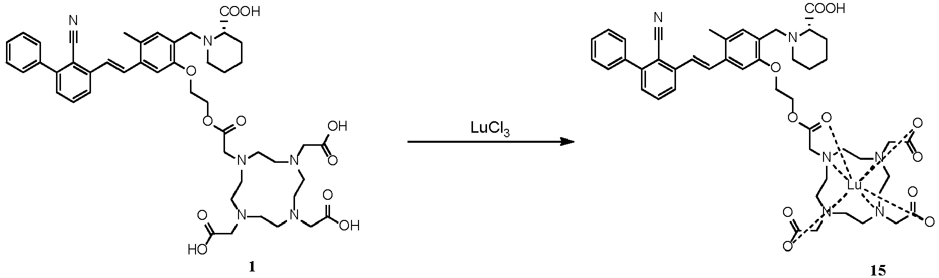

### Synthesis of compound 15

Compound **1** (15 mg, 0.017 mmol) and lutetium trichloride (23.89 mg, 0.085 mmol) were dissolved in sodium acetate-acetic acid buffer (pH=4.5) (1.0 mL), and the reaction solution was stirred at room temperature for 1 hour. The reaction solution was directly separated by Prep-HPLC to obtain **15** (8.6 mg, yield: 36%). LC-MS (ESI): m/z = 1055.79(M+H)⁺.

¹H-NMR (400 MHz, D₂O)δ: 7.20 (s, 1H), 7.15-6.87 (m, 6H), 6.81 (s, 1H), 6.75-6.22 (m, 4H), 4.50-3.82 (m, 3H), 3.78-2.89 (m, 17H), 2.86-2.18 (m, 13H), 2.08-0.96 (m, 9H).

### Embodiment 16

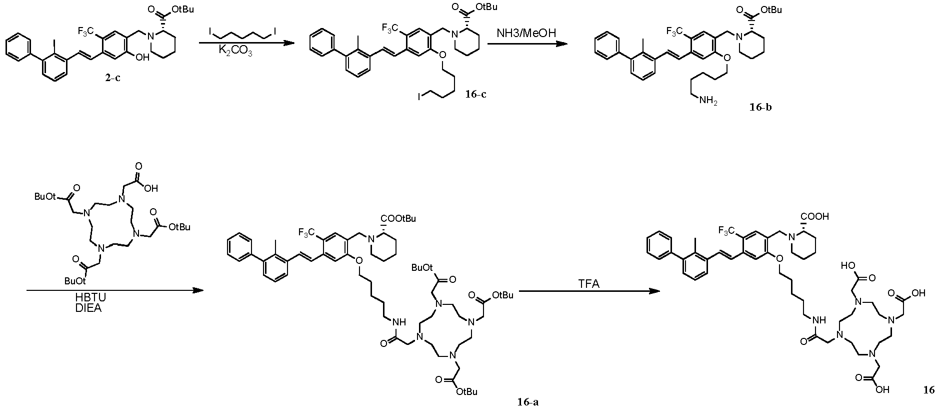

### Synthesis of compound 16-c

Compound **2-c** (500 mg, 0.910 mmol) was dissolved in *N,* N-dimethylformamide (15 mL), and 1,5-diiodopentane (294.66 mg, 0.910 mmol) and potassium carbonate (314.30 mg, 2.274 mmol) were added, and the reaction solution was stirred at room temperature for 2 hours. The reaction solution was diluted with ethyl acetate (50 mL), and washed sequentially with water (20 mL×2) and saturated brine (20 mL×1). The organic phase was separated, dried over anhydrous sodium sulfate, filtered, and concentrated under reduced pressure, and the residue was separated by column chromatography to obtain **16-c** (171 mg, yield: 25.20%).

### Synthesis of compound 16-b

Compound **16-c** (171 mg, 0.229 mmol) was dissolved in tetrahydrofuran (2 mL), and ammonia methanol solution (7 M, 10 mL) was added. The reaction solution was stirred at 50°C for 12 hours. The reaction solution was cooled to room temperature, and the reaction solution was diluted with ethyl acetate (5 mL), and washed sequentially with water (2 mL×2) and saturated brine (2 mL×1). The organic phase was separated, dried over anhydrous sodium sulfate, filtered, and concentrated under reduced pressure to obtain **16-b** (145 mg, yield: 99.61%).

### Synthesis of compound 16-a

Compound **16-b** (145 mg, 0.228 mmol) was dissolved in *N*, N-dimethylformamide (5 mL), and [4,7,10-tris(4,4-dimethyl-2-oxopentyl)-1,4,7,10-tetraazacyclododecan-1-yl] acetic acid (155.36 mg, 0.274 mmol), benzotriazole-*N,N,N',N'*-tetramethyluronium hexafluorophosphate (129.70 mg, 0.342 mmol), and *N, N*-diisopropylethylamine (0.075 mL, 0.456 mmol) were added. The reaction solution was stirred at room temperature for 1 hour, then the reaction solution was diluted with ethyl acetate (5 mL), and washed sequentially with water (2 mL×2) and saturated brine (2 mL×1). The organic phase was separated, dried over anhydrous sodium sulfate, filtered, and concentrated under reduced pressure to obtain **16-a** (243 mg, yield: 98.10%).

### Synthesis of compound 16

Compound **16-a** (243 mg, 0.224 mmol) was dissolved in dichloromethane (10 mL), and trifluoroacetic acid (10 mL) was added. The reaction solution was stirred at room temperature for 18 hours, and the reaction solution was concentrated under reduced pressure, and the residue was directly separated by prep-HPLC to obtain **16** (104.77 mg, yield: 48.35%). LC-MS (ESI): m/z =968.4 (M+H)⁺.

### Embodiment 17

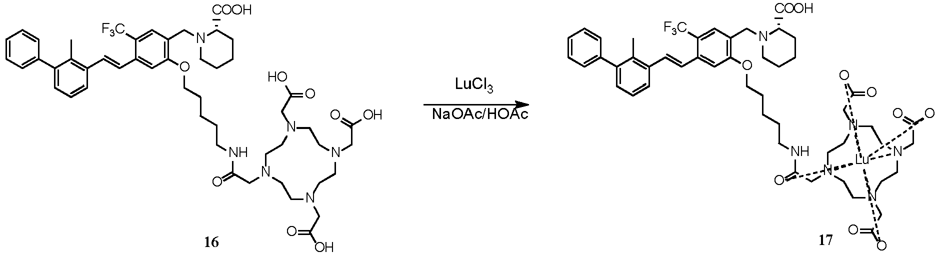

### Synthesis of compound 17

Compound **16** (49 mg, 0.051 mmol) was dissolved in sodium acetate-acetic acid buffer (pH=4.5) (10 mL), and lutetium trichloride (28.69 mg, 0.102 mmol) was added. The reaction solution was stirred at room temperature for 10 minutes, and directly separated by prep-HPLC to obtain **17** (29.67 mg, yield: 51.41%). LC-MS (ESI): m/z =1140.7 (M+H)⁺.

### Embodiment 18

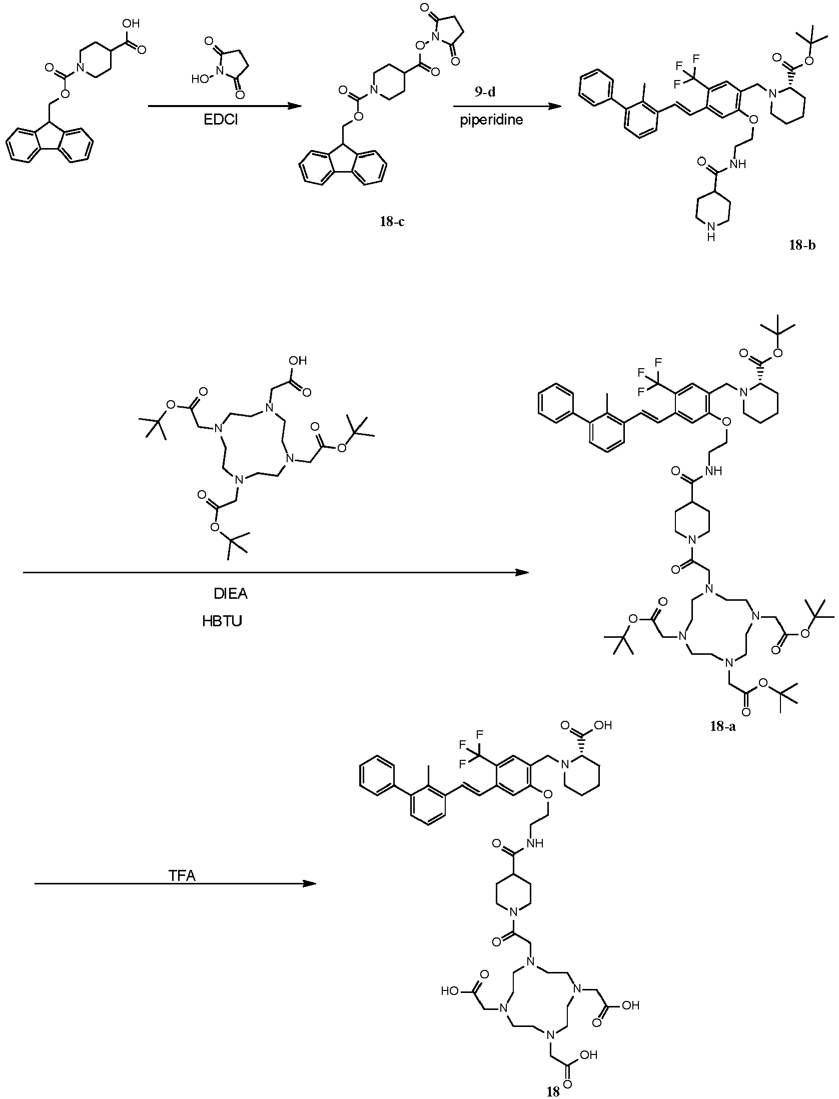

### Synthesis of compound 18-c

1-{[(9*H-*Fluoren-9-ylmethyl)oxy]carbonyl}hexahydropyridine-4-carboxylic acid (702.80 mg, 2.00 mmol), 1-hydroxytetrahydro-1*H*-pyrrole-2,5-dione (230.18 mg, 2.000 mmol), 1-(3-dimethylaminopropyl)-3-ethylcarbodiimide hydrochloride (383.40 mg, 2.000 mmol) were dissolved in acetonitrile (7.0 mL) and dichloromethane (7.0 mL), and the reaction solution was stirred at room temperature for 5 hours, and directly separated by reversed-phase chromatography to obtain **18-c** (800 mg, yield: 89.19%).

### Synthesis of compound 18-b

Compound **9-d** (59.47 mg, 0.100 mmol) and compound **18-c** (57.41 mg, 0.128 mmol) were dissolved in acetonitrile (1.0 mL), and the reaction solution was stirred at room temperature for 16 hours. Piperidine (85.15 mg, 1.000 mmol) was added, and the stirring was continued for 1 hour, and the reaction solution was concentrated under reduced pressure, and the residue was purified by silica gel column (petroleum ether : ethyl acetate=5:1, 6 CV, dichloromethane : methanol=7:3, 6 CV) to obtain **18-b** (50 mg, yield: 70.83%).

### Synthesis of compound 18-a

*N, N-*Diisopropylethylamine (0.023 mL, 0.142 mmol), compound **18-b** (50 mg, 0.071 mmol), (10-{1-[(2-methylpropan-2-yl)oxy]-1-oxoethyl-2-yl}-4,7-bis{2-[(2-methylpropan-2-yl)oxy]-2-oxoethyl}-1,4,7,10-tetraazacyclododecan-1-yl)acetic acid (44.63 mg, 0.078 mmol), and O-benzotriazole-tetramethyluronium hexafluorophosphate (34.92 mg, 0.092 mmol) were dissolved in *N, N*-dimethylformamide (1.0 mL), and the reaction solution was stirred at room temperature for 1 hour, then directly purified by silica gel column (petroleum ether:ethyl acetate=5:1, 6 CV, dichloromethane:methanol=10:1, 6 CV, CV is column volume) to obtain **18-a** (80 mg, yield: 89.59%).

### Synthesis of compound 18

Compound **18-a** (80 mg, 0.063 mmol) was dissolved in dichloromethane (2.0 mL), and trifluoroacetic acid (2.0 mL) was added. The reaction solution was stirred at room temperature for 24 hours, and the reaction solution was concentrated under reduced pressure to obtain compound **18** (30 mg, yield: 45.62%). LC-MS (ESI): m/z =1037.3 (M+H)⁺.

### Embodiment 19

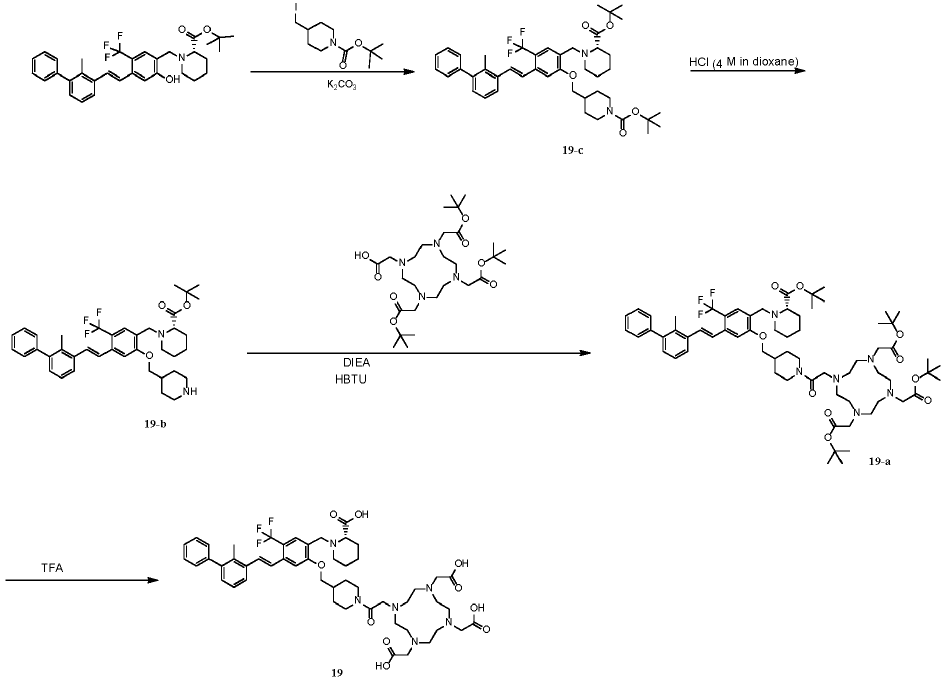

### Synthesis of compound 19-c

Compound **2-c** (500 mg, 0.906 mmol) and 2-methylpropan-2-yl 4-(iodomethyl) hexahydropyridine-1-carboxylate (1473.11 mg, 4.530 mmol) were dissolved in *N, N-*dimethylformamide (3.0 mL), and potassium carbonate (180 mg, 1.302 mmol) was added. The reaction solution was stirred at 80°C for 60 hours, and the reaction solution was cooled to room temperature. The reaction solution was diluted with ethyl acetate (40 mL), and washed sequentially with water (20 mL×2) and saturated brine (20 mL×1). The organic phase was dried over anhydrous sodium sulfate, filtered, and concentrated under reduced pressure. The residue was separated by silica gel column chromatography (petroleum ether: ethyl acetate = 5:1) to obtain **19-c** (620 mg, yield: 91.34%).

### Synthesis of compound 19-b

Compound **19-c** (284 mg, 0.379 mmol) was dissolved in tetrahydrofuran (2 mL) and hydrochloric acid in 1,4-dioxane (4 M in dioxane) (1.5 mL) was added. The reaction solution was stirred at room temperature for 5 hours. The reaction solution was quenched with potassium carbonate aqueous solution (20% aqueous, 5 mL). The mixture was extracted with dichloromethane (20 mL×2), and the organic phase was dried over anhydrous sodium sulfate, filtered, and concentrated under reduced pressure to obtain **19-b** (246 mg, yield: 99.99%), which was directly carried out in the next step of reaction without purification.

### Synthesis of compound 19-a

Compound **19-b** (246 mg, 0.379 mmol), (10-{1-[(2-methylpropan-2-yl)oxy]-1-oxoethyl-2-yl}-4,7-bis{2-[(2-methylpropan-2-yl)oxy]-2-oxoethyl}-1,4,7,10-tetraazacyclododecan-1-yl)acetic acid (238.87 mg, 0.417 mmol), and benzotriazole-*N,N,N',N'-*tetramethyluronium hexafluorophosphate (186.93 mg, 0.493 mmol) were dissolved in *N, N-*dimethylformamide (2.0 mL), and *N, N-*diisopropylethylamine (0.125 mL, 0.758 mmol) was added. The reaction solution was stirred at room temperature for 1 hour. The reaction solution was concentrated under reduced pressure, and the residue was separated by silica gel column chromatography (petroleum ether: ethyl acetate: ethanol = 10:20:1) to obtain **19-a** (360 mg, yield: 78.89%).

### Synthesis of compound 19

Compound **19-a** (360 mg, 0.299 mmol) was dissolved in dichloromethane (3 mL), and trifluoroacetic acid (3 mL) was added. The reaction solution was stirred at room temperature for 16 hours, and the reaction solution was concentrated under reduced pressure, and the residue was directly separated by pre-HPLC to obtain **19** (194 mg, yield: 66.24%). LC-MS (ESI): m/z =980.7 (M+H)⁺.

### Embodiment 20

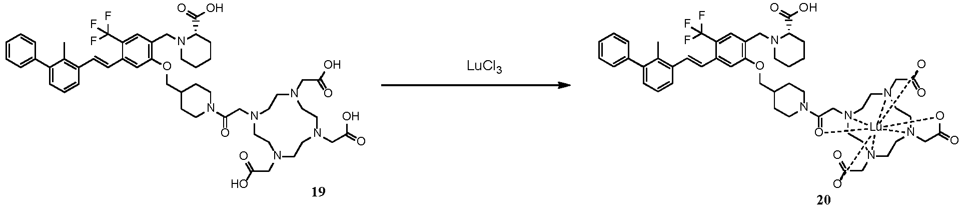

### Synthesis of compound 20

Compound **19** (23.50 mg, 0.024 mmol) and lutetium trichloride (13.50 mg, 0.048 mmol) were dissolved in sodium acetate-acetic acid buffer (pH=4.5) (1 mL), and the reaction solution was stirred at 90°C for 5 minutes. The reaction solution was cooled to room temperature, and directly separated by pre-HPLC to obtain **20** (23 mg, yield: 83.24%). LC-MS (ESI): m/z =1152.1 (M+H)⁺.

### Embodiment 21

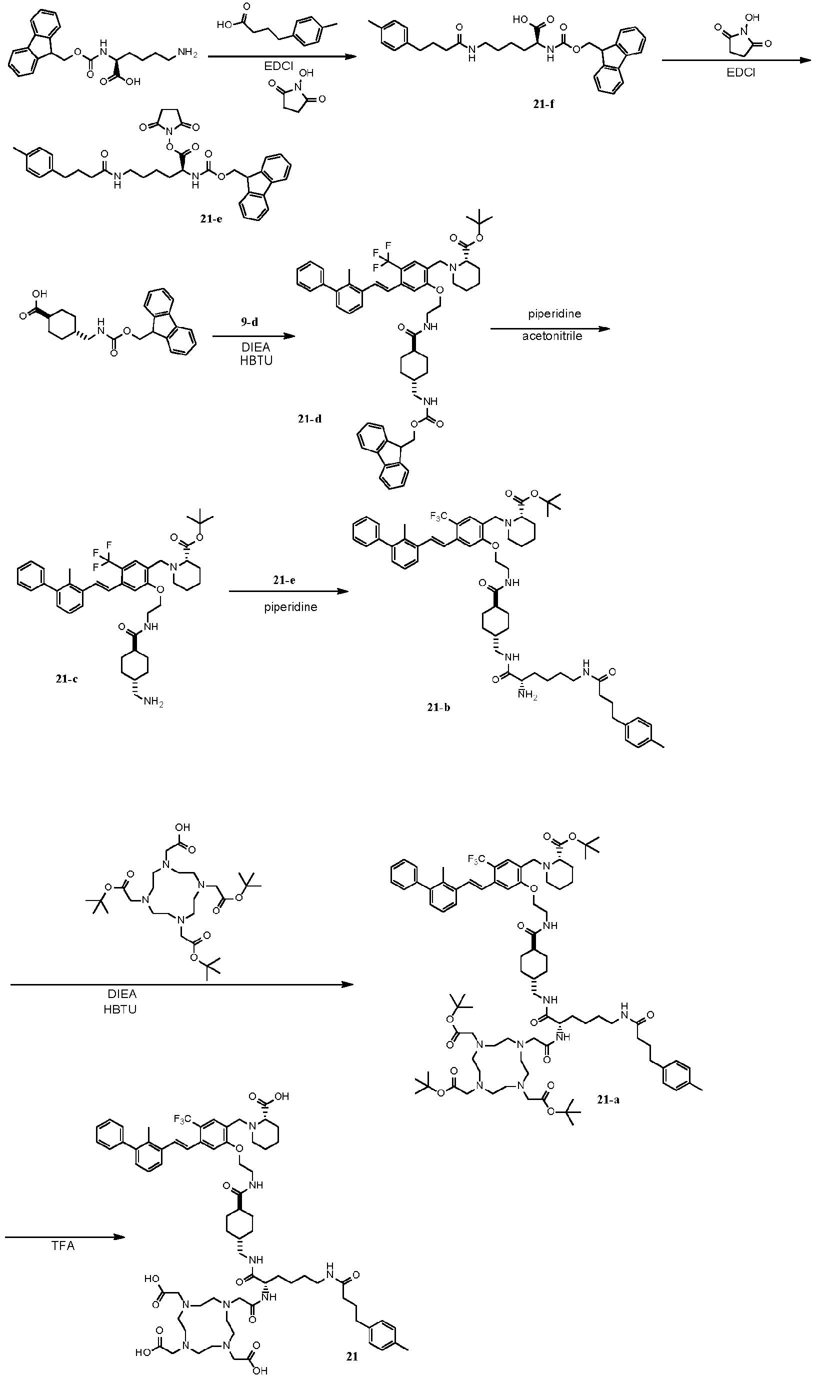

### Synthesis of compound 21-f

1-(3-Dimethylaminopropyl)-3-ethylcarbodiimide hydrochloride (383.40 mg, 2.000 mmol), 4-(4-methylphenyl) butyric acid (356.46 mg, 2.00 mmol), and 1-hydroxytetrahydro-1*H*-pyrrole-2,5-dione (230.18 mg, 2.000 mmol) were added to dimethyl sulfoxide (4 mL). The reaction was stirred at room temperature for 48 hours. (2*S*)-6-Amino-2-({[(9*H*-fluoren-9-ylmethyl)oxy]carbonyl}amino)hexanoic acid (736.86 mg, 2.00 mmol) was added, and the reaction solution was continuously stirred at room temperature for 1 hour. The reaction solution was directly separated by reversed-phase chromatography (120 g of C18, water (0.1% formic acid), 0-95% acetonitrile, 6 CV, CV is column volume) to obtain **21-f** (860 mg, yield: 81.34%).

### Synthesis of compound 21-e

1-(3-Dimethylaminopropyl)-3-ethylcarbodiimide hydrochloride (498.97 mg, 2.603 mmol), compound **21-f** (860 mg, 1.627 mmol) and 1-hydroxytetrahydro-1*H*-pyrrole-2,5-dione (252.76 mg, 2.196 mmol) were added to dimethyl sulfoxide (2 mL). The reaction solution was stirred at room temperature for 2 hours. The reaction solution was directly separated by reversed-phase chromatography (120 g of C18, water (0.1% formic acid), 0-95% acetonitrile, 6 CV, CV is column volume) to obtain **21-e** (900 mg, yield: 88.42%).

### Synthesis of compound 21-d

*N, N-*Diisopropylethylamine (0.040 mL, 0.240 mmol), compound **9-d** (118.94 mg, 0.20 mmol), (1r,4r)-4-(((((9*H*-fluoren-9-yl)methoxy)carbonyl)amino)methyl)cyclohexane-1-carboxylic acid (75.89 mg, 0.200 mmol), and benzotriazole-*N,N,N',N'*-tetramethyluronium hexafluorophosphate (91.02 mg, 0.240 mmol) were dissolved in *N, N*-dimethylformamide (1.0 mL). The reaction was stirred at room temperature for 30 minutes. The reaction solution was diluted with ethyl acetate (20 mL), and washed sequentially with water (20 mL×2) and saturated brine (10 mL×1). The organic phase was dried over anhydrous sodium sulfate, filtered, and concentrated under reduced pressure, and the residue was separated by silica gel column chromatography (petroleum ether: ethyl acetate: ethanol = 20:10:1) to obtain **21-d** (164 mg, yield: 85.76%).

### Synthesis of compound 21-c

Piperidine (0.047 mL, 0.515 mmol) and compound **21-d** (164 mg, 0.172 mmol) were dissolved in acetonitrile (2 mL). The reaction solution was stirred at room temperature for 2 hours. The reaction solution was concentrated under reduced pressure, and the residue was separated by silica gel column (dichloromethane: methanol: ammonia methanol (7M in MeOH) = 400:100:1) to obtain **21-c** (110 mg, yield: 87.38%).

### Synthesis of compound 21-b

Compound **21-c** (110 mg, 0.150 mmol) and compound **21-e** (112.63 mg, 0.180 mmol) were dissolved in acetonitrile (2 mL). The reaction solution was stirred at room temperature for 1 hour, and dichloromethane (2.0 mL) was added to continue stirring for 1 hour. Piperidine (0.069 mL, 0.749 mmol) was added, and the reaction solution was continuously stirred at room temperature for 2 hours. The reaction solution was concentrated under reduced pressure, and the residue was separated by silica gel column chromatography (petroleum ether: ethyl acetate = 5:1, then, dichloromethane: methanol: ammonia methanol (7 M in MeOH) = 100:10:2) to obtain **21-b** (127 mg, yield: 82.82%).

### Synthesis of compound 21-a

Compound **21-b** (127 mg, 0.124 mmol), (4,7,10-tris{2-[(2-methylpropan-2-yl)oxy]-2-oxoethyl}-1,4,7,10-tetraazacyclododecan-1-yl)acetic acid (92.33 mg, 0.161 mmol) and O-benzotriazole-tetramethyluronium hexafluorophosphate (70.54 mg, 0.186 mmol) were dissolved in *N, N-*dimethylformamide (1.0 mL), and *N, N*-diisopropylethylamine (0.041 mL, 0.248 mmol) was added. The reaction solution was stirred at room temperature for 1 hour, and the reaction solution was concentrated under reduced pressure, and the residue was separated by silica gel column (petroleum ether: ethyl acetate=4:1, 6 CV, then 100% PE, 5 CV, then dichloromethane: methanol=10:1, 8 CV, CV is column volume) to obtain **21-a** (195 mg, yield: 99.54%).

### Synthesis of compound 21

Compound **21-b** (195 mg, 0.124 mmol) was dissolved in dichloromethane (2 mL), and trifluoroacetic acid (2 mL) was added. The reaction solution was stirred at room temperature for 16 hours, and the reaction solution was concentrated under reduced pressure, and directly separated by pre-HPLC to obtain **21** (100 mg, yield: 59.79%). LC-MS (ESI): m/z =1353.61 (M+H)⁺.

### Embodiment 22

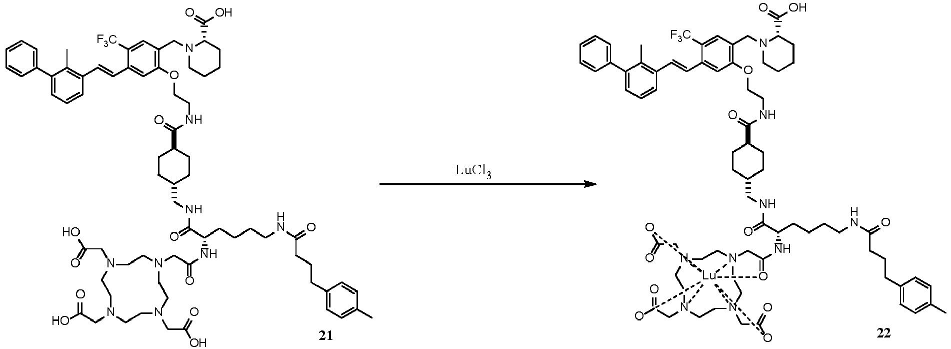

### Synthesis of compound 22

Compound **21** (13.53 mg, 0.01 mmol) was dissolved in sodium acetate-acetic acid buffer (pH=4.5) (1 mL), and lutetium trichloride (5.63 mg, 0.020 mmol) was added, and the reaction solution was heated and stirred at 90°C for 10 minutes. The reaction solution was directly separated by Prep-HPLC to obtain **22** (10 mg, yield: 65.59%). LC-MS (ESI): m/z =1525.55 (M+H)⁺.

### Embodiment 23

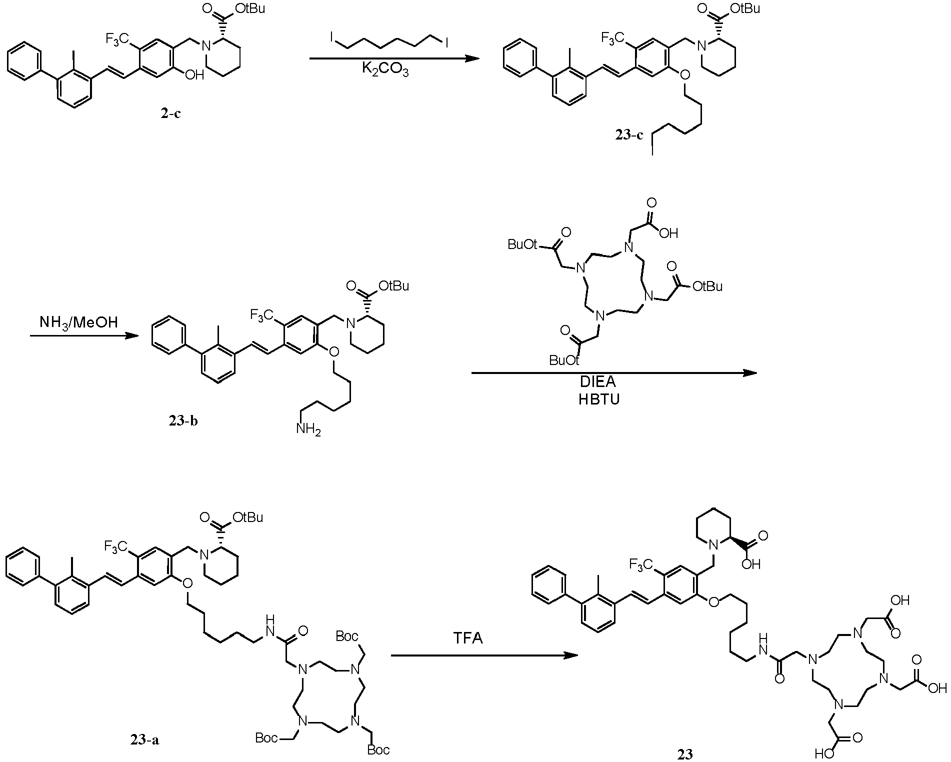

### Synthesis of compound 23-c

Compound **2-c** (500 mg, 0.910 mmol) was dissolved in *N, N*-dimethylformamide (15 mL), and 1,6-diiodohexane (307.55 mg, 0.910 mmol) and potassium carbonate (314.43 mg, 2.275 mmol) were added, and the reaction solution was stirred at room temperature for 2 hours. The reaction solution was diluted with ethyl acetate (50 mL), and washed sequentially with water (20 mL × 2) and saturated saline (20 mL × 1). The organic phase was separated, dried over anhydrous sodium sulfate, filtered, and concentrated under reduced pressure, and the residue was separated by column chromatography to obtain **23-c** (462 mg, yield: 66.82%).

### Synthesis of compound 23-b

Compound **23-c** (462 mg, 0.608 mmol) was dissolved in tetrahydrofuran (2 mL), and ammonia methanol solution (7 M, 15 mL) was added. The reaction solution was stirred at 50°C for 16 hours. The reaction solution was cooled to room temperature, and the reaction solution was diluted with ethyl acetate (5 mL), and washed sequentially with water (2 mL × 2) and saturated saline (2 mL × 1). The organic phase was separated, dried over anhydrous sodium sulfate, filtered, and concentrated under reduced pressure to obtain crude product **23-b** (483 mg, yield: 122.41%), which was directly carried out in the next step of reaction without purification.

### Synthesis of compound 23-a

Compound **23-b** (145 mg, 0.228 mmol) was dissolved in *N, N*-dimethylformamide (5 mL), and [4,7,10-tris(4,4-dimethyl-2-oxopentyl)-1,4,7,10-tetraazacyclododecan-1-yl] acetic acid (104.83 mg, 0.185 mmol), benzotriazole-*N,N,N', N'*-tetramethyluronium hexafluorophosphate (87.67 mg, 0.231 mmol), and *N, N-*diisopropylethylamine (0.051 mL, 0.308 mmol) were added. The reaction solution was stirred at room temperature for 1 hour, and the reaction solution was diluted with ethyl acetate (5 mL) and washed sequentially with water (2 mL×2) and saturated brine (2 mL×1). The organic phase was separated, dried over anhydrous sodium sulfate, filtered, and concentrated under reduced pressure to obtain **23-a** (91 mg, yield: 49.10%).

### Synthesis of compound 23

Compound **23-a** (91 mg, 0.076 mmol) was dissolved in dichloromethane (10 mL), and trifluoroacetic acid (10 mL) was added. The reaction solution was stirred at room temperature for 18 hours, and the reaction solution was concentrated under reduced pressure, and the residue was directly separated by prep-HPLC to obtain **23** (55.42 mg, yield: 74.34%). LC-MS (ESI): m/z =982.12 (M+H)⁺.

### Embodiment 24

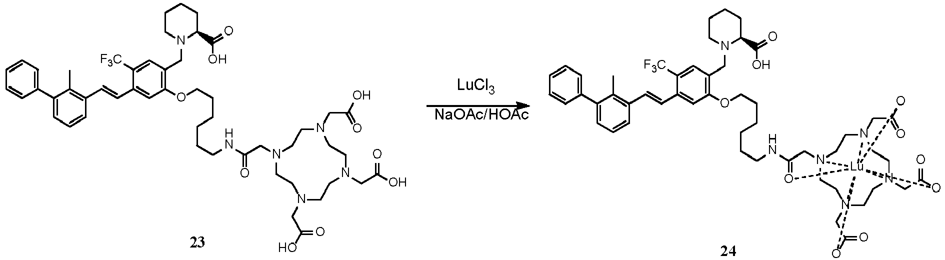

### Synthesis of compound 24

Compound **23** (30 mg, 0.031 mmol) was dissolved in sodium acetate-acetic acid buffer (pH=4.5) (10 mL), and lutetium trichloride (17.20 mg, 0.061 mmol) was added. The reaction solution was stirred at room temperature for 10 minutes, and directly separated by prep-HPLC to obtain **24** (28.12 mg, yield: 78.67%). LC-MS (ESI): m/z =1154.3 (M+H)⁺.

### Embodiment 25

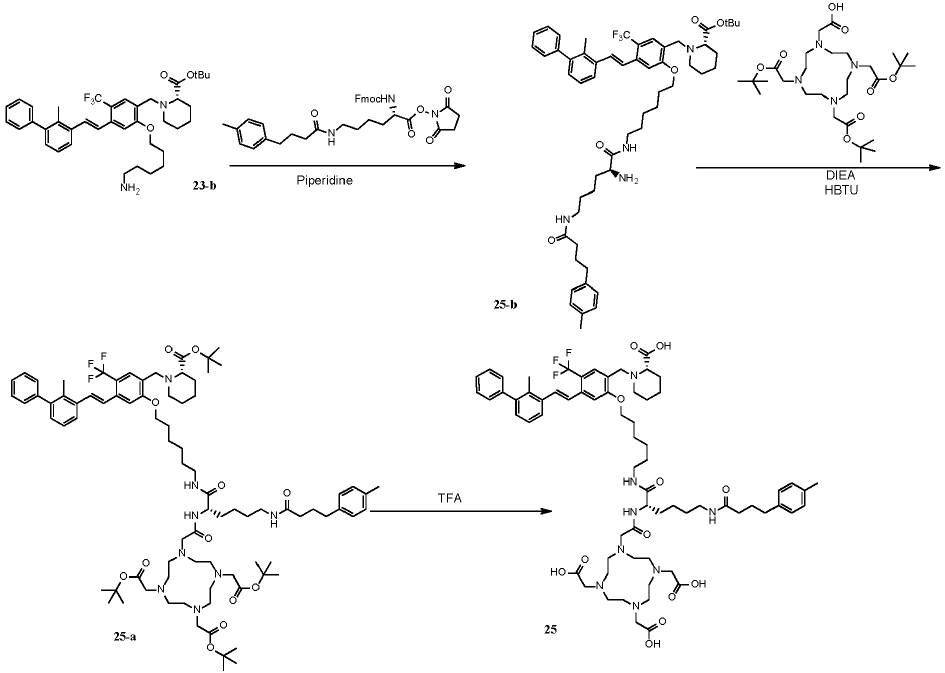

### Synthesis of compound 25-b

Compound **23-b** (200 mg, 0.308 mmol) was dissolved in acetonitrile (5 mL) and dichloromethane (5 mL), and 9*H*-fluoren-9-ylmethyl{[(2*S*)-125-b-[(2,5-dioxytetrahydro-1*H-*pyrrole-1-yl)oxy]-6-{[4-(4-methylphenyl)-1-oxybutyl]amino}-1-oxohex-2-yl]amino}formate (231.44 mg, 0.370 mmol) was added, and the reaction solution was stirred at room temperature for 1 hour. Piperidine (0.102 mL, 0.616 mmol) was added to continue stirring for 1 hour, and the reaction solution was diluted with ethyl acetate (5 mL), and washed sequentially with water (2 mL × 2) and saturated brine (2 mL × 1). The organic phase was separated, dried over anhydrous sodium sulfate, filtered, and concentrated under reduced pressure to obtain **25-b** (184 mg, yield: 63.69%).

### Synthesis of compound 25-a

Compound **25-b** (184 mg, 0.196 mmol) was dissolved in *N, N*-dimethylformamide (5 mL), and (4,7,10-tris{2-[(2-methylpropan-2-yl)oxy]-2-oxoethyl}-1,4,7,10-tetraazacyclododecan-1-yl)acetic acid (112.44 mg, 0.196 mmol), benzotriazole-*N,N,N',N'-*tetramethyluronium hexafluorophosphate (111.68 mg, 0.294 mmol), and *N, N-*diisopropylethylamine (0.065 mL, 0.393 mmol) were added, and the reaction solution was stirred at room temperature for 1 hour. The reaction solution was diluted with ethyl acetate (5 mL), and washed sequentially with water (2 mL×2) and saturated brine (2 mL×1). The organic phase was separated, dried over anhydrous sodium sulfate, filtered, and concentrated under reduced pressure to obtain **25-a** (158.85 mg, yield: 54.16%).

### Synthesis of compound 25

Compound **25-a** (158.85 mg, 0.106 mmol) was dissolved in dichloromethane (10 mL), and trifluoroacetic acid (10 mL) was added. The reaction solution was stirred at room temperature for 18 hours, and the reaction solution was concentrated under reduced pressure, and the residue was directly separated by prep-HPLC to obtain **25** (84.5 mg, yield: 62.60%). LC-MS (ESI): m/z =1270.5 (M+H)⁺.

### Embodiment 26

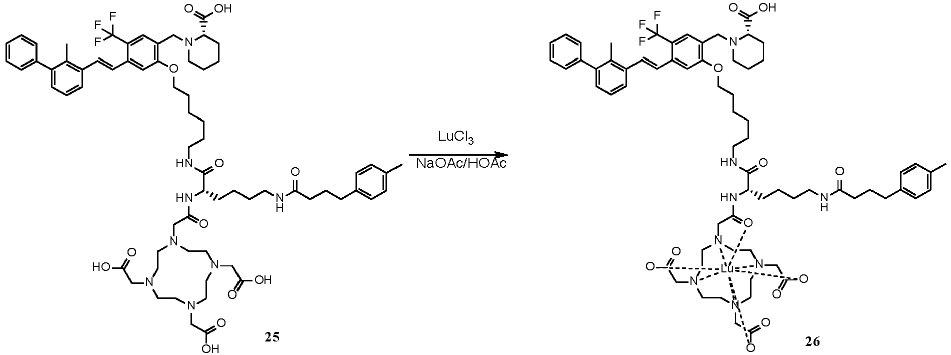

### Synthesis of compound 26

Compound **25** (20 mg, 0.016 mmol) was dissolved in sodium acetate-acetic acid buffer (pH=4.5) (3 mL), and lutetium trichloride (8.86 mg, 0.032 mmol) was added. The reaction solution was stirred at room temperature for 10 minutes, and directly separated by prep-HPLC to obtain **26** (19.02 mg, yield: 83.75%). LC-MS (ESI): m/z = 1142.46(M+H)⁺.

### Embodiment 27

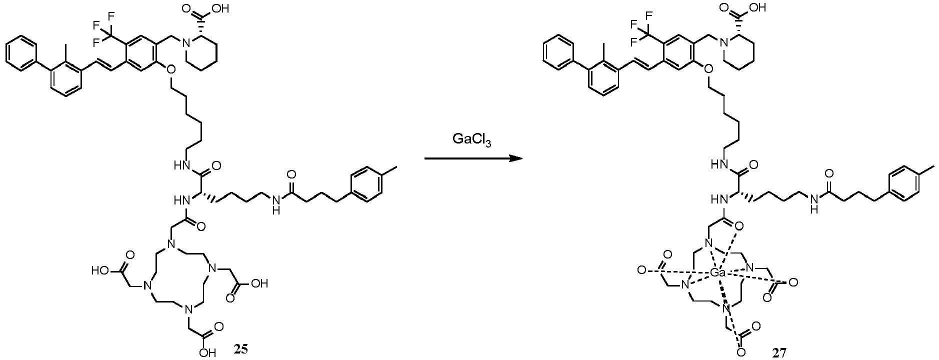

### Synthesis of compound 27

Compound **25** (20 mg, 0.016 mmol) was added to water (2 mL), and gallium trichloride (0.640 mL, 0.032 mmol) was added. The reaction solution was stirred at 90°C for 10 minutes, cooled to room temperature, and directly separated by prep-HPLC to obtain **27** (17.37 mg, yield: 81.24%). LC-MS (ESI): m/z = 1337.21(M+H)⁺.

### Embodiment 28

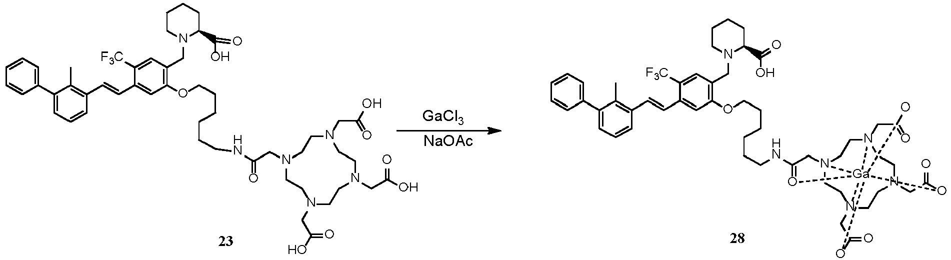

### Synthesis of compound 28

Compound **23** (8 mg, 0.008 mmol) was added to water (2 mL), and sodium acetate (0.164 mL, 0.024 mmol) was added, and the reaction solution was stirred for 1 minute, and gallium trichloride (0.319 mL, 0.016 mmol) was added. The reaction solution was stirred at 90°C for 10 minutes, and the reaction solution was cooled to room temperature, and directly separated by prep-HPLC to obtain **28** (4.92 mg, yield: 57.61%). LC-MS (ESI): m/z = 1048.82(M+H)⁺.

### Embodiment 29

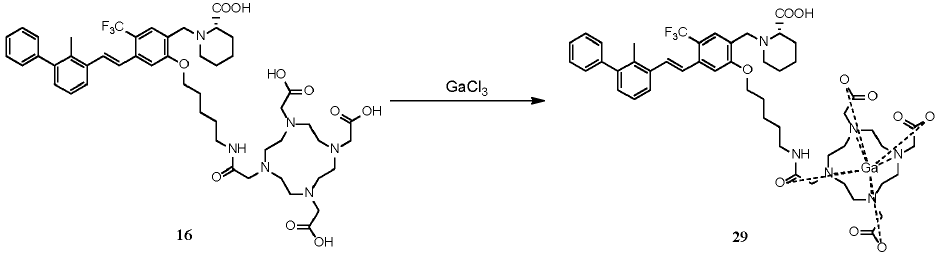

### Synthesis of compound 29

Compound **16** (20 mg, 0.021 mmol) was added to water (2 mL), and gallium trichloride (0.809 mL, 0.041 mmol) was added. The reaction solution was stirred at 90°C for 10 minutes, and the reaction solution was cooled to room temperature, and directly separated by prep-HPLC to obtain **29** (19.64 mg, yield: 90.47%). LC-MS (ESI): m/z = 1334.8(M+H)⁺.

### Embodiment 30

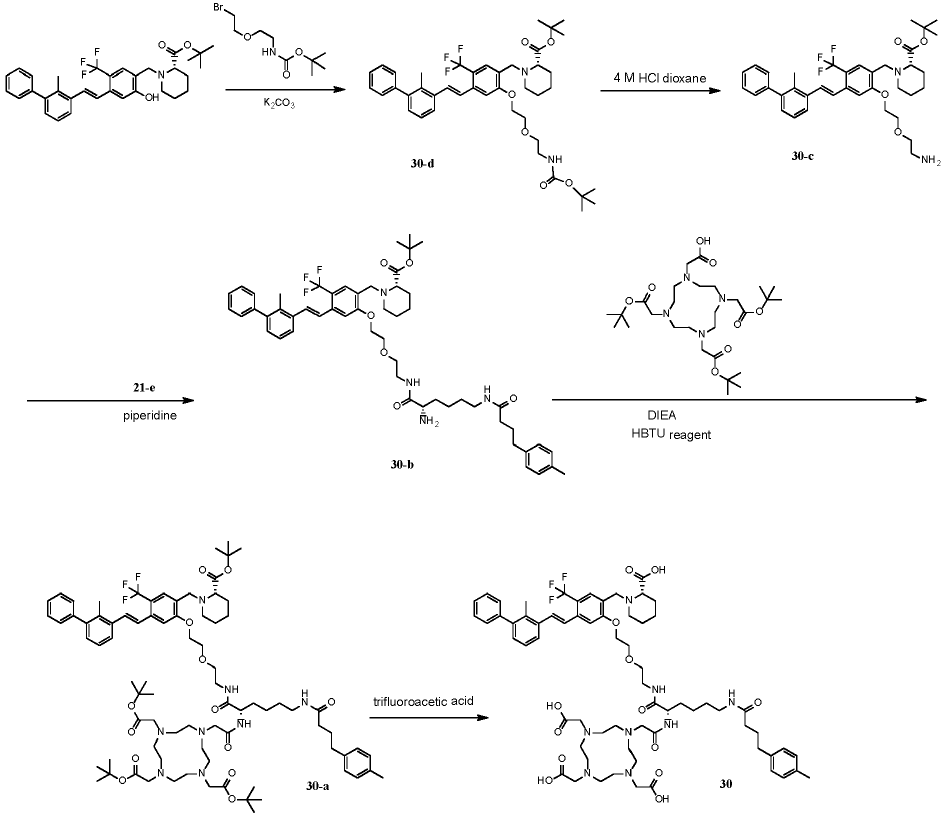

### Synthesis of compound 30-d

Compound **2-c** (551.65 mg, 1.00 mmol), 2-methylpropan-2-yl ({2-[(2-bromoethyl)oxy]ethyl}amino) formate (321.78 mg, 1.200 mmol), and potassium carbonate (179.66 mg, 1.300 mmol) were dissolved in *N, N*-dimethylformamide (3.0 mL). The reaction solution was stirred at 80°C for 60 hours. The reaction solution was cooled to room temperature, diluted with ethyl acetate (40 mL), and washed sequentially with water (20 mL×2) and saturated brine (20 mL×1). The organic phase was dried over anhydrous sodium sulfate, filtered, and concentrated under reduced pressure, and the residue was separated by silica gel column chromatography (petroleum ether: ethyl acetate = 10:3) to obtain **30-d** (438 mg, yield: 59.28%).

### Synthesis of compound 30-c

Compound **30-d** (438 mg, 0.593 mmol) was dissolved in tetrahydrofuran (4 mL), and hydrochloric acid in 1,4-dioxane (4 M) (4 mL) was added, and the reaction solution was stirred at room temperature for 1 hour, and the reaction solution was concentrated under reduced pressure to obtain **30-c** (260 mg, yield: 68.64%).

### Synthesis of compound 30-b

Compound **30-c** (127.75 mg, 0.2 mmol), compound **21-e** (150.17 mg, 0.240 mmol) were dissolved in acetonitrile (2.0 mL) and dichloromethane (2.0 mL). The reaction solution was stirred at room temperature for 1 hour, and piperidine (0.183 mL, 2.00 mmol) was added, and the reaction solution was continuously stirred at room temperature for 2 hours. The reaction solution was concentrated under reduced pressure, and the residue was separated by silica gel column (petroleum ether: ethyl acetate = 5:1, 6 CV, then, dichloromethane: methanol: ammonia methanol (7 M in MeOH) = 100:10:2, 8 CV, CV is column volume) to obtain **30-b** (160 mg, yield: 86.29%).

### Synthesis of compound 30-a

*N, N*-Diisopropylethylamine (0.057 mL, 0.346 mmol), compound **30-b** (160 mg, 0.173 mmol), (4,7,10-tris{2-[(2-methylpropan-2-yl)oxy]-2-oxoethyl}-1,4,7,10-tetraazacyclododecan-1-yl)acetic acid (118.61 mg, 0.207 mmol), and benzotriazole-*N,N,N',N'-*tetramethyluronium hexafluorophosphate (85.29 mg, 0.225 mmol) were dissolved in *N, N-*dimethylformamide (2.0 mL), and the reaction solution was stirred at room temperature for 1 hour. The reaction solution was concentrated under reduced pressure. The residue was separated by column chromatography (petroleum ether: ethyl acetate = 4:1, 6 CV, then 100% PE, 5 CV, then dichloromethane: methanol = 10:1, 8 CV, CV is the column volume) to obtain **30-a** (255 mg, 0.172 mmol, 99.71%).

### Synthesis of compound 30

Trifluoroacetic acid (3 mL) and compound **30-a** (255 mg, 0.172 mmol) were dissolved in dichloromethane (3 mL). The reaction solution was stirred at room temperature for 16 hours. The reaction solution was concentrated under reduced pressure, and the residue was separated by Pre-HPLC to obtain **30** (121 mg, yield: 55.92%). LC-MS (ESI): m/z = 1258.46(M+H)⁺.

### Embodiment 31

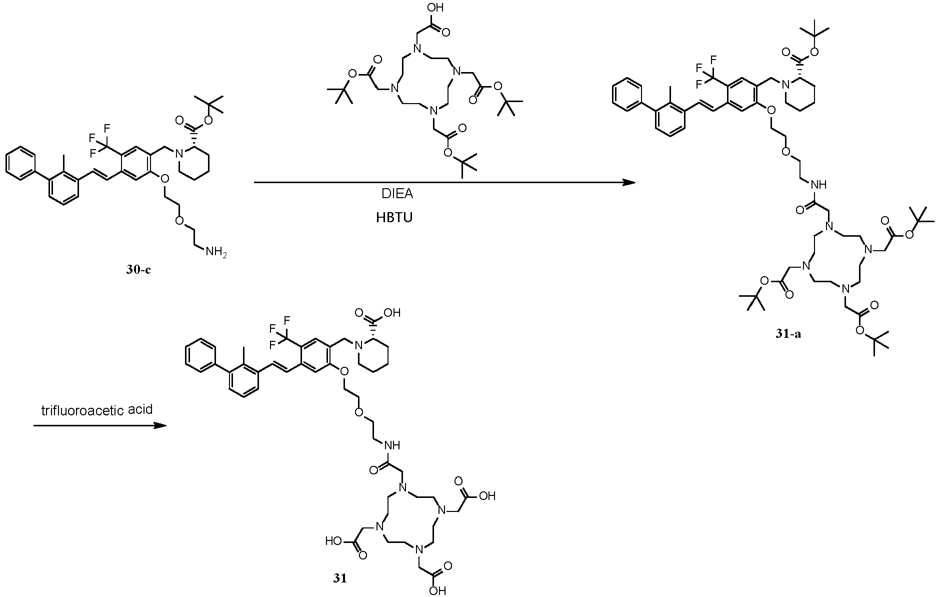

### Synthesis of compound 31-a

*N, N*-Diisopropylethylamine (0.033 mL, 0.200 mmol), compound **30-c** (63.88 mg, 0.1 mmol), (4,7,10-tris{2-[(2-methylpropan-2-yl)oxy]-2-oxoethyl}-1,4,7,10-tetraazacyclododecan-1-yl)acetic acid (68.73 mg, 0.120 mmol) and benzotriazole-*N,N,N',N'-*tetramethyluronium hexafluorophosphate (49.30 mg, 0.130 mmol) were dissolved in acetonitrile (1.0 mL), and the reaction solution was stirred at room temperature for 0.5 hour. The reaction solution was concentrated under reduced pressure, and the residue was separated by column chromatography (petroleum ether: ethyl acetate = 5:1, then, dichloromethane: methanol: ammonia methanol (7 M in MeOH) = 200:20:1) to obtain **31-a** (119 mg, yield: 99.71%).

### Synthesis of compound 31

Trifluoroacetic acid (2.0 mL) and compound **31-a** (119 mg, 0.100 mmol) were dissolved in dichloromethane (2.0 mL). The reaction solution was reacted at room temperature for 16 hours. The reaction solution was concentrated under reduced pressure, and the residue was separated by Pre-HPLC to obtain **31** (47 mg, yield: 48.64%). LC-MS (ESI): m/z = 970.13(M+H)⁺.

### Embodiment 32

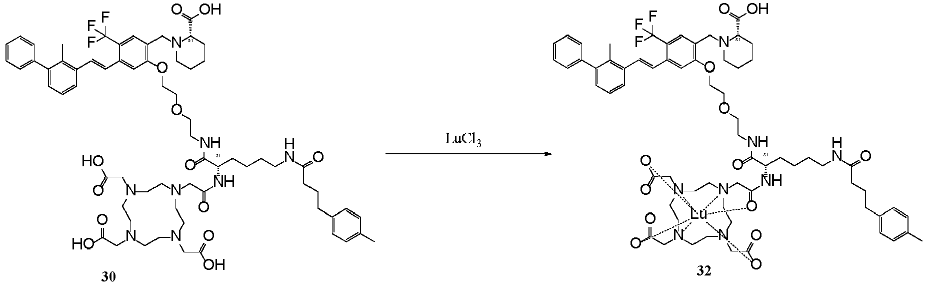

### Synthesis of compound 32

Compound **30** (12.57 mg, 0.01 mmol) and lutetium trichloride (5.63 mg, 0.020 mmol) were added to sodium acetate-acetic acid buffer (pH=4.5) (1.0 mL), and the reaction solution was stirred at 90°C for 10 minutes. The reaction solution was cooled to room temperature, and directly separated by reversed-phase chromatography to obtain **32** (7.2 mg, yield: 50.38%). LC-MS (ESI): m/z = 1430.4(M+H)⁺.

### Embodiment 33

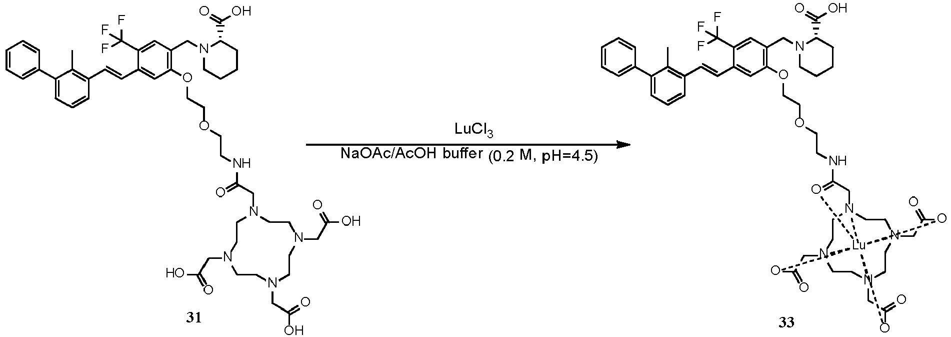

### Synthesis of compound 33

Lutetium trichloride (5.63 mg, 0.020 mmol) and compound **31** (9.69 mg, 0.01mmol) were added to sodium acetate-acetic acid buffer (pH=4.5) (1.0 mL), and the reaction solution was stirred at 90°C for 5 minutes, and the reaction solution was cooled to room temperature. The reaction solution was directly separated by Pre-HPLC to obtain **33** (8.7 mg yield: 76.25%). LC-MS (ESI): m/z = 1142.01(M+H)⁺.

### Embodiment 34

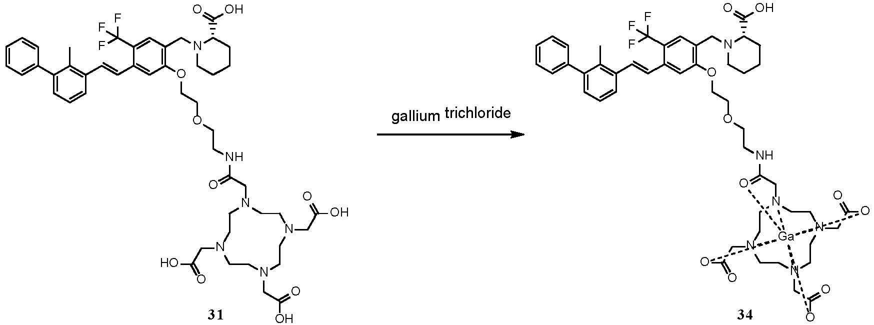

### Synthesis of compound 34

Compound **31** (4.85 mg, 0.005 mmol) and gallium trichloride (1.76 mg, 0.010 mmol) were added to water (0.2 mL), and the reaction solution was heated and stirred at 90°C for 5 minutes. The reaction solution was cooled to room temperature, and the reaction solution was directly separated by Pre-HPLC to obtain **34** (3.5 mg, yield: 67.57%). LC-MS (ESI): m/z = 1036.77(M+H)⁺.

### Embodiment 35

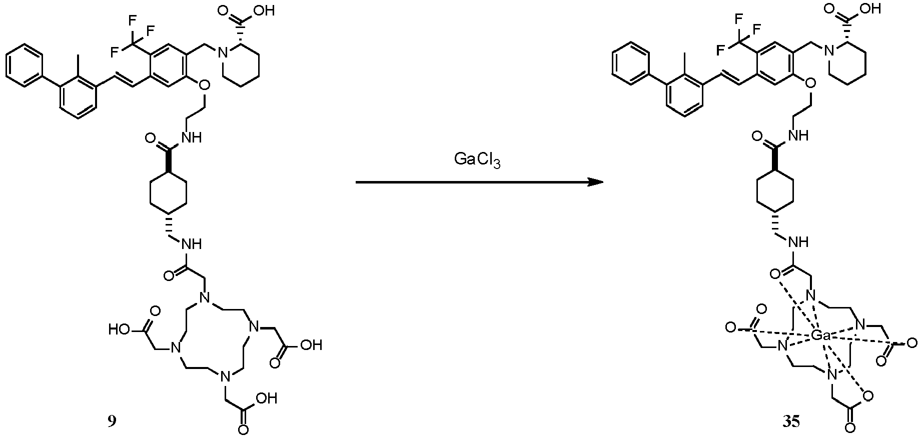

### Synthesis of compound 35

Compound **9** (5.32 mg, 0.005 mmol) and gallium trichloride (1.76 mg, 0.010 mmol) were added to water (0.2 mL), and the reaction solution was heated and stirred at 90°C for 10 minutes. The reaction solution was cooled to room temperature, and directly separated by Pre-HPLC to obtain **35** (2.3 mg, yield: 40.71%). LC-MS (ESI): m/z = 1131.91(M+H)⁺.

### Embodiment 36

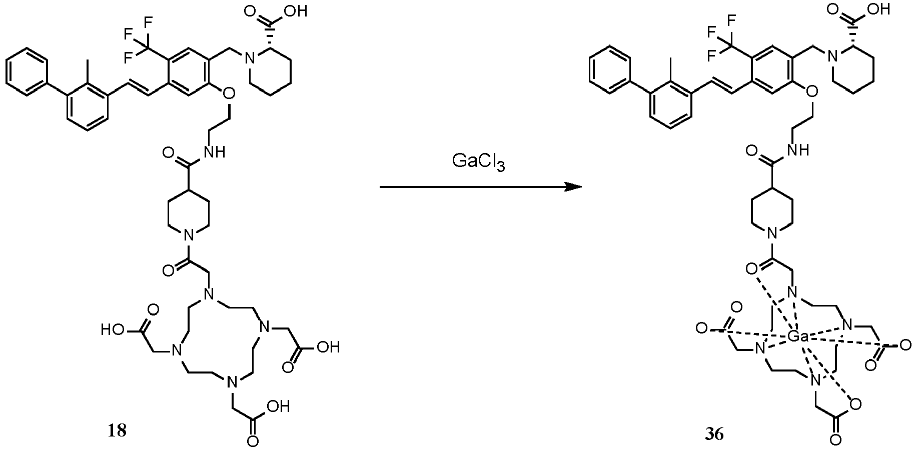

### Synthesis of compound 36

Compound **18** (5.18 mg, 0.005 mmol) and gallium trichloride (1.76 mg, 0.010 mmol) were added to water (0.2 mL), and the reaction solution was stirred at 90°C for 10 minutes. The reaction solution was cooled to room temperature, and directly separated by Pre-HPLC to obtain **36** (2.7 mg, yield: 49.00%). LC-MS (ESI): m/z = 1103.86(M+H)⁺.

### Embodiment 37

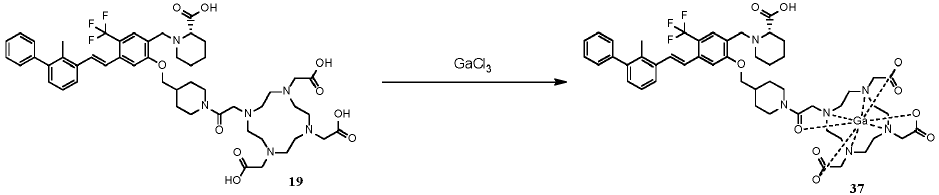

### Synthesis of compound 37

Compound **19** (9.79 mg, 0.01 mmol) and gallium trichloride (3.52, 0.020 mmol) were added to water (0.5 mL), and the reaction solution was heated and stirred at 90°C for 10 minutes, and the reaction solution was cooled to room temperature. The reaction solution was directly separated by Pre-HPLC to obtain **37** (8.5 mg, yield: 81.26%). LC-MS (ESI): m/z = 1146.81(M+H)⁺.

### Embodiment 38

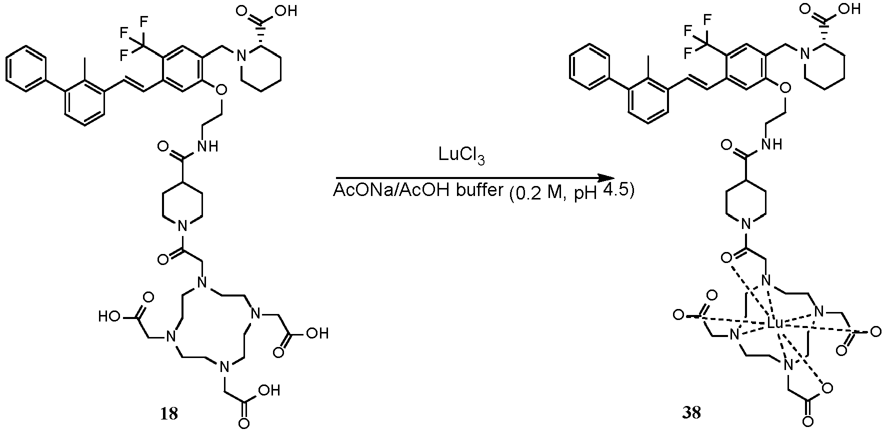

### Synthesis of compound 38

Compound **18** (15.54 mg, 0.015 mmol) and lutetium trichloride (8.44 mg, 0.030 mmol) were added to sodium acetate-acetic acid buffer (pH=4.5) (1.0 mL), and the reaction solution was stirred at room temperature for 10 minutes, and directly separated by Pre-HPLC to obtain **38** (8 mg, yield: 44.15%). LC-MS (ESI): m/z = 1209.1(M+H)⁺.

### Embodiment 39

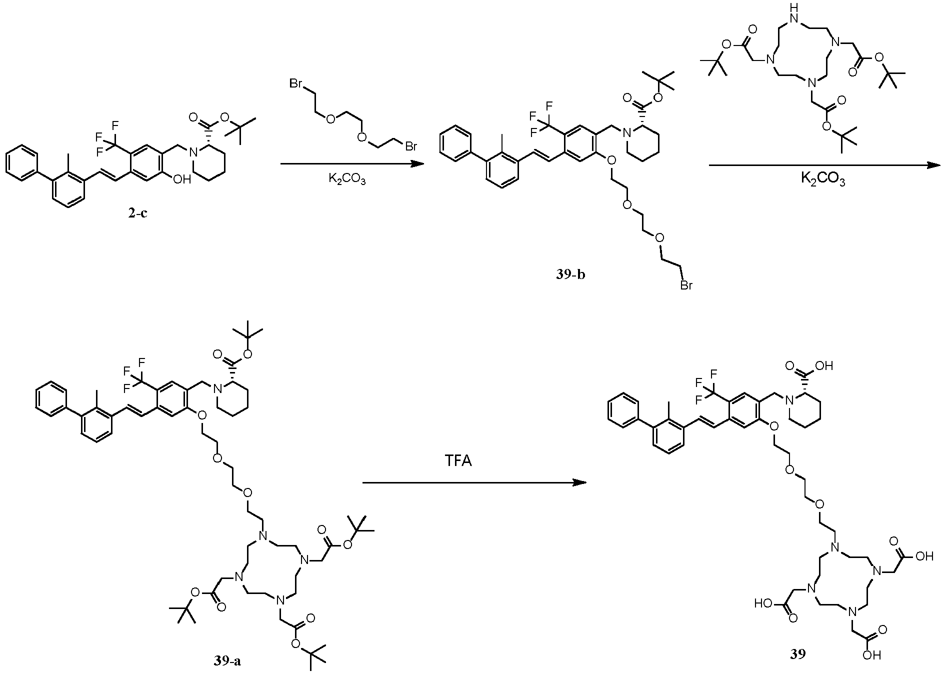

### Synthesis of compound 39-b

Compound **2-c** (441.32 mg, 0.80 mmol) and 1,8-dibromo-3,6-dioxoctane (242.85 mg, 0.880 mmol) were dissolved in *N, N-*dimethylformamide (3.0 mL), and potassium carbonate (143.73 mg, 1.040 mmol) was added. The reaction solution was stirred at 80°C for 60 hours. The reaction solution was cooled to room temperature, diluted with ethyl acetate (40 mL), and the reaction solution was washed sequentially with water (20 mL×2) and saturated brine (20 mL×1). The organic phase was dried over anhydrous sodium sulfate, filtered, and concentrated under reduced pressure. The residue was separated by silica gel column chromatography (petroleum ether: ethyl acetate = 3:1) to obtain **39-b** (240 mg, yield: 40.18%).

### Synthesis of compound 39-a

Compound **39-b** (140 mg, 0.187 mmol) and 2-methylpropan-2-yl (4,7-bis{2-[(2-methylpropan-2-yl)oxy]-2-oxoethyl}-1,4,7,10-tetraazacyclododecan-1-yl) acetate (96.50 mg, 0.187 mmol) were dissolved in acetonitrile (2 mL), and potassium carbonate ( 25.91 mg, 0.187 mmol) was added, and the reaction solution was stirred at 60°C for 16 hours. The reaction solution was cooled to room temperature, and the reaction solution was concentrated under reduced pressure, and the residue was purified by Pre-HPLC to obtain **39-a** (240 mg, yield: 84.29%).

### Synthesis of compound 39

Compound **39-a** (240 mg, 0.158 mmol) and trifluoroacetic acid (3.0 mL) were dissolved in dichloromethane (3.0 mL), and the reaction solution was stirred at room temperature for 16 hours. The reaction solution was concentrated under reduced pressure. The residue was purified by Pre-HPLC to obtain **39** (78 mg, yield: 51.76%). LC-MS (ESI): m/z = 956.24(M+H)⁺.

### Embodiment 40

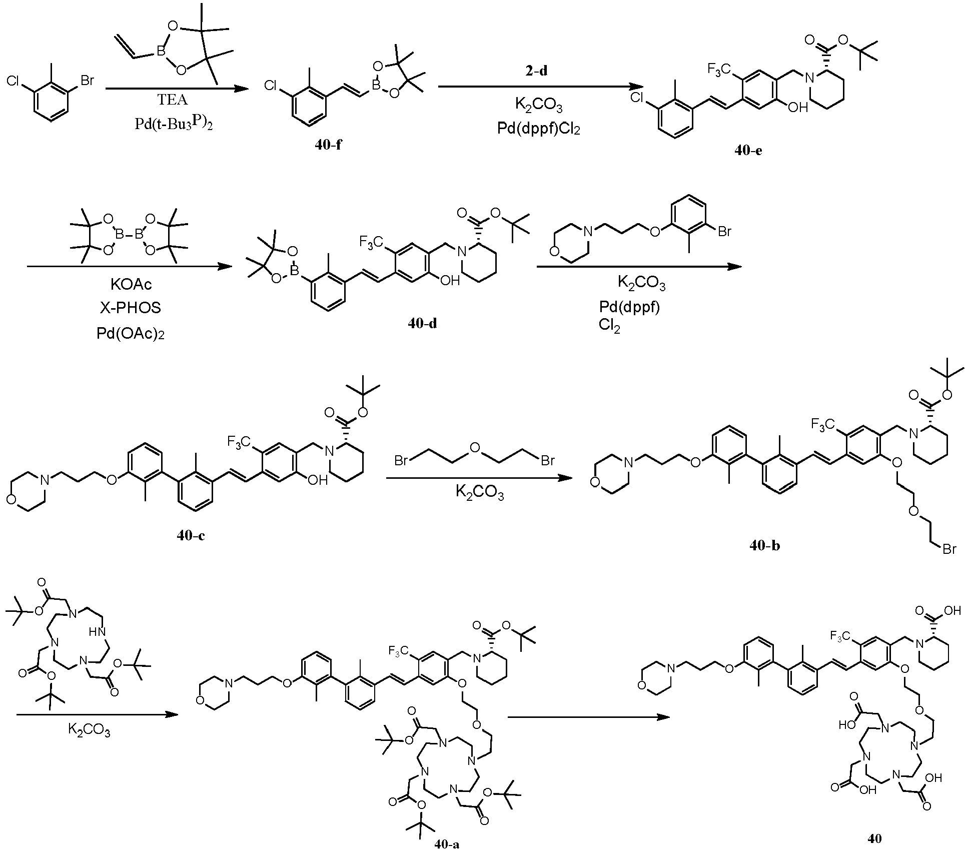

### Synthesis of compound 40-f

3-Bromo-1-chloro-2-toluene (3082.20 mg, 15.00 mmol), 4,4,5,5-tetramethyl-2-vinyl-1,3,2-dioxyborane (2772.36 mg, 18.000 mmol), and triethylamine (20.850 mL, 150.000 mmol) were dissolved in toluene (30 mL), and bis(tri-tert-butylphosphine) palladium (383.29 mg, 0.750 mmol) was added, and the reaction solution was replaced with nitrogen for three times, and the reaction solution was heated at 80°C for 16 hours. The reaction solution was cooled to room temperature, filtered, and the filtrate was concentrated under reduced pressure, and the residue was separated by silica gel column chromatography (petroleum ether: ethyl acetate=10:1) to obtain **40-f** (1000 mg, yield: 23.93%).

### Synthesis of compound 40-e

Compound **2-d** (1200 mg, 2.738 mmol) and compound **40-f** (991.60 mg, 3.559 mmol) were dissolved in dioxane (10.0 mL) and water (2.0 mL), and (1,1'-bis(diphenylphosphino)ferrocene) dichloropalladium (100.17 mg, 0.137 mmol) and potassium carbonate (756.83 mg, 5.476 mmol) were added, and the reaction solution was heated and stirred at 80°C for 16 hours under nitrogen protection. The reaction solution was cooled to room temperature, and the reaction solution was diluted with water (10 mL), and the mixture was extracted with dichloromethane (30 mL × 2). The organic phases were combined, dried over anhydrous sodium sulfate, and concentrated under reduced pressure. The residue was separated by column chromatography (petroleum ether: ethyl acetate = 20:1) to obtain **40-e** (220 mg, yield: 15.76%). LC-MS (ESI): m/z =510.39 (M+H)⁺.

### Synthesis of compound 40-d

Compound **40-e** (220 mg, 0.431 mmol) and 4,4,5,5-tetramethyl-2-(4,4,5,5-tetramethyl-1,3,2-dioxaborolan-2-yl)-1,3,2-dioxaborolane (219.09 mg, 0.863 mmol) were dissolved in toluene (3 mL), and palladium acetate (9.68 mg, 0.043 mmol), 2-dicyclohexylphosphino-2',4',6'-triisopropylbiphenyl (41.13 mg, 0.086 mmol), and potassium acetate (84.60 mg, 0.862 mmol) were added, and the reaction solution was heated at 100°C for 16 hours under nitrogen protection. The reaction solution was cooled to room temperature, and the reaction solution was diluted with dichloromethane (40 mL), and the mixture was washed with water (10 mL×1). The organic phase was dried over anhydrous sodium sulfate, filtered, and concentrated under reduced pressure, and the residue was separated by column chromatography (petroleum ether: ethyl acetate=10:1) to obtain **40-d** (220 mg, yield: 84.86%).

### Synthesis of compound 40-c

Compound **40-d** (220 mg, 0.366 mmol) and 4-{3-[(3-bromo-2-methylphenyl)oxy]propyl}-1,4-oxazinane (137.91 mg, 0.439 mmol) were dissolved in dioxane (2.0 mL) and water (0.4 mL), and (1,1'-bis(diphenylphosphino)ferrocene) dichloropalladium (26.76 mg, 0.037 mmol) and potassium carbonate (101.10 mg, 0.731 mmol) were added, and the reaction solution was heated and stirred at 90°C for 16 hours under nitrogen protection. The reaction solution was cooled to room temperature, and the reaction solution was diluted with water (10 mL), and the obtained mixture was extracted with dichloromethane (40 mL). The organic phase was dried over anhydrous sodium sulfate, concentrated under reduced pressure, and the residue was separated by column chromatography (100% ethyl acetate) to obtain **40-c** (195 mg, yield: 75.21%).

### Synthesis of compound 40-b

Compound **40-c** (195 mg, 0.275 mmol) and 1-bromo-2-[(2-bromoethyl)oxy]ethane (191.39 mg, 0.825 mmol) were dissolved in *N, N-*dimethylformamide (2.0 mL), and potassium carbonate (76.02 mg, 0.550 mmol) was added, and the reaction solution was heated and stirred at 70°C for 20 hours. The reaction solution was cooled to room temperature and concentrated under reduced pressure, and the residue was separated by column chromatography (ethyl acetate: ethanol=20:1) to obtain **40-b** (150 mg, yield: 63.44%).

### Synthesis of compound 40-a

Compound **40-b** (150 mg, 0.174 mmol) and 2-methylpropan-2-yl (4,7-bis{2-[(2-methylpropan-2-yl)oxy]-2-oxoethyl}-1,4,7,10-tetraazacyclododecan-1-yl) acetate (134.68 mg, 0.262 mmol) were dissolved in acetonitrile (2.0 mL), and potassium carbonate (36.17 mg, 0.262 mmol) was added, and the reaction solution was stirred at 60°C for 16 hours, and 13% of **40-b** was remained and the mixture was continuously heated and stirred for 8 hours. The reaction solution was evaporated to dryness, and the residue was separated by column chromatography (methanol: ammonia methanol (7 M) = 5:1) to obtain **40-a** (170 mg, yield: 75.33%).

### Synthesis of compound 40

Compound **40-a** (170 mg, 0.131 mmol) was dissolved in dichloromethane (5.0 mL), and trifluoroacetic acid (5.0 mL) was added. The reaction solution was stirred at room temperature for 16 hours. The reaction solution was concentrated under reduced pressure, and the residue was separated by Pre-HPLC to obtain **40** (40 mg, yield: 21.64%).

### Embodiment 41

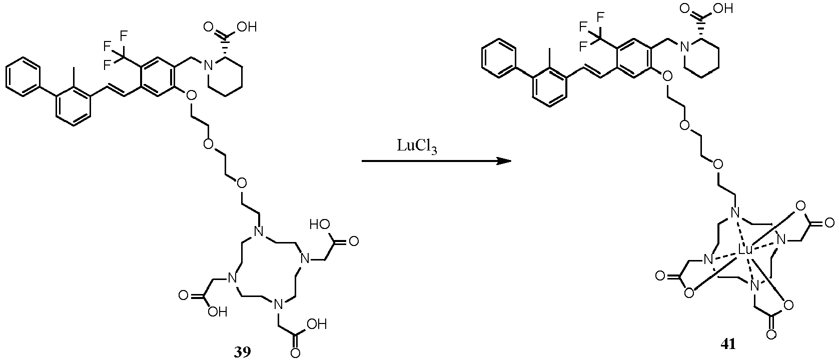

### Synthesis of compound 41

Compound **39** (11.2 mg, 0.012 mmol) and lutetium trichloride (7.25 mg, 0.026 mmol) were dissolved in sodium acetate-acetic acid buffer (pH=4.5) (0.5 mL), and the reaction solution was heated at 90°C for 20 minutes. The reaction solution was cooled to room temperature, and purified directly by Pre-HPLC to obtain **41** (11 mg, yield: 83.27%).

### Embodiment 42

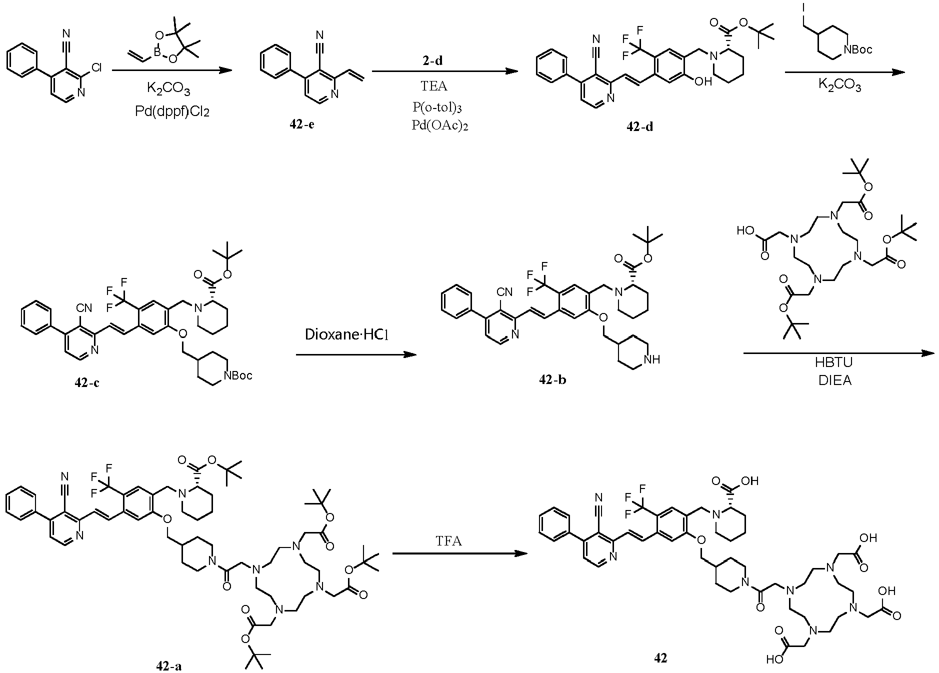

### Synthesis of compound 42-e

2-Chloro-4-phenylpyridine-3-carbonitrile (4293.20 mg, 20.00 mmol) and 4,4,5,5-tetramethyl-2-vinyl-1,3,2-dioxyborane (7701.00 mg, 50.00 mmol) were dissolved in dioxane (50.0 mL) and water (10.0 mL), and potassium carbonate (8292.60 mg, 60.00 mmol) and (1,1'-bis(diphenylphosphino)ferrocene) dichloropalladium (1463.40 mg, 2.00 mmol) were mixed in a microwave tube, and the microwave tube was sealed after 1 minute of nitrogen substitution. The reaction solution was stirred at 100°C for 16 hours, and the reaction solution was cooled to room temperature. The reaction solution was directly diluted with water (50 mL), and extracted with dichloromethane (100 mL). The organic phase was dried over anhydrous sodium sulfate, filtered, and concentrated under reduced pressure, and the residue was separated by column chromatography (petroleum ether: ethyl acetate=85:15) to obtain **42-e** (3150 mg, yield: 76.36%). LC-MS (ESI): m/z = 207.29(M+H)⁺.

### Synthesis of compound 42-d

Compound **42-e** (412.50 mg, 2.0 mmol) and compound **2-d** (876.56 mg, 2.00 mmol) were dissolved in *N, N*-dimethylacetamide (6.0 mL), and palladium acetate (44.90 mg, 0.20 mmol), tri(o-tolyl)phosphine (121.75 mg, 0.40 mmol), and triethylamine (2.0 mL, 14.389 mmol) were added, and the mixture was heated via microwave at 160°C for 0.5 hour under nitrogen protection. The reaction solution was cooled to room temperature and concentrated under reduced pressure, and the residue was separated by column chromatography (petroleum ether: ethyl acetate=5:1) to obtain **42-d** (550 mg, yield: 48.79%).

### Synthesis of compound 42-c

Compound **42-d** (225.45 mg, 0.4 mmol) and 2-methylpropan-2-yl-4-(iodomethyl) hexahydropyridine-1-carboxylate (650.38 mg, 2.00 mmol) were dissolved in *N, N-*dimethylformamide (2 mL), and potassium carbonate (276.42 mg, 2.00 mmol) was added, and the reaction solution was heated and stirred at 80°C for 60 hours. The reaction solution was cooled to room temperature, diluted with ethyl acetate (40 mL), and washed sequentially with water (20 mL×2) and saturated brine (20 mL). The organic phase was dried over anhydrous sodium sulfate, filtered, and concentrated under reduced pressure. The residue was separated by silica gel column chromatography (petroleum ether: ethyl acetate = 5:1) to obtain **42-c** (248 mg, yield: 81.48%).

### Synthesis of compound 42-b

Compound **42-c** (248 mg, 0.326 mmol) was dissolved in tetrahydrofuran (3 mL), and dioxane solution of hydrochloric acid (1 mL) was added. The reaction solution was stirred at room temperature for 6 hours. 1 M K₂CO₃ solution (10 mL) was added. The mixture was extracted with dichloromethane (30 mL). The separated organic phase was dried over anhydrous sodium sulfate, filtered, and concentrated under reduced pressure to obtain **42-b** (215 mg, yield: 99.83%). The product 42-b was directly used in the next step of reaction without purification.

### Synthesis of compound 42-a

Compound **42-b** (100 mg, 0.151 mmol), (10-{1-[(2-methylpropan-2-yl)oxy]-1-oxoethyl-2-yl}-4,7-bis{2-[(2-methylpropan-2-yl)oxy)-2-oxoethyl}-1,4,7,10-tetraazacyclododecan-1-yl) acetic acid (104.01 mg, 0.182 mmol) and O-benzotriazole-tetramethyluronium hexafluorophosphate (74.61 mg, 0.197 mmol) were dissolved in *N, N-*dimethylformamide (1.0 mL), and diisopropylethylamine (0.050 mL, 0.303 mmol) was added, and the reaction solution was stirred at room temperature for 1 hour. The reaction solution was concentrated under reduced pressure, and the residue was separated by silica gel column chromatography (petroleum ether: ethyl acetate: ethanol=10:20:1) to obtain **42-a** (360 mg, yield: 78.89%).

### Synthesis of compound 42

Compound **42-a** (150 mg, 0.096 mmol) was dissolved in dichloromethane (2.0 mL), and trifluoroacetic acid (2.0 mL) was added. The reaction solution was stirred at room temperature for 20 hours. The reaction solution was concentrated under reduced pressure, and the residue was separated by Pre-HPLC to obtain 42 (63 mg, yield: 66.01%).

### Embodiment 43

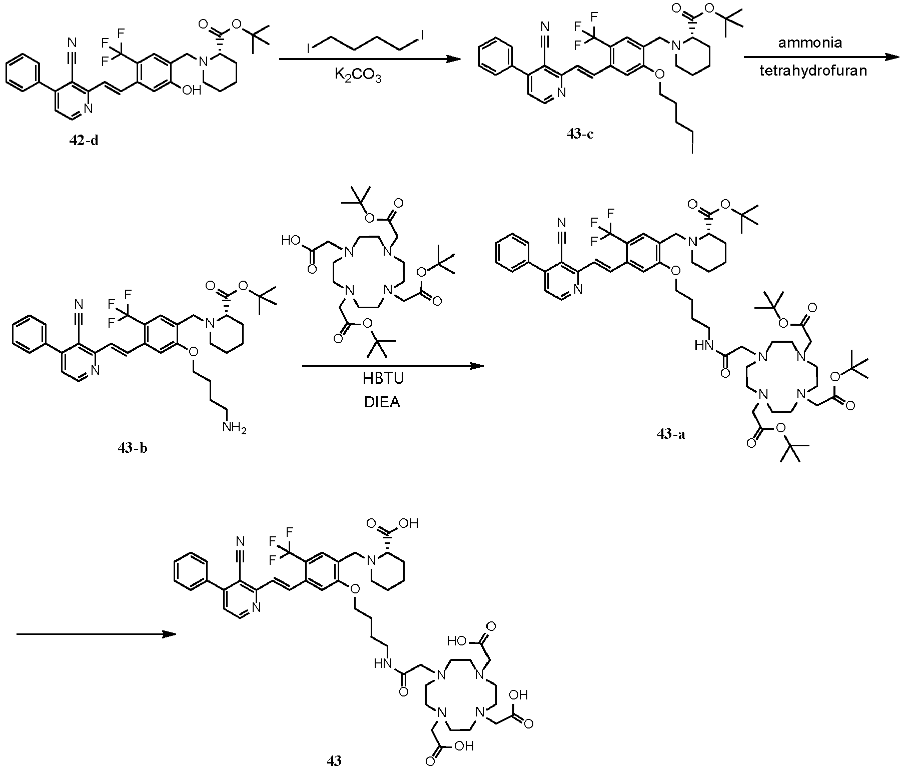

### Synthesis of compound 43-c

Compound **42-d** (112.72 mg, 0.2 mmol), 1,4-diiodobutane (247.94 mg, 0.80 mmol) was dissolved in *N, N*-dimethylformamide (1.0 mL), and potassium carbonate (55.28 mg, 0.40 mmol) was added, and the reaction solution was stirred at 50°C for 1 hour. The reaction solution was cooled to room temperature, and the reaction solution was diluted with ethyl acetate (50 mL), washed sequentially with water (20 mL×2) and saturated brine (20 mL). The organic phase was separated, dried over anhydrous sodium sulfate, filtered, and concentrated under reduced pressure, and the residue was separated by column chromatography (petroleum ether: ethyl acetate=5:1) to obtain **43-c** (124 mg, yield: 83.15%).

### Synthesis of compound 43-b

Compound **43-c** (124 mg, 0.166 mmol) was dissolved in tetrahydrofuran (3 mL), and ammonia methanol solution (10 mL, 70.00 mmol) was added. The reaction solution was heated and stirred at 50 °C for 16 hours. The reaction solution was cooled to room temperature and concentrated under reduced pressure, and the residue was dissolved in dichloromethane (30 mL) and washed with 1 M K₂CO₃ solution (10 mL). The separated organic phase was dried over anhydrous sodium sulfate, filtered, and concentrated under reduced pressure to obtain **43-b** (105 mg, yield: 99.47%), which was directly used in the next step of reaction without purification.

### Synthesis of compound 43-a

Compound **43-b** (105 mg, 0.165 mmol), (4,7,10-tris{2-[(2-methylpropan-2-yl)oxy]-2-oxoethyl}-1,4,7,10-tetraazacyclododecan-1-yl)acetic acid (113.69 mg, 0.199 mmol), and O-benzotriazole-tetramethyluronium hexafluorophosphate (81.56 mg, 0.215 mmol) were dissolved in acetonitrile (1.0 mL), and diisopropylethylamine (0.055 mL, 0.331 mmol) was added. The reaction solution was stirred at room temperature for 1 hour. The reaction solution was concentrated under reduced pressure, and the residue was separated by column chromatography (dichloromethane: methanol = 94:6) to obtain **43-a** (196 mg, yield: 99.61%).

### Synthesis of compound 43

Compound **43-a** (196 mg, 0.165 mmol) was dissolved in dichloromethane (3.0 mL). Trifluoroacetic acid (3.0 mL) was added. The reaction solution was stirred at room temperature for 20 hours. The reaction solution was concentrated under reduced pressure, and the residue was separated by Pre-HPLC to obtain compound **43** (80 mg, yield: 50.31%). LC-MS (ESI): m/z =965.45 (M+H)⁺.

### Embodiment 44

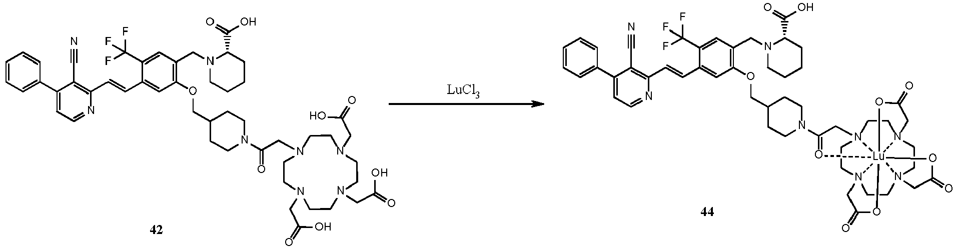

### Synthesis of compound 44

Compound **42** (9.91 mg, 0.01 mmol) was dissolved in sodium acetate-acetic acid buffer (pH=4.5) (0.5mL), and lutetium trichloride (5.63 mg, 0.020 mmol) was added, and the reaction solution was stirred at 90°C for 10 minutes. The reaction solution was cooled to room temperature, and separated directly by Pre-HPLC to obtain **44** (10.6 mg, yield: 91.14%). LC-MS (ESI): m/z = 1163.12 (M+H)⁺.

### Embodiment 45

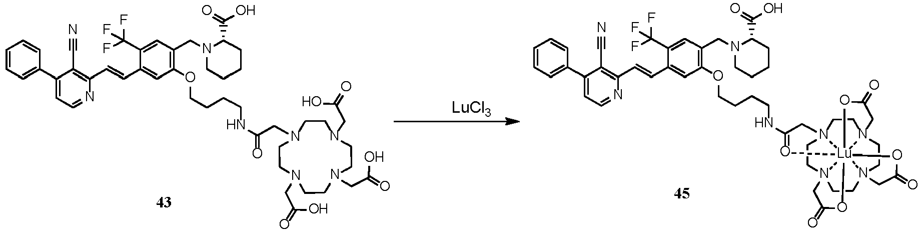

### Synthesis of compound 45

Compound **43** (11 mg, 0.011 mmol) and lutetium trichloride (9.28 mg, 0.033 mmol) were dissolved in sodium acetate-acetic acid buffer (pH=4.5) (0.5 mL), and the reaction solution was stirred at 90°C for 10 minutes. The reaction solution was directly separated by Pre-HPLC to obtain **45** (9 mg, yield: 71.94%). LC-MS (ESI): m/z = 1137.80 (M+H)⁺.

### Embodiment 46

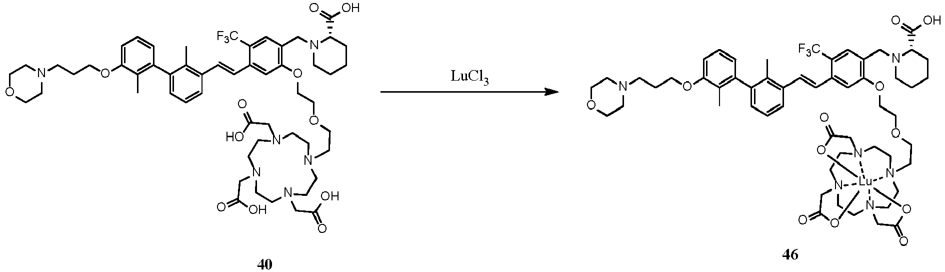

### Synthesis of compound 46

Compound **40** (15 mg, 0.011 mmol) was dissolved in sodium acetate-acetic acid buffer (pH=4.5) (0.5 mL), and lutetium trichloride (5.98 mg, 0.021 mmol) was added. The reaction solution was heated and stirred at 90°C for 10 minutes. The reaction solution was cooled to room temperature, and purified directly by Pre-HPLC to obtain **46** (5.5 mg, yield: 41.70%).

### Embodiment 47

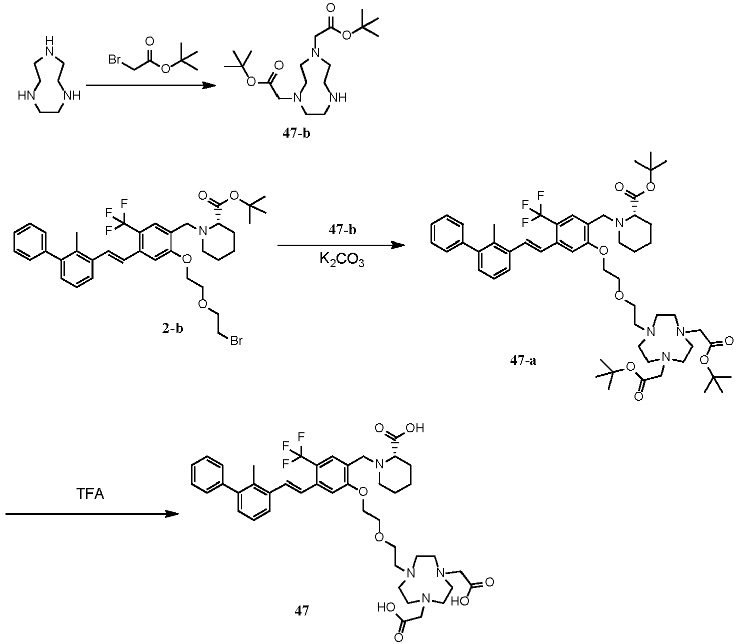

### Synthesis of compound 47-b

A solution of 2-methylpropan-2-yl bromoacetate (3019.14 mg, 15.478 mmol) dissolved in acetonitrile (40 mL) was added dropwise into a solution of 1,4,7-triazane (1000 mg, 7.739 mmol) dissolved in acetonitrile (10 mL) at 0°C. After dropwise addition, the reaction solution was stirred at room temperature for 24 hours, and the reaction solution was concentrated under reduced pressure, and diluted with pure water (5 mL). The mixture was adjusted to pH=3 with 1M hydrochloric acid. The reaction solution was extracted with methyl tert-butyl ether (15 mL×3), and the mixture was adjusted to PH=8 with 1M sodium hydroxide solution, extracted with dichloromethane (20 mL×2), and the organic phases were combined, dried over anhydrous sodium sulfate, filtered, and concentrated under reduced pressure to obtain **47-b** (1200 mg, yield: 43.37%).

### Synthesis of compound 47-a

Compound **2-b** (140.53 mg, 0.20 mmol) and compound **47-b** were dissolved in acetonitrile (1.0 mL), and potassium carbonate (55.28 mg, 0.400 mmol) was added, and the reaction solution was stirred at 60°C overnight. The reaction solution was cooled to room temperature, concentrated under reduced pressure, and the residue was separated by column chromatography (dichloromethane: methanol = 5:1) to obtain **47-a** (153 mg, yield: 78.12%).

### Synthesis of compound 47

Compound **47-a** (153 mg, 0.156 mmol) was dissolved in dichloromethane (2.5 mL), and trifluoroacetic acid (2.5 mL) was added. The reaction solution was stirred at room temperature overnight. The reaction solution was concentrated under reduced pressure, and the residue was separated by Pre-HPLC to obtain **47** (75 mg, yield: 59.19%).

### Embodiment 48

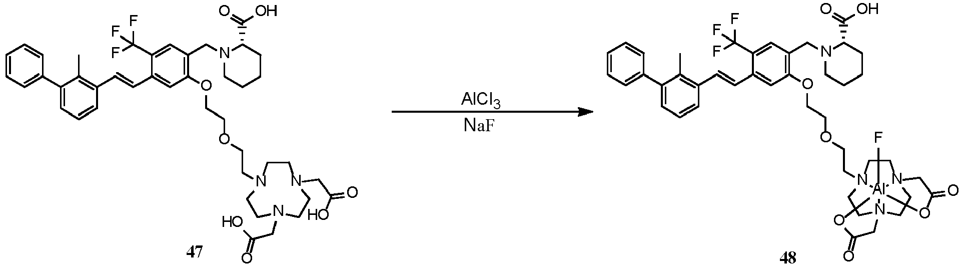

### Synthesis of compound 48

Aluminum chloride (0.80 mg, 0.006 mmol) was dissolved in 0.2 M sodium acetate-acetic acid buffer (pH=4.5) (0.60 mL), and sodium fluoride (0.25 mg, 0.006 mmol) was added, and the reaction solution was stirred at room temperature for 5 minutes. Compound **47** (4.05 mg, 0.005 mmol) was added, and the mixture was heated at 100°C for 15 minutes. The reaction solution was cooled to room temperature, and the reaction solution was purified directly by Pre-HPLC to obtain **48** (3.0 mg, yield: 70.26%).

### Embodiment 49

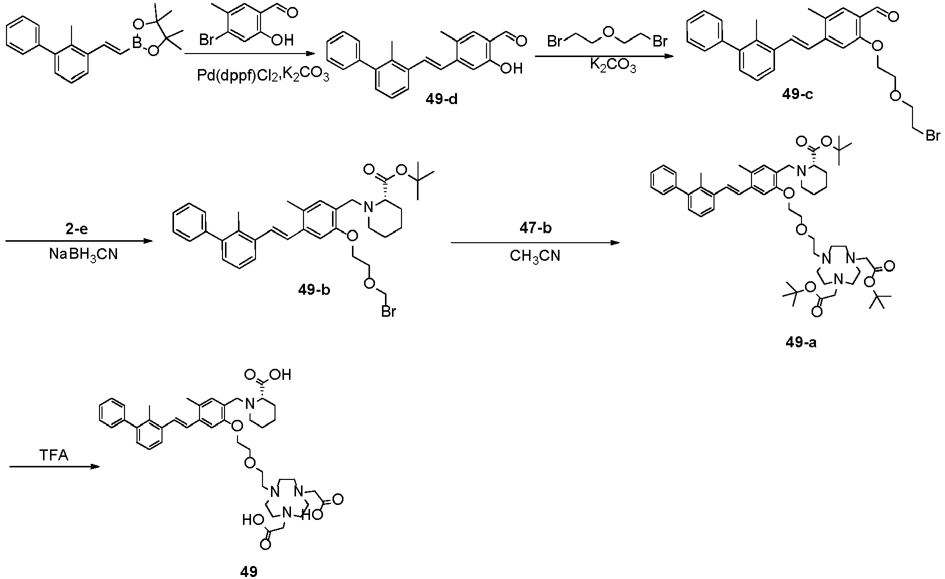

### Synthesis of compound 49-d

4,4,5,5-Tetramethyl-2-[(1*E*)-2-(2-methyl-3-phenylphenyl)vinyl]-1,3,2-dioxyborane (960.7 mg, 3.0 mmol) and 4-bromo-2-hydroxy-5-methylphenyl-1-formaldehyde (645.1 mg, 3.00 mmol) were dissolved in dioxane (100.0 mL) and water (20.0 mL), and (1,1'-bis(diphenylphosphino)ferrocene)dichloropalladium (219.3 mg, 0.3 mmol) and potassium carbonate (1243.8 mg, 9.00 mmol) were added. The reaction solution was heated and stirred at 90°C for 12 hours, and the reaction solution was cooled to room temperature, concentrated under reduced pressure, and the residue was purified by column chromatography (petroleum ether: ethyl acetate = 10:1) to obtain **49-d** (950 mg, yield: 86.78). LC-MS (ESI): m/z = 329.1(M+Na)⁺.

### Synthesis of compound 49-c

Compound **49-d** (620 mg, 2.89 mmol) and 1-bromo-2-[(2-bromoethyl)oxy]ethane (875.6 mg, 3.77 mmol) were dissolved in *N, N*-dimethylformamide (50 ml), and potassium carbonate (782.7 mg, 5.66 mmol) was added, and the reaction solution was stirred at 70°C for 12 hours. The reaction solution was cooled to room temperature, concentrated under reduced pressure, and the residue was dissolved with dichloromethane (100 mL), and the mixture was washed sequentially with water (50 ml×2) and saturated brine (50 ml), dried over anhydrous sodium sulfate, filtered, and concentrated under reduced pressure, and the residue was purified by column chromatography (petroleum ether: ethyl acetate=3:1) to obtain **49-c** (220 mg, yield: 23.09%).

### Synthesis of compound 49-b

Compound **49-c** (280.0 mg, 0.58 mmol) and compound **2-e** (216.41 mg, 1.16 mmol) were dissolved in methanol (40 mL), and sodium cyanoborohydride (110.39 mg, 1.752 mmol) and acetic acid (35.0 mg, 0.584 mmol) were added, and the reaction solution was stirred at 60°C for 6 hours. The reaction solution was cooled to room temperature and concentrated under reduced pressure, and the concentrate was separated by column chromatography (petroleum ether: ethyl acetate = 10:1) to obtain **49-b** (280 mg, yield: 70.21%). LC-MS (ESI): m/z = 648.3 (M+H)⁺.

### Synthesis of compound 49-a

Compound **49-b** (380 mg, 0.586 mmol) and compound **47-b** (418.8 mg, 1.172 mmol) were dissolved in acetonitrile (30.0 mL), and potassium carbonate (323.36 mg, 2.343 mmol) was added. The reaction solution was stirred at 60°C for 12 hours, and the reaction solution was cooled to room temperature and concentrated under reduced pressure, and the residue was dissolved in dichloromethane (100 mL). The mixture was washed sequentially with water (50 ml×2) and saturated brine (50 ml), dried over anhydrous sodium sulfate, filtered, and concentrated under reduced pressure, and the residue was purified by column chromatography (petroleum ether: ethyl acetate = 10:1) to obtain **49-a** (320 mg, yield: 56.09%).

### Synthesis of compound 49

Compound **49-a** (320 mg, 0.346 mmol) was dissolved in dichloromethane (20.0 mL) and trifluoroacetic acid (20 mL) was added. The reaction solution was stirred at room temperature for 12 hours and concentrated under reduced pressure, and the residue was directly separated by pre-HPLC to obtain **49** (52 mg, yield: 19.86%). LC-MS (ESI): m/z = 757.5(M+H)⁺.

### Embodiment 50

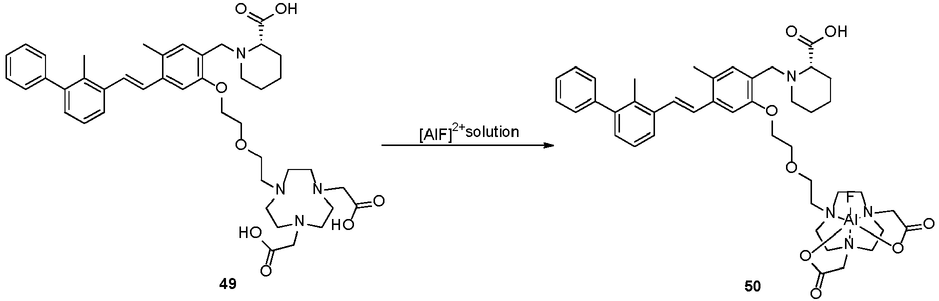

### Synthesis of compound 50

### 1. Preparation of [AlF]²⁺ solution

AlCl₃ (100 mg) was dissolved in 0.2 M AcONa/AcoH buffer (50 mL), and the solution was shaken well and set aside. NaF (50 mg) and 0.2 M AcONa/AcoH buffer (40 mL) were added to a plastic (centrifuge) tube with cover, and the solution was shaken well and set aside. The above AlCl₃ solution (10.0 mL) was added to the plastic (centrifuge) tube, then NaF solution (5.0 mL) was added. The mixture was shaken for 2 minutes, and set aside.

2. Compound **49** (1.0 mg, 0.13 mmol) was dissolved in anhydrous ethanol (0.6 mL), and [AlF]²⁺ solution (0.016 mmol, 1.6 mL) prepared above was added, and the reaction solution was stirred at 100 °C for 30 minutes. The reaction solution was cooled to room temperature and concentrated under reduced pressure, and the residue was directly separated by prep-HPLC to obtain **50** (6.2 mg, yield: 58.6%). LC-MS (ESI): m/z = 801.2(M+H)⁺.

### Embodiment 51

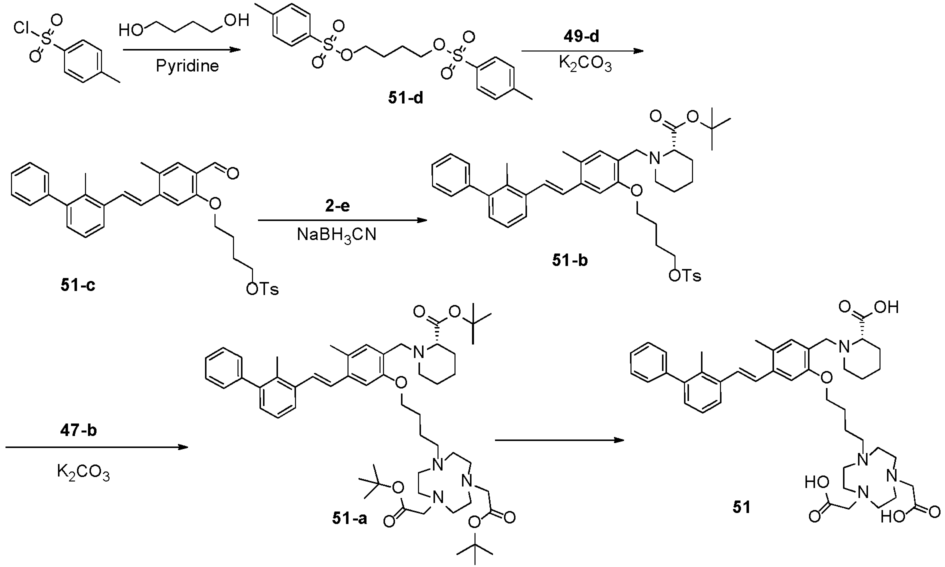

### Synthesis of compound 51-d

The compound butane-1,4-diol (901.2 mg, 10.0 mmol) was dissolved in dichloromethane (50.0 mL), and pyridine (3160.0 mg, 40.0 mmol) was added, and the reaction solution was cooled to 0°C, then 4-methylbenzenesulfonyl chloride (5719.5 mg, 30.00 mmol) was added. The reaction solution was stirred at room temperature overnight, concentrated under reduced pressure, and the residue was dissolved with ethyl acetate (100 mL), and the mixture was washed with water (50.0 mL×2), dried over anhydrous sodium sulfate, filtered, and concentrated under reduced pressure, and the residue was purified by column chromatography (petroleum ether: ethyl acetate=15:1) to obtain **51-d** (670 mg, yield: 16.8%). LC-MS (ESI): m/z = 329.1(M+Na)⁺.

### Synthesis of compound 51-c

Compound **49-d** (656.8 mg, 2.0 mmol) and compound **51-d** (1594.0 mg, 4.0 mmol) were dissolved in *N, N-*dimethylformamide (50.0 mL), and potassium carbonate (1382.0 mg, 10.0 mmol) was added. The reaction solution was heated and stirred at 50°C for 6 hours, and the reaction solution was cooled to room temperature. The reaction solution was concentrated under reduced pressure, and the residue was dissolved in dichloromethane (100 mL), and the mixture was washed sequentially with water (50 ml×2) and saturated brine (50 ml), dried over anhydrous sodium sulfate, filtered, and concentrated under reduced pressure, and the residue was purified by column chromatography (petroleum ether: ethyl acetate = 3:1) to obtain **51-c** (610 mg, yield: 54.98%). LC-MS (ESI): m/z = 555.2(M+H)⁺.

### Synthesis of compound 51-b

Compound **51-c** (554.7 mg, 1.0 mmol) and compound **2-e** (555.8 mg, 3.0 mmol) were dissolved in methanol (40 mL), and sodium cyanoborohydride (126.0 mg, 2.0 mmol) and acetic acid (120.0 mg, 2.0 mmol) were added. The reaction solution was stirred at 60 °C for 6 hours. The reaction solution was cooled to room temperature and concentrated under reduced pressure, and the residue was separated by column chromatography (petroleum ether: ethyl acetate = 10:1) to obtain **51-b** (370 mg, yield: 51.1%). LC-MS (ESI): m/z = 724.6(M+H)⁺.

### Synthesis of compound 51-a

Compound **57-b** (370 mg, 0.511 mmol) and compound **47-b** (365.4 mg, 1.02 mmol) were dissolved in acetonitrile (30.0 mL), and potassium carbonate (282.1 mg, 2.04 mmol) was added. The reaction solution was heated and stirred at 60°C for 12 hours, and the reaction solution was cooled to room temperature and concentrated under reduced pressure. The residue was dissolved in dichloromethane (100 mL), and the mixture was washed sequentially with water (50 ml×2) and saturated brine (50 ml), dried over anhydrous sodium sulfate, filtered, and concentrated under reduced pressure, and the residue was purified by column chromatography (dichloromethane: methanol = 10:1) to obtain **51-a** (350 mg, yield: 75.32%). LC-MS (ESI): m/z = 926.2(M+H)⁺.

### Synthesis of compound 51

Compound **51-a** (350 mg, 0.385 mmol) was dissolved in dichloromethane (20.0 mL), and trifluoroacetic acid (20 mL) was added, and the reaction solution was stirred at room temperature for 12 hours and concentrated under reduced pressure, and the residue was directly separated by pre-HPLC to obtain **51** (158 mg, yield: 55.4%). LC-MS (ESI): m/z = 741.5(M+H)⁺.

### Embodiment 52

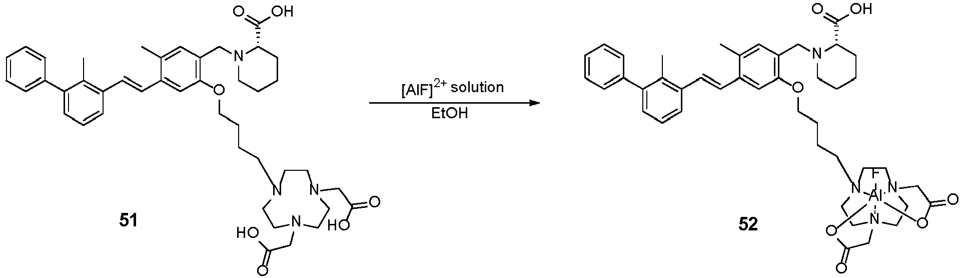

### Synthesis of compound 52

Compound **51** (20 mmol, 0.027 mg) was dissolved in anhydrous ethanol (0.5 mL), and the [AlF]²⁺ solution (0.016 mmol, 1.6 mL) prepared in Embodiment 50 was added, and the reaction solution was stirred at 100°C for 30 minutes. The reaction solution was cooled to room temperature, and directly separated by prep-HPLC to obtain **52** (15 mg, yield: 70.79%). LC-MS (ESI): m/z = 785.2(M+H)⁺.

### Embodiment 53

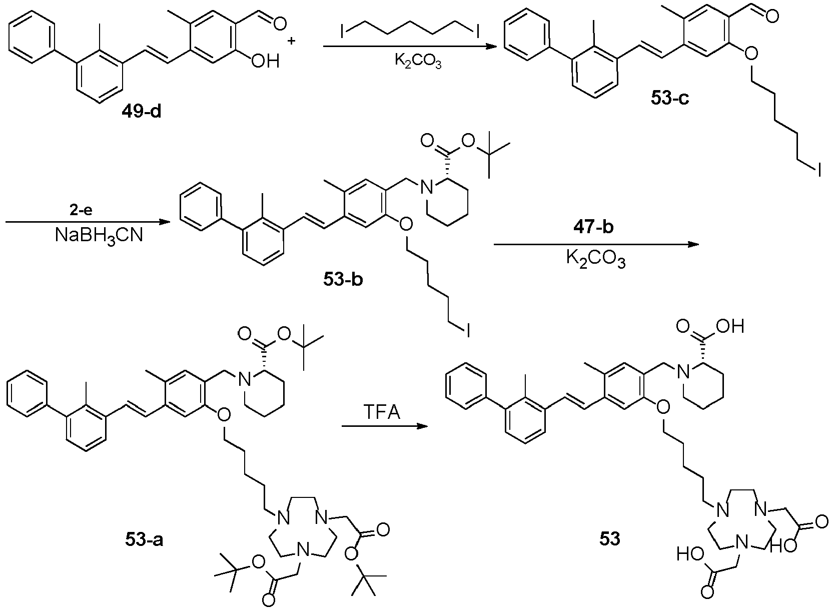

### Synthesis of compound 53-c

Compound **49-d** (328.4 mg, 1.0 mmol) and 1,5-diiodopentane (647.8 mg, 2.0 mmol) were dissolved in *N, N*-dimethylformamide (20.0 mL), and potassium carbonate (414.6 mg, 3.0 mmol) was added. The reaction solution was stirred at room temperature for 12 hours, and water (60 mL) was added, and the mixture was extracted with dichloromethane (150 mL). The organic phases were combined, washed sequentially with water (50 ml × 2) and saturated brine (50 ml), dried over anhydrous sodium sulfate, filtered, and concentrated under reduced pressure, and the residue was purified by column chromatography (petroleum ether: ethyl acetate=10:1) to obtain **53-c** (55 mg, yield: 9.96%). LC-MS (ESI): m/z = 525.2 (M+H)⁺.

### Synthesis of compound 53-b

Compound **53-c** (340 mg, 0.648 mmol) and compound **2-e** (240.23 mg, 1.297 mmol) were dissolved in methanol (40 mL), and sodium cyanoborohydride (81.69 mg, 1.29 mmol) and acetic acid (77.8 mg, 1.29 mmol) were added. The reaction solution was heated and stirred at 60°C for 5 hours, and the reaction solution was cooled to room temperature and concentrated under reduced pressure, and the residue was separated by column chromatography (petroleum ether: ethyl acetate = 10:1) to obtain **53-b** ( 158 mg, yield: 55.4%). LC-MS (ESI): m/z =694.8 (M+H)⁺.

### Synthesis of compound 53-a

Compound **53-b** (230 mg, 0.332 mmol) and compound **47-b** (237.0 mg, 0.663 mmol) were dissolved in acetonitrile (30.0 mL), and potassium carbonate (137.2 mg, 0.995 mol) was added. The reaction solution was heated and stirred at 65°C for 12 hours, and the reaction solution was cooled to room temperature, concentrated under reduced pressure, and the residue was dissolved in dichloromethane (100 mL). The mixture was washed sequentially with water (50 ml×2) and saturated brine (50 ml), dried over anhydrous sodium sulfate, filtered, and concentrated under reduced pressure, and the residue was purified by column chromatography (petroleum ether: ethyl acetate = 10:1) to obtain **53-a** (230 mg, yield: 75.13%). LC-MS (ESI): m/z =923.7 (M+H)⁺.

### Synthesis of compound 53

Compound **53-a** (230 mg, 0.249 mmol) was dissolved in dichloromethane (20.0 mL), and trifluoroacetic acid (10.0 mL) was added. The reaction solution was stirred at room temperature for 18 hours, and the reaction solution was concentrated under reduced pressure, and the residue was directly separated by pre-HPLC to obtain **53** (110.3 mg, yield: 58.65%). LC-MS (ESI): m/z =755.7 (M+H)⁺.

### Embodiment 54

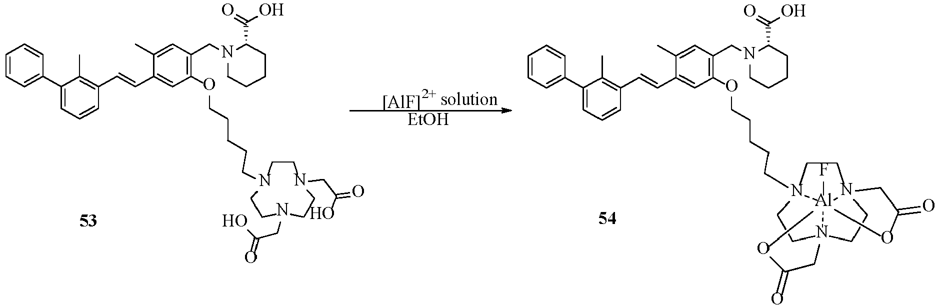

### Synthesis of compound 54

Compound **53** (0.033 mmol, 25.0 mg) was dissolved in anhydrous ethanol (0.5 mL), and the [AlF]²⁺ solution (0.016 mmol, 1.6 mL) prepared in Embodiment 50 was added, and the reaction solution was heated and stirred at 100°C for 30 minutes. The reaction solution was cooled to room temperature, and directly separated by prep-HPLC to obtain **54** (14.1 mg, yield: 53.29%). LC-MS (ESI): m/z =800.6 (M+H)⁺.

### Embodiment 55

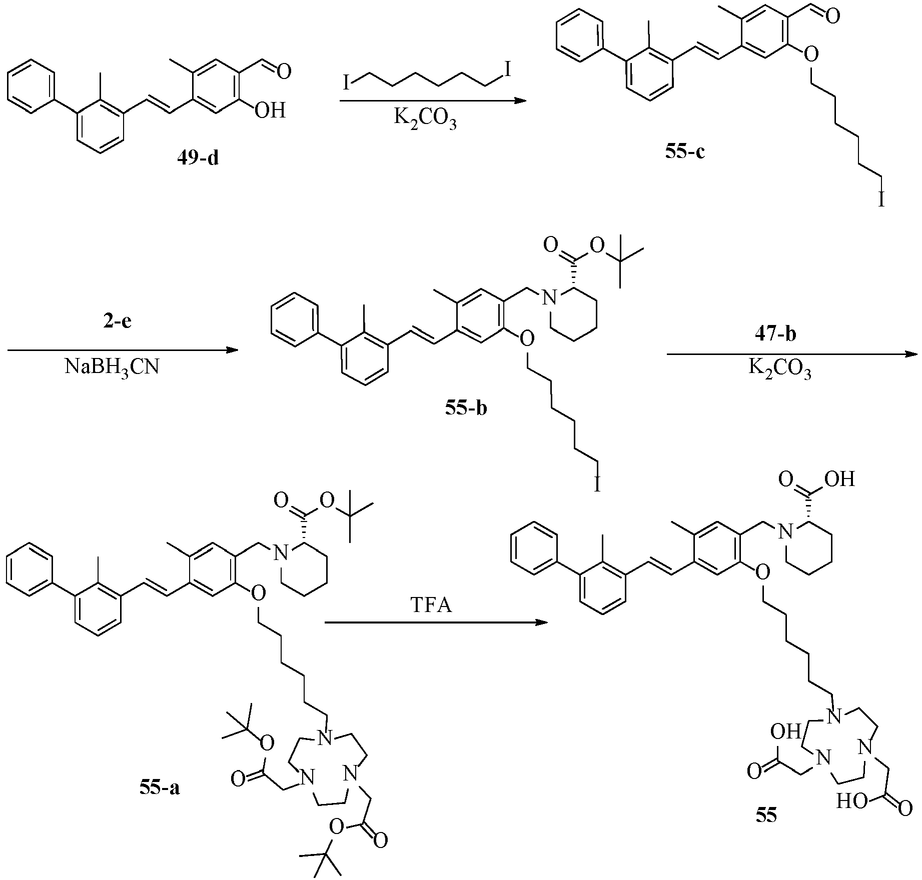

### Synthesis of compound 55-c

Compound **49-d** (328.41 mg, 1.0 mmol) and 1,6-diiodohexane (675.94 mg, 2.0 mmol) were dissolved in *N, N*-dimethylformamide (30.0 mL), and potassium carbonate (414.60 mg, 3.0 mmol) was added. The reaction solution was stirred at room temperature for 12 hours and concentrated under reduced pressure, and the residue was dissolved in dichloromethane (100 mL). The mixture was washed sequentially with water (50 ml × 2) and saturated brine (50 ml), dried over anhydrous sodium sulfate, filtered, and concentrated under reduced pressure, and the residue was purified by column chromatography (petroleum ether: ethyl acetate = 10:1) to obtain **55-c** (380 mg, yield: 67.04%). LC-MS (ESI): m/z =539.2 (M+H)⁺.

### Synthesis of compound 55-b

Compound **55-c** (340 mg, 0.631 mmol) and compound **2-e** (240.0 mg, 1.263 mmol) were dissolved in methanol (40 mL), and sodium cyanoborohydride (79.56 mg, 1.26 mmol) and acetic acid (75.8 mg, 1.26 mmol) were added. The reaction solution was heated and stirred at 60°C for 5 hours, and the reaction solution was cooled to room temperature and concentrated under reduced pressure, and the residue was purified by column chromatography (petroleum ether: ethyl acetate = 10:1) to obtain **55-b** (230 mg, yield: 51.47%). LC-MS (ESI): m/z =709.8 (M+H)⁺.

### Synthesis of compound 55-a

Compound **55-b** (380 mg, 0.586 mmol) and compound **47-b** (418.8 mg, 1.172 mmol) were dissolved in acetonitrile (30.0 mL), and potassium carbonate (323.36 mg, 2.343 mmol) was added, and the reaction solution was heated and stirred at 60°C for 12 hours. The reaction solution was cooled to room temperature and concentrated under reduced pressure, and the residue was dissolved in dichloromethane (100 mL). The mixture was washed sequentially with water (50 ml×2) and saturated brine (50 ml×1), dried over anhydrous sodium sulfate, filtered, and concentrated under reduced pressure, and the residue was purified by column chromatography (petroleum ether: ethyl acetate = 10:1) to obtain **55-a** (180 mg, yield: 61.78%). LC-MS (ESI): m/z =926.2 (M+H)⁺.

### Synthesis of compound 55

Compound **55-a** (220 mg, 0.235 mmol) was dissolved in dichloromethane (20.0 mL), and trifluoroacetic acid (10.0 mL) was added, and the reaction solution was stirred at room temperature for 12 hours and concentrated under reduced pressure, and the residue was directly separated by pre-HPLC to obtain **55** (160 mg, yield: 88.65%). LC-MS (ESI): m/z =769.6 (M+H)⁺.

### Embodiment 56

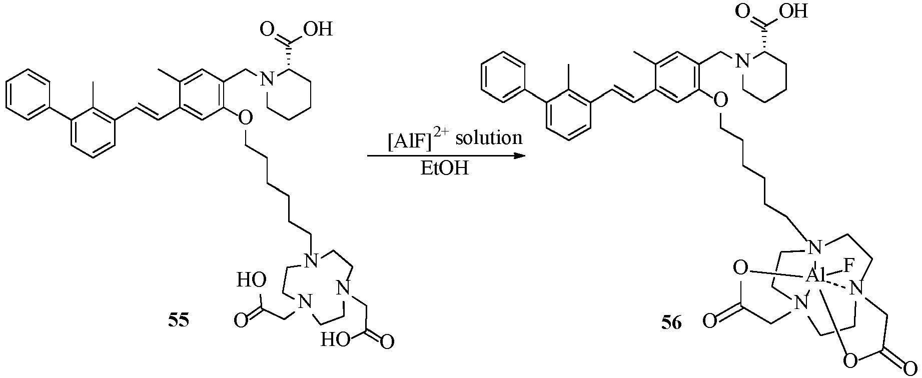

### Synthesis of compound 56

Compound **55** (0.039 mmol, 6.0 mg) was dissolved in anhydrous ethanol (1.0 mL), and the [AlF]²⁺ solution (0.05 mmol, 5.0 mL) prepared in Embodiment 50 was added, and the reaction solution was heated and stirred at 100°C for 30 minutes. The reaction solution was cooled to room temperature, and directly separated by prep-HPLC to obtain **56** (6 mg, yield: 18.92%). LC-MS (ESI): m/z =814.6 (M+H)⁺.

### Embodiment 57

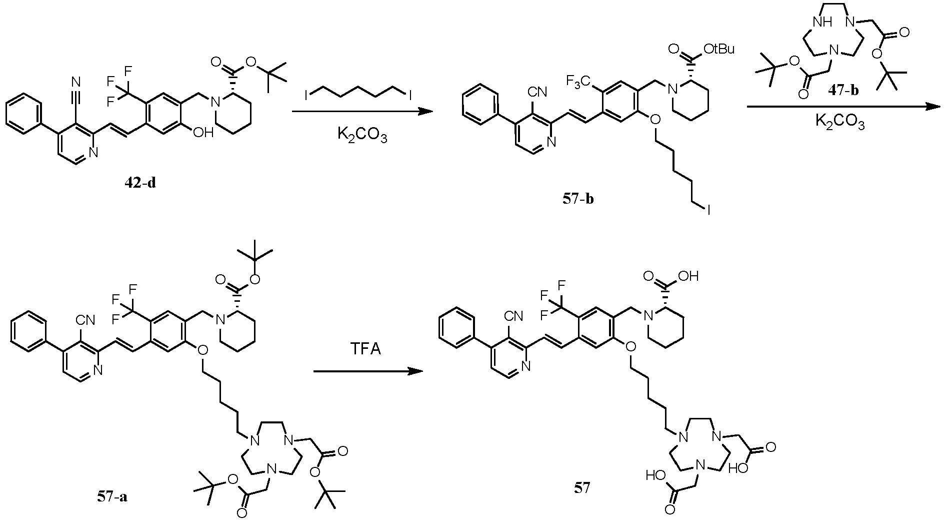

### Synthesis of compound 57-b

Compound **42-d** (195 mg, 0.346 mmol) and 1,5-diiodopentane (224.15 mg, 0.692 mmol) were dissolved in *N,* N dimethylformamide (5 mL), and potassium carbonate (239.09 mg, 1.730 mmol) was added, and the reaction solution was stirred at 60°C for 1 hour. The reaction solution was cooled to room temperature, and the reaction solution was extracted with ethyl acetate (10 mL), washed sequentially with water (10 mL×3) and saturated brine (10 mL×1), and the organic phases were combined, dried over anhydrous sodium sulfate, filtered, and concentrated under reduced pressure to obtain **57-b** (211 mg, yield: 80.49%).

### Synthesis of compound 57-a

Compound **57-b** (211 mg, 0.278 mmol) and compound **47-b** were dissolved in acetonitrile (10 mL), and potassium carbonate (76.98 mg, 0.557 mmol) was added, and the reaction solution was stirred at 60°C overnight. The reaction solution was cooled to room temperature and concentrated under reduced pressure, and the residue was dissolved with ethyl acetate (10 mL), and the mixture was washed sequentially with water (10 mL×3) and saturated brine (10 mL×1). The organic phases were combined, dried over anhydrous sodium sulfate, filtered, and concentrated under reduced pressure to obtain **57-a** (242 mg, yield: 87.85%).

### Synthesis of compound 57

Compound **57-a** (242 mg, 0.245 mmol) was dissolved in dichloromethane (9 mL), and trifluoroacetic acid (9 mL, 1.223 mmol) was added. The reaction solution was stirred at room temperature for 30 minutes, and the reaction solution was concentrated under reduced pressure, and the residue was separated by prep-HPLC to obtain **57** (93.61 mg, yield: 46.61%).

### Embodiment 58

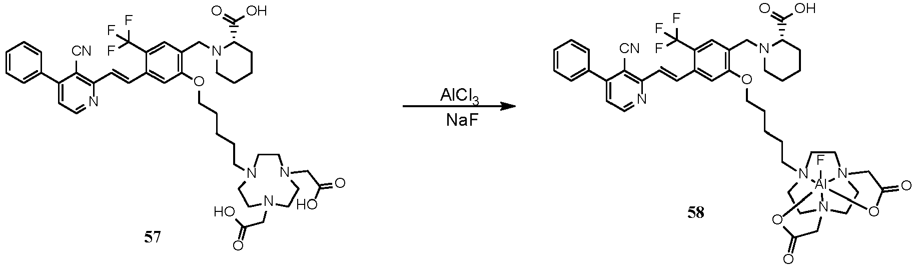

### Synthesis of compound 58

Compound **57** (20 mg, 0.024 mmol) was dissolved in ethanol (0.50 mL), and 0.2 M sodium acetate-acetic acid buffer (pH=4.6) (1.0 mL), aluminum chloride (2 mg, 0.029 mmol), and sodium fluoride (1 mg, 0.029 mmol) were added, and the reaction solution was stirred at 100°C for 30 minutes. The reaction solution was cooled to room temperature and concentrated under reduced pressure, and the residue was separated by prep-HPLC to obtain **58** (11.68 mg, yield: 55.43%).

### Embodiment 59

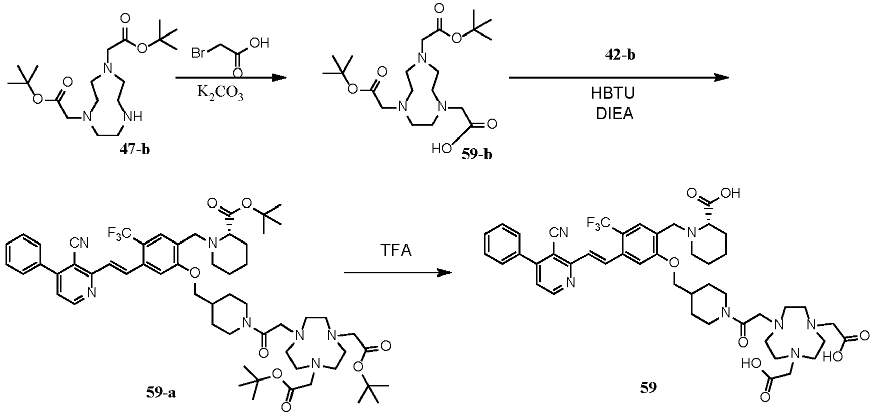

### Synthesis of compound 59-b

Compound **47-b** (715.00 mg, 2.0 mmol) and potassium carbonate (552.84 mg, 4.00 mmol) were dissolved in methanol (10 mL) and water (10 mL), and bromoacetic acid (383.48 mg, 2.76 mmol) was added. The reaction solution was stirred at room temperature overnight, and the pH was adjusted to 6 with hydrochloric acid (1 mol/L), and the reaction solution was extracted with dichloromethane (50 mL×2). The organic phases were combined, dried over anhydrous sodium sulfate, filtered, and concentrated under reduced pressure to obtain **59-b** (610 mg, yield: 73.40%).

### Synthesis of compound 59-a

Compound **42-b** (115 mg, 0.174 mmol) was dissolved in *N, N*-dimethylformamide (5 mL), and **59-b** (86.78 mg, 0.209 mmol), O-benzotriazole-tetramethyluronium hexafluorophosphate (85.80 mg, 0.226 mmol), and diisopropylethylamine (0.058 mL, 0.348 mmol) were added, and the reaction solution was stirred at room temperature for 1 hour. The reaction solution was extracted with ethyl acetate (10 mL), washed sequentially with water (10 mL×3) and saturated brine (10 mL×1), and the organic phases were combined, dried over anhydrous sodium sulfate, filtered, and concentrated under reduced pressure to obtain **59-a** (45.66 mg, yield: 24.79%).

### Synthesis of compound 59

Compound **59-a** (45.66 mg, 0.043 mmol) was dissolved in dichloromethane (9 mL), and trifluoroacetic acid (9 mL, 1.223 mmol) was added, and the reaction solution was stirred at room temperature for 30 minutes. The reaction solution was concentrated under reduced pressure, and directly separated by pre-HPLC to obtain **59** (22.57 mg, yield: 58.78%). LC-MS (ESI): m/z =890 (M+H)⁺.

### Embodiment 60

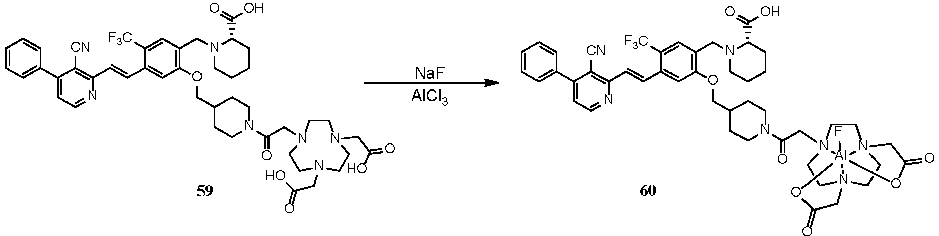

### Synthesis of compound 60

Compound **59** (5 mg, 0.006 mmol) was dissolved in 0.2 M sodium acetate-acetic acid buffer (pH=4.6) (1.0 mL), and aluminum chloride (AlCl₃, 0.6 mL, 0.002 mmol) and sodium fluoride (0.3 mL, 0.002 mmol) were added, and the reaction solution was stirred at 100°C for 30 minutes. The reaction solution was cooled to room temperature, and separated directly by prer-HPLC to obtain **60** (1.96 mg, yield: 37.33%). LC-MS (ESI): m/z =934 (M+H)⁺.

### Embodiment 61

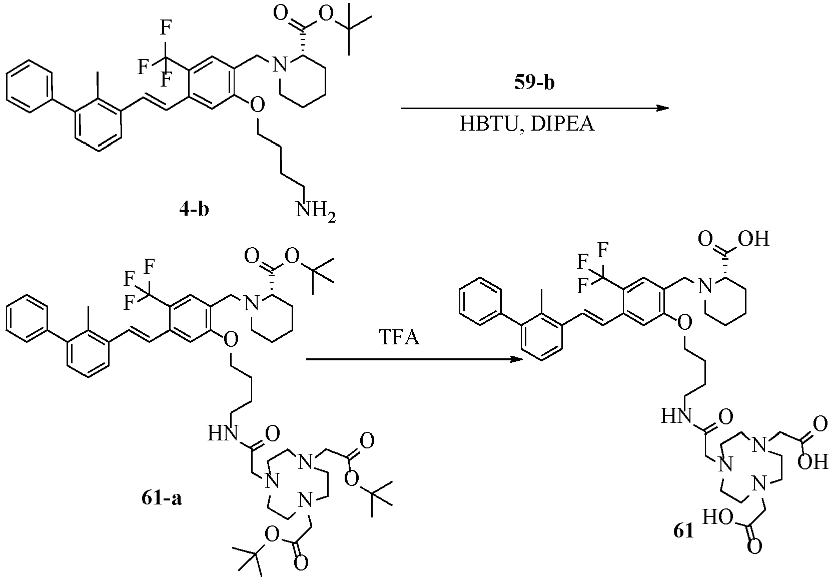

### Synthesis of compound 61-a

Compound **4-b** (124.5 mg, 0.20 mmol) and compound **59-b** (108.0 mg, 0.26 mmol) were dissolved in *N, N*-dimethylformamide (15.0 mL), and O-benzotriazole-tetramethyluronium hexafluorophosphate (113.7 mg, 0.30 mmol) and diisopropylethylamine (77.4 mg, 0.60 mmol) were added, and the reaction solution was stirred at room temperature for 18 hours, diluted with dichloromethane (100 mL), washed sequentially with water (50 ml×3) and saturated brine (50 ml×1), dried over anhydrous sodium sulfate, filtered, and concentrated under reduced pressure, and the residue was purified by column chromatography (dichloromethane: methanol=10:1) to obtain **61-a** (60 mg, yield: 29.81%). LC-MS (ESI): m/z =1020.7 (M+H)⁺.

### Synthesis of compound 61

Compound **61-a** (60 mg, 0.059 mmol) was dissolved in dichloromethane (12.0 mL), and trifluoroacetic acid (6.0 mL) was added, and the reaction solution was stirred at room temperature for 6 hours and concentrated under reduced pressure, and the residue was directly separated by pre-HPLC to obtain **61** (15 mg, yield: 29.94%). LC-MS (ESI): m/z =852.8 (M+H)⁺.

### Embodiment 62

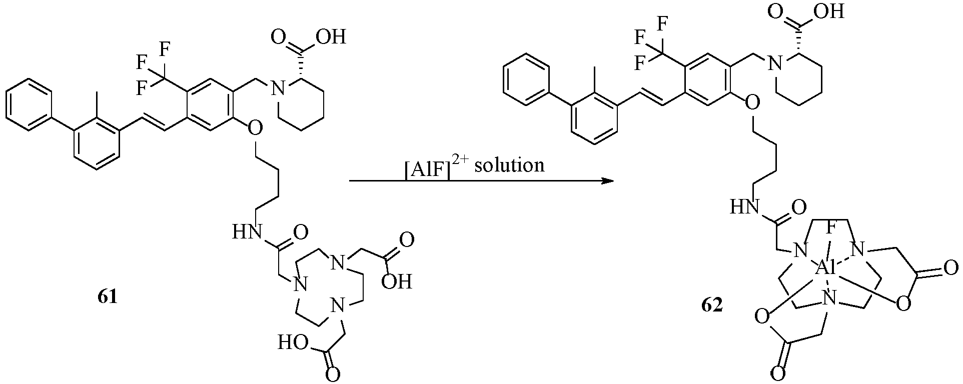

### Synthesis of compound 62

Compound **61** (0.008 mmol, 7.0 mg) was dissolved in anhydrous ethanol (1.0 mL), and the [AlF]²⁺ solution (0.016 mmol, 1.6 mL) prepared in Embodiment 50 was added, and the reaction solution was heated and stirred at 100°C for 2 hours. The reaction solution was cooled to room temperature, and directly separated by prep-HPLC to obtain **62** (1.6 mg, yield: 21.74%). LC-MS (ESI): m/z =897.4 (M+H)⁺.

### Embodiment 63

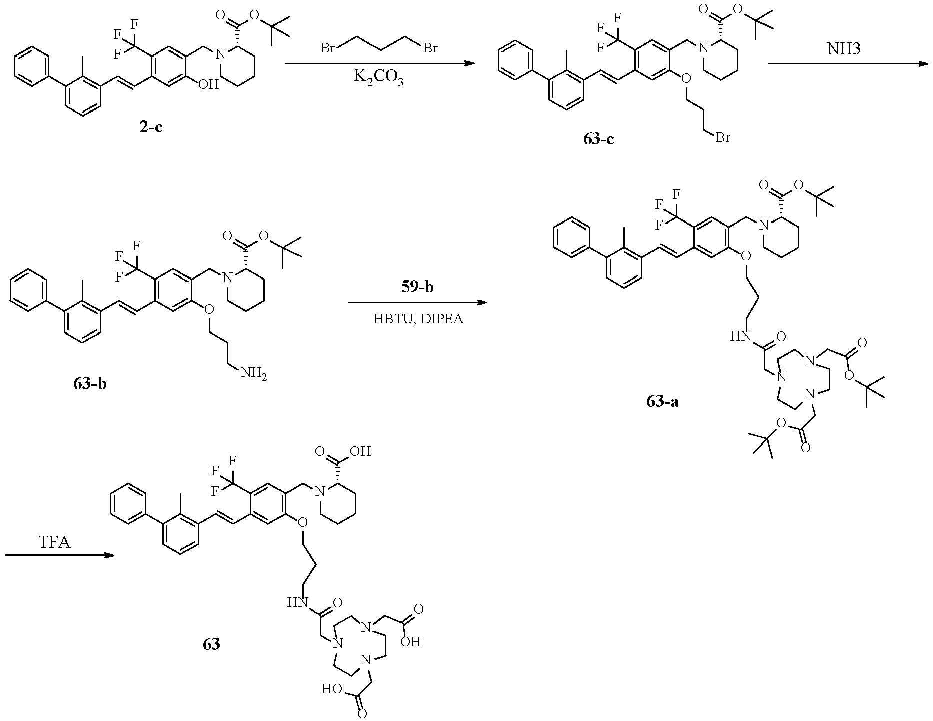

### Synthesis of compound 63-c

Compound **2-c** (551.65 mg, 1.0 mmol) and 1,3-dibromopropane (0.305 mL, 3.00 mmol) were dissolved in DMF (5.0 mL), and potassium carbonate (276.42 mg, 2.00 mmol) was added, and the reaction solution was heated and stirred at 50°C for 7 hours. The reaction solution was cooled to room temperature, diluted with ethyl acetate (50 mL), and washed sequentially with water (20 mL×2) and saturated brine (20 mL×1). The organic phases were combined, dried over anhydrous sodium sulfate, filtered, and concentrated under reduced pressure, and the residue was separated by column chromatography (petroleum ether: ethyl acetate=20: 1) to obtain **63-c** (461 mg, yield: 68.54%).

### Synthesis of compound 63-b

Compound **63-c** (461 mg, 0.807 mmol) and ammonia methanol solution (7 M) (23 mL) were sealed in a microwave tube, and the reaction solution was heated and stirred at 50°C for 60 hours. The reaction solution was cooled to room temperature and concentrated under reduced pressure, and the residue was dissolved in dichloromethane (50 mL) and washed with 10% K₂CO₃ solution (10 mL). The organic phase was separated, dried over anhydrous sodium sulfate, filtered, and concentrated under reduced pressure to obtain **63-b** (409 mg, yield: 99.89%), which was directly used in the next step of reaction without purification.

### Synthesis of compound 63-a

Compound **63-b** (121.7 mg, 0.20 mmol) and compound **59-b** (108.0 mg, 0.26 mmol) were dissolved in *N, N*-dimethylformamide (15.0 mL), and O-benzotriazole-tetramethyluronium hexafluorophosphate (113.7 mg, 0.30 mmol) and diisopropylethylamine (77.4 mg, 0.60 mmol) were added, and the reaction solution was stirred at room temperature for 18 hours, diluted with dichloromethane (100 mL), washed sequentially with water (50 ml×3) and saturated brine (50 ml×1), dried over anhydrous sodium sulfate, filtered, and concentrated under reduced pressure, and the residue was separated by column chromatography (dichloromethane: methanol=10:1) to obtain **63-a** (90 mg, yield: 44.72%). LC-MS (ESI): m/z =1006.7 (M+H)⁺.

### Synthesis of compound 63

Compound **63-a** (90 mg, 0.089 mmol) was dissolved in dichloromethane (20.0 mL), and trifluoroacetic acid (10.0 mL) was added, and the reaction solution was stirred at room temperature for 12 hours and concentrated under reduced pressure, and the residue was directly separated by pre-HPLC to obtain **63** (25 mg, yield: 33.36%). LC-MS (ESI): m/z =838.4 (M+H)⁺.

### Embodiment 64

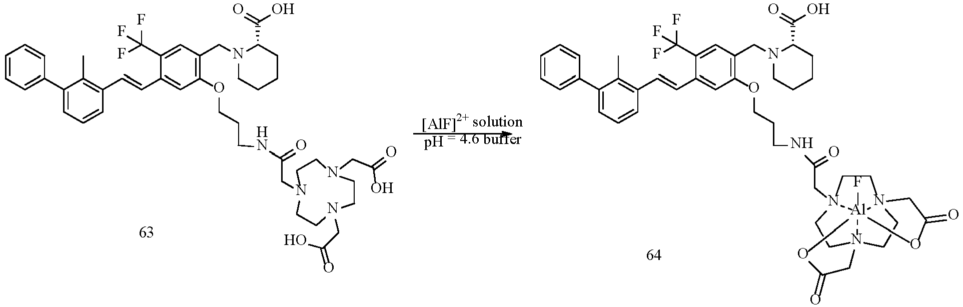

### Synthesis of compound 64

Compound **63** (0.008 mmol, 7.0 mg) was dissolved in anhydrous ethanol (1.0 mL), and the [AlF]²⁺ solution (0.016 mmol, 1.6 mL) prepared in Embodiment 50 was added, and the reaction solution was heated and stirred at 100°C for 2 hours. The reaction solution was cooled to room temperature, and directly separated by prep-HPLC to obtain **64** (6.67 mg, yield: 70.42%). LC-MS (ESI): m/z =883.4 (M+H)⁺.

### Embodiment 65

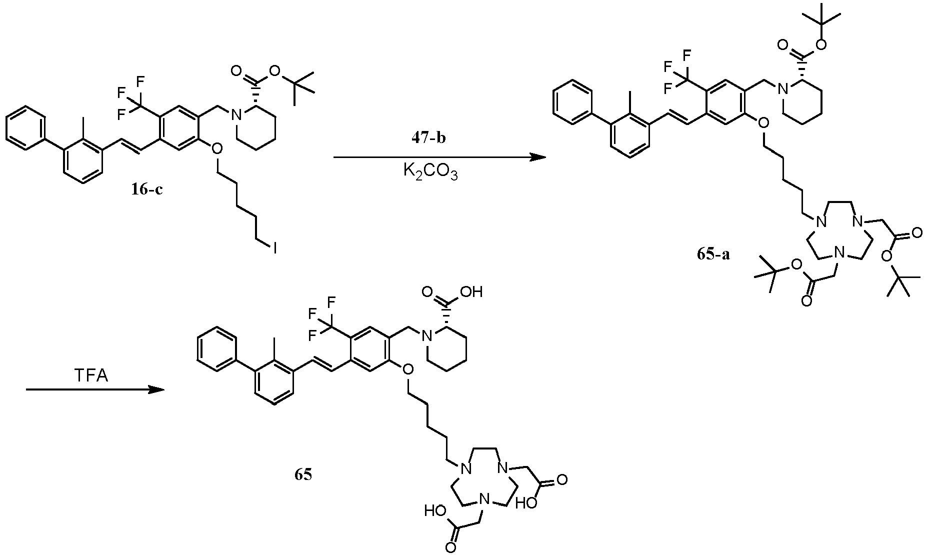

### Synthesis of compound 65-a

Compound **16-c** (252 mg, 0.337 mmol) and compound **47-b** (156.64 mg, 0.438 mmol) were dissolved in acetonitrile (3.0 mL), and potassium carbonate (72 mg, 0.521 mmol) was added, and the reaction solution was stirred at room temperature overnight. The reaction solution was concentrated under reduced pressure, and the residue was separated by silica gel column chromatography (dichloromethane: methanol = 10: 1) to obtain **65-a** (190 mg, yield: 57.68%).

### Synthesis of compound 65

Compound **65-a** (190 mg, 0.194 mmol) was dissolved in dichloromethane (3.0 mL), and trifluoroacetic acid (3.0 mL) was added. The reaction solution was stirred at room temperature overnight, and the reaction solution was concentrated under reduced pressure, and directly separated by pre-HPLC to obtain **65** (74 mg, yield: 47.05%). LC-MS (ESI): m/z =809.5(M+H)⁺.

¹H NMR (400 MHz, MeOD) δ 7.90 (s, 1H), 7.62 (d, *J* = 15.9 Hz, 1H), 7.57 - 7.49 (m, 2H), 7.46 - 7.38 (m, 2H), 7.38 - 7.23 (m, 5H), 7.18 (d, *J* = 7.5 Hz, 1H), 4.59 (d, *J* = 13.0 Hz, 1H), 4.42 (d, *J* = 13.0 Hz, 1H), 4.32 (t, *J* = 6.3 Hz, 2H), 3.59 (dd, *J* = 9.9, 3.6 Hz, 1H), 3.56 - 3.35 (m, 5H), 3.24 - 2.78 (m, 15H), 2.30 (s, 3H), 2.28 - 2.16 (m, 1H), 2.06 - 1.67 (m, 8H), 1.67 - 1.51 (m, 3H).

### Embodiment 66

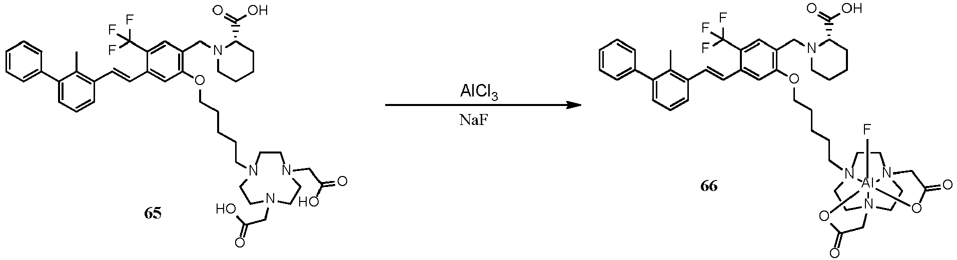

### Synthesis of compound 66

Aluminum chloride (1.60 mg, 0.012 mmol) was dissolved in 0.2 M sodium acetate-acetic acid buffer (pH=4.6) (0.8 mL), and sodium fluoride (0.50 mg, 0.012 mmol) was added, and the reaction solution was stirred at room temperature for 5 minutes. Ethanol (0.60 mL) and compound **65** (8.09 mg, 0.01 mmol) were added, and the reaction solution was heated at 100°C for 30 minutes. The reaction solution was cooled to room temperature, and directly purified by Pre-HPLC to obtain **66** (4.6 mg, yield: 53.93%).

### Embodiment 67

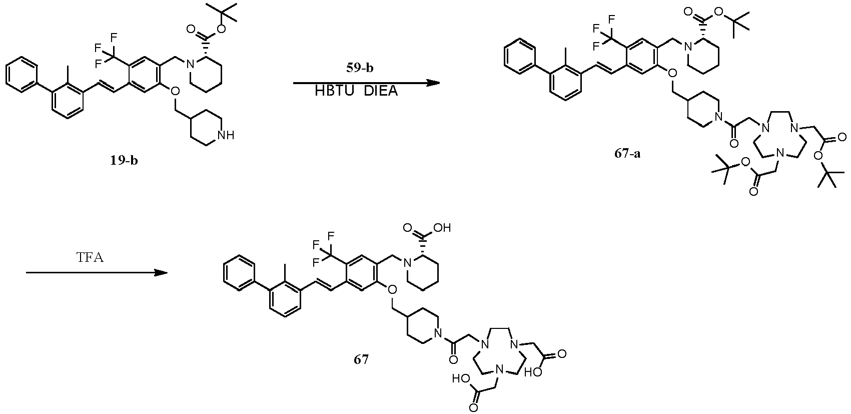

### Synthesis of compound 67-a

Compound **19-b** (64.88 mg, 0.1 mmol), compound **59-b** (54.02 mg, 0.130 mmol), and O-benzotriazole-tetramethyluronium hexafluorophosphate (75.85 mg, 0.200 mmol) were dissolved in *N, N*-dimethylformamide (1.0 mL), and diisopropylethylamine (0.050 mL, 0.30 mmol) was added, and the reaction solution was stirred at room temperature for 1 hour. The reaction solution was directly purified by Pre-HPLC to obtain **67-a** (58 mg, yield: 55.43%).

### Synthesis of compound 67

Compound **67-a** (58 mg, 0.055 mmol) was dissolved in dichloromethane (2 mL), and trifluoroacetic acid (2 mL) was added, and the reaction solution was stirred at room temperature for 24 hours. The reaction solution was directly purified by Pre-HPLC to obtain **67** (25 mg, yield: 41.10%).

¹H NMR (400 MHz, MeOD) δ: 7.94 (s, 1H), 7.62 (d, *J* = 15.8Hz, 1H), 7.54 (t, *J*= 3.4 Hz, 2H), 7.47-7.39 (m, 2H), 7.39-7.23 (m, 5H), 7.18 (d, *J*= 7.5 Hz, 1H), 4.63-4.49 (m, 2H), 4.40 (d, *J*= 13.3 Hz, 1H), 4.16 (d, *J*= 6.3 Hz, 2H), 3.95 (d, *J*= 12.7 Hz, 1H), 3.84-3.53 (m, 7H), 3.44-3.33 (m, 1H), 3.26-2.61 (m, 15H), 2.30 (s, 3H), 2.29-2.17 (m, 2H), 2.06-1.49 (m, 7H), 1.49-1.23 (m, 2H).

### Embodiment 68

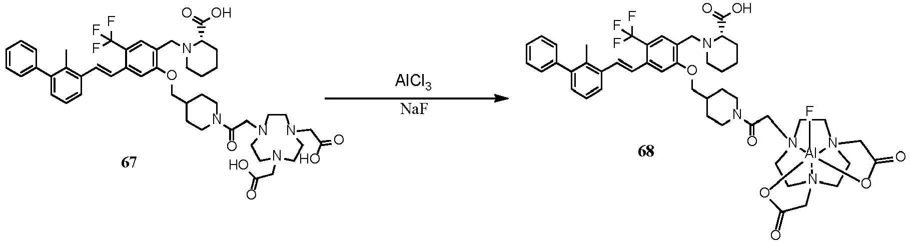

### Synthesis of compound 68

Aluminum chloride (1.60 mg, 0.012 mmol) was dissolved in 0.2 M sodium acetate-acetic acid buffer (pH=4.6) (0.8 mL), and sodium fluoride (0.50 mg, 0.012 mmol) was added, and the reaction solution was stirred at room temperature for 5 minutes. Ethanol (0.60 mL) and compound **67** (8.78 mg, 0.01 mmol) were added, and the reaction solution was heated at 100°C for 30 minutes. The reaction solution was cooled to room temperature, and directly purified by Pre-HPLC to obtain **68** (4.74 mg, yield: 51.41%).

### Embodiment 69

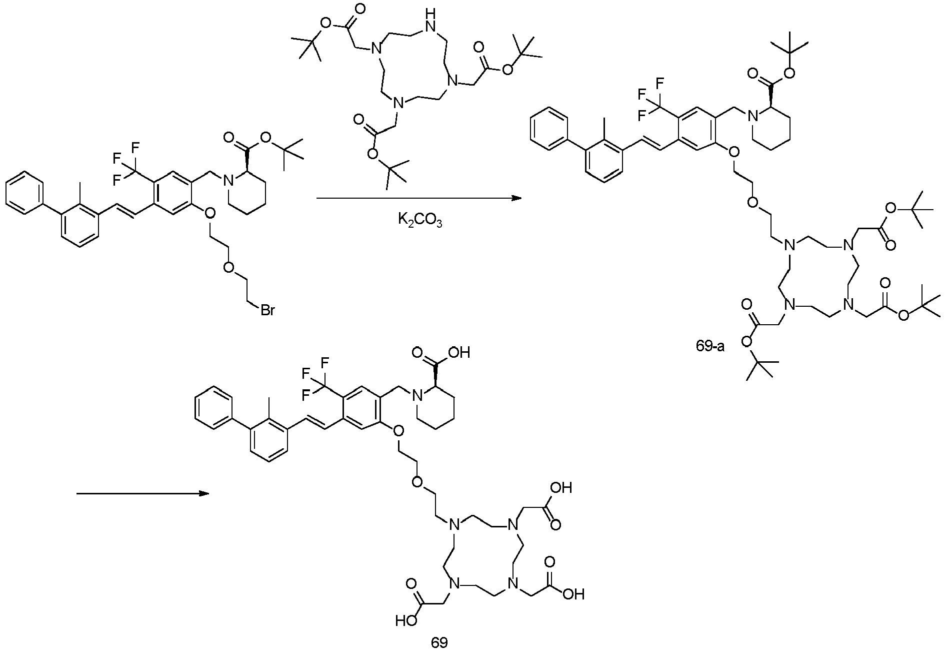

### Synthesis of compound 69-a

2-methylpropan-2-yl (2*R*)-1-{[2-({2-[(2-bromoethyl)oxy]ethyl}oxy)-4-[(1*E*)-2-(2-methyl-3-phenylphenyl)vinyl]-5-(trifluoromethyl)phenyl]methyl}hexahydropyridine-2-carboxylate (140.53 mg, 0.2 mmol) and compound *tert*-butyl 2,2',2"-(1,4,7,10-tetraazacyclododecan-1,4,7-triyl)triacetate (154.41 mg, 0.300 mmol) were dissolved in acetonitrile (5.0 mL), and potassium carbonate (55.28 mg, 0.400 mmol) was added. The reactant was heated and stirred at 60 °C for 16 hours. The reaction solution was cooled to room temperature, filtered, and concentrated under reduced pressure to obtain **69-a** (226 mg, yield: 99.43%), which was directly used in the next step of reaction without purification.

### Synthesis of compound 69

Compound **69-a** (226 mg, 0.199 mmol) was dissolved in dichloromethane (3.0 mL), and trifluoroacetic acid (3.0 mL) was added, and the reaction solution was stirred at room temperature overnight, and directly separated by Pre-HPLC to obtain **69** (127 mg, yield: 70.02%).

### Embodiment 70

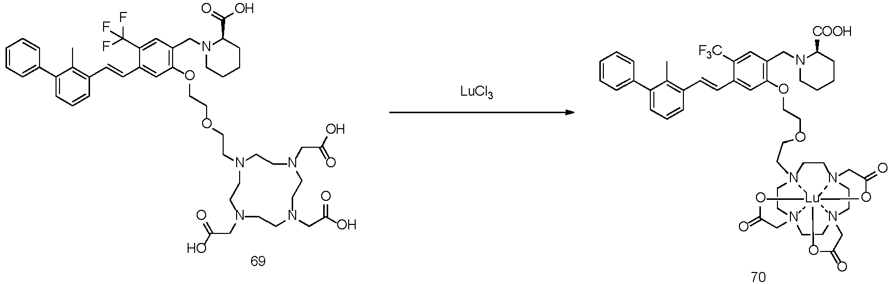

### Synthesis of compound 70

Compound **69** (13.68 mg, 0.015 mmol) and lutetium trichloride (8.44 mg, 0.030 mmol) were dissolved in 0.2 M AcONa/AcOH buffer (pH=4.5, 1.0 mL), and the reaction solution was stirred at room temperature for 1 hour. The reaction solution was directly purified by Prep-HPLC to obtain **70** (9.77 mg, yield: 60.09%). LC-MS (ESI): m/z =1084.68(M+H)⁺.

### Embodiment 71

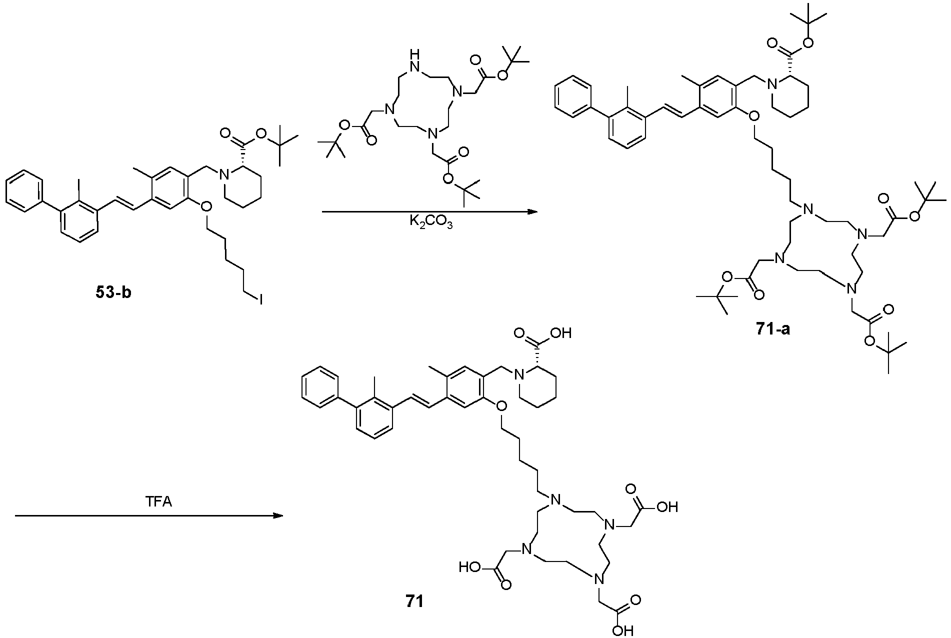

### Synthesis of compound 71-a

Compound **53-b** (180 mg, 0.259 mmol) and *tert*-butyl 2,2',2"-(1,4,7,10-tetraazacyclododecan-1,4,7-triyl) triacetate (237.0 mg, 0.519 mmol) were dissolved in acetonitrile (30.0 mL), and potassium carbonate (35.81 mg, 0.259mmol) was added. The reaction solution was heated and stirred at 60°C for 3 hours, and the reaction solution was cooled to room temperature, concentrated under reduced pressure, and the residue was dissolved in dichloromethane (100 mL). The mixture was washed sequentially with water (50 ml×2) and saturated brine (50 ml), dried over anhydrous sodium sulfate, filtered, and concentrated under reduced pressure, and the residue was purified by column chromatography (petroleum ether: ethyl acetate = 10:1) to obtain **71-a** (230 mg, yield: 75.13%). LC-MS (ESI): m/z =1080.5 (M+H)⁺.

### Synthesis of compound 71

Compound **71-a** (170 mg, 0.157 mmol) was dissolved in dichloromethane (15.0 mL), and trifluoroacetic acid (15.0 mL) was added, and the reaction solution was stirred at room temperature for 16 hours and concentrated under reduced pressure, and the residue was directly separated by pre-HPLC to obtain **71** (85.7 mg, yield: 63.6%). LC-MS (ESI): m/z = 856.0(M+H)⁺.

### Embodiment 72

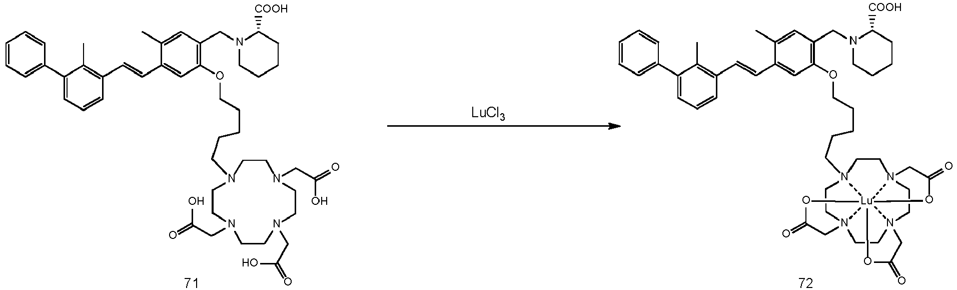

Compound **71** (13.68 mg, 0.015 mmol) and lutetium trichloride (8.44 mg, 0.030 mmol) were dissolved in 0.2 M AcONa/AcOH buffer (pH=4.5, 1.0 mL), and the reaction solution was stirred at room temperature for 1 hour. The reaction solution was directly purified by prep-HPLC to obtain **72** (9.60 mg, yield: 53.3%). LC-MS (ESI): m/z =1028.02(M+H)⁺.

### Embodiment 73

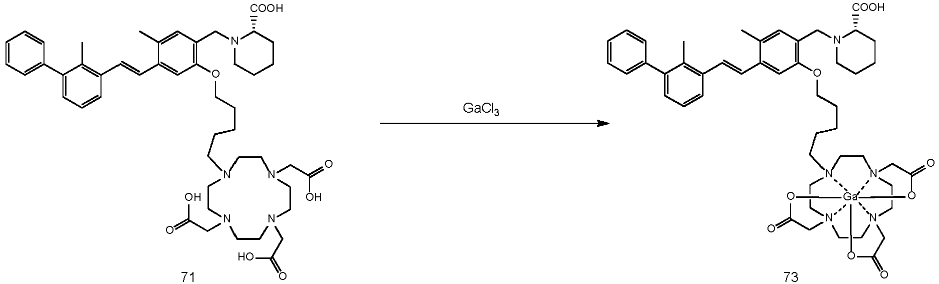

Compound **71** (11.0 mg, 0.013 mmol) and gallium trichloride (1.76 mg, 0.010 mmol) were added to water (0.2 mL), and the reaction solution was heated and stirred at 90°C for 5 minutes. The reaction solution was cooled to room temperature, and the reaction solution was directly separated by Pre-HPLC to obtain **73** (11.1 mg, yield: 92.5%). LC-MS (ESI): m/z = 922.77(M+H)⁺.

### Embodiment 74

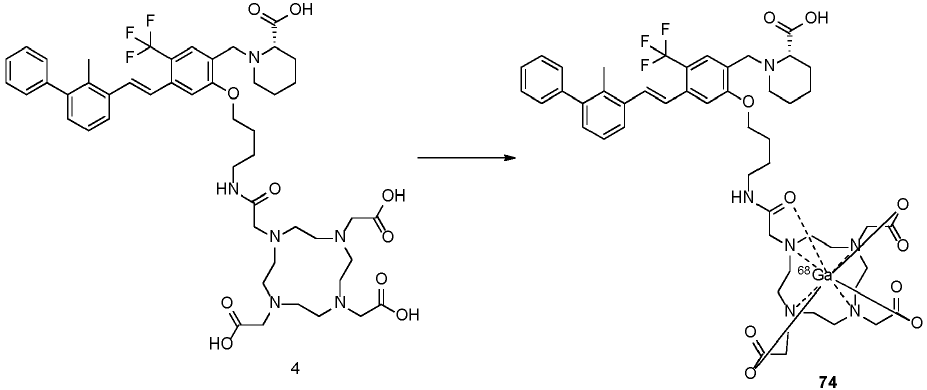

### Synthesis of compound 74

Compound **4** was dissolved in DMSO to a 10 mg/mL solution, and the solution was taken out a portion to dilute to 0.1 mg/mL with sodium acetate buffer which is metal-free and pH=5.5. The germanium-gallium generator was eluted with 5mL of 0.1M HCl in segments, and the highest activity portion (0.5mL) was taken and 0.5mL of sodium acetate buffer was added thereto, then 20-fold equivalent of compound 4 (0.1mg/mL) was added. The mixture was vortexed and mixed for 10s, and heated and shaken at 95 °C and 800rpm for 30 minutes. The C18 cartridge was activated with ethanol, then eluted clean with pure water and eluted dry, and the solution after the reaction completed was passed through the C18 cartridge, and the C18 cartridge was eluted with pure water and eluted dry and then eluted with ethanol. TLC purity detection was carried out using 1% EDTA as a developing agent, and compound 4 labeled with ⁶⁸Ga was detected by TLC scanning, and its radiochemical purity was 99.67%.

Appropriate amounts of ⁶⁸Ga-labeled compound 4 and compound 8 were taken for HPLC characterization according to the following conditions, and chromatographic conditions were as follows:
Mobile phase A: 0.1%TFA-H₂O
Mobile phase B: ACN
Chromatographic column: Waters XBridge, 19*150mm, Sum
Flow rate: 1ml/min
Method: Time: 0 min 25% B, 10 min 95% B

After HPLC characterization, the UV peak time of compound 8 and the radioactive peak time of compound 4 labeled with ⁶⁸Ga are both around 7:07 (mm:ss), indicating that the labeling of compound 4 by ⁶⁸Ga was successful (namely, compound 74), which was consistent with the resluts of TLC scanning.

### Embodiment 75

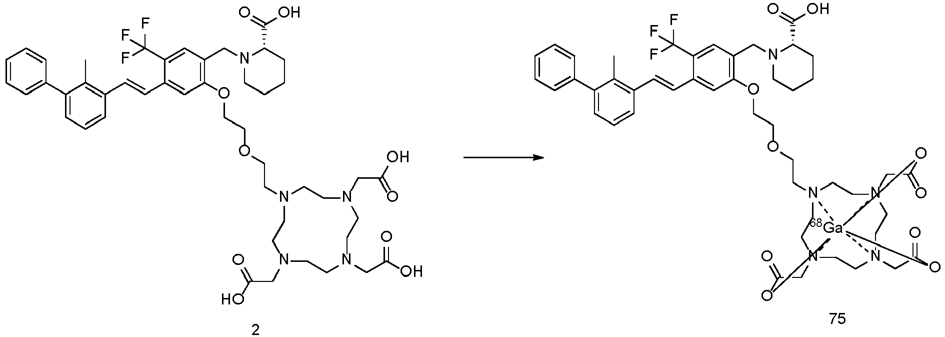

### Synthesis of compound 75

Compound 2 was dissolved in DMSO to a 10 mg/mL solution, and the solution was taken out a portion to dilute to 0.1 mg/mL with sodium acetate buffer which is metal-free and pH=5.5. The germanium-gallium generator was eluted with 5mL of 0.1M HCl in segments, and the highest activity portion (0.5mL) was taken and 0.5mL of sodium acetate buffer was added thereto, then 20-fold equivalent of compound 2 (0.1mg/mL) was added. The mixture was vortexed and mixed for 10s, and heated and shaken at 95 °C and 800rpm for 30 minutes. The C18 cartridge was activated with ethanol, then eluted clean with pure water and eluted dry, and the solution after the reaction completed was passed through the C18 cartridge, and the C18 cartridge was eluted with pure water and eluted dry, and then eluted with ethanol. The compound **2** labeled with ⁶⁸Ga was detected by TLC scanning, and its radiochemical purity was 99.74%.

Appropriate amounts of ⁶⁸Ga-labeled compound 2 and compound 7 were taken for HPLC characterization according to the following conditions, and chromatographic conditions were as follows:
Mobile phase A: 0.1%TFA-H₂O
Mobile phase B: ACN
Chromatographic column: Waters XBridge, 19*150mm, Sum
Flow rate: 1ml/min
Method: Time: 0 min 25% B, 10 min 95% B

After HPLC characterization, the UV peak time of compound 7 and the radioactive peak time of compound 2 labeled with ⁶⁸Ga are both around 7:03 (mm:ss), indicating that the labeling of compound 2 by ⁶⁸Ga was successful (namely, compound 75), which was consistent with the resluts of TLC scanning.

### Embodiment 76

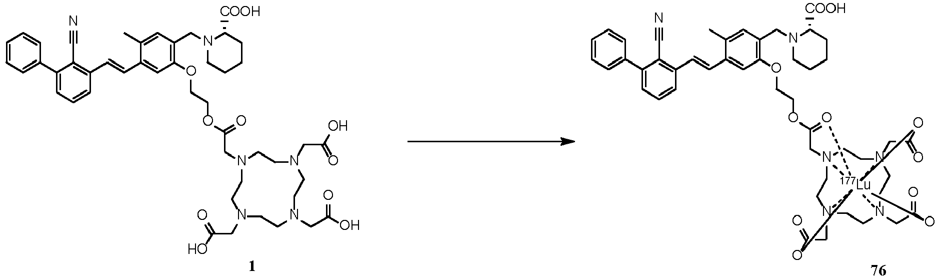

### Synthesis of compound 76

Compound 1 was dissolved in DMSO to a 10 mg/mL solution, and the solution was taken out a portion to dilute to 0.1 mg/mL with sodium acetate buffer which is metal-free and pH=5.5. The metal bath reactor was turned on and preheated to 25°C. A solution of 1mCi (about 50pmol) ¹⁷⁷LuCl₃ was taken, and then a solution of compound 1 with 20-fold equivalent was added, and the solution was supplemented to 50µL via sodium acetate buffer, and the mixture was reacted at 25°C and 800 rpm for 120 minutes. TLC purity detection was carried out using 1% EDTA as a developing agent, and compound **1** labeled with ¹⁷⁷Lu was detected by TLC scanning, and its radiochemical purity was 96.90%.

Appropriate amounts of compound 15 and ¹⁷⁷Lu-labeled compound 1 were taken for HPLC characterization according to the following conditions, and chromatographic conditions were as follows:
Mobile phase A: 0.1% TFA-water;
Mobile phase B: acetonitrile;
Method: 25%B--95%B, 10min, UV214nm;
Flow rate: 1mL/min;
Chromatographic column: Velch Xtimate C18, 4.6*150mm, 5µm.

The ¹⁷⁵Lu-labeled compound 1 was characterized by HPLC and the peak time was confirmed to be 5.731 minutes.

The amount of ¹⁷⁷Lu-labeled compound 1 was so low that UV light could not be seen, so a tube of mobile phase was collected every 0.5 minute, and a total of 20 tubes were collected. The radioactivity count of each tube of mobile phase was detected, and the radioactivity count-time curve was drawn. Compared with the HPLC of compound 15, it could be found that the peak time of ¹⁷⁷Lu-labeled compound 1 was consistent with compound 15, indicating that ¹⁷⁷Lu-labeled compound 1 (namely, compound 76) was successfully prepared.

| Time/min | 0-0.5 | 0.5-1 | 1-1.5 | 1.5-2 | 2-2.5 | 0.5-3 | 3-3.5 | 3.5-4 | 4-4.5 | 4.5-5 |
|---|---|---|---|---|---|---|---|---|---|---|
| Radioactivity count | 80 | 123 | 253 | 199 | 156 | 177 | 85 | 101 | 139 | 192 |

| Time/min | 5-5.5 | 5.5-6 | 6-6.5 | 6.5-7 | 7-7.5 | 7.5-8 | 8-8.5 | 8.5-9 | 9-9.5 | 9.5-10 |
|---|---|---|---|---|---|---|---|---|---|---|
| Radioactivity count | 2091 | 33721 | 3054 | 868 | 879 | 407 | 349 | 413 | 421 | 123 |

### Embodiment 77

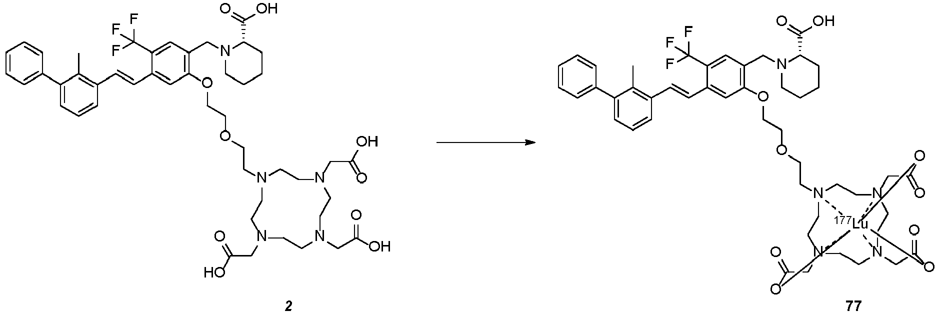

### Synthesis of compound 77

Compound **2** was dissolved in DMSO to a 10 mg/mL solution, and the solution was taken out a portion to dilute to 0.1 mg/mL with sodium acetate buffer which is metal-free and pH=5.5. The metal bath reactor was turned on and preheated to 95°C. A solution of 1mCi (about 50pmol) ¹⁷⁷LuCl₃ was taken, and then a solution of compound 2 with 20-fold equivalent was added, and the solution was supplemented to 50µL via sodium acetate buffer, and the mixture was reacted at 95°C and 800 rpm for 30 minutes. TLC purity detection was carried out using 1% EDTA as a developing agent, and compound 2 labeled with ¹⁷⁷Lu was detected by TLC scanning, and its radiochemical purity reached 100%, which could meet the requirements for animal administration and no further purification was needed.

Appropriate amounts of compound 6 and ¹⁷⁷Lu-labeled compound 2 were taken for HPLC characterization according to the following conditions, and chromatographic conditions were as follows:
Mobile phase A: 0.1% TFA-water;
Mobile phase B: acetonitrile;
Method: 25%B--95%B, 10min, UV214nm;
Flow rate: 1mL/min;
Chromatographic column: Velch Xtimate C18, 4.6*150mm, 5µm.

The ¹⁷⁵Lu-labeled compound 2 was characterized by HPLC and the peak time was confirmed to be 6.457 minutes.

The amount of ¹⁷⁷Lu-labeled compound 2 was so low that UV light could not be seen, so a tube of mobile phase was collected every 0.5 minute, and a total of 20 tubes were collected. The radioactivity count of each tube of mobile phase was detected, and the radioactivity count-time curve was drawn. Compared with the HPLC of ¹⁷⁵Lu-labeled compound 2, it could be found that the peak time of ¹⁷⁷Lu-labeled compound 2 was consistent with compound 6, indicating that ¹⁷⁷Lu-labeled compound 2 (namely, compound 77) was successfully prepared.

| Time/min | 0-0.5 | 0.5-1 | 1-1.5 | 1.5-2 | 2-2.5 | 0.5-3 | 3-3.5 | 3.5-4 | 4-4.5 | 4.5-5 |
|---|---|---|---|---|---|---|---|---|---|---|
| Radioactivity count | 67 | 70 | 55 | 177 | 82 | 65 | 106 | 58 | 58 | 51 |

| Time/min | 5-5.5 | 5.5-6 | 6-6.5 | 6.5-7 | 7-7.5 | 7.5-8 | 8-8.5 | 8.5-9 | 9-9.5 | 9.5-10 |
|---|---|---|---|---|---|---|---|---|---|---|
| Radioactivity count | 92 | 134 | 2559 6 | 2218 3 | 114 6 | 63 | 55 | 60 | 116 | 105 |

### Embodiment 78

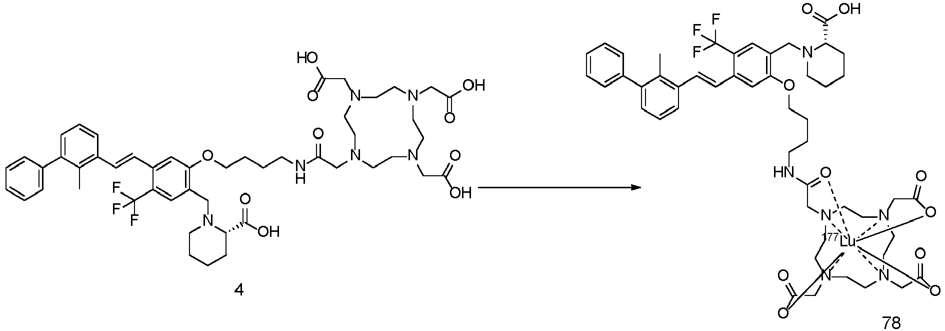

### Synthesis of compound 78

Compound **4** was dissolved in DMSO to a 10 mg/mL solution, and the solution was taken out a portion to dilute to 0.1 mg/mL with sodium acetate buffer which is metal-free and pH=5.5. The metal bath reactor was turned on and preheated to 95°C. A solution of 1mCi (about 50pmol) ¹⁷⁷LuCl₃ was taken, and then compound 4 with 20-fold equivalent was added, and the solution was supplemented to 50µL via sodium acetate buffer, and the mixture was reacted at 95°C and 800 rpm for 30 minutes. TLC purity detection was carried out using 1% EDTA as a developing agent, and compound 4 labeled with ¹⁷⁷Lu was detected by TLC scanning, and its radiochemical purity reached 100%, which could meet the requirements for animal administration and no further purification was needed.

Appropriate amounts of compound 5 and ¹⁷⁷Lu-labeled compound 4 were taken for HPLC characterization according to the following conditions, and chromatographic conditions were as follows:
Mobile phase A: 0.1% TFA-water;
Mobile phase B: acetonitrile;
Method: 25%B--95%B, 10min, UV214nm;
Flow rate: 1mL/min;
Chromatographic column: Velch Xtimate C18, 4.6*150mm, 5µm.
The ¹⁷⁵Lu-labeled compound 4 was characterized by HPLC and the peak time was confirmed to be 6.690 minutes.

The amount of ¹⁷⁷Lu-labeled compound 4 was so low that UV light could not be seen, so a tube of mobile phase was collected every 0.5 minute, and a total of 20 tubes were collected. The radioactivity count of each tube of mobile phase was detected, and the radioactivity count-time curve was drawn. Compared with the HPLC of compound 5, it can be found that the peak time of ¹⁷⁷Lu-labeled compound 4 was consistent with compound 5, indicating that ¹⁷⁷Lu-labeled compound 4 (namely, compound 78) was successfully prepared.

| Time/min | 0-0.5 | 0.5-1 | 1-1.5 | 1.5-2 | 2-2.5 | 0.5-3 | 3-3.5 | 3.5-4 | 4-4.5 | 4.5-5 |
|---|---|---|---|---|---|---|---|---|---|---|
| Radioactivity count | 70 | 46 | 59 | 74 | 80 | 203 | 92 | 95 | 296 | 240 |

| Time/min | 5-5.5 | 5.5-6 | 6-6.5 | 6.5-7 | 7-7.5 | 7.5-8 | 8-8.5 | 8.5-9 | 9-9.5 | 9.5-10 |
|---|---|---|---|---|---|---|---|---|---|---|
| Radioactivity count | 199 | 479 | 139 9 | 2720 3 | 513 4 | 129 | 115 | 109 | 86 | 96 |

### Embodiment 79

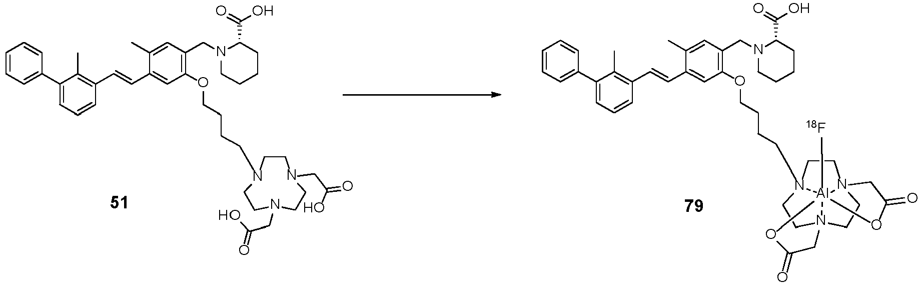

Buffer system: sodium acetate-acetic acid buffer solution with a pH of around 4.0 and a concentration of 0.5M (freshly prepared).

QMA column activation: 5mL of water, 5mL of freshly prepared sodium acetate-acetic acid buffer solution with a pH of around 4.0 and a concentration of 0.5M.

F-18 ion purification: the QMA column was passed through target water and eluted sequentially with 0.3mL buffer.

Precursor solution: 100µL buffer, 6µL AlCl₃ buffer solution with a concentration of 10mM, 300µL acetonitrile (reaction-promoting solvent), 20µL precursor solution (3mg/mL).

The precursor solution was mixed evenly and let stand to equilibrate for 5 minutes.

300µL of QMA column eluent containing F-18 was added to the reaction solution.

The plastic centrifuge tube was sealed and heated for 15 minutes, and the heating temperature was 100 °C.

C18 Light cartridge purification: the reaction solution was diluted with 5mL of water, then the sample was loaded to the C18 Light cartridge, and the product was eluted with 1mL of ethanol-water solution at a concentration of 50% (v/v). The eluent was injected into HPLC for analysis.

The chromatographic conditions were as follows

| | | | |
|---|---|---|---|
| Chromatographic column: | Phenomenex, Luna 5µC18(2) 110A, 150x4.60 mm | | |
| Detection wavelength: | 300nm | | |
| Flow rate | 1mL/min; | | |
| Mobile phase: | Time/min | Acetonitrile | 0.1% TFA aqueous solution |
| | 0 | 40 | 60 |
| | 3 | 70 | 30 |
| | 10 | 70 | 30 |
| | 12 | 40 | 60 |
| | 15 | 40 | 60 |

### Quality control analysis of compound 79

The radiochemical purity of compound 79 is 95.28%.

### Embodiment 80

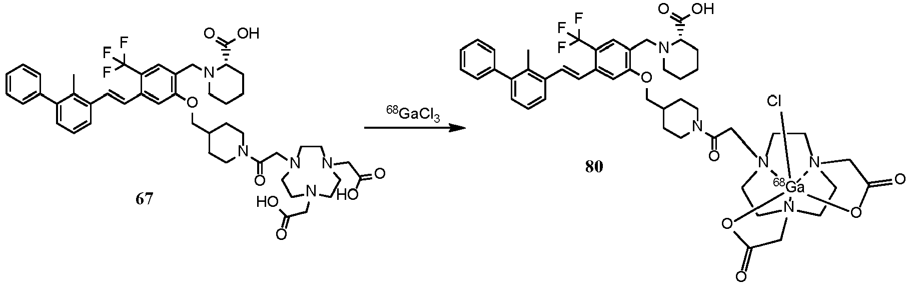

Buffer system: sodium acetate-acetic acid buffer solution with a pH of around 4.0 and a concentration of 0.5M (freshly prepared).

Compound 67 was dissolved in buffer solution, and 300µL of ⁶⁸Ga³⁺ solution was added. The plastic centrifuge tube was sealed and heated for 15 minutes, and the heating temperature was 100 °C.

C18 Light cartridge purification: the reaction solution was diluted with 5mL of water, then the sample was loaded to the C18 Light cartridge, and the product was eluted with 1mL of ethanol-water solution at a concentration of 50% (v/v) to obtain compound 80.

### Embodiment 81

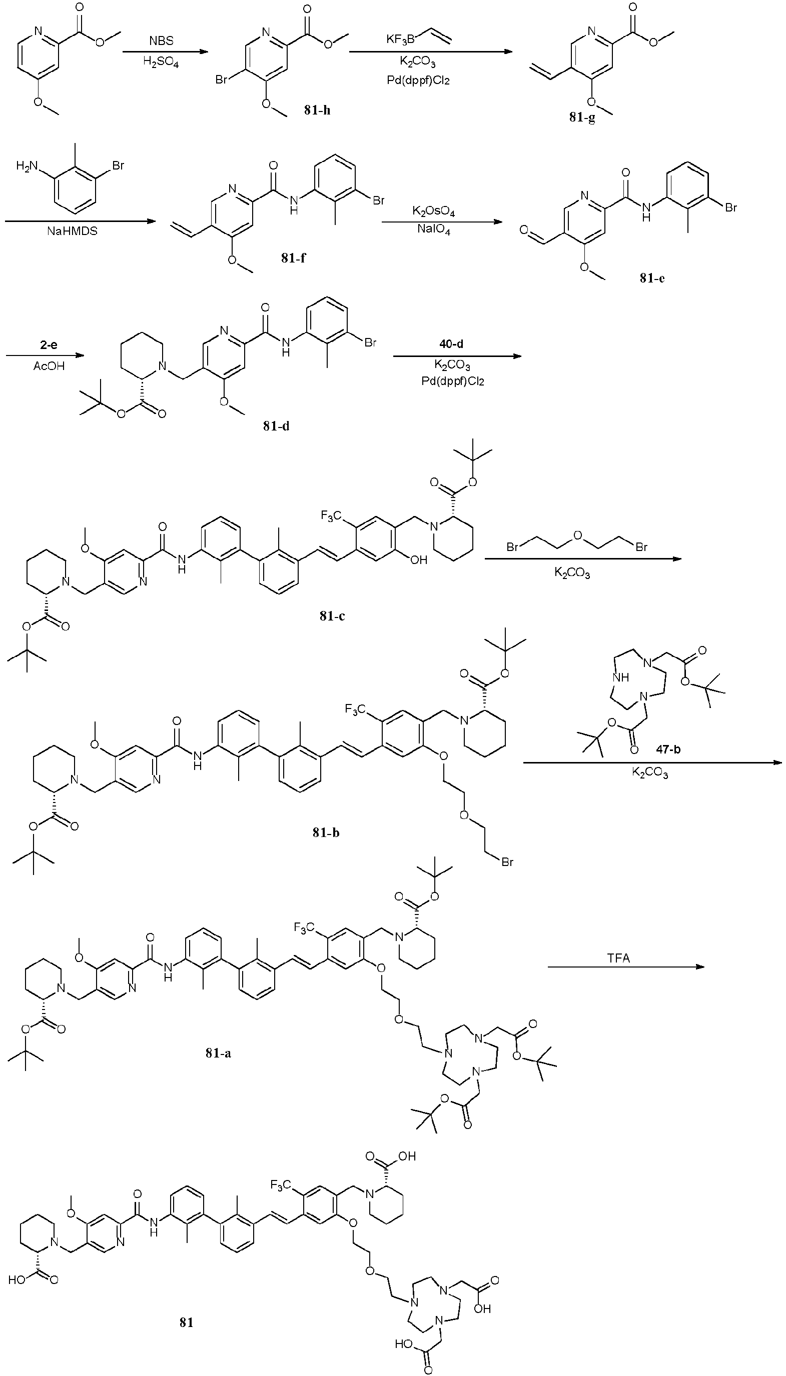

### Synthesis of compound 81-h

Methyl 4-methoxypyridinecarboxylate (5000 mg, 29.94 mmol) was added to concentrated sulfuric acid (100 mL), and N-bromosuccinimide (7993.98 mg, 44.91 mmol) was added in batches at room temperature, and the reaction solution was stirred at room temperature for 16 hours. The reaction solution was cooled to 0°C, and then the reaction solution was slowly added into ice water (1.0 L), and the pH of the mixture was adjusted to 7-9 with saturated aqueous sodium bicarbonate. The mixture was extracted with ethyl acetate (1.0 L), and the organic phase was collected, washed sequentially with water (500 mL×3) and saturated brine (500 mL×1), dried over anhydrous sodium sulfate, filtered, and concentrated under reduced pressure, and the residue was separated by column chromatography (petroleum ether: ethyl acetate=3:1) to obtain **81-h** (7 g, yield: 95.04%). LC-MS (ESI): m/z = 248.3(M+H)⁺.

### Synthesis of compound 81-g

Compound **81-h** (7.0 g, 28.448 mmol) and potassium trifluoro(vinyl)borate (5675.45 mg, 42.673 mmol) were dissolved in dioxane (60.0 mL) and water (6.0 mL), and 1,1'-bis(diphenylphosphino)ferrocene dichloropalladium (2081.57 mg, 2.845 mmol) and potassium carbonate ( 11795.5 mg, 85.3 mmol) were added. The reaction solution was heated and stirred at 90°C for 16 hours, and the reaction solution was cooled to room temperature and concentrated under reduced pressure, and the residue was separated by column chromatography (petroleum ether: ethyl acetate=5:1) to obtain **81-g** (3.4 g, yield: 61.86%). LC-MS (ESI): m/z = 194.22(M+H)⁺.

### Synthesis of compound 81-f

Compound **81-g** (966.0 mg, 5.0 mmol) and 3-bromo-2-methylphenyl-1-amine (1023.2 mg, 5.50 mmol) were dissolved in tetrahydrofuran (30 mL), and the reaction solution was cooled to 0°C, and sodium bis(trimethylsilyl)amide (6.5 mL, 1.0 M) was added dropwise to the reaction solution. After dropwise addition, the reaction solution was stirred at room temperature for 1 hour, and ammonium chloride aqueous solution (100 mL) was slowly added to the reaction solution to quench the reaction. The mixture was extracted with ethyl acetate (150 mL), and the organic phase was collected, washed sequentially with water (100 mL×3) and saturated brine (100 mL×1), dried over anhydrous sodium sulfate, filtered, and concentrated under reduced pressure, and the residue was separated by column chromatography (petroleum ether: ethyl acetate=10:1) to obtain **81-f** (1.4 g, yield: 80.64%). LC-MS (ESI): m/z = 349.3(M+H)⁺.

### Synthesis of compound 81-e

Compound **81-f** (850 mg, 2.448 mmol) and potassium osmate (90.09 mg, 0.245 mmol) were dissolved in dioxane (60 mL) and water (20 mL), and sodium periodate (1570.86 mg, 7.344 mmol) was added in batches, and the reaction solution was stirred at room temperature for 6 hours. The reaction solution was filtered, and the filter cake was washed with dioxane (50 mL), and the filter cake was purified by column chromatography (petroleum ether: ethyl acetate=5:1) to obtain **81-e** (360 mg, yield: 42.11%). LC-MS (ESI): m/z = 351.2(M+H)⁺.

### Synthesis of compound 81-d

Compound **81-e** (360 mg, 1.03 mmol) and compound **2-e** (382.02 mg, 2.06 mmol) were dissolved in methanol (60.0 mL), and sodium cyanoborohydride (129.91 mg, 2.06 mmol) and acetic acid (129.90 mg, 2.16 mmol) were added, and the reaction solution was heated and stirred at 60 °C for 6 hours. The reaction solution was cooled to room temperature and concentrated under reduced pressure, and the residue was separated by column chromatography (petroleum ether: ethyl acetate=5:1) to obtain **81-d** (280 mg, yield: 52.38%). LC-MS (ESI): m/z = 520.3(M+H)⁺.

### Synthesis of compound 81-c

Compound **81-d** (280.0 mg, 0.54 mmol) and compound **40-d** (487.2 mg, 0.81 mmol) were dissolved in dioxane (50.0 mL) and water (5.0 mL), and 1,1'-bis(diphenylphosphino)ferrocene dichloropalladium (39.52 mg, 0.054 mmol) and potassium carbonate (223.9 mg, 1.62 mmol) were added, and the reaction solution was heated and stirred at 110 °C for 12 hours. The reaction solution was cooled to room temperature and concentrated under reduced pressure, and the residue was separated by column chromatography (petroleum ether: ethyl acetate=5:1) to obtain **81-c** (210 mg, yield: 38.46%). LC-MS (ESI): m/z =913.53 (M+H)⁺.

### Synthesis of compound 81-b

Compound **81-c** (198 mg, 0.217 mmol) and 1-bromo-2-[(2-bromoethyl)oxy]ethane (150.8 mg, 0.651 mmol) were dissolved in *N, N*-dimethylformamide (50.0 mL), and potassium carbonate (89.77 mg, 0.651 mmol) was added, and the reaction solution was heated and stirred at 80°C for 12 hours. The reaction solution was cooled to room temperature and concentrated under reduced pressure, and the residue was dissolved with dioxane (60 mL), and the mixture was washed sequentially with water (40 mL×3) and saturated brine (40 mL×1), dried over anhydrous sodium sulfate, filtered, and concentrated under reduced pressure, and the residue was separated by column chromatography (petroleum ether: ethyl acetate=5:1) to obtain **81-b** (70 mg, yield: 28.6%). LC-MS (ESI): m/z = 1065.14(M+H)⁺.

### Synthesis of compound 81-a

Compound **81-b** (70 mg, 0.066 mmol) and compound **47-b** (47.07 mg, 0.132 mmol) were dissolved in acetonitrile (10.0 mL), and potassium carbonate (36.37 mg, 0.263 mmol) was added, and the reaction solution was heated and stirred at 60°C for 3 hours. The reaction solution was cooled to room temperature and concentrated under reduced pressure, and the residue was separated by column chromatography (dichloromethane: methanol=10:1) to obtain **81-a** (80 mg, yield: 90.7%). LC-MS (ESI): m/z = 1341.0(M+H)⁺.

### Synthesis of compound 81

Compound **81-a** (80 mg, 0.060 mmol) was dissolved in dichloromethane (10.0 mL), and trifluoroacetic acid (5.0 mL) was added, and the reaction solution was stirred at room temperature for 6 hours and concentrated under reduced pressure, and the residue was directly separated by pre-HPLC to obtain **81** (28 mg, yield: 42.04%). LC-MS (ESI): m/z = 1116.64(M+H)⁺.

### Embodiment 82

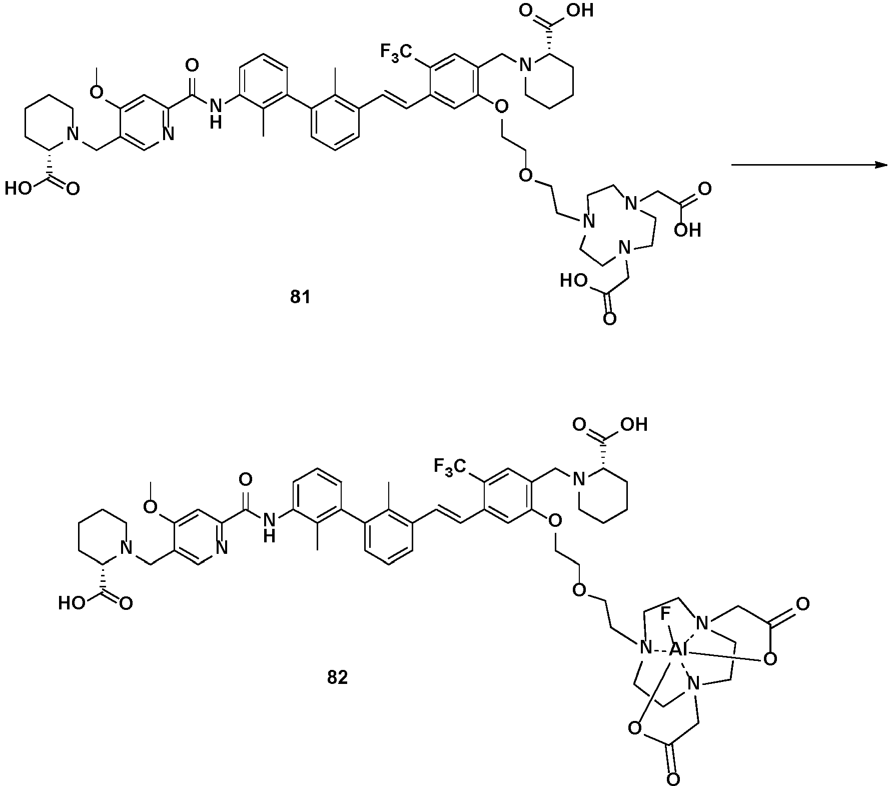

### Synthesis of compound 82

Compound **81** (8.0 mg, 0.007 mmol) was dissolved in ethanol (0.5 mL), and the [AlF]²⁺ solution (0.016 mmol, 1.6 mL) was added, and the reaction solution was stirred at 100°C for 1 hour. The reaction solution was cooled to room temperature, and directly separated by pre-HPLC to obtain **82** (4 mg, yield: 48.08%). LC-MS (ESI): m/z = 1160.8(M+H)⁺.

### Effect embodiment 1

Homogenouse Time-Resolved Fluorescence (HTRF) binding assay was used to detect the binding ability of the compound of the present disclosure to PD-1/PD-L1.

The purchased kit (CisBio, #64CUS000C-1) contains reagents required for experiments such as PD-1, PD-L1, anti-tag1-Eu, Anti-tag2-XL665, Dilute Buffer, and Detection Buffer.

### Experimental steps

1. The compound was configured with 100%DMSO into 10 concentrations with a concentration gradient of 3 times.
2. The DMSO solution of the compound was added to Dilute Buffer solution, and the mixture was mixed well and transferred to a 96-well plate.
3. PD-L1 was diluted with Dilute Buffer solution and then added to the above 96-well plate.
4. PD-1 was diluted with Dilute Buffer solution and then added to the above 96-well plate, and incubated at room temperature for 30 minutes.
5. One part of anti-tag1-Eu and one part of Anti-tag2-XL665 were added to Detection Buffer solution, and the mixture was mixed well and transferred to the above 96-well plate.
6. The mixture in the 96-well plate was incubated at room temperature for 1 to 24 hours.
7. The HTRF values were read with Envision.

### Experimental results

The biological activity of the compounds of the present disclosure were determined by the above tests, and the results obtained are as follows (Table 1):

Table 1 IC₅₀ values of PD-1/PD-L1 binding of some compounds in the present disclosure.

| Compound | IC₅₀ (nM) | Compound | IC₅₀ (nM) |
|---|---|---|---|
| 2 | 1.29 | 29 | 0.5711 |
| 3 | 3.16 | 30 | 9.217 |
| 4 | 0.8881 | 31 | 0.6027 |
| 6 | 2.658 | 32 | 12.37 |
| 7 | 0.9691 | 33 | 0.6012 |
| 8 | 0.5216 | 34 | 0.4048 |
| 9 | 1.072 | 35 | 0.7659 |
| 10 | 169.3 | 36 | 0.5214 |
| 11 | 32.57 | 37 | 0.7292 |
| 12 | 32.52 | 38 | 1.134 |
| 13 | 299.5 | 48 | 0.8853 |
| 14 | 0.95 | 50 | 3.316 |
| 15 | 5.33 | 52 | 4.377 |
| 16 | 1.422 | 54 | 7.365 |
| 17 | 1.191 | 56 | 11.46 |
| 18 | 0.9012 | 57 | 4.834 |
| 19 | 0.869 | 59 | 5.471 |
| 20 | 1.012 | 62 | 0.5288 |
| 21 | 3.741 | 64 | 0.7003 |
| 22 | 5.029 | 66 | 1.08 |
| 23 | 0.8334 | 68 | 1.249 |
| 24 | 0.7852 | 69 | 24.85 |
| 25 | 16.17 | 70 | 47.7 |
| 26 | 33.21 | 71 | 13.24 |
| 27 | 13.61 | 72 | 112.5 |
| 28 | 0.5616 | | |

### Effect embodiment 2:

1. Inhibitory effect of compound 78 on tumor
1.1 Experimental animal information: tumor-bearing C57BL6 mice; SPF grade; weight range: 17-20g; Shanghai Qi'up Biomedical Technology Co., Ltd.
1.2 Experimental process

Ten tumor-bearing mice with tumor volumes of 10-100 mm³ were systematically and randomly divided into 2 groups according to tumor size, with 5 mice in each group, corresponding to PBS and compound 78 respectively. After anesthesia with isoflurane, tumor-bearing mice were injected into the tail vein with compound 78 with 45 MBq (approximately 1.2 mCi) per mouse and a volume of 0.2 mL. After administration, the tumor size and weight of the animals were measured periodically, and the tumor growth curve was drawn to understand the inhibitory effect of the drug on tumor.
1.3 Experimental results

Experimental results show that compared with the control group, compound 78 has a very good therapeutic effects on inhibiting tumor growth. (see figure 1).

The experiments were operated according to the above, and the experimental results show that: the injection of compound 5, compound 6, compound 12, compound 13, compound 14, compound 15, compound 17, compound 20, compound 22, compound 24, compound 26, compound 32, compound 33, compound 38, compound 41, compound 44, compound 45, compound 46, compound 70, compound 72, compound 76, and compound 77 respectively also has very good therapeutic effects on inhibiting tumor growth.

### Effect embodiment 3:

Tumor targeting study of compound 79 in Micro-PET/CT scanning 1. Experimental animal information: C57BL/6J mice, SPF grade; 15-21g; model mouse construction: bilateral tumor

### Construction of bilateral tumor model mice

### Cell culture

Human-derived PD-L1 gene knock-in MC-38 cells (MC-38-hPD-Ll cells) were adherently cultured in vitro, and the culture conditions were DMEM medium plus 10% heat-inactivated fetal bovine serum and hygromycinB (final concentration was 100µL/mL), and the cells were cultured at 37°C with 5% CO₂. Subculture of 2-3 times a week. When the cells were in the exponential growth phase, the cells were harvested, counted, and the left side of the mice was inoculated subcutaneously.

Mouse-derived MC-38 cells were adherently cultured in vitro, and the culture conditions were DMEM medium plus 10% heat-inactivated fetal bovine serum, and the cells were cultured at 37 °C with 5% CO₂. Subculture of 2-3 times a week. When the cells were in the exponential growth phase, the cells were collected, counted, and the right side of the mice was inoculated subcutaneously.

### Inoculation of tumor cell

100 µL of 1×10⁶ MC-38-hPD-Ll cell suspension was inoculated subcutaneously on the left dorsal side of C57BL/6J mice. The same mice were subcutaneously inoculated with 100 µL of 1×10⁶ MC-38 cell suspension on the right dorsal side on the second day. After inoculation, the mice were fed normally, and after a certain number of days, tumor-bearing mice with the volume of bilateral transplanted tumor in the range of 150mm³-350mm³ were selected for test.

### Experiment procedure

After quality control, compound 79 was diluted with 10% ethanol in physiological saline, and the drug was extracted and injected into each animal through tail vein. The administration volume was 100µL/animal, and the administration dose was 100-200µCi/animal. The study of Micro-PET/CT imaging was performed at 0.5 h, 1.5 h, 2.5 h, 3.5 h after injection of compound 79.

### Experimental results

The experimental results show that the left side of bilateral tumor mice scanned by compound 79 was MC38-PDL1 and the right side was MC3 8. The results show that the tumor uptake value on the left side is significantly higher than that on the right side, as shown in Table 2.

**Table 2 The values of tumor target uptake (unit: ID%/g)**

| Time | Compound 79 | |
|---|---|---|
| | bilateral tumor model | |
| | MC-38 | MC38-PDL1 |
| 30min | 0.75 | 1.3 |
| 90min | 0.3 | 0.65 |
| 150min | 0.07 | 0.54 |
| 210min | 0.06 | 0.66 |

The experiments were operated according to the above, and the experimental results show that: compound 48, compound 50, compound 52, compound 54, compound 56, compound 58, compound 60, compound 62, compound 64, compound 66, compound 68, and compound 82 were injected respectively for Micro-PET/CT imaging, and it can be found that the uptake value of the mouse left tumor (MC38-PDL1) is significantly higher than that of the mouse right tumor (MC38).

### Effect embodiment 4

### Tumor targeting study of compound 80 in Micro-PET/CT scanning

Experimental animal information: C57BL/6J mice, SPF grade; 15-21g;
Construction of bilateral tumor model mice:

### Cell culture

Human-derived PD-L1 gene knock-in MC-38 cells (MC-38-hPD-Ll cells) were adherently cultured in vitro, and the culture conditions were DMEM medium plus 10% heat-inactivated fetal bovine serum and hygromycinB (final concentration was 100µL/mL), and the cells were cultured at 37°C with 5% CO₂. Subculture of 2-3 times a week. When the cells were in the exponential growth phase, the cells were harvested, counted, and the left side of the mice was inoculated subcutaneously.

Mouse-derived MC-38 cells were adherently cultured in vitro, and the culture conditions were DMEM medium plus 10% heat-inactivated fetal bovine serum, and the cells were cultured at 37 °C with 5% CO₂. Subculture of 2-3 times a week. When the cells were in the exponential growth phase, the cells were collected, counted, and the right side of the mice was inoculated subcutaneously.

### Inoculation of tumor cell

100 µL of 1×10⁶ MC-38-hPD-Ll cell suspension was inoculated subcutaneously on the left dorsal side of C57BL/6J mice. The same mice were subcutaneously inoculated with 100 µL of 1×10⁶ MC-38 cell suspension on the right dorsal side on the second day. After inoculation, the mice were fed normally, and after a certain number of days, tumor-bearing mice with the volume of bilateral transplanted tumor in the range of 150mm³-350mm³ were selected for test.

### Experiment procedure

After quality control, compound 80 was diluted with 10% ethanol in physiological saline, and the drug was extracted and injected into each animal through tail vein. The administration volume was 100µL/animal, and the administration dose was 100-200µCi/animal. The study of Micro-PET/CT imaging was performed at 0.5 h, 1.5 h, 2.5 h, 3.5 h after injection of compound 80.

### Experimental results:

The experimental results show that the uptake value of the left side tumor (MC38-PDL1) in the model mice scanned with compound 80 is significantly higher than that of the right side (MC-38), as shown in Table 3.

**Table 3 The values of tumor target uptake (unit: ID%/g)**

| Time | Compound 80 | |
|---|---|---|
| | bilateral tumor model | |
| | MC-38 | MC38-PDL1 |
| 30min | 0.98 | 2.6 |
| 90min | 1.2 | 2.8 |
| 150min | 0.74 | 2.2 |
| 210min | 0.46 | 2.3 |

The experiments were operated according to the above, and the experimental results show that: compound 7, compound 8, compound 27, compound 28, compound 29, compound 34, compound 35, compound 36, compound 37, compound 73, compound 74, and compound 75 were injected respectively for Micro-PET/CT imaging, and it can be found that the uptake value of the mouse left tumor (MC38-PDL1) is significantly higher than that of the mouse right tumor (MC38).

Although specific embodiments of the present disclosure are described above, it should be understood by those skilled in the art that these are merely illustrative and that a variety of changes or modifications can be made to these embodiments without departing from the principles and substance of the present disclosure. Therefore, the scope of protection of the present disclosure is defined by the appended claims.

## Claims

1. A compound represented by general formula II or a metal complex thereof, or their tautomers thereof, or their stereoisomers thereof, or their pharmaceutically acceptable salts thereof, or their solvates thereof: 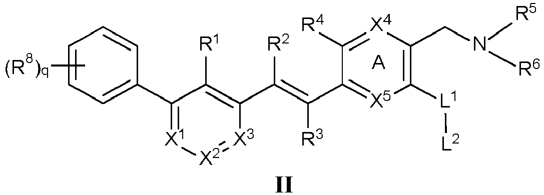
X¹, X², X³, X⁴, and X⁵ are each independently CH or N;
R¹ is hydrogen, halogen, cyano, C₁-C₄ alkyl, or C₁-C₄ alkyl substituted by one or more R^{a};
R² and R³ are each independently hydrogen or halogen;
R⁴ is hydrogen, halogen, C₁-C₄ alkyl, or C₁-C₄ alkyl substituted by one or more R^{b};
R⁵ and R⁶ are each independently hydrogen, deuterium, C₁-C₄ alkyl, or C₁-C₄ alkyl substituted by one or more R^{c}; or, R⁵, R⁶ together with the nitrogen atom to which they are attached form a 5- to 7-membered heterocyclic ring, or a 5- to 7-membered heterocyclic ring substituted by one or more R^{c}; in the 5- to 7-membered heterocyclic ring, the number of heteroatoms is 1, 2, 3, or 4, and each heteroatom is independently selected from N, O, and S;
each R^{a}, each R^{b} and each R^{c} are independently deuterium, halogen, hydroxyl, amino, C₁-C₄ alkyl, C₁-C₄ alkyl-O-, -COOH, or -C(O)OR^{g};
each R⁸ is independently hydrogen, deuterium, halogen, hydroxyl, amino, C₁-C₄ alkyl, C₁-C₄ alkyl-S-, C₁-C₄ alkyl-O-, -C(O) NH2, -C(O)OC₁₋₄ alkyl, -OR^{8a}, -NHR^{8a}, -NR^{8a}R^{8b}, -NH-C(O)-R^{8d}, C₁-C₄ alkyl substituted by one or more R^{8c}, C₁-C₄ alkyl-S- substituted by one or more R^{8c}, or C₁-C₄ alkyl-O- substituted by one or more R^{8c};
or, two adjacent R⁸ together with the carbon atoms on the benzene ring to which they are attached form a 5- to 7-membered carbocyclic ring, a 5- to 7-membered heterocyclic ring, a 5-to 7-membered carbocyclic ring substituted by one or more C₁₋₄ alkyl, or a 5- to 7-membered heterocyclic ring substituted by one or more C₁₋₄ alkyl; in the 5- to 7-membered heterocyclic ring, the number of heteroatoms is 1, 2, 3, or 4, and each heteroatom is independently selected from N and O;
each R^{8a}, R^{8b}, and each R^{8c} are independently C₁-C₄ alkyl-S-, halogen, C₁-C₄ alkyl, C₁-C₄ alkyl-O-, -COOH, -(C₁-C₄ alkylene)-COOH, -C(O)OC₁-C₄ alkyl, -C(O)NH₂, -C(O)NHC₁-C₄ alkyl, 5- to 7-membered heterocyclic ring, or -NR^{8e}R^{8f}; in the 5- to 7-membered heterocyclic ring, the species of heteroatoms are independently selected from one or more of N, O, and S, and the number of heteroatoms is independently 1, 2, or 3;
R^{8e} and R^{8f} are each independently hydrogen, halogen, C₁-C₄ alkyl, or C₁-C₄ alkyl substituted by one or more R^{8g};
each R^{8g} is independently halogen, C₁-C₄ alkyl, hydroxyl, -NR^{8h}R^{8k}, C₁-C₄ alkyl-O-, - COOH, -(C₁-C₄ alkylene)-COOH, -C(O)OC₁-C₄ alkyl, -C(O)NH₂, or -C(O)NHC₁-C₄ alkyl;
R^{8h} and R^{8k} are each independently hydrogen or C₁-C₄ alkyl;
each R^{8d} is independently C₆-C₁₀ aryl substituted by one or more R^{8d-1}, or 5- to 10-membered heteroaryl substituted by one or more R^{8d-2}; in the 5- to 10-membered heteroaryl, the species of heteroatoms are independently selected from one or more of N, O, and S, and the number of heteroatoms is independently 1, 2, or 3;
each R^{8d-1} and R^{8d-2} are independently C₁-C₄ alkoxy or C₁-C₄ alkyl substituted by one or more R^{8d-1-1};
each R^{8d-1-1} is independently 5- to 7-membered heterocyclic ring substituted by one carboxyl; in the 5- to 7-membered heterocyclic ring, the species of heteroatoms are independently selected from one or more of N, O, and S, and the number of heteroatoms is independently 1, 2, or 3;
q is 0, 1, 2, or 3;
L¹ is
(i) a single bond or -(CH₂)ₙ-;
(ii) -(CH₂)ₘ-, in which 1, 2, 3, 4, or 5 non-adjacent CH₂ are independently replaced by - Y₁-, and each Y₁ is independently -C(O)-, -C(O)O-, -O-, -NH-, -C(O)NH-, or -NHC(O)NH-; or
(iii) -(CH₂)ₚ-, in which one CH₂ is replaced by -Y₂-, and the other 0, 1, 2, 3, or 4 non-adjacent CH₂ are independently replaced by -Y₃-; each Y₃ is independently -C(O)-, -C(O)O-, - O-, -NH-, -C(O)NH-, or -NHC(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
L¹ is unsubstituted or 1, 2, or 3 H contained in L¹ are each independently substituted by R⁷;
n, m, and p are each independently 1, 2, 3, 4, 5, 6, 7, 8, 9, 10, 11, 12, 13, or 14;
each R⁷ is independently C₁-C₄ alkyl or -L³-R⁹;
L³ is
(i) -(CH₂)ⱼ-; or
(ii) -(CH₂)ₖ-, in which 1, 2, 3, or 4 non-adjacent CH₂ are independently replaced by -Y₄-, and each Y₄ is independently -C(O)-, -C(O)O-, -O-, -NH-, -C(O)NH-, or -NHC(O)NH-;
L³ is unsubstituted or 1, 2, or 3 H contained in L³ are each independently substituted by R¹⁰;
j and k are each independently 1, 2, 3, 4, 5, 6, 7, 8, 9, 10, 11, 12, 13, or 14;
R⁹ is hydrogen, C₆-C₁₀ aryl, or C₆-C₁₀ aryl substituted by one or more R^{d};
each R¹⁰ is independently C₁-C₄ alkyl;
each R^{d} is independently C₁-C₄ alkyl or C₁-C₄ alkyl substituted by one or more R^{e};
each R^{e} is independently hydroxyl, amino, C₁-C₄ alkyl, C₁-C₄ alkyl-O-, -COOH, or - C(O)OR^{h};
R^{g} and R^{h} are each independently C₁-C₄ alkyl or halogenated C₁-C₄ alkyl;
L² is metal chelating group;
the metal complex is a complex chelated by the compound represented by general formula II with a metal atom or ion.

2. The compound represented by general formula II or the metal complex thereof, or their tautomers thereof, or their stereoisomers thereof, or their pharmaceutically acceptable salts thereof, or their solvates thereof according to claim 1, wherein, it satisfies one or more of the following conditions:
a) q is 0, 1, or 2;
each R⁸ is independently C₁-C₄ alkyl, -NH-C(O)-R^{8d}, or C₁-C₄ alkyl-O- substituted by one or more R^{8c};
each R^{8c} is independently 5- to 7-membered heterocyclic ring; in the 5- to 7-membered heterocyclic ring, the species of heteroatoms are independently selected from one or more of N, O, and S, and the number of heteroatoms is independently 1, 2, or 3;
each R^{8d} is independently C₆-C₁₀ aryl substituted by one or more R^{8d-1}, or 5- to 10-membered heteroaryl substituted by one or more R^{8d-2}; in the 5- to 10-membered heteroaryl, the species of heteroatoms are independently selected from one or more of N, O, and S, and the number of heteroatoms is independently 1, 2, or 3;
each R^{8d-1} and R^{8d-2} are independently C₁-C₄ alkoxy or C₁-C₄ alkyl substituted by one or more R^{8d-1-1};
each R^{8d-1-1} is independently 5- to 7-membered heterocyclic ring substituted by one carboxyl; in the 5- to 7-membered heterocyclic ring, the species of heteroatoms are independently selected from one or more of N, O, and S, and the number of heteroatoms is independently 1, 2, or 3;
b) R¹ is cyano or C₁-C₄ alkyl; X¹, X², and X³ are each independently CH or N;
c) Rand R³ are hydrogen;
d) R⁴ is C₁-C₄ alkyl or C₁-C₄ alkyl substituted by one or more R^{b}; each R^{b} is independently halogen; X⁴ and X⁵ are each independently CH or N;
e) R⁵, R⁶ together with the nitrogen atom to which they are attached form a 5- to 7-membered heterocyclic ring substituted by one or more R^{c}; in the 5- to 7-membered heterocyclic ring, the number of heteroatoms is 1, 2, 3, or 4, and each heteroatom is independently selected from N, O, and S; R^{c} is -COOH;
f) L¹ is
(ii) -(CH₂)ₘ-, in which 1, 2, 3, 4, or 5 non-adjacent CH₂ are independently replaced by - Y₁-, and each Y₁ is independently -C(O)O-, -O-, or -C(O)NH-; or
(iii) -(CH₂)ₚ-, in which one CH₂ is replaced by -Y₂-, and the other 1, 2, 3, or 4 non-adjacent CH₂ are independently replaced by -Y₃-; each Y₃ is independently -C(O)-, -O-, or -C(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
L¹ is unsubstituted or one H contained in L¹ is each independently substituted by R⁷;
m and p are each independently 5, 6, 7, 8, 9, 10, or 11;
R⁷ is -L³-R⁹;
L³ is
(ii) -(CH₂)ₖ-, in which 1, 2, 3, or 4 non-adjacent CH₂ are independently replaced by -Y₄-, and each Y₄ is -C(O)NH-;
L³ is unsubstituted;
k is 7, 8, or 9;
R⁹ is C₆-C₁₀ aryl substituted by one or more R^{d}; each R^{d} is independently C₁-C₄ alkyl;
g) the metal complex is a complex chelated by the compound represented by general formula II with the metal ion;
h) the metal ion does not bind to non-metal nuclide.

3. The compound represented by general formula II or the metal complex thereof, or their tautomers thereof, or their stereoisomers thereof, or their pharmaceutically acceptable salts thereof, or their solvates thereof according to claim 2, wherein, it satisfies one or more of the following conditions:
i) q is 0;
j) R¹ is cyano or C₁-C₄ alkyl; X¹ and X² are CH, and X³ is N;
k) R⁴ is C₁-C₄ alkyl or C₁-C₄ alkyl substituted by one or more R^{b}; each R^{b} is independently halogen; X⁴ and X⁵ are each independently CH.

4. The compound represented by general formula II or the metal complex thereof, or their tautomers thereof, or their stereoisomers thereof, or their pharmaceutically acceptable salts thereof, or their solvates thereof according to claim 1, wherein, the definition of which is any one of the following schemes:
scheme 1:
X¹, X², X³, X⁴, and X⁵ are each independently CH or N;
R¹ is cyano or C₁-C₄ alkyl;
R² and R³ are hydrogen;
R⁴ is C₁-C₄ alkyl or C₁-C₄ alkyl substituted by one or more R^{b}; each R^{b} is independently halogen;
R⁵, R⁶ together with the nitrogen atom to which they are attached form a 5- to 7-membered heterocyclic ring substituted by one or more R^{c}; in the 5- to 7-membered heterocyclic ring, the number of heteroatoms is 1, 2, 3, or 4, and each heteroatom is independently selected from N, O, and S;
R^{c} is -COOH;
q is 0, 1, or 2;
each R⁸ is independently C₁-C₄ alkyl, -NH-C(O)-R^{8d}, or C₁-C₄ alkyl-O- substituted by one or more R^{8c};
each R^{8c} is independently 5- to 7-membered heterocyclic ring; in the 5- to 7-membered heterocyclic ring, the species of heteroatoms are independently selected from one or more of N, O, and S, and the number of heteroatoms is independently 1, 2, or 3;
each R^{8d} is independently C₆-C₁₀ aryl substituted by one or more R^{8d-1}, or 5- to 10-membered heteroaryl substituted by one or more R^{8d-2}; in the 5- to 10-membered heteroaryl, the species of heteroatoms are independently selected from one or more of N, O, and S, and the number of heteroatoms is independently 1, 2, or 3;
each R^{8d-1} and R^{8d-2} are independently C₁-C₄ alkoxy or C₁-C₄ alkyl substituted by one or more R^{8d-1-1};
each R^{8d-1-1} is independently 5- to 7-membered heterocyclic ring substituted by one carboxyl; in the 5- to 7-membered heterocyclic ring, the species of heteroatoms are independently selected from one or more of N, O, and S, and the number of heteroatoms is independently 1, 2, or 3;
L¹ is
(ii) -(CH₂)ₘ-, in which 1, 2, 3, 4, or 5 non-adjacent CH₂ are independently replaced by - Y₁-, and each Y₁ is independently -C(O)O-, -O-, or -C(O)NH-; or
(iii) -(CH₂)ₚ-, in which one CH₂ is replaced by -Y₂-, and the other 1, 2, 3, or 4 non-adjacent CH₂ are independently replaced by -Y₃-; each Y₃ is independently -C(O)-, -O-, or -C(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
L¹ is unsubstituted or one H contained in L¹ is each independently substituted by R⁷;
m and p are each independently 5, 6, 7, 8, 9, 10, or 11;
R⁷ is -L³-R⁹;
L³ is
(ii) -(CH₂)ₖ-, in which 1, 2, 3, or 4 non-adjacent CH₂ are independently replaced by -Y₄-, and each Y₄ is -C(O)NH-;
L³ is unsubstituted;
k is 7, 8, or 9;
R⁹ is C₆-C₁₀ aryl substituted by one or more R^{d}; each R^{d} is independently C₁-C₄ alkyl;
L² is metal chelating group;
the metal complex is a complex chelated by the compound represented by general formula II with the metal atom or ion;
scheme 2:
X¹, X², X⁴ and X⁵ are CH;
X³ is CH or N;
R¹ is cyano or C₁-C₄ alkyl;
R² and R³ are hydrogen;
R⁴ is C₁-C₄ alkyl or C₁-C₄ alkyl substituted by one or more R^{b}; each R^{b} is independently halogen;
R⁵, R⁶ together with the nitrogen atom to which they are attached form a 5- to 7-membered heterocyclic ring substituted by one or more R^{c}; in the 5- to 7-membered heterocyclic ring, the number of heteroatoms is 1, 2, 3, or 4, and each heteroatom is independently selected from N, O, and S;
R^{c} is -COOH;
q is 0;
L¹ is
(ii) -(CH₂)ₘ-, in which 1, 2, 3, 4, or 5 non-adjacent CH₂ are independently replaced by - Y₁-, and each Y₁ is independently -C(O)O-, -O-, or -C(O)NH-; or
(iii) -(CH₂)ₚ-, in which one CH₂ is replaced by -Y₂-, and the other 1, 2, 3, or 4 non-adjacent CH₂ are independently replaced by -Y₃-; each Y₃ is independently -C(O)-, -O-, or -C(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
L¹ is unsubstituted or one H contained in L¹ is each independently substituted by R⁷;
m and p are each independently 5, 6, 7, 8, 9, 10, or 11;
R⁷ is -L³-R⁹;
L³ is
(ii) -(CH₂)ₖ-, in which 1, 2, 3, or 4 non-adjacent CH₂ are independently replaced by -Y₄-, and each Y₄ is -C(O)NH-;
L³ is unsubstituted;
k is 7, 8, or 9;
R⁹ is C₆-C₁₀ aryl substituted by one or more R^{d}; each R^{d} is independently C₁-C₄ alkyl;
L² is metal chelating group;
the metal complex is a complex chelated by the compound represented by general formula II with the metal ion.

5. The compound represented by general formula II or the metal complex thereof, or their tautomers thereof, or their stereoisomers thereof, or their pharmaceutically acceptable salts thereof, or their solvates thereof according to claim 1, wherein, it satisfies one or more of the following conditions:
l) the stereoisomer is chiral isomer;
m) the metal complex has a structure represented by the following general formula I: 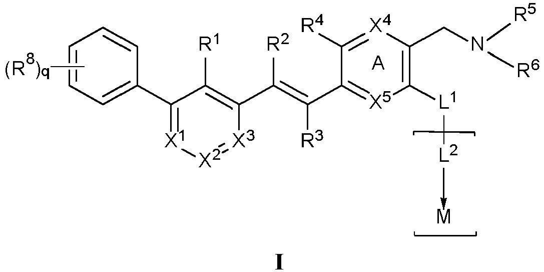 wherein, M is the metal atom or ion;
n) in R¹, the C₁-C₄ alkyl is methyl or ethyl;
o) in R⁴, the C₁-C₄ alkyl is methyl or ethyl;
p) in R⁴, the C₁-C₄ alkyl in the C₁-C₄ alkyl substituted by one or more R^{b} is methyl or ethyl;
q) in R^{b}, the halogen is fluorine or chlorine;
r) in the 5- to 7-membered heterocyclic ring substituted by one or more R^{c}, which is formed by R⁵, R⁶ together with the nitrogen atom they are attached, the 5- to 7-membered heterocyclic ring is a 6-membered saturated monocyclic heterocyclic ring;
s) in the 5- to 7-membered heterocyclic ring substituted by one or more R^{c}, which is formed by R⁵, R⁶ together with the nitrogen atom they are attached, the number of heteroatoms is 1;
t) in the 5- to 7-membered heterocyclic ring substituted by one or more R^{c}, which is formed by R⁵, R⁶ together with the nitrogen atom they are attached, the heteroatom is N;
u) L¹ is connected to ring A through -Y₁-, and Y₁ connected to ring A is -O-;
v) L¹ is connected to L² via -CH₂-;
w) L¹ is -O(CH₂)ₙ₂-, -O(CH₂)ₙ₂O(CH₂)ₘ₂-, -O(CH₂)ₙ₂O(CH₂)ₘ₂O(CH₂)ₘ₃-, - O(CH₂)ₙ₂OC(O)(CH₂)ₘ₂-, -O(CH₂)ₙ₂NHC(O)(CH₂)ₘ₂-, -O(CH₂)ₙ₂NHC(O)-(CH₂)ₙ₃-NHC(O)(CH₂)ₘ₃-, -O(CH₂)ₙ₂-O(CH₂)ₙ₃-NHC(O)-(CH₂)ₙ₄NHC(O)-(CH₂)ₘ₃-, - O(CH₂)ₙ₂NHC(O)-Y₂-(CH₂)ₙ₃NHC(O)-(CH₂)ₘ₃-, -O(CH₂)ₙ₂-Y₂-C(O)-(CH₂)ₘ₃-, - O(CH₂)ₙ₂NHC(O)-Y₂-C(O)-(CH₂)ₘ₃-, or -O(CH₂)ₙ₂NHC(O)-Y₂-(CH₂)ₙ₃NHC(O)-(CH₂)ₙ₄NHC(O)-(CH₂)ₘ₃-; the right end of L¹ is connected to L²; each n2, each n3, each n4, each m2 and each m3 are independently 1, 2, 3, 4, 5, or 6; L¹ is unsubstituted or one H contained in L¹ is substituted by R⁷;
x) when Y₂ is 5- to 7-membered carbocyclic ring, the 5- to 7-membered carbocyclic ring is 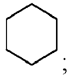
y) when Y₂ is 5- to 7-membered heterocyclic ring, the 5- to 7-membered heterocyclic ring is 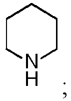
z) R⁹ is phenyl or phenyl substituted by one or more R^{d};
aa) in R^{d}, the C₁-C₄ alkyl is methyl or ethyl;
bb) L² is R¹¹ or -L⁴-(CH₂)ₛ-R¹¹;
s is 1, 2, or 3;
L⁴ is 5- to 7-membered carbocyclic ring or 5- to 7-membered heterocyclic ring; in the 5-to 7-membered heterocyclic ring, the number of heteroatoms is 1, 2, 3, or 4, and each heteroatom is independently selected from N, O, and S;
R¹¹ is 8- to 20-membered saturated monocyclic or bridged carbocyclic ring, and 3, 4, 5, or 6 non-adjacent CH₂ of the monocyclic or bridged carbocyclic ring are independently replaced by -Y₅-, and each Y₅ is independently -O-, -NH-, or-N (R^{11a})-;
each R^{11a} is independently C₁-C₄ alkyl or C₁-C₄ alkyl substituted by one or more -COOH; when L² is -L⁴-(CH₂)ₛ-R¹¹, the -L⁴-(CH₂)ₛ-R¹¹ is 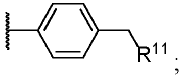
each Y₅ is independently -NH- or -N(R^{11a})-;
each R^{11a} is independently C₁-C₄ alkyl substituted by one or more -COOH;
cc) in the metal complex, the molar ratio of the compound represented by general formula II to the metal atom or ion is 1: 1;
dd) the metal ion is ion of the following metals: Al, Cu, Ga, Y, Zr, Tc, In, Lu, Re, At, Bi, or Tl;
ee) the valence state of the metal ion is monovalent, divalent, trivalent, or tetravalent;
ff) the metal ion is radioactive metal ion or non-radioactive metal ion;
gg) the metal ion further binds to non-metal nuclide;
hh) the metal ion is radioactive as a whole.

6. The compound represented by general formula II or the metal complex thereof, or their tautomers thereof, or their stereoisomers thereof, or their pharmaceutically acceptable salts thereof, or their solvates thereof according to claim 5, wherein, it satisfies one or more of the following conditions:
ii) the metal complex has the structure represented by the following general formula I: 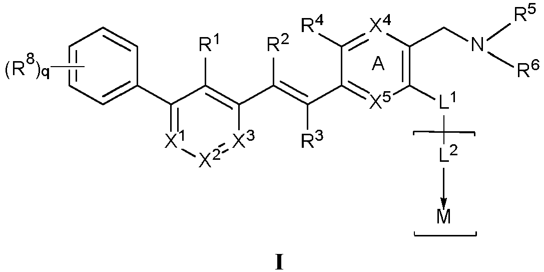 wherein, M is the metal ion;
jj) in R⁴, the C₁-C₄ alkyl substituted by one or more R^{b} is trifluoromethyl;
kk) in the 5- to 7-membered heterocyclic ring substituted by one or more R^{c}, which is formed by R⁵, R⁶ together with the nitrogen atom they are attached, the 5- to 7-membered heterocyclic ring is piperidine ring;
ll) m2 is 1 or 2;
mm) m3 is 1 or 2;
nn) when Y₂ is 5- to 7-membered carbocyclic ring, the 5- to 7-membered carbocyclic ring is 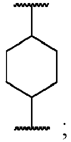
oo) when Y₂ is 5- to 7-membered heterocyclic ring, the 5- to 7-membered heterocyclic ring is 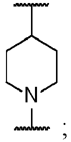
pp) R⁷ is 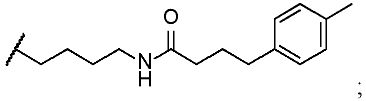
qq) L² is R¹¹;
rr) in L⁴, the 5- to 7-membered carbocyclic ring is 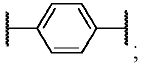
ss) in R¹¹, the 8- to 20-membered is 8-, 9-, 10-, 11-, 12-, 13-, 14-, 15-, or 16-membered;
tt) in R^{11a}, the C₁-C₄ alkyl substituted by one or more -COOH is -CH₂-COOH;
uu) the metal ion is ion of the following metals: ²⁷Al, ⁶³Cu, ⁶⁴Cu, ⁶⁸Ga, ⁷⁰Ga, ⁸⁹Y, ⁹⁰Y, ⁸⁹Zr, ⁹¹Zr, ^{99m}Tc, ¹¹¹In, ¹¹³In, ¹⁷⁵Lu, ¹⁷⁷Lu, ¹⁸⁶Re, ¹⁸⁸Re, ²¹¹At, ²¹²Bi, ²¹³Bi, ²⁰¹Tl, or ²⁰³Tl;
vv) the valence state of the metal ion is trivalent;
ww) the non-metal nuclide is radioactive non-metal nuclide or non-radioactive non-metal nuclide, and the radioactive non-metal nuclide for example ¹⁸F.

7. The compound represented by general formula II or the metal complex thereof, or their tautomers thereof, or their stereoisomers thereof, or their pharmaceutically acceptable salts thereof, or their solvates thereof according to claim 6, wherein, it satisfies one or more of the following conditions:
xx) the 5- to 7-membered heterocyclic ring substituted by one or more R^{c}, which is formed by R⁵, R⁶, and together with the nitrogen atom they are attached is 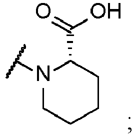
yy) when Y₂ is 5- to 7-membered carbocyclic ring, the 5- to 7-membered carbocyclic ring is 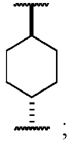
zz) L² is 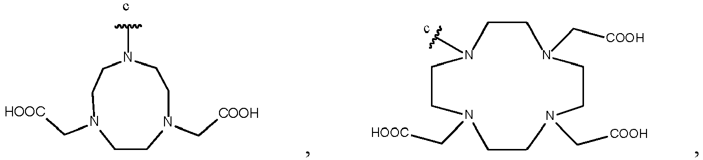 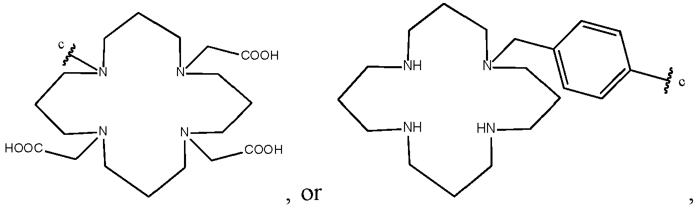 wherein the c-terminal is connected to L¹;
aaa)the metal ion is [Al¹⁸F]²⁺, [⁶⁸GaCl]²⁺, [¹⁷⁷LuCl]²⁺, ⁶⁸Ga³⁺, or ¹⁷⁷Lu³⁺.

8. The compound represented by general formula II or the metal complex thereof, or their tautomers thereof, or their stereoisomers thereof, or their pharmaceutically acceptable salts thereof, or their solvates thereof according to claim 1, wherein, it satisfies one or more of the following conditions:
bbb) 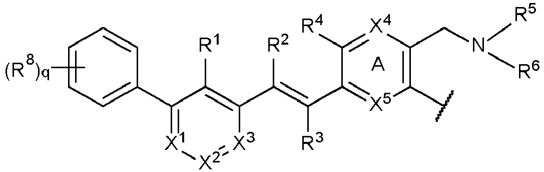 is 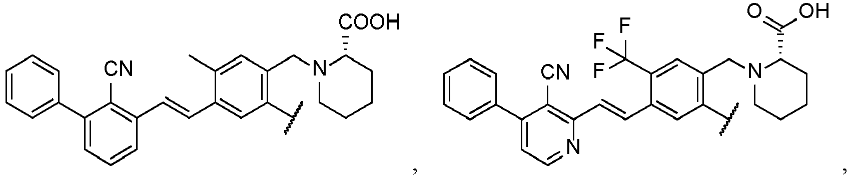 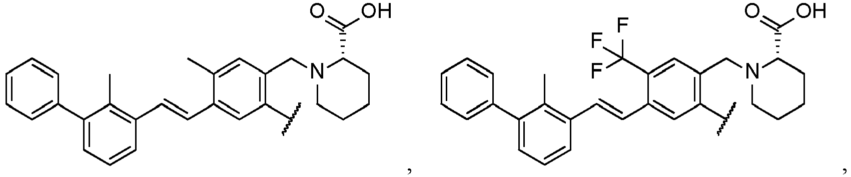 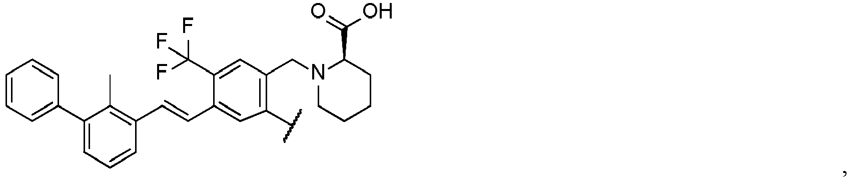 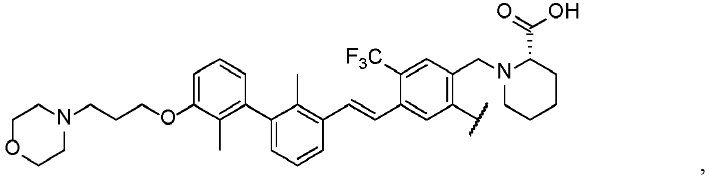 or 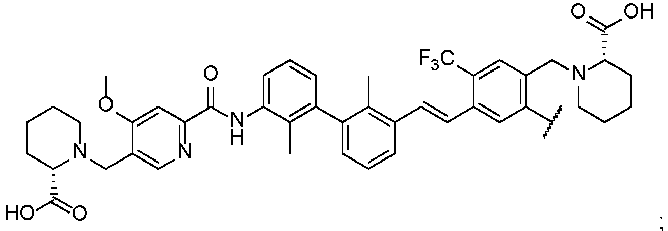
ccc)L¹ is 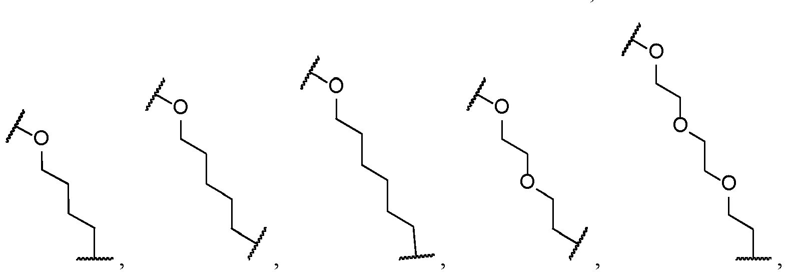 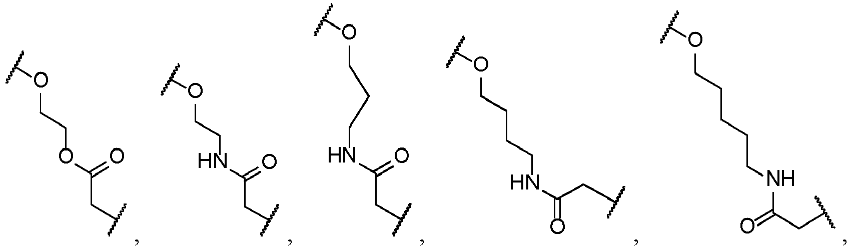 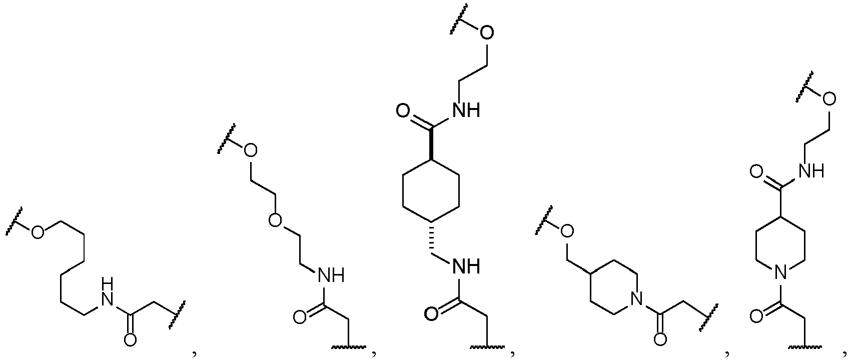 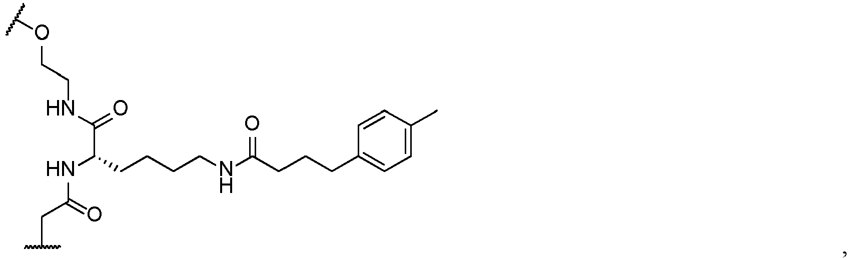 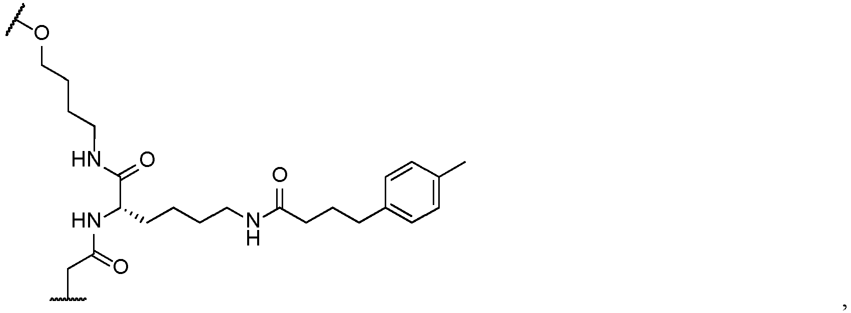 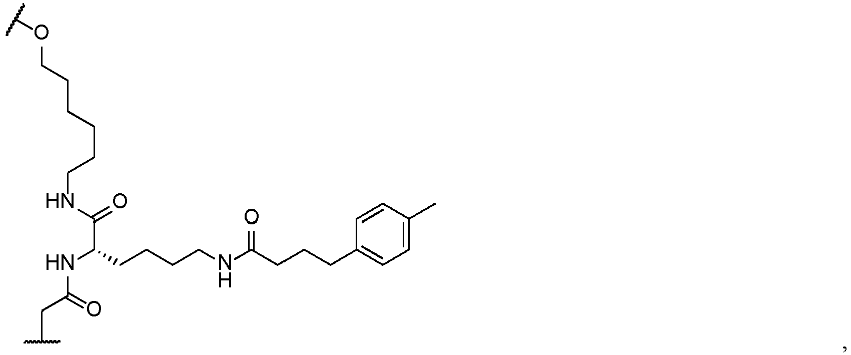 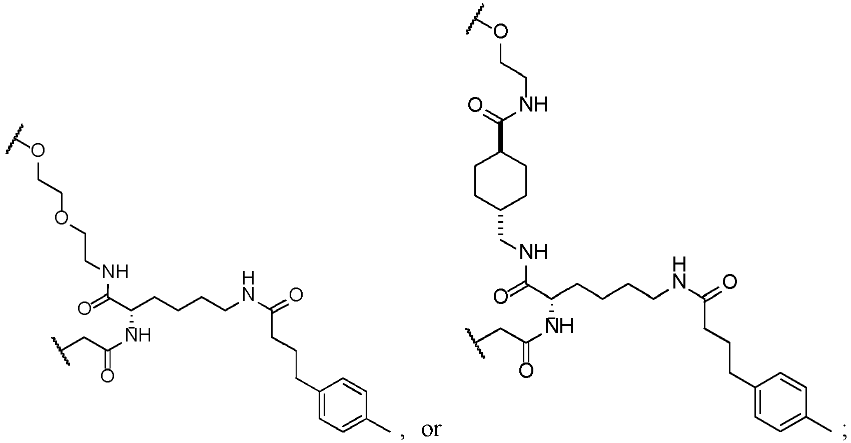 wherein the upper end is connected to ring A and the lower end is connected to L²;
ddd) L² is 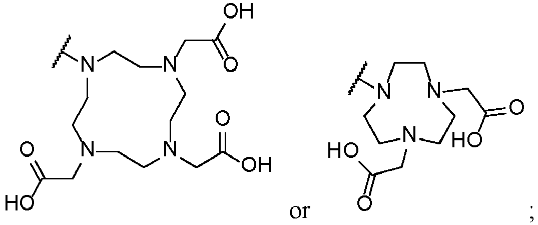
eee)the L² and the metal ion form any one of the following groups: 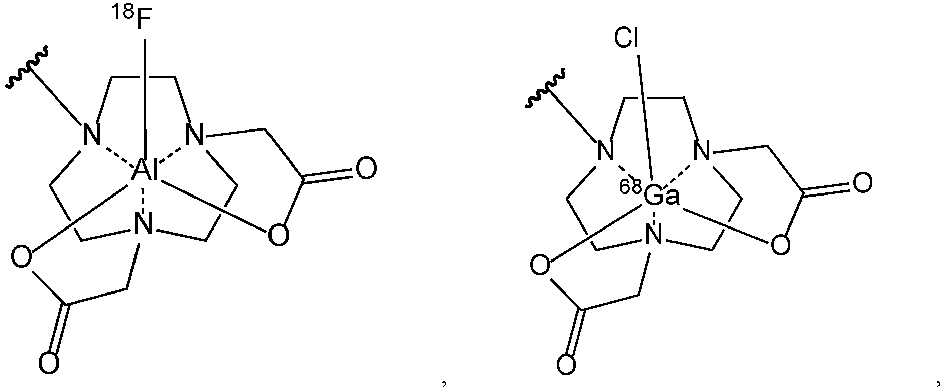 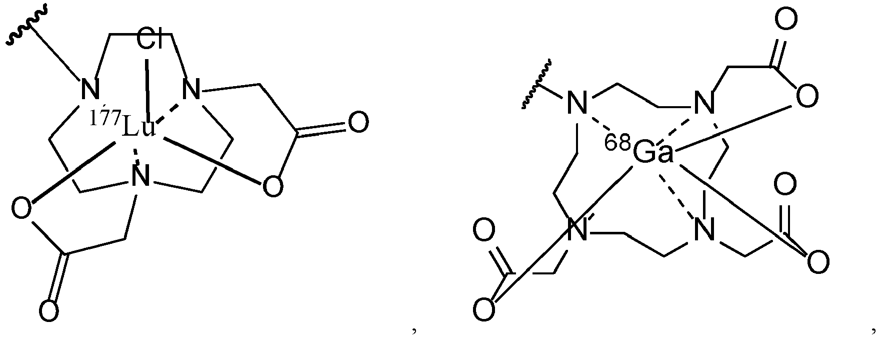 or

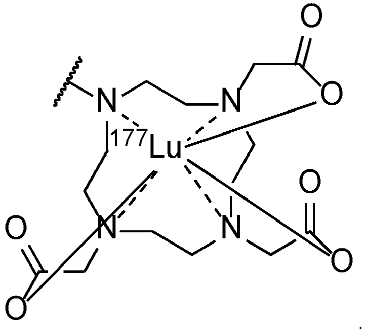

9. The compound represented by general formula II or the metal complex thereof, or their tautomers thereof, or their stereoisomers thereof, or their pharmaceutically acceptable salts thereof, or their solvates thereof according to claim 1, wherein: 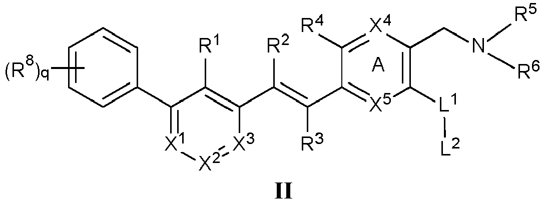
X¹, X², X³, X⁴, and X⁵ are each independently CH or N;
R¹ is hydrogen, halogen, cyano, C₁-C₄ alkyl, or C₁-C₄ alkyl substituted by one or more R^{a};
R² and R³ are each independently hydrogen or halogen;
R⁴ is hydrogen, halogen, C₁-C₄ alkyl or C₁-C₄ alkyl substituted by one or more R^{b};
R⁵ and R⁶ are each independently hydrogen, deuterium, C₁-C₄ alkyl, or C₁-C₄ alkyl substituted by one or more R^{c}; or, R⁵, R⁶ together with the nitrogen atom to which they are attached form a 5- to 7-membered heterocyclic ring, or a 5- to 7-membered heterocyclic ring substituted by one or more R^{c}; in the 5- to 7-membered heterocyclic ring, the number of heteroatoms is 1, 2, 3, or 4, and each heteroatom is independently selected from N, O, and S;
each R^{a}, each R^{b} and each R^{c} are independently deuterium, halogen, hydroxyl, amino, C₁-C₄ alkyl, C₁-C₄ alkyl-O-, -COOH, or -C(O)OR^{g};
each R⁸ is independently hydrogen, deuterium, halogen, hydroxyl, amino, C₁-C₄ alkyl, C₁-C₄ alkyl-S-, C₁-C₄ alkyl-O-, -C(O)ONH₂, -C(O)OC₁₋₄ alkyl, -OR^{8a}, -NHR^{8a}, -NR^{8a}R^{8b}, C₁-C₄ alkyl substituted by one or more R^{8c}, C₁-C₄ alkyl-S- substituted by one or more R^{8c}, or C₁-C₄ alkyl-O- substituted by one or more R^{8c};
or, two adjacent R⁸ together with the carbon atoms on the benzene ring to which they are attached form a 5- to 7-membered carbocyclic ring, a 5- to 7-membered heterocyclic ring, a 5-to 7-membered carbocyclic ring substituted by one or more C₁₋₄ alkyl, or a 5- to 7-membered heterocyclic ring substituted by one or more C₁₋₄ alkyl; in the 5- to 7-membered heterocyclic ring, the number of heteroatoms is 1, 2, 3, or 4, and each heteroatom is independently selected from N and O;
each R^{8a}, R^{8b}, and each R^{8c} are independently C₁-C₄ alkyl-S-, halogen, C₁-C₄ alkyl, C₁-C₄ alkyl-O-, -COOH, -(C₁-C₄ alkylene)-COOH, -C(O)OC₁-C₄ alkyl, -C(O)NH₂, -C(O)NHC₁-C₄ alkyl, or -NR^{8e}R^{8f};
R^{8e} and R^{8f} are each independently hydrogen, halogen, C₁-C₄ alkyl, or C₁-C₄ alkyl substituted by one or more R^{8g};
each R^{8g} is independently halogen, C₁-C₄ alkyl, hydroxyl, -NR^{8h}R^{8k}, C₁-C₄ alkyl-O-, - COOH, -(C₁-C₄ alkylene)-COOH, -C(O)OC₁-C₄ alkyl, -C(O)NH₂, or -C(O)NHC₁-C₄ alkyl;
R^{8h} and R^{8k} are each independently hydrogen or C₁-C₄ alkyl;
q is 0, 1, 2, or 3;
L¹ is
(i) a single bond or -(CH₂)ₙ-;
(ii) -(CH₂)ₘ-, in which 1, 2, 3, 4, or 5 non-adjacent CH₂ are independently replaced by - Y₁-, and each Y₁ is independently -C(O)-, -C(O)O-, -O-, -NH-, -C(O)NH-, or -NHC(O)NH-; or
(iii) -(CH₂)ₚ-, in which one CH₂ is replaced by -Y₂-, and the other 0, 1, 2, 3, or 4 non-adjacent CH₂ are independently replaced by -Y₃-; each Y₃ is independently -C(O)-, -C(O)O-, - O-, -NH-, -C(O)NH-, or -NHC(O)NH-; Y₂ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S;
L¹ is unsubstituted or 1, 2, or 3 H contained in L¹ are each independently substituted by R7;
n, m, and p are each independently 1, 2, 3, 4, 5, 6, 7, 8, 9, 10, 11, 12, 13, or 14;
each R⁷ is independently C₁-C₄ alkyl or -L³-R⁹;
L³ is
(i) -(CH₂)ⱼ-; or
(ii) -(CH₂)ₖ-, in which 1, 2, 3, or 4 non-adjacent CH₂ are independently replaced by -Y₄-, and each Y₄ is independently -C(O)-, -C(O)O-, -O-, -NH-, -C(O)NH-, or -NHC(O)NH-;
L³ is unsubstituted or 1, 2, or 3 H contained in L³ are each independently substituted by R¹⁰;
j and k are each independently 1, 2, 3, 4, 5, 6, 7, 8, 9, 10, 11, 12, 13, or 14;
R⁹ is hydrogen, C₆-C₁₀ aryl, or C₆-C₁₀ aryl substituted by one or more R^{d};
each R¹⁰ is independently C₁-C₄ alkyl;
each R^{d} is independently C₁-C₄ alkyl or C₁-C₄ alkyl substituted by one or more R^{e};
each R^{e} is independently hydroxyl, amino, C₁-C₄ alkyl, C₁-C₄ alkyl-O-, -COOH, or - C(O)OR^{h};
R^{g} and R^{h} are each independently C₁-C₄ alkyl or halogenated C₁-C₄ alkyl;
L² is metal chelating group;
the metal complex is a complex chelated by the compound represented by general formula II with the metal atom or ion.

10. The compound represented by general formula II or the metal complex thereof, or their tautomers thereof, or their stereoisomers thereof, or their pharmaceutically acceptable salts thereof, or their solvates thereof according to claim 1 or 9, wherein, the metal is Cu, Ga, Y, Zr, Tc, In, Lu, Re, At, Bi, Tl, or their radioactive or non-radioactive isotopes thereof;
and/or, the valence state of the metal ion is monovalent, divalent, trivalent, or tetravalent;
and/or, X¹ is CH;
and/or, X² is CH;
and/or, X³ is CH;
and/or, X⁴ is CH;
and/or, X⁵ is CH;
and/or, q is 0;
and/or, R¹ is cyano or C₁-C₄ alkyl;
and/or, R² is hydrogen;
and/or, R³ is hydrogen;
and/or, R⁴ is C₁-C₄ alkyl or C₁-C₄ alkyl substituted by one or more R^{b};
and/or, each R^{b} is independently halogen;
and/or, when R⁵, R⁶ together with the nitrogen atom to which they are attached form a 5-to 7-membered heterocyclic ring or a 5- to 7-membered heterocyclic ring substituted by one or more R^{c}, the 5- to 7-membered heterocyclic ring is piperidine;
and/or, n, m, and p are each independently 5, 6, 7, 8, 9, 10, 11, or 12;
and/or, L¹ is a single bond, -(CH₂)ₙ₁NH(CH₂)ₘ₁-, -(CH₂)ₙ₁O(CH₂)ₘ₁-, - (CH₂)ₙ₁NHC(O)NH(CH₂)ₘ₁-, -O(CH₂)ₙ₁NH-, -O(CH₂)ₙ₁O-, -O(CH₂)ₙ₁O(CH₂)ₘ₁O-, - NH(CH₂)ₙ₁NH(CH₂)ₘ₁NH-, -NH(CH₂)ₙ₁O(CH₂)ₘ₁O-, -NH(CH₂)ₙ₁NH(CH₂)ₘ₁O-, - O(CH₂)ₙ₁NH(CH₂)ₘ₁O-, -O(CH₂)ₙ₁O(CH₂)ₘ₁NH-, -O(CH₂)ₙ₁NH(CH₂)ₘ₁NH-, - NH(CH₂)ₙ₁O(CH₂)ₘ₁NH-, -O-Y₂-(CH₂)ₙ₁NH-, -O(CH₂)ₙ₂OC(O)(CH₂)ₘ₂-, - O(CH₂)ₙ₂O(CH₂)ₘ₂-, -O(CH₂)ₙ₂NHC(O)(CH₂)ₘ₂-, -O(CH₂)ₙ₂NHC(O)-(CH₂)ₙ₃-NHC(O)(CH₂)ₘ₃-, -O-(CH₂)ₙ₂-Y₂-C(O)-(CH₂)ₘ₂-, -O(CH₂)ₙ₂NHC(O)-Y₂-(CH₂)ₙ₃NHC(O)-(CH₂)ₘ₃-, -O(CH₂)ₙ₂NHC(O)-Y₂-C(O)-(CH₂)ₘ₃-, -O(CH₂)ₙ₂-Y₂-C(O)-(CH₂)ₘ₃-, - O(CH₂)ₙ₂NHC(O)-Y₂-(CH₂)ₙ₃NHC(O)-(CH₂)ₙ₄NHC(O)-(CH₂)ₘ₃, -O(CH₂)ₙ₂NHC(O)-(CH₂)ₙ₃NHC(O)-(CH₂)ₘ₃-, or -O(CH₂)ₙ₂-O(CH₂)ₙ₃-NHC(O)-(CH₂)ₙ₄NHC(O)-(CH₂)ₘ₃-, each n1, each n2, each n3, n4, each m1, each m2 and each m3 are each independently 1, 2, 3, 4, 5, or 6; L¹ is unsubstituted or 1, 2, or 3 H contained in L¹ are each independently substituted by R7;
and/or, when Y₂ is 5- to 7-membered carbocyclic ring, the 5- to 7-membered carbocyclic ring is 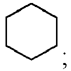
and/or, when Y₂ is 5- to 7-membered heterocyclic ring, the 5- to 7-membered heterocyclic ring is 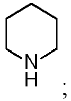
and/or, L² is R¹¹ or -L⁴-(CH₂)ₛ-R¹¹; s is 1, 2, or 3; L⁴ is 5- to 7-membered carbocyclic ring or 5- to 7-membered heterocyclic ring; in the 5- to 7-membered heterocyclic ring, the number of heteroatoms is 1, 2, 3, or 4, and each heteroatom is independently selected from N, O, and S; R¹¹ is 8- to 20-membered saturated monocyclic or bridged carbocyclic ring, and 3, 4, 5, or 6 non-adjacent CH₂ of the monocyclic or bridged carbocyclic ring are independently replaced by -Y₅-; each Y₅ is independently -O-,-NH-, or-N (R^{11a})-; each R^{11a} is independently C₁-C₄ alkyl or C₁-C₄ alkyl substituted by one or more -COOH;
and/or, each R⁷ is independently -L³-R⁹;
and/or, each Y₄ is independently -C(O)NH-;
and/or, L³ is -(CH₂)ₖ-, in which one CH₂ is replaced by -Y₄-; L³ is unsubstituted or 1, 2, or 3 H contained in L³ are each independently substituted by R¹⁰;
and/or, j and k are each independently 7, 8, or 9;
and/or, each Y₄ is independently -C(O)NH-;
and/or, R⁹ is phenyl or phenyl substituted by one or more R^{d};
and/or, each R^{d} is independently C₁-C₄ alkyl.

11. The compound represented by general formula II or the metal complex thereof, or their tautomers thereof, or their stereoisomers thereof, or their pharmaceutically acceptable salts thereof, or their solvates thereof according to claim 1 or 9, wherein, the metal is ⁶³Cu, ⁶⁴Cu, ⁶⁸Ga, ⁷⁰Ga, ⁸⁹Y, ⁹⁰Y, ⁸⁹Zr, ⁹¹Zr, ^{99m}Tc, ¹¹¹In, ¹¹³In, ¹⁷⁵Lu, ¹⁷⁷Lu, ¹⁸⁶Re, ¹⁸⁸Re, ²¹¹At, ²¹²Bi, ²¹³Bi, ²⁰¹Tl, or ²⁰³Tl;
and/or, X¹, X², X³, X⁴, and X⁵ are CH;
and/or, R¹ is cyano or methyl;
and/or, R⁴ is methyl or trifluoromethyl;
and/or, R⁵, R⁶ together with the nitrogen atom to which they are attached form 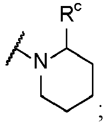
and/or, each R^{c} is independently -COOH;
and/or, L¹ is connected to ring A through -Y₁-, and Y₁ connected to ring A is -O-;
and/or, L¹ is connected to L² via -CH₂-;
and/or, L¹ is unsubstituted or one H contained in L¹ is substituted by R⁷;
and/or, R⁷ is 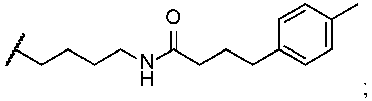
and/or, when Y₂ is 5- to 7-membered carbocyclic ring, the 5- to 7-membered carbocyclic ring is 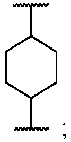
and/or, when Y₂ is 5- to 7-membered heterocyclic ring, the 5- to 7-membered heterocyclic 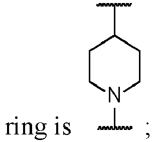
and/or, L² is 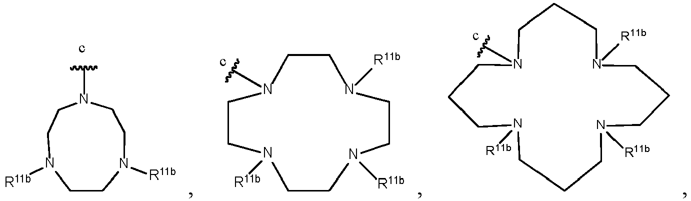 or 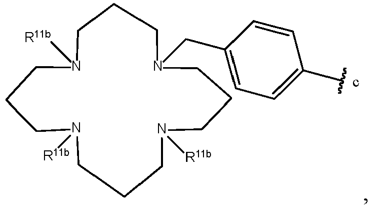 and each R^{11b} is independently H or R^{11a}; each R^{11a} is independently C₁-C₄ alkyl or C₁-C₄ alkyl substituted by one or more -COOH.

12. The compound represented by general formula II or the metal complex thereof, or their tautomers thereof, or their stereoisomers thereof, or their pharmaceutically acceptable salts thereof, or their solvates thereof according to claim 1 or 9, wherein, the metal complex is a complex chelated by the compound represented by general formula II with the metal ion;
and/or, the metal is Ga, Lu, or radioactive or their non-radioactive isotopes thereof;
and/or, R⁵, R⁶ together with the nitrogen atom to which they are attached form 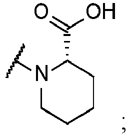
and/or, when Y₂ is 5- to 7-membered carbocyclic ring, the 5- to 7-membered carbocyclic ring is 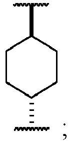
and/or, L¹ is 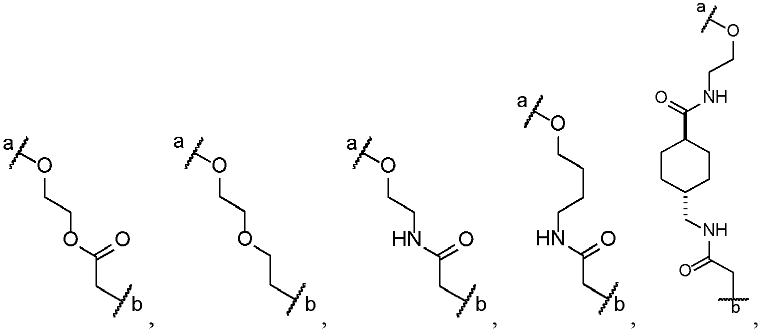 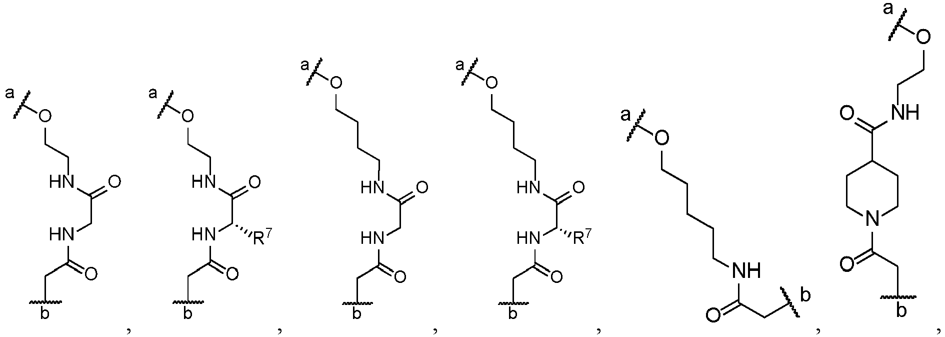 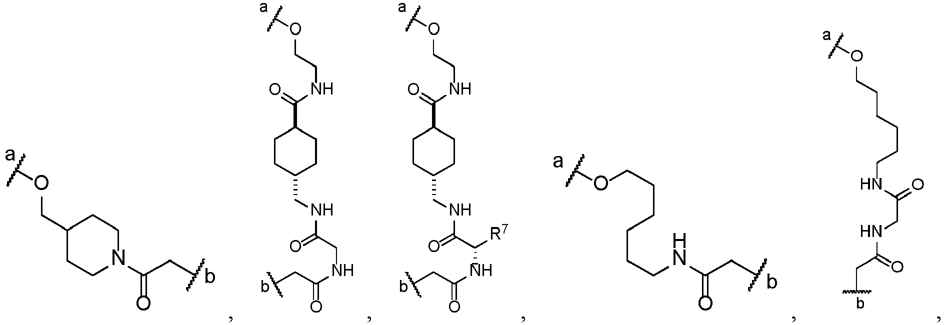 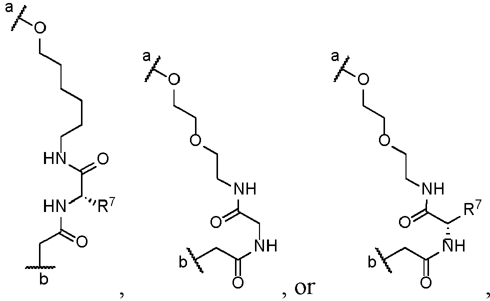 wherein the a-terminal is connected to ring A
and the b-terminal is connected to L²;
and/or, L² is 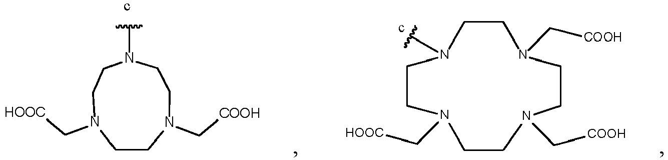 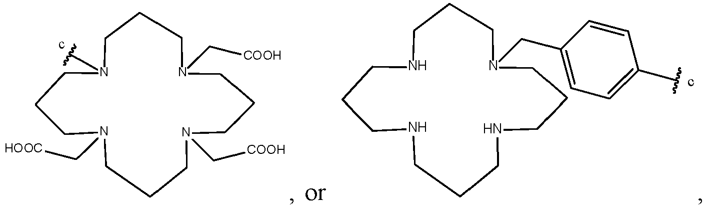 wherein the c-terminal is connected to L¹.

13. The compound represented by general formula II or the metal complex thereof, or their tautomers thereof, or their stereoisomers thereof, or their pharmaceutically acceptable salts thereof, or their solvates thereof according to claim 1 or 9, wherein, the metal ion is Ga³⁺ and Lu³⁺;
and/or, L¹ is 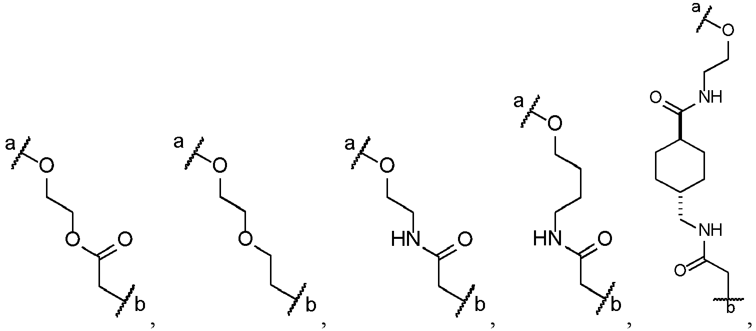 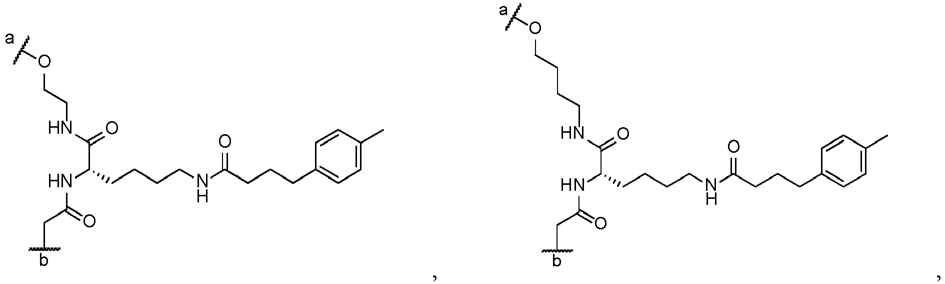 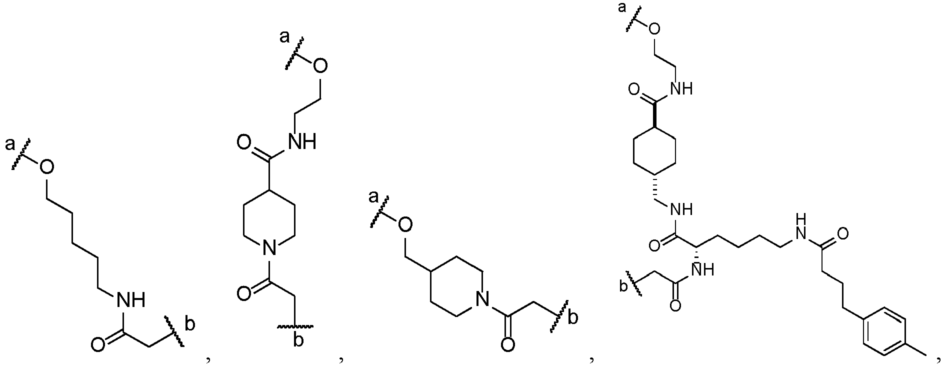 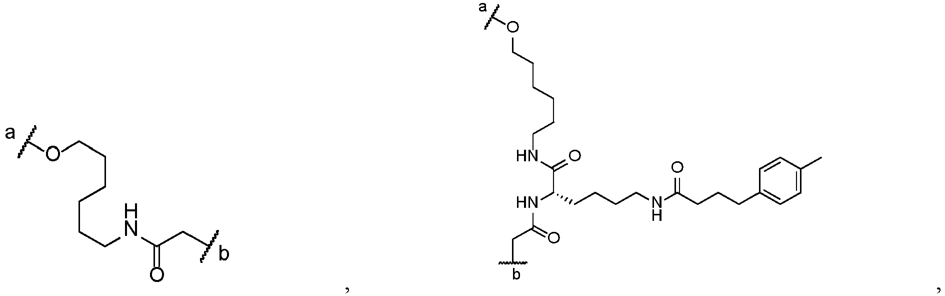 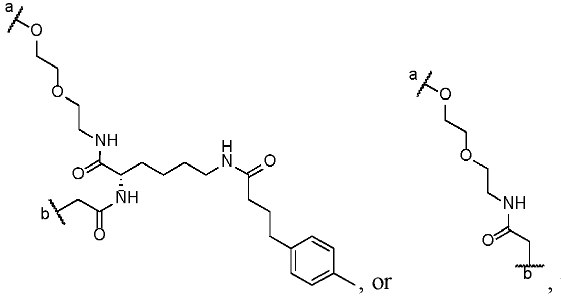 wherein the a-terminal is connected to ring A and the b-terminal is connected to L²; preferably, L¹ is 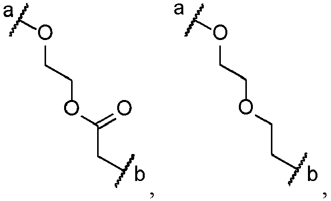 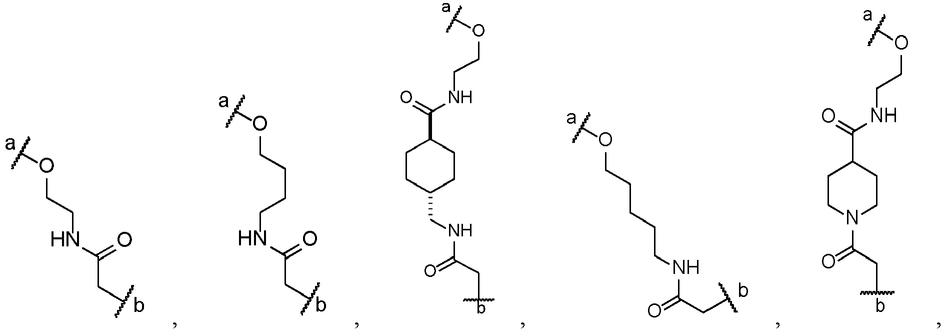 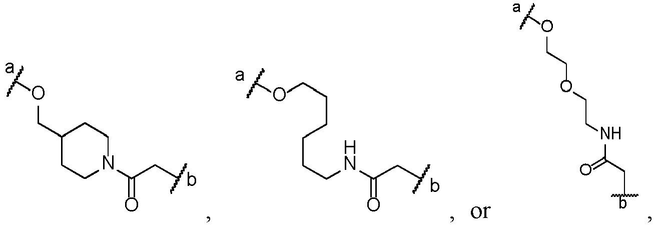 wherein the a-terminal is connected to ring A and the b-terminal is connected to L²;
and/or, L² is 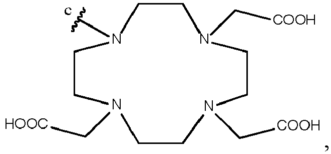 where the c-terminal is connected to L¹.

14. The compound represented by general formula II or the metal complex thereof, or their tautomers thereof, or their stereoisomers thereof, or their pharmaceutically acceptable salts thereof, or their solvates thereof according to claim 1 or 9, wherein, the metal complex has the following structure: 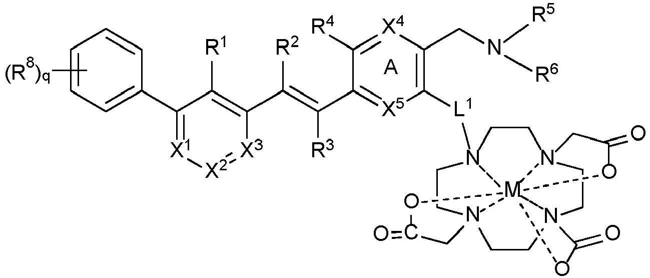 M is a trivalent metal ion.

15. The compound represented by general formula II or the metal complex thereof, or their tautomers thereof, or their stereoisomers thereof, or their pharmaceutically acceptable salts thereof, or their solvates thereof according to claim 1 or 9, wherein, the C₁-C₄ alkyl is independently methyl, ethyl, n-propyl, isopropyl, n-butyl, isobutyl, sec-butyl, or tert-butyl at any one occurrence;
and/or, the "one or more" is independently 1, 2, 3, 4, 5, or 6 at any one occurrence;
and/or, the C₆-C₁₀ aryl is independently phenyl or naphthyl at any one occurrence;
and/or, the 5- to 7-membered carbocyclic ring is independently 5, 6, or 7-membered saturated monocyclic carbocyclic ring at any one occurrence;
and/or, the 5- to 7-membered heterocyclic ring is independently 5, 6, or 7-membered saturated monocyclic heterocyclic ring at any one occurrence;
and/or, the halogen is independently F, Cl, Br, or I at any one occurrence.

16. The compound represented by general formula II or the metal complex thereof, or their tautomers thereof, or their stereoisomers thereof, or their pharmaceutically acceptable salts thereof, or their solvates thereof according to claim 1 or 9, wherein, the compound represented by the general formula II has any one of the following structures: 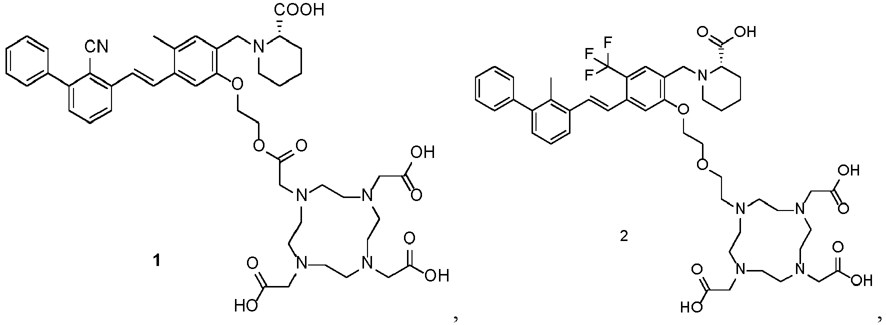 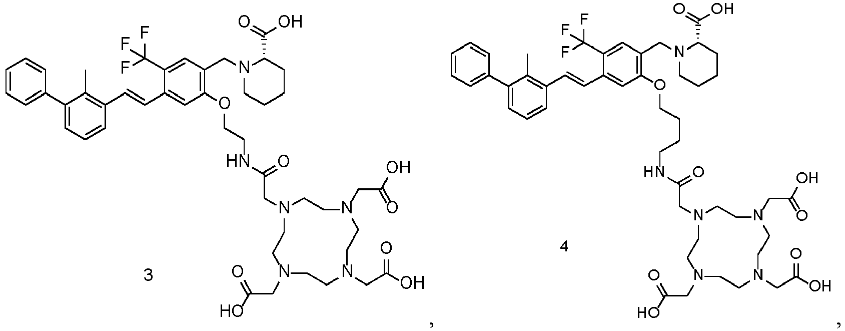 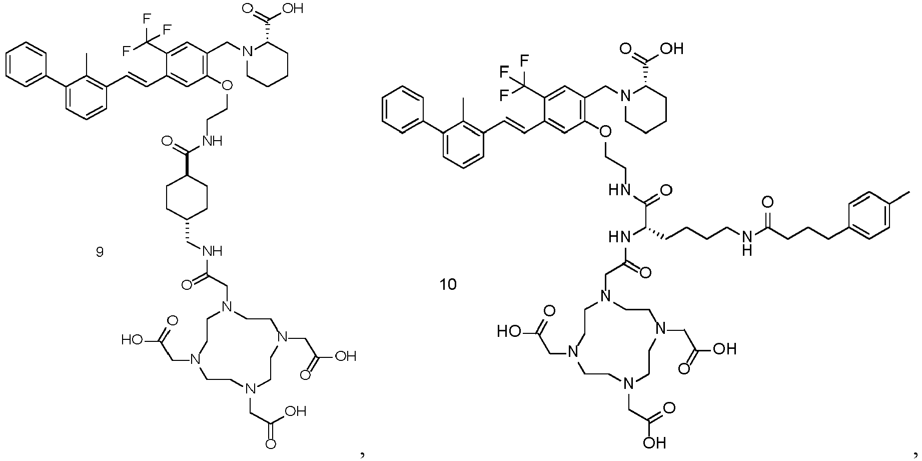 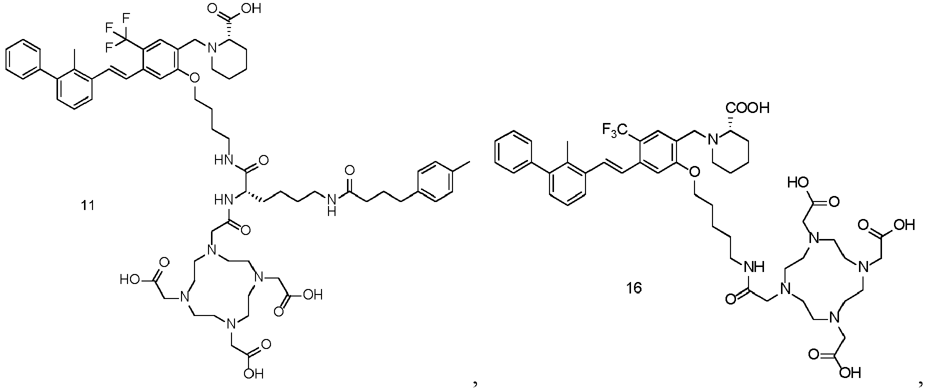 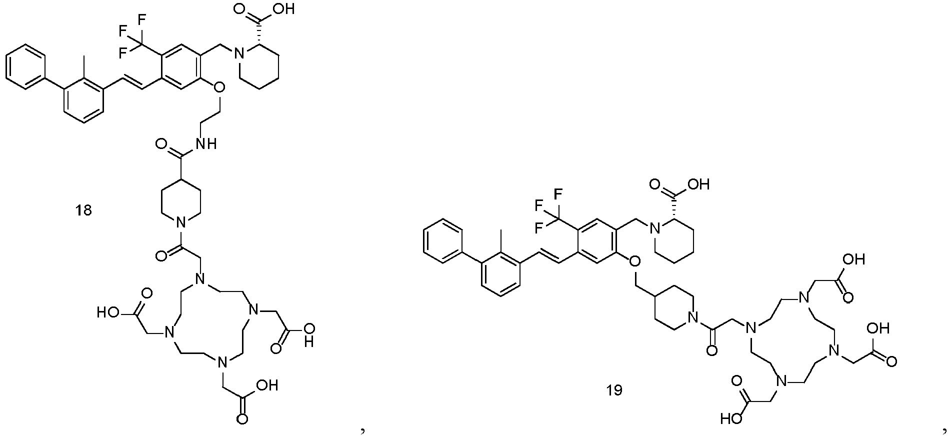 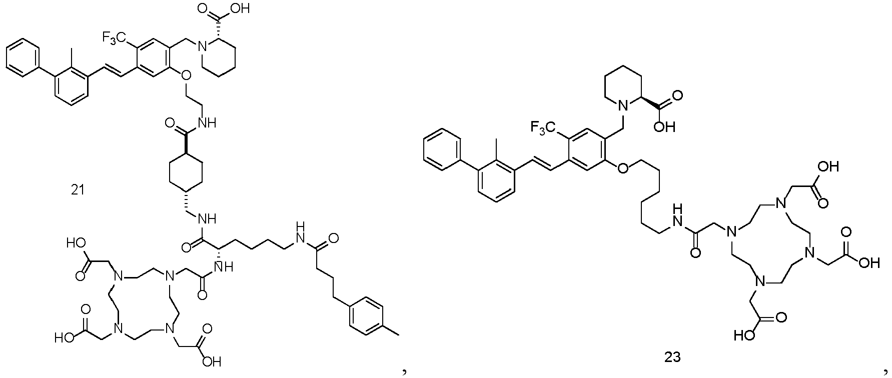 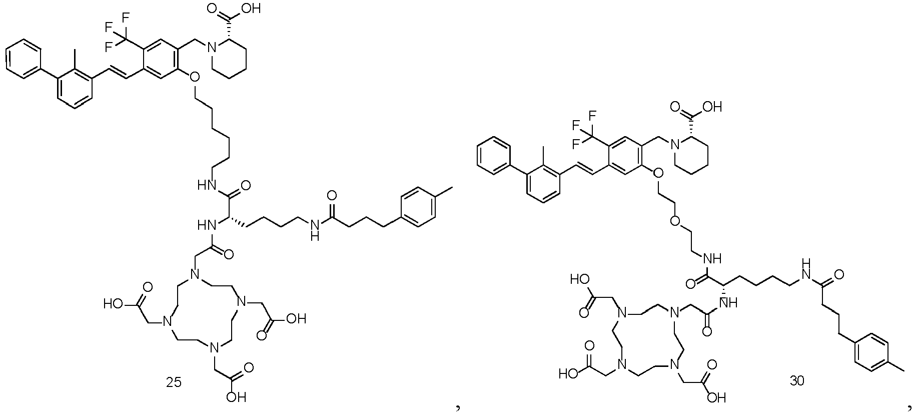 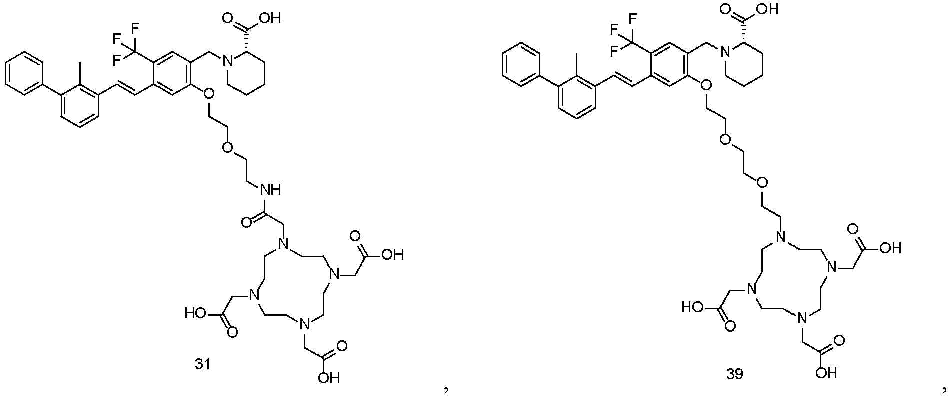 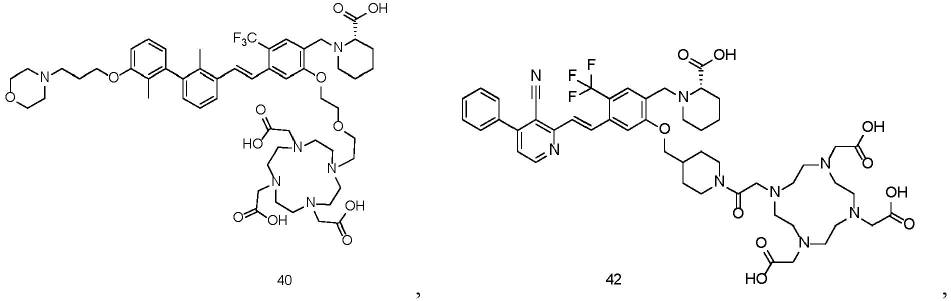 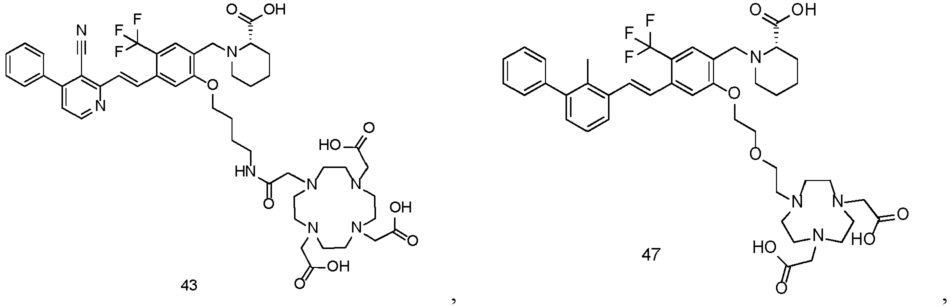 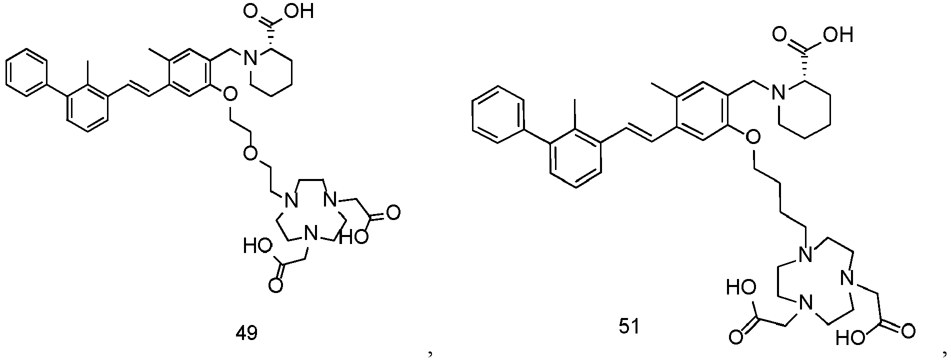 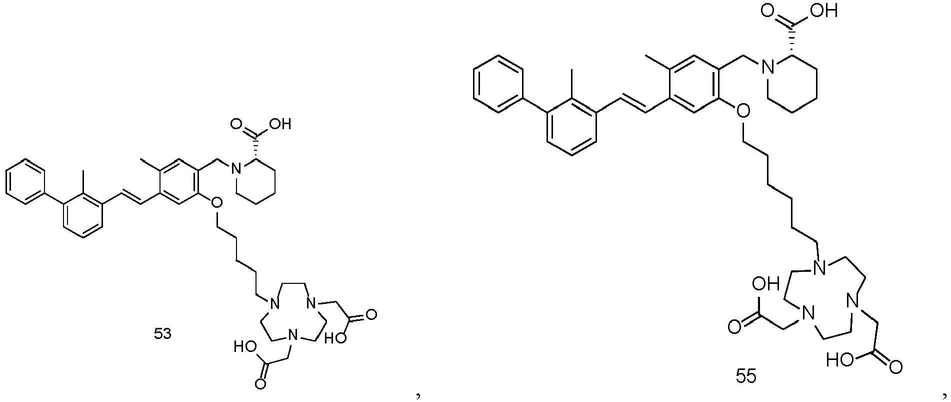 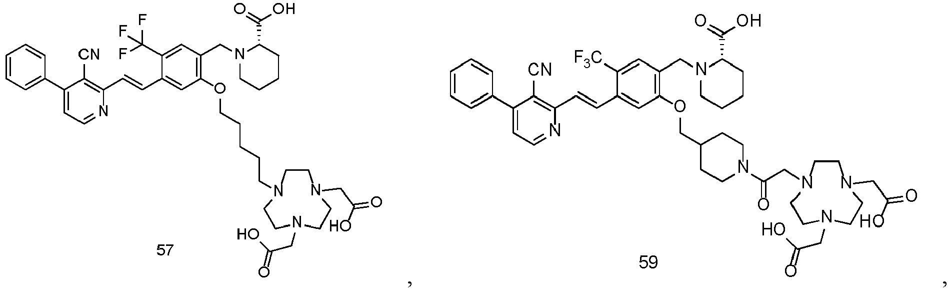 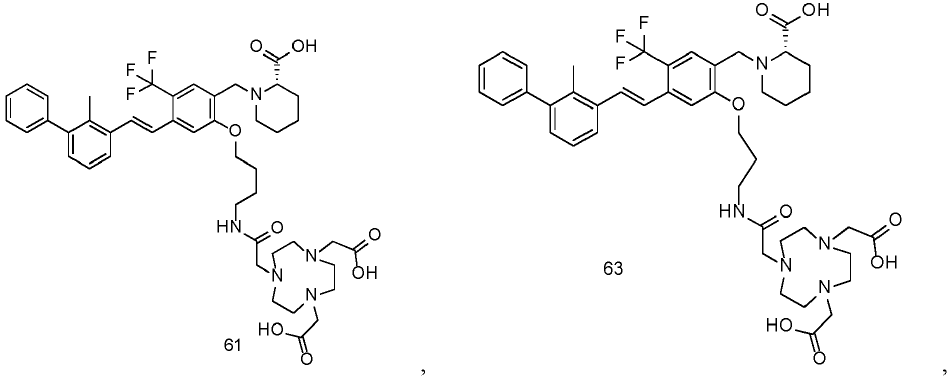 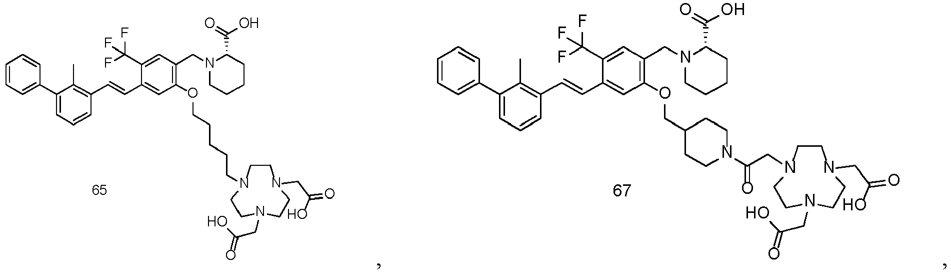 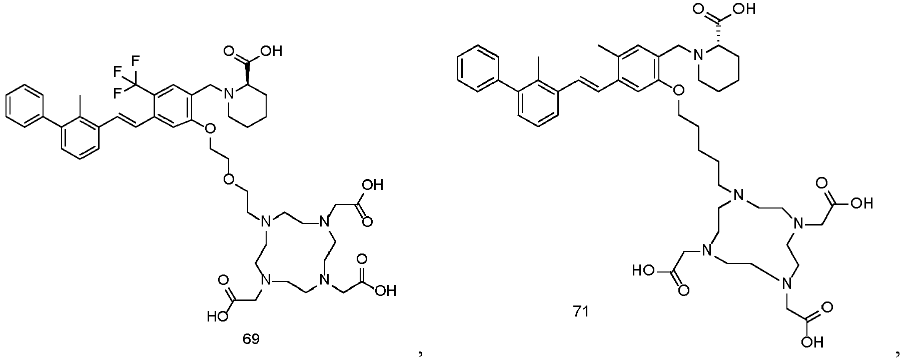 or 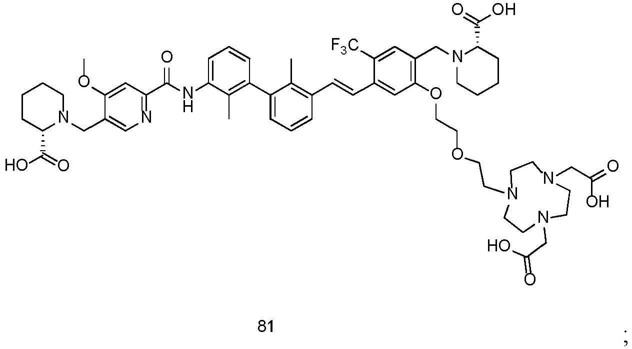
or, the metal complex is a complex formed by any one of the following compounds with ¹⁷⁷Lu³⁺: 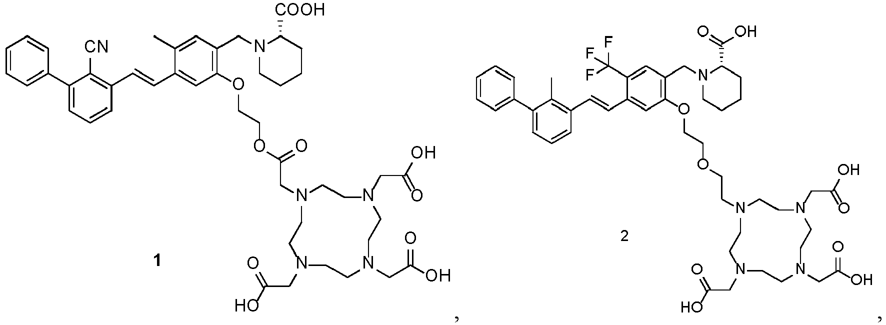 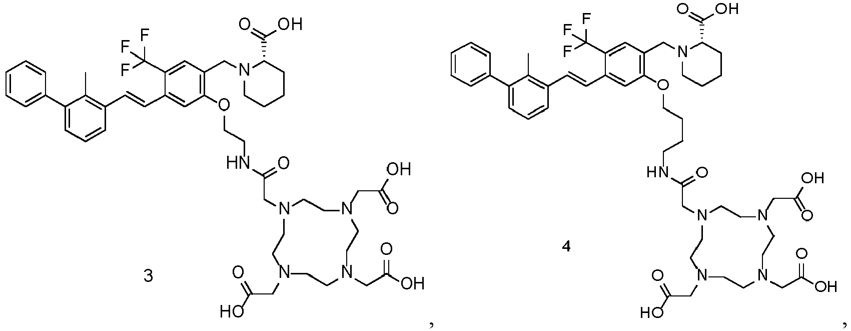 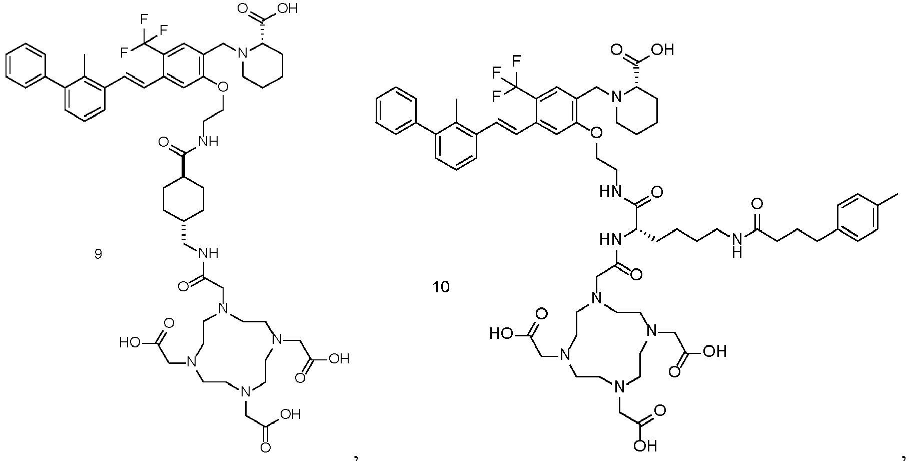 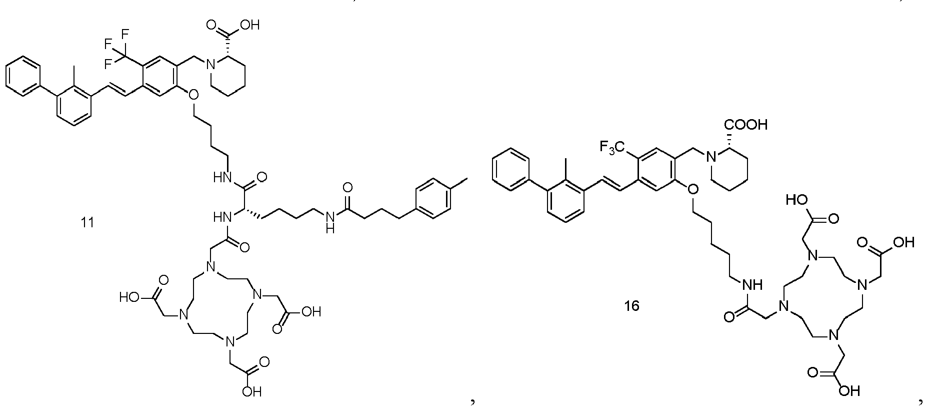 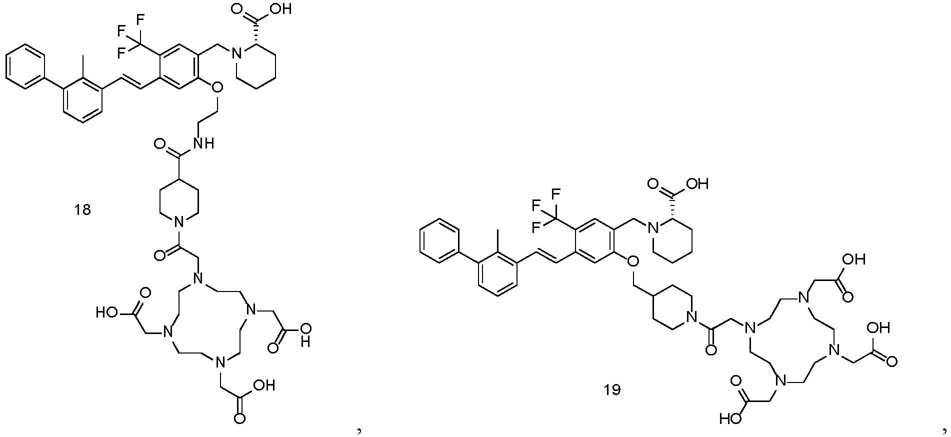 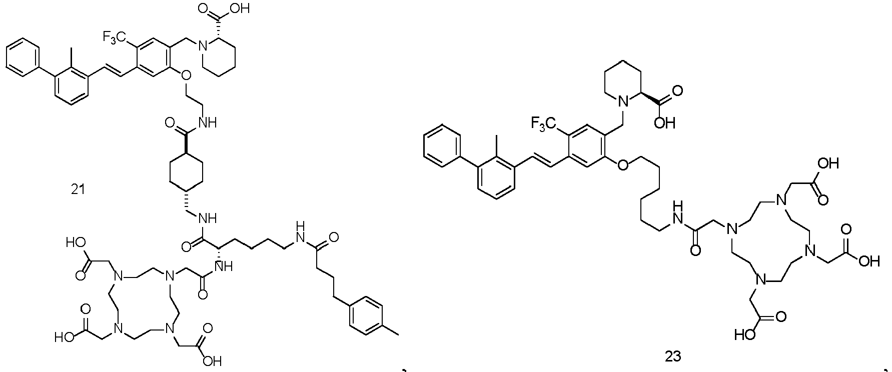 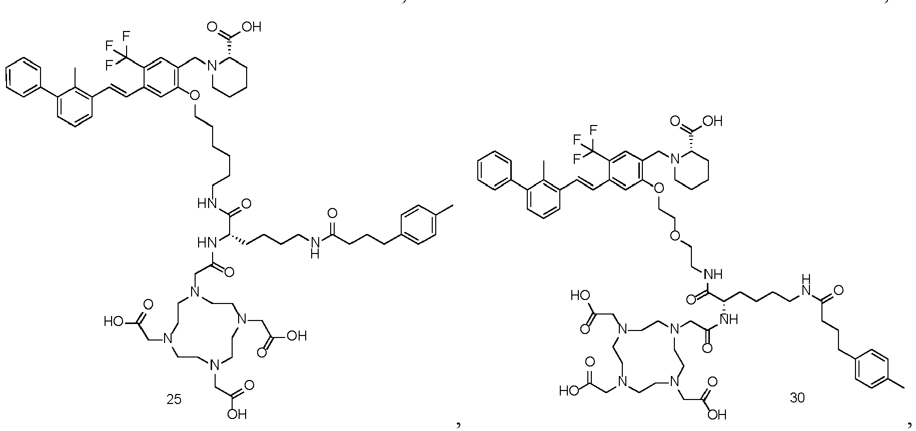 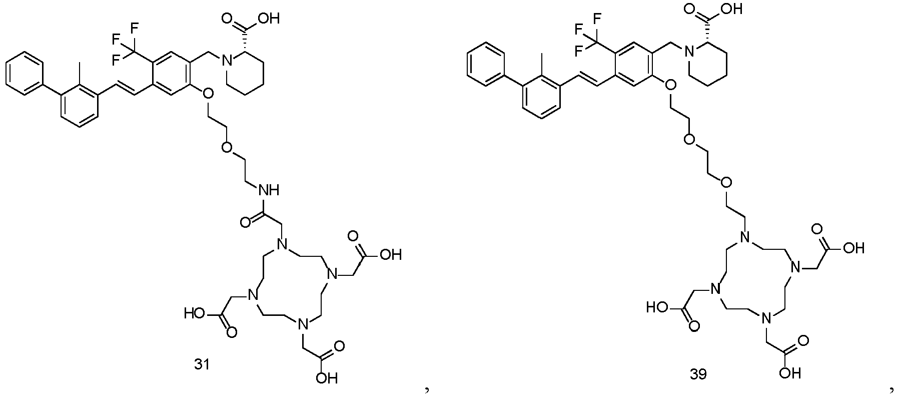 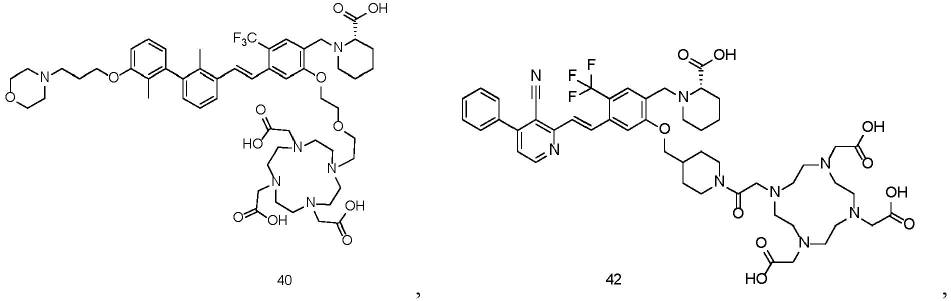 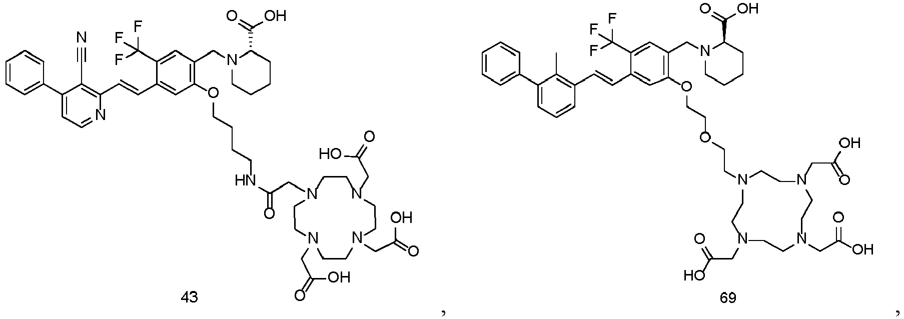 or 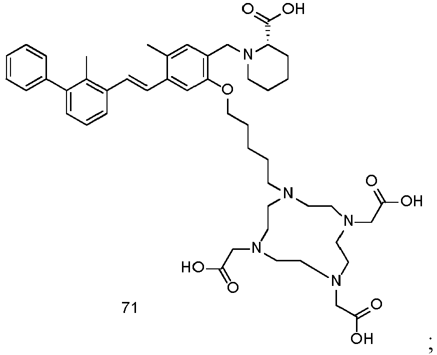
or, the metal complex is a complex formed by any one of the following compounds with ⁶⁸Ga³⁺: 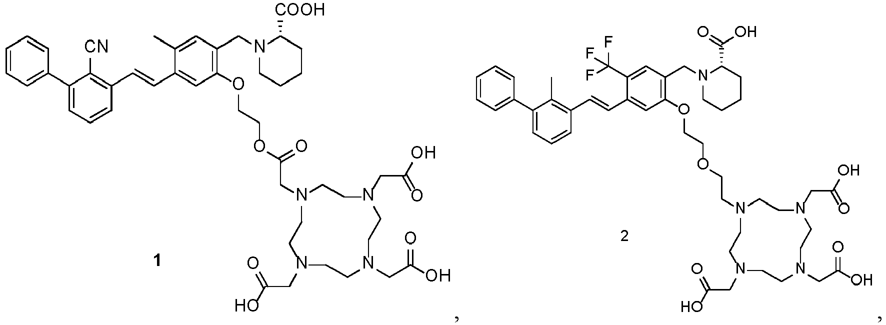 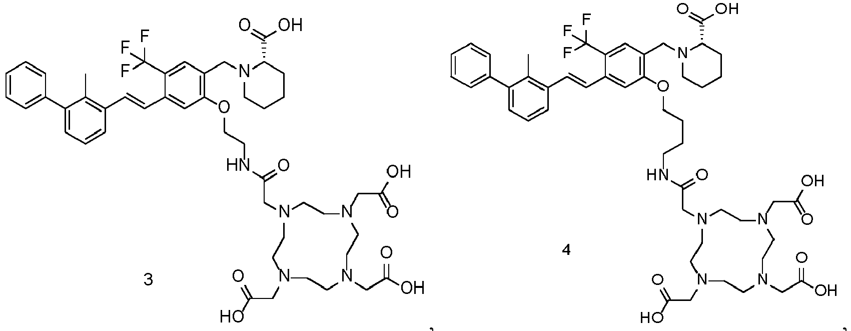 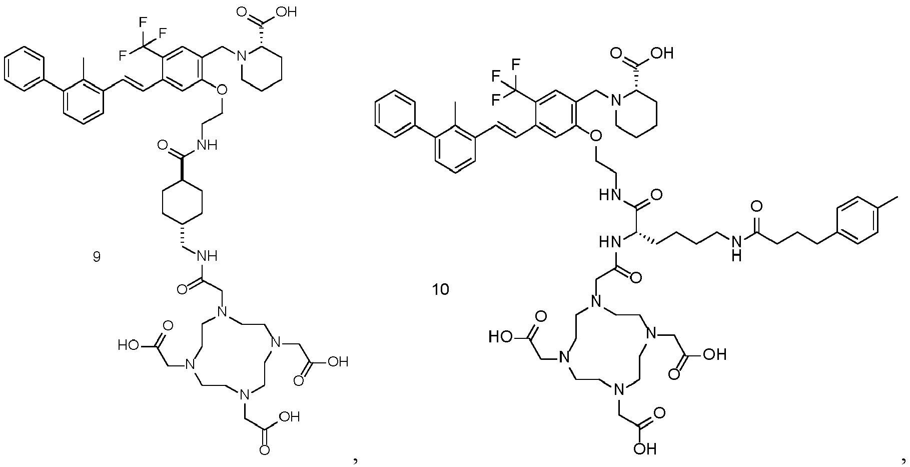 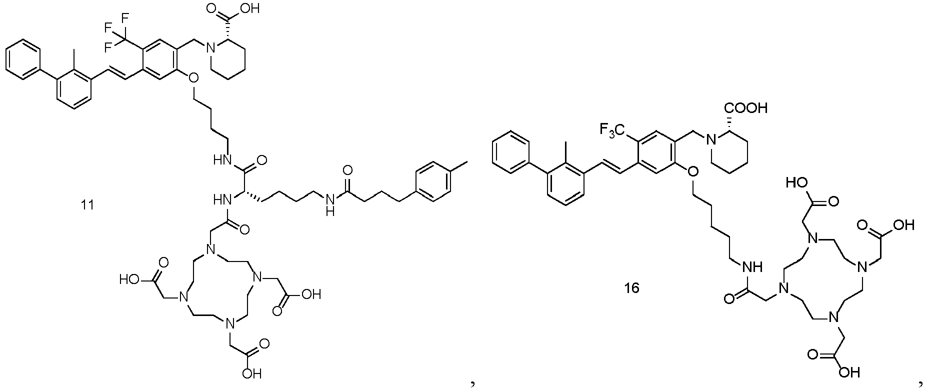 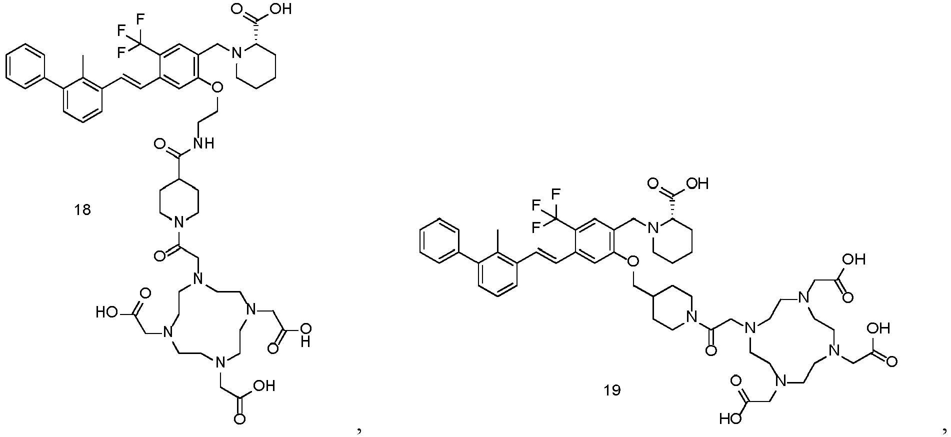 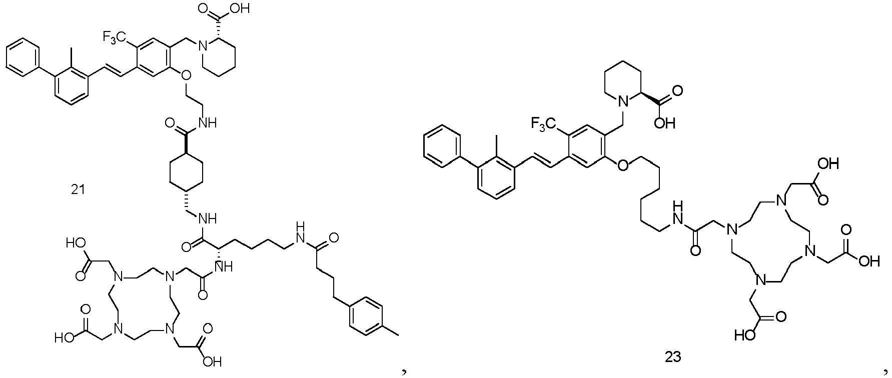 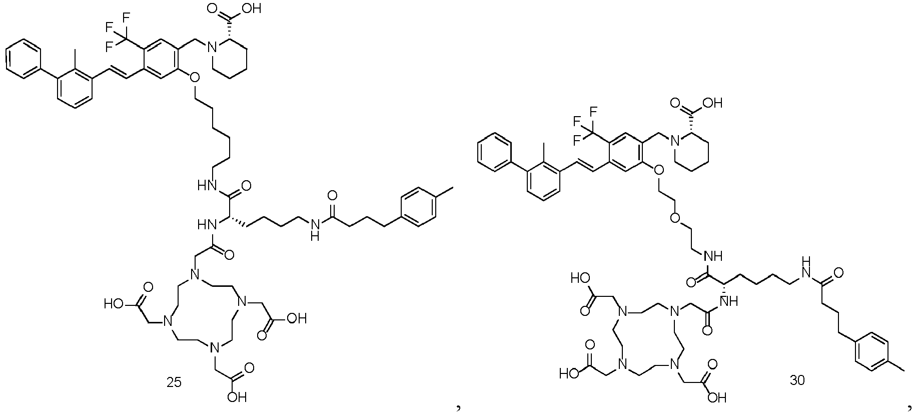 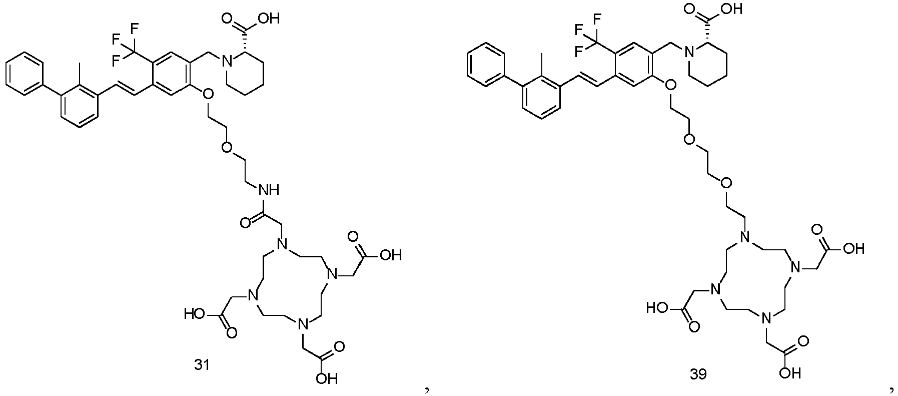 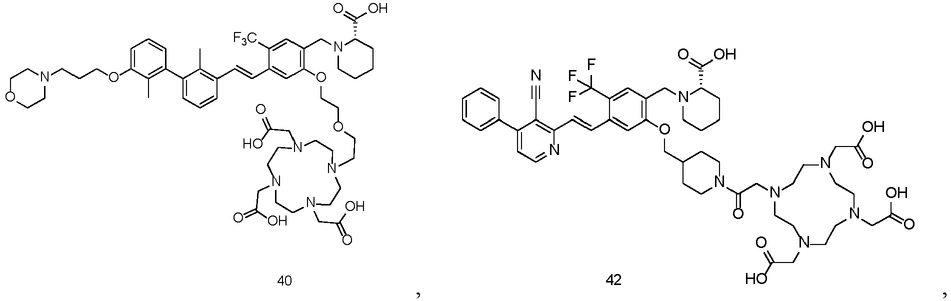 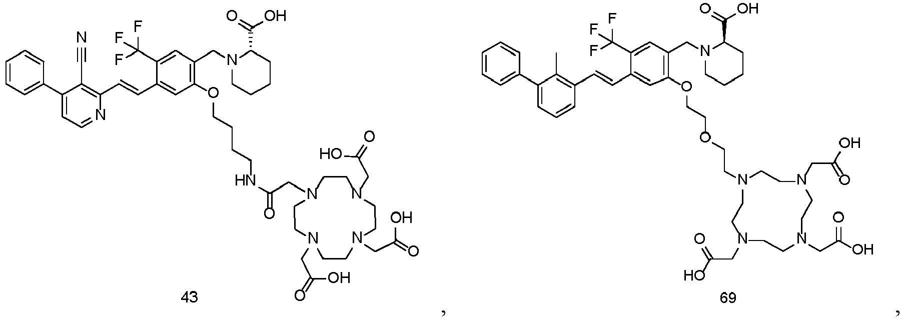 or 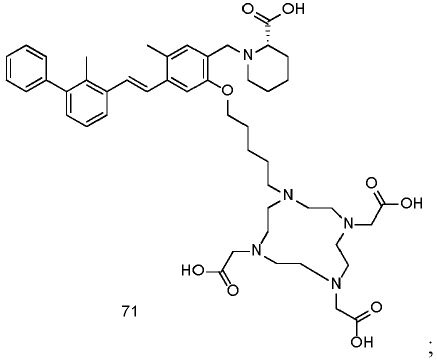
or, the metal complex is a complex formed by any one of the following compounds with [Al¹⁸F]²⁺: 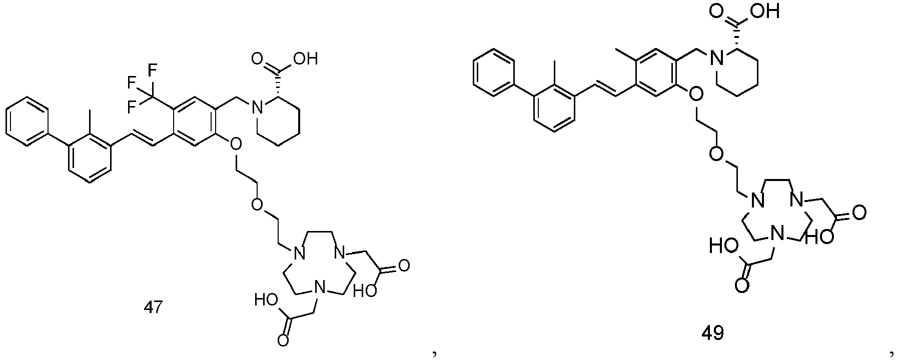 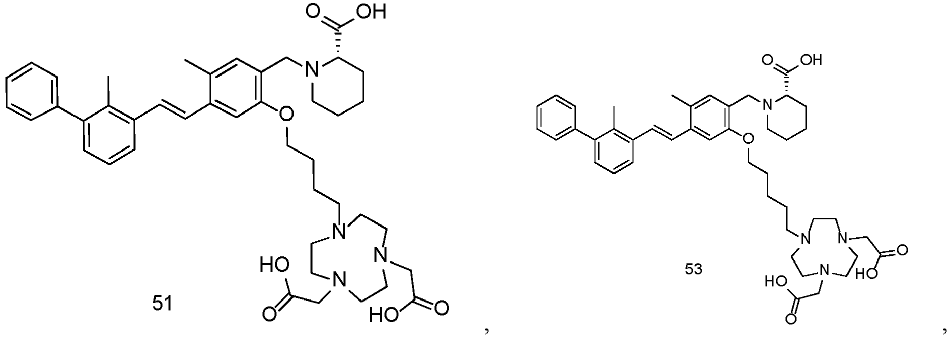 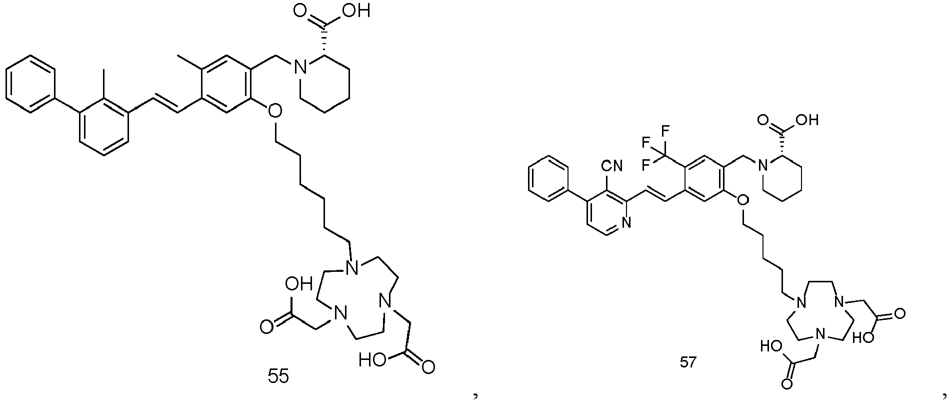 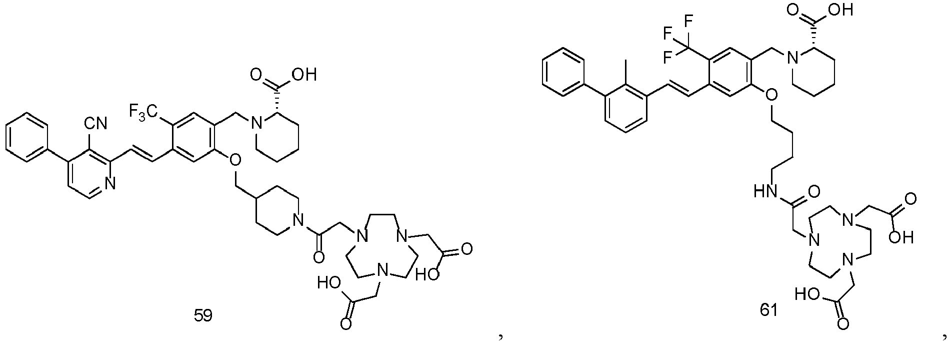 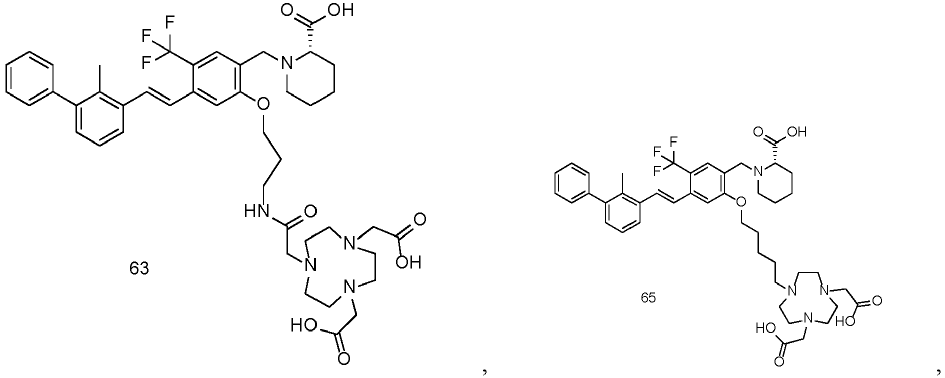 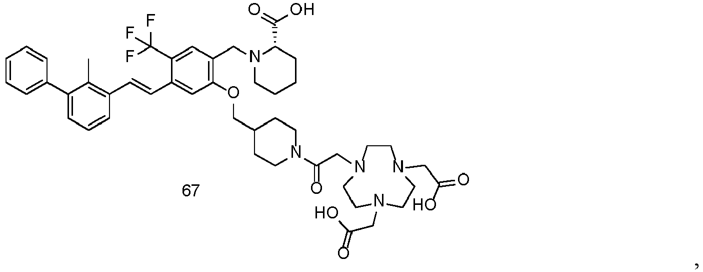 or 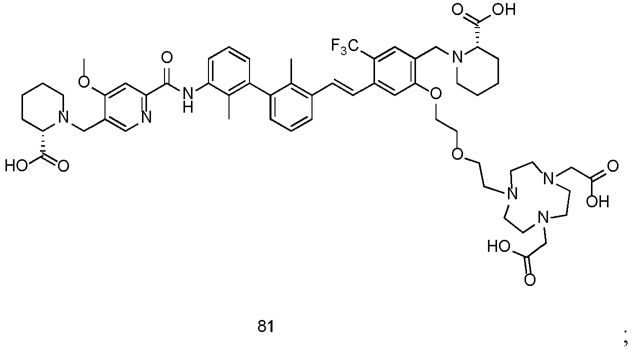
or, the metal complex is a complex formed by any one of the following compounds with [⁶⁸GaCl]²⁺: 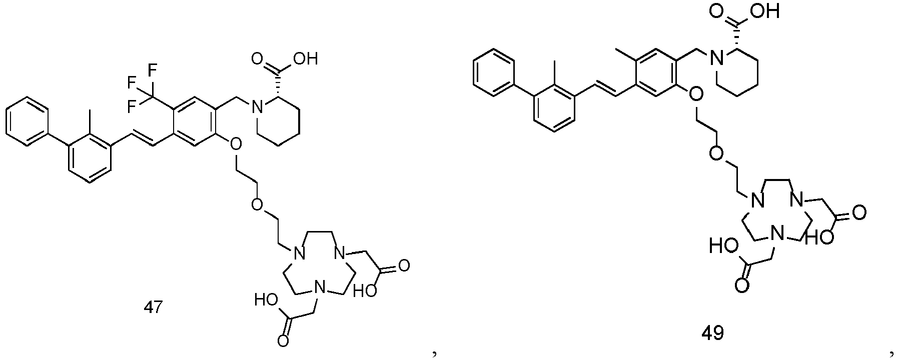 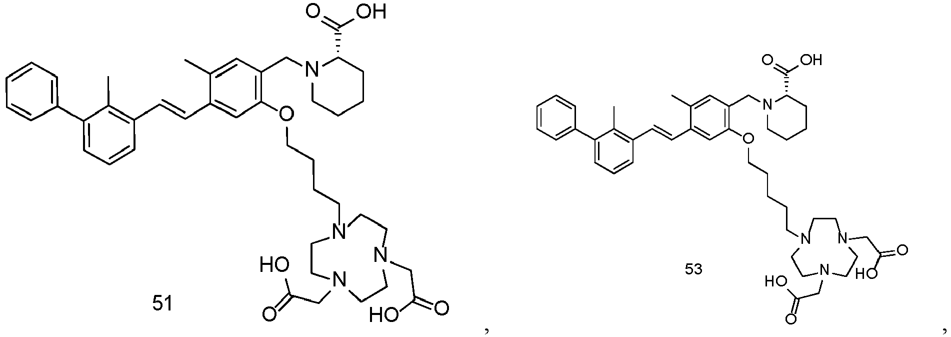 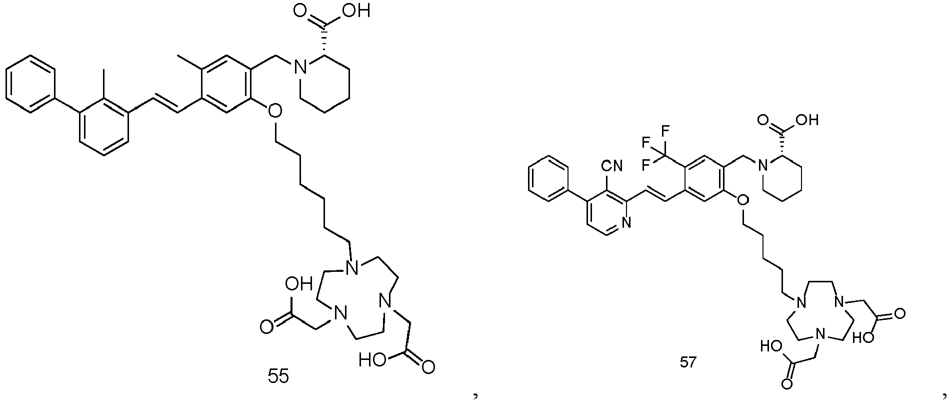 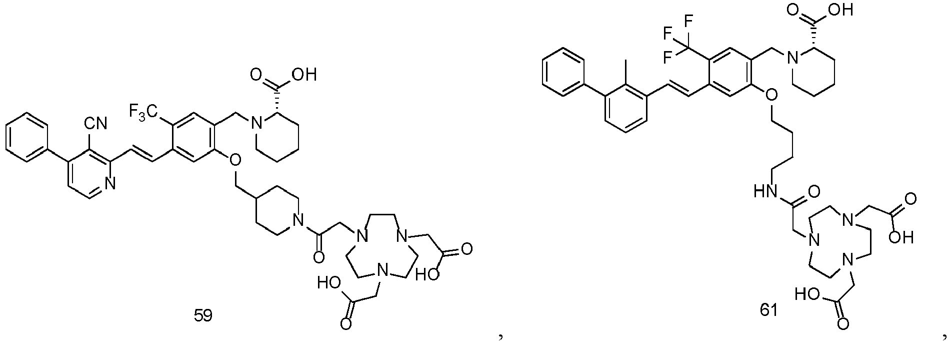 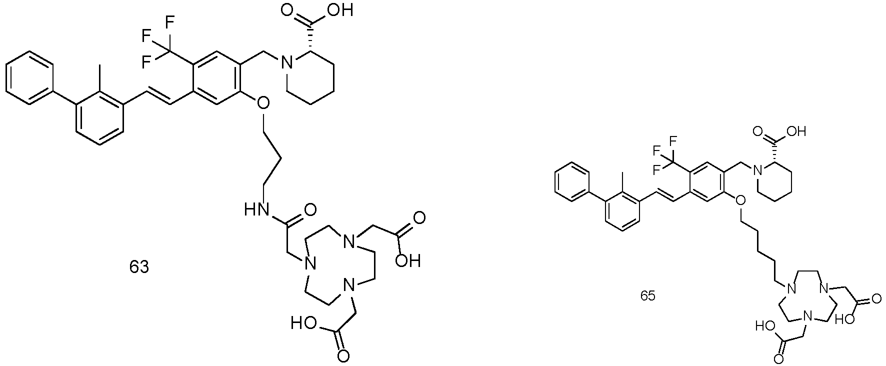 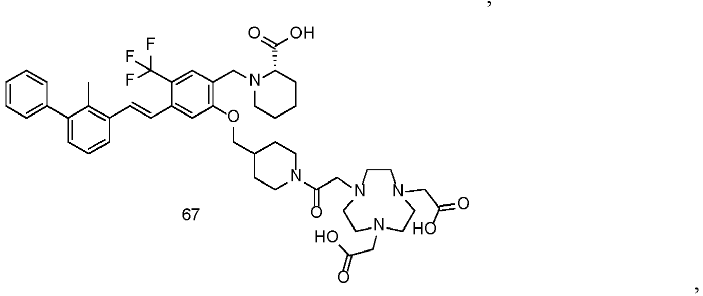 , or 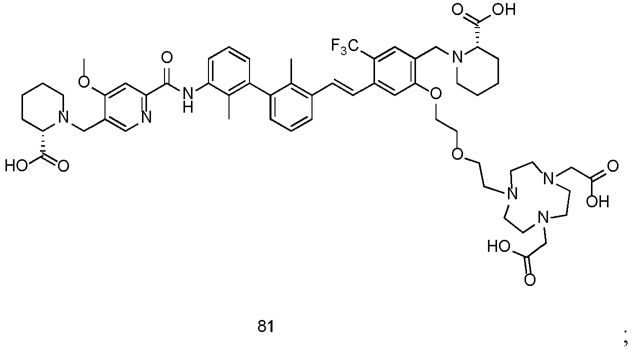
or, the metal complex is a complex formed by any one of the following compounds with [¹⁷⁷LuCl]²⁺:

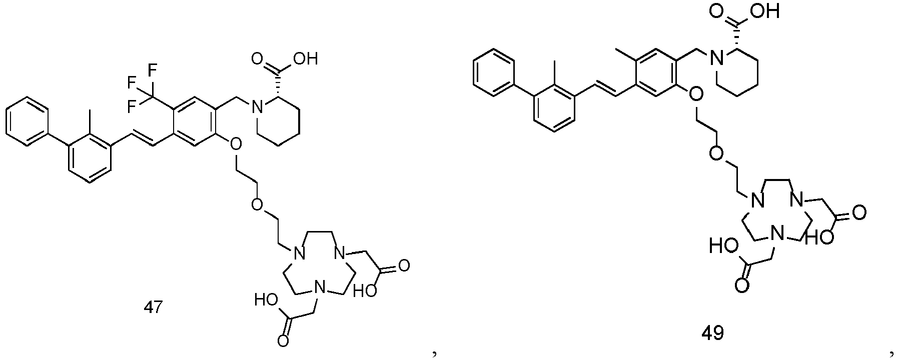

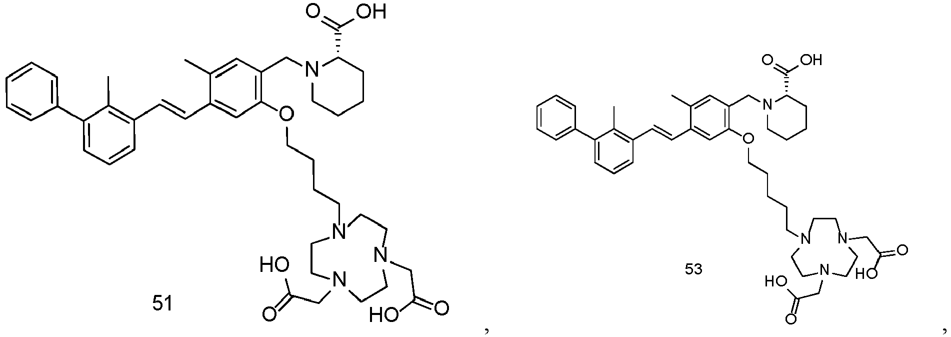

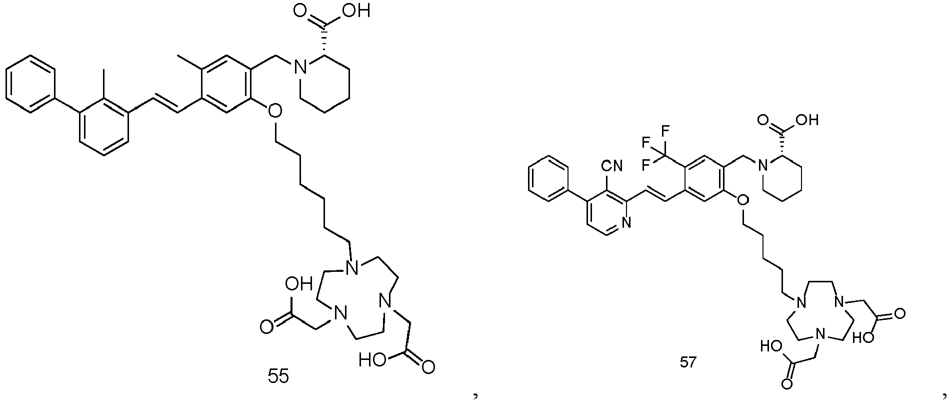

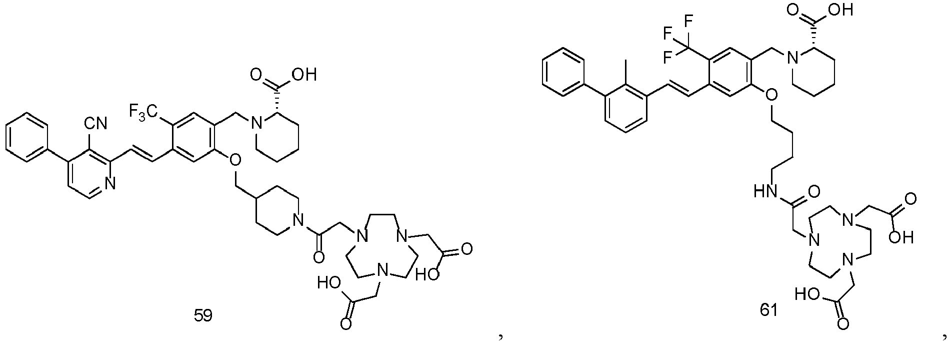

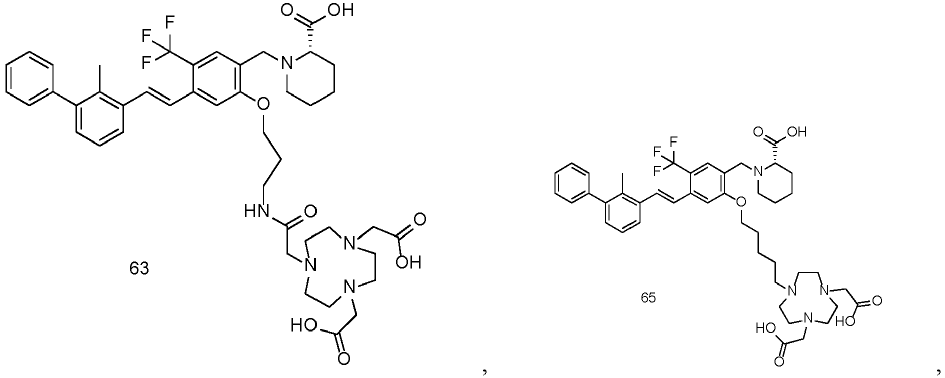

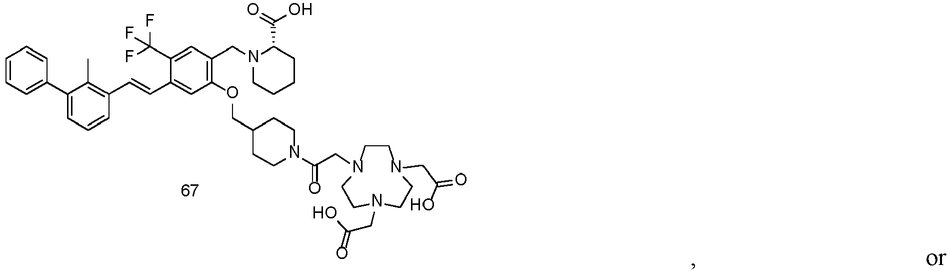

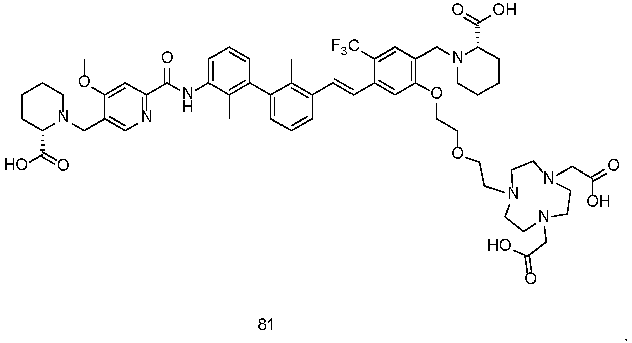

17. The compound represented by general formula II or the metal complex thereof, or their tautomers thereof, or their stereoisomers thereof, or their pharmaceutically acceptable salts thereof, or their solvates thereof according to claim 1 or 9, wherein, the metal complex has any one of the following structures: 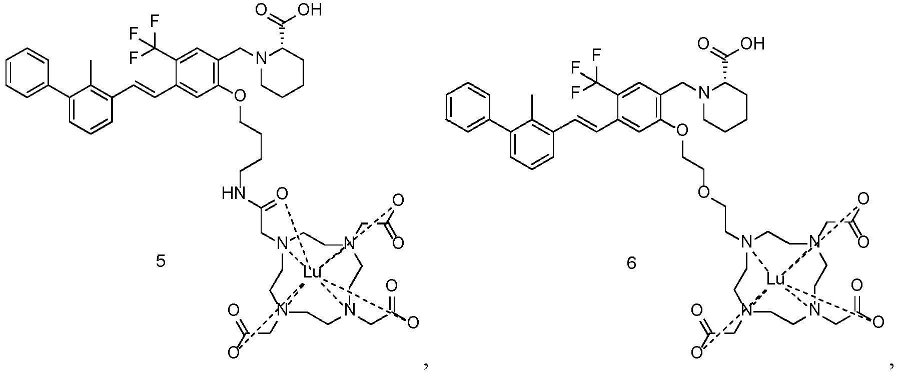 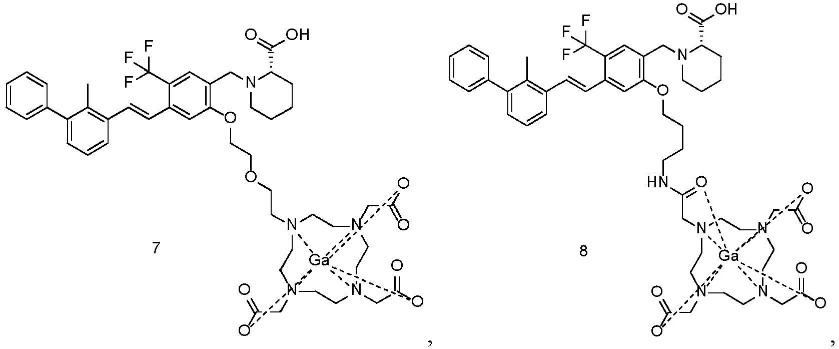 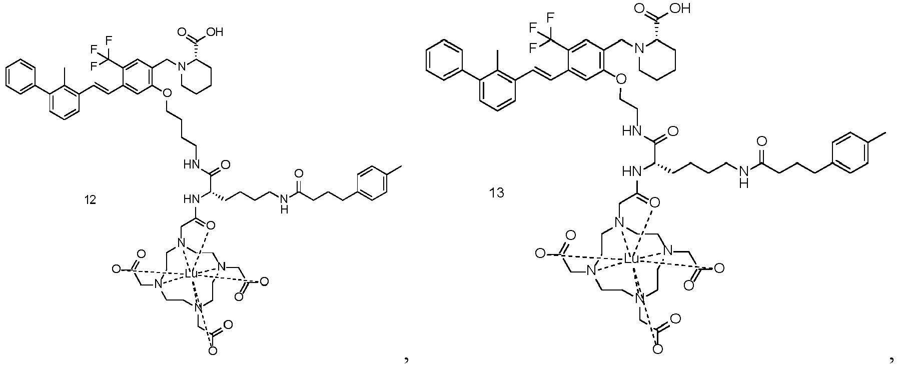 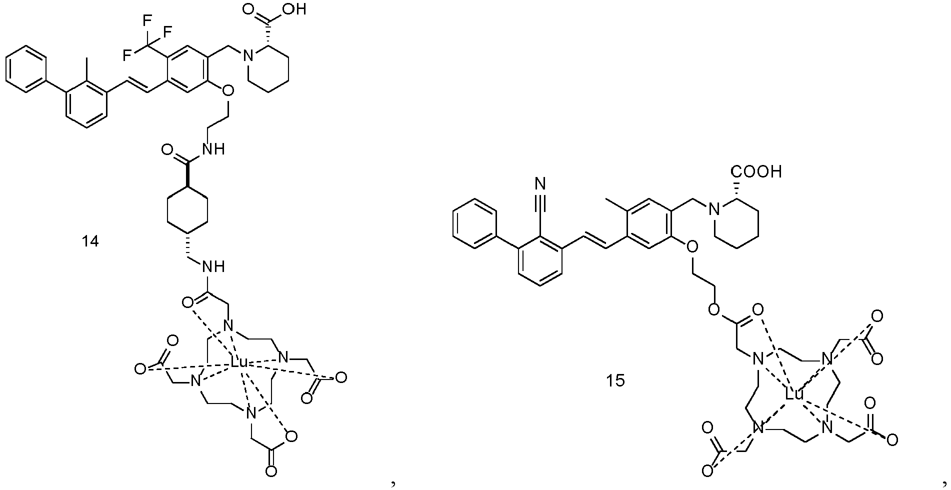 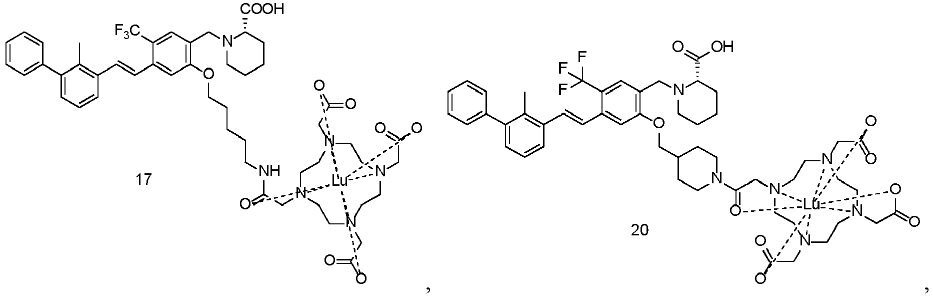 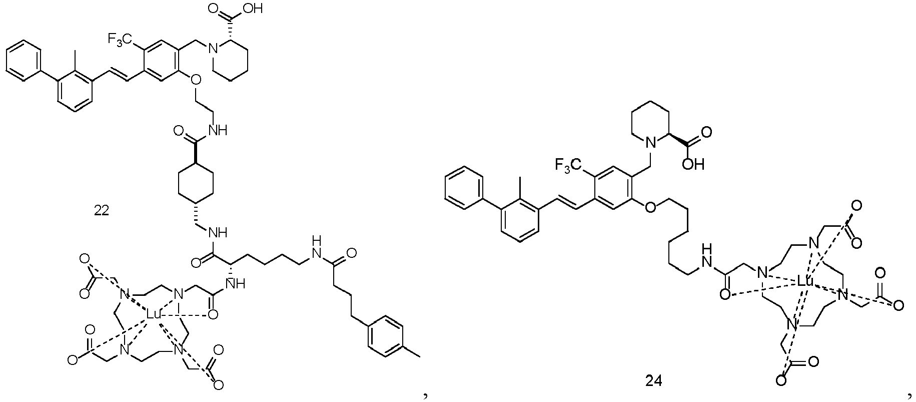 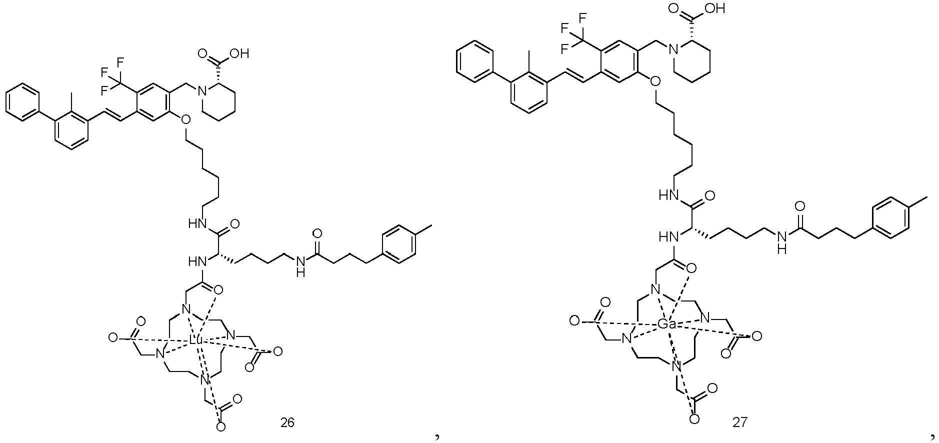 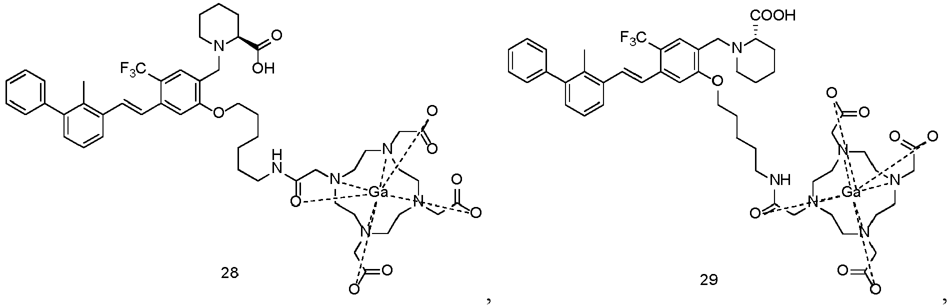 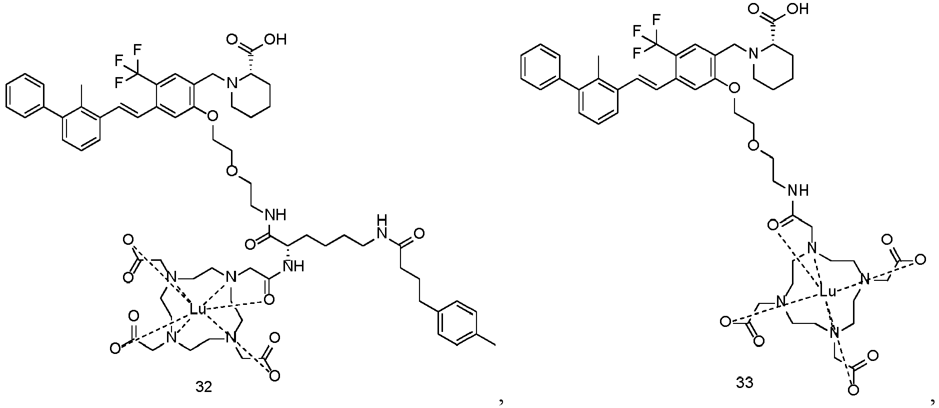 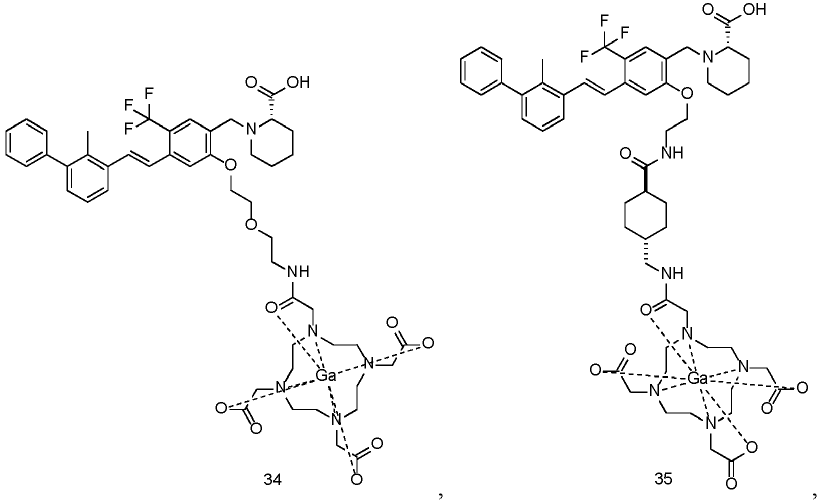 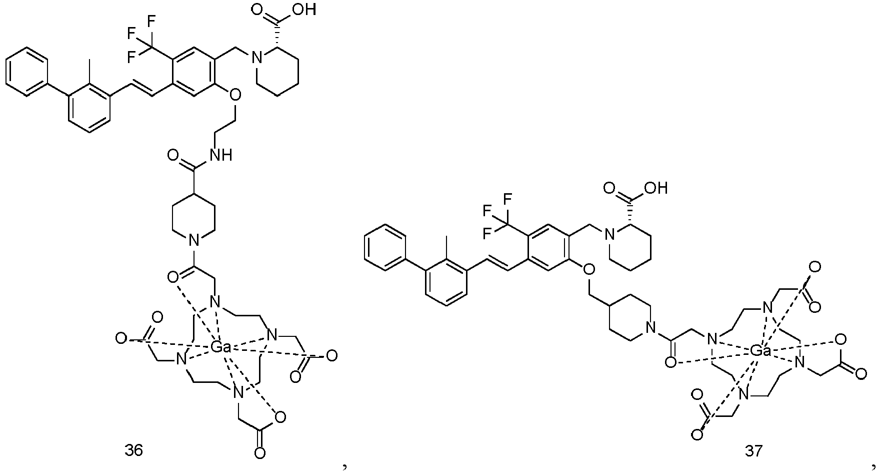 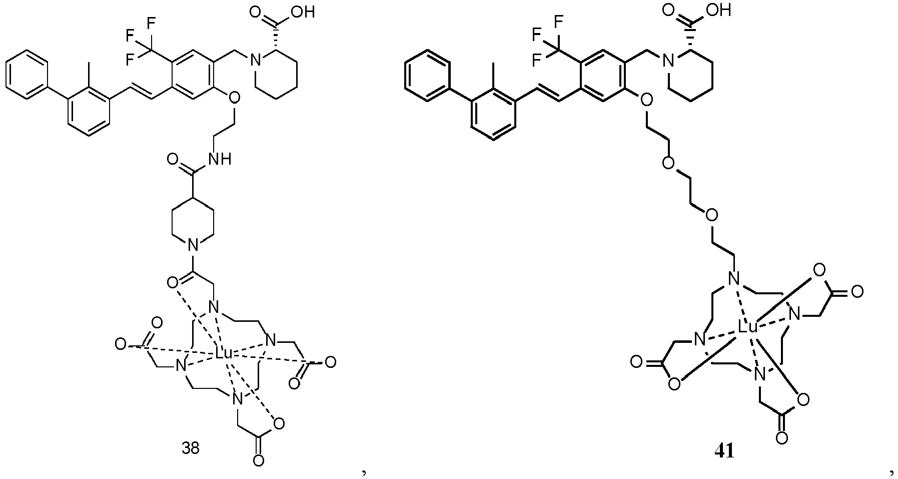 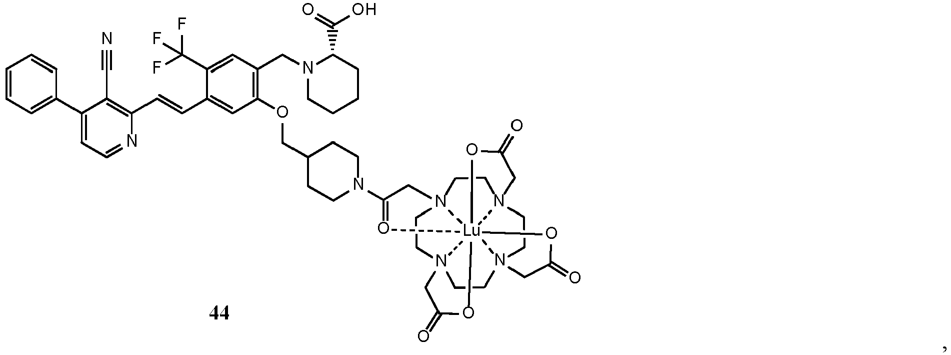 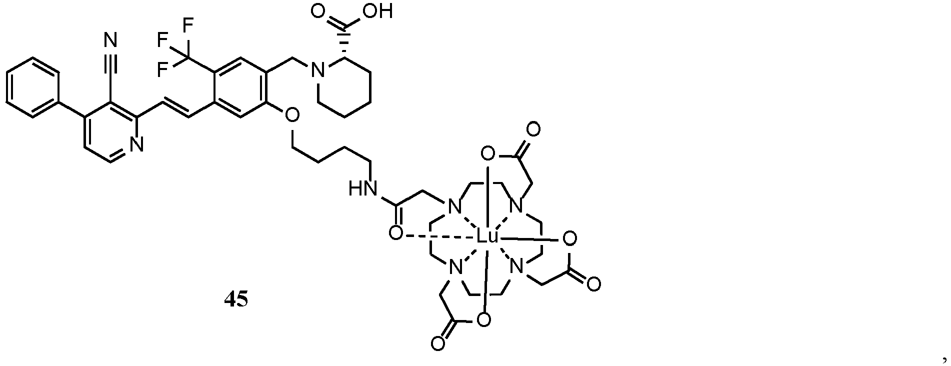 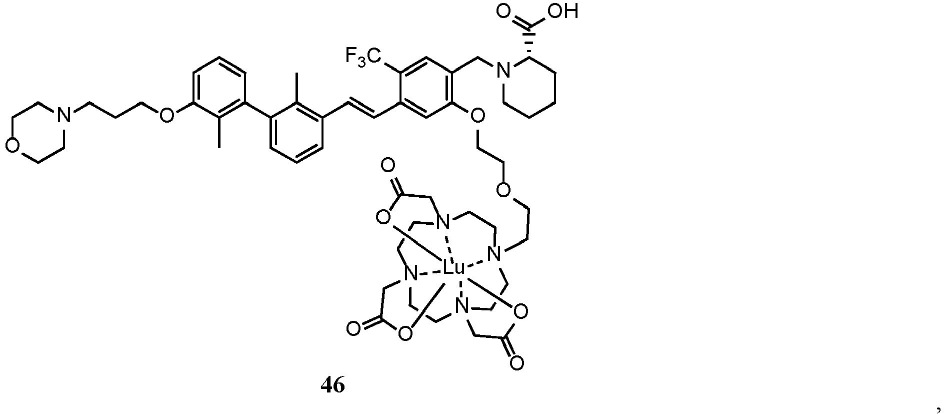 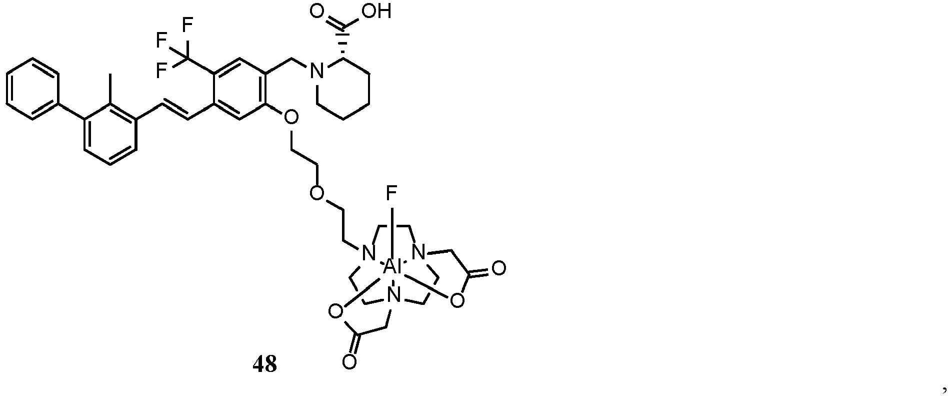 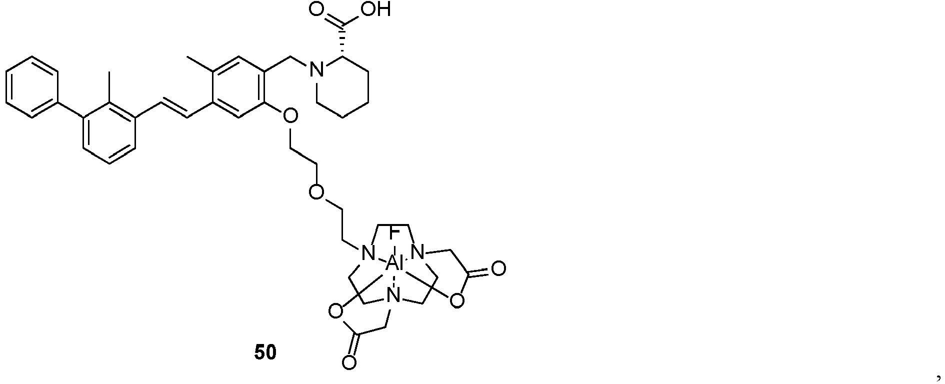 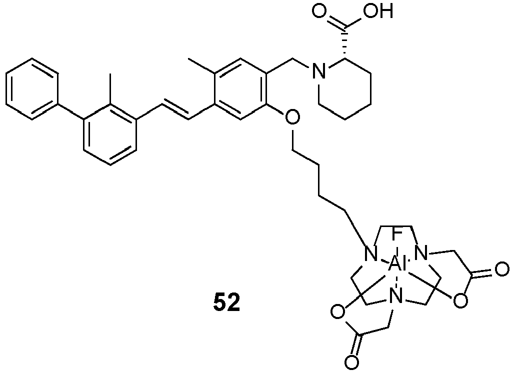 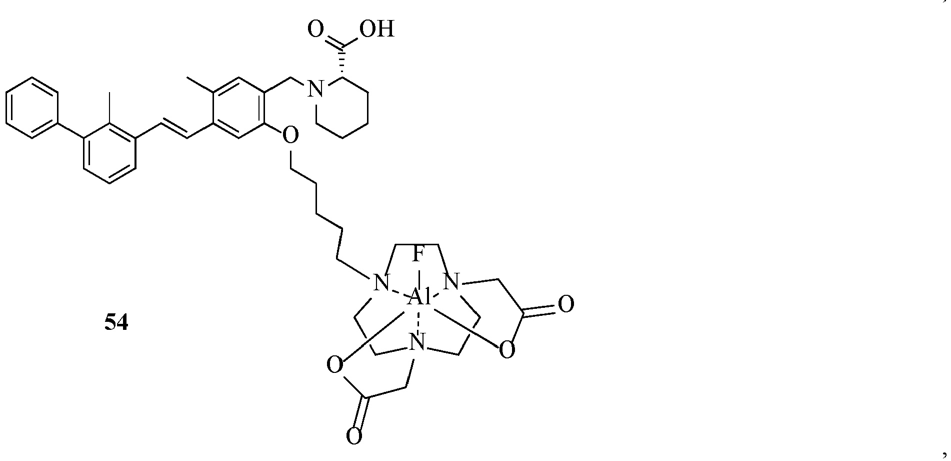 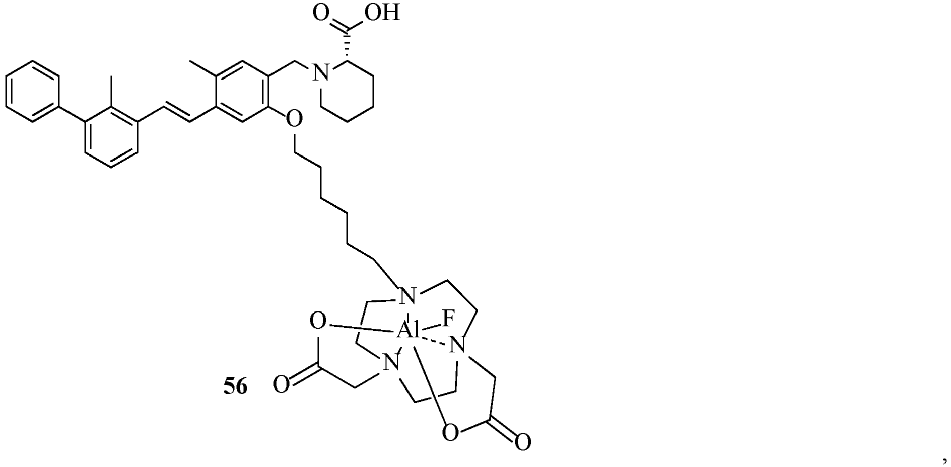 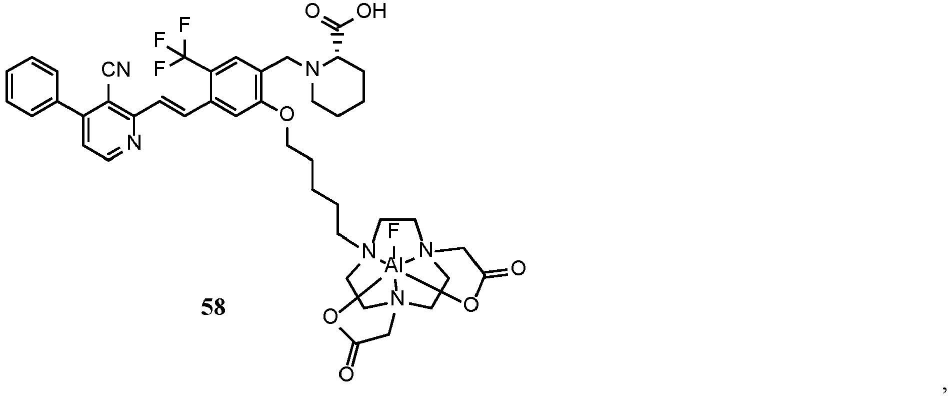 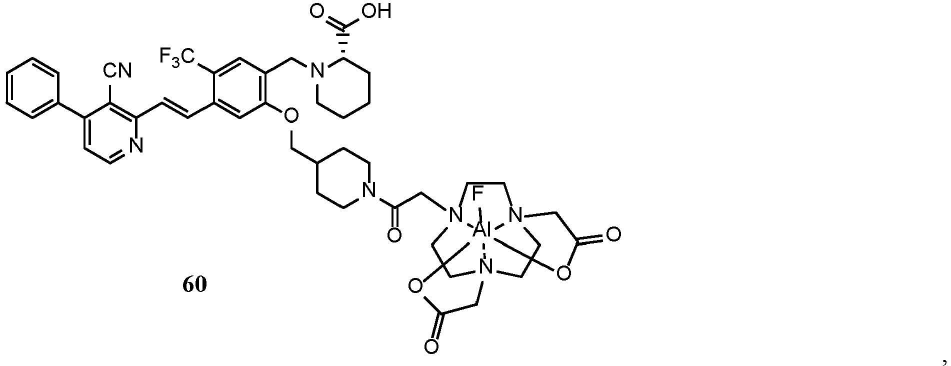 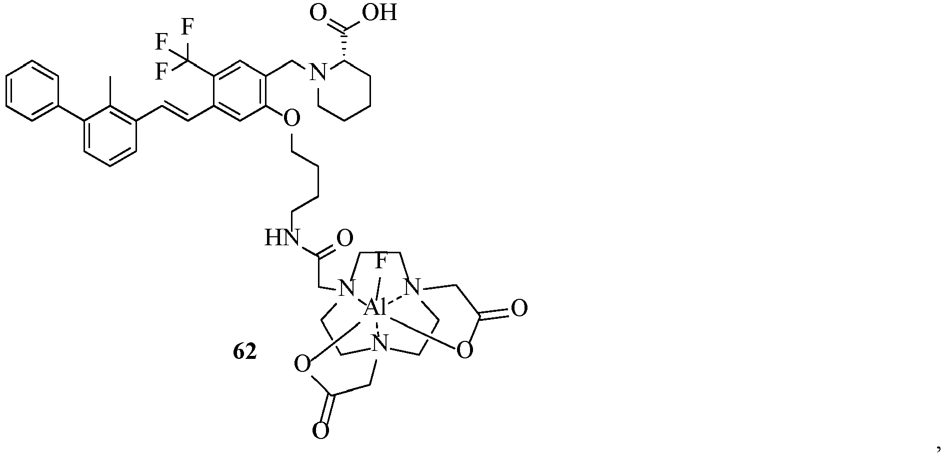 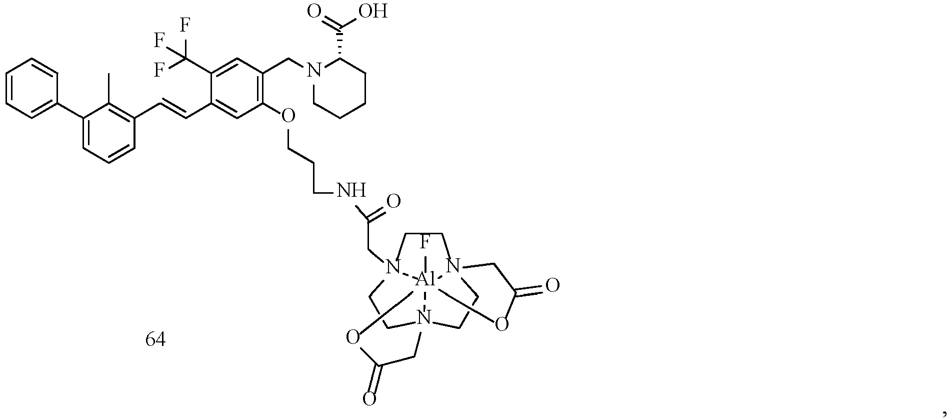 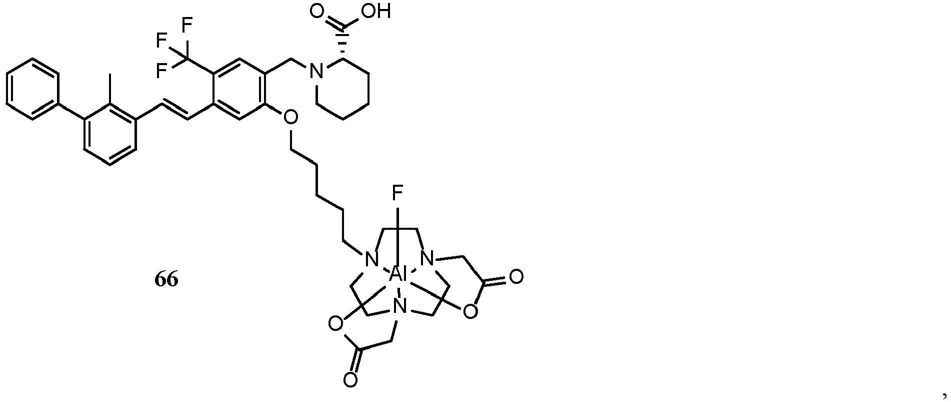 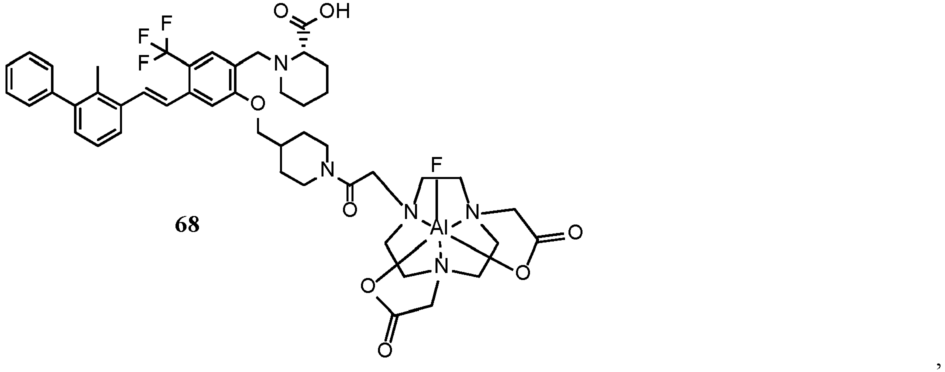 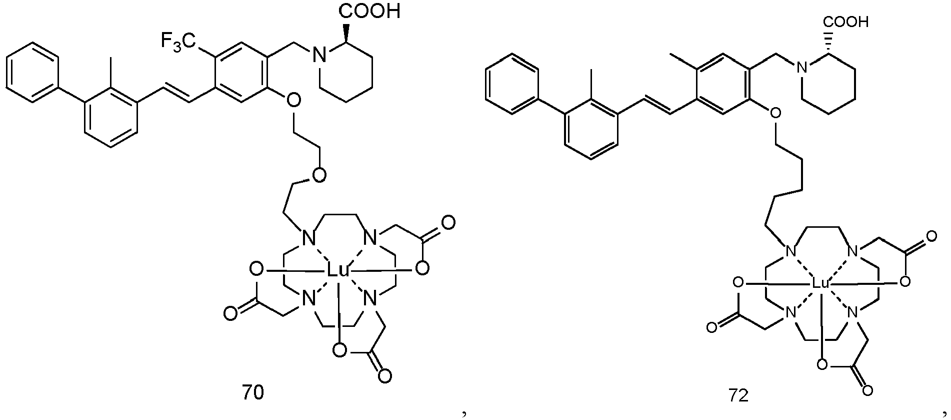 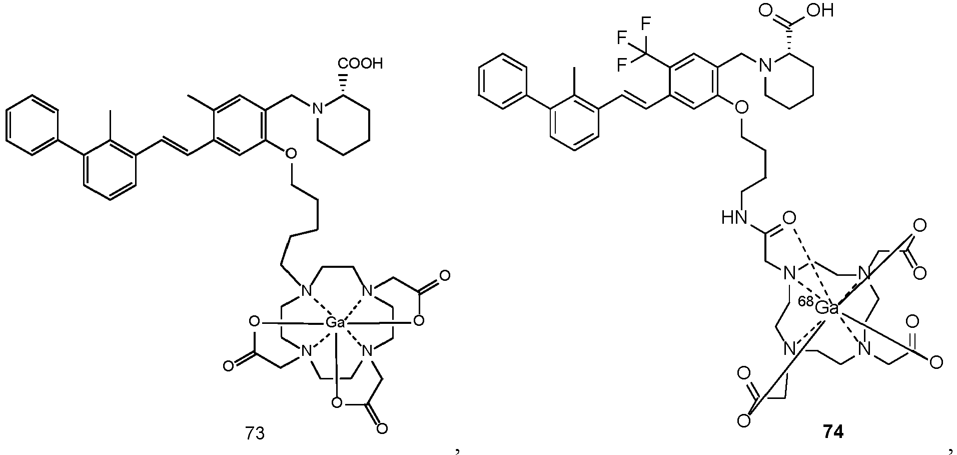 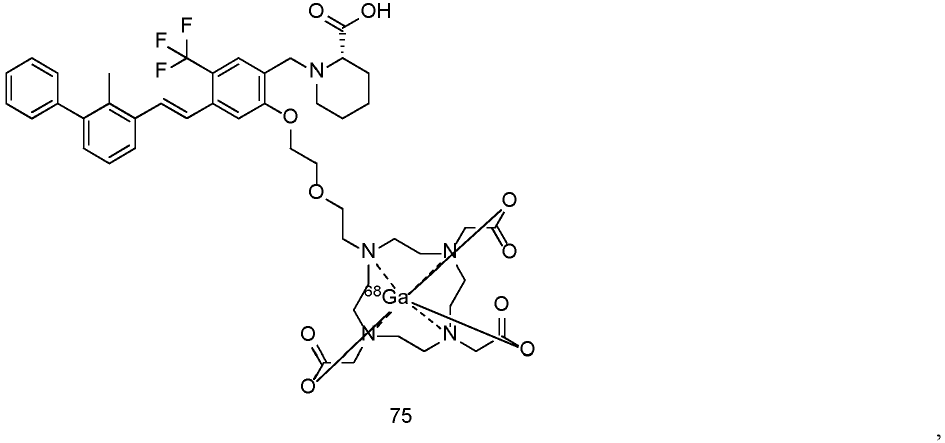 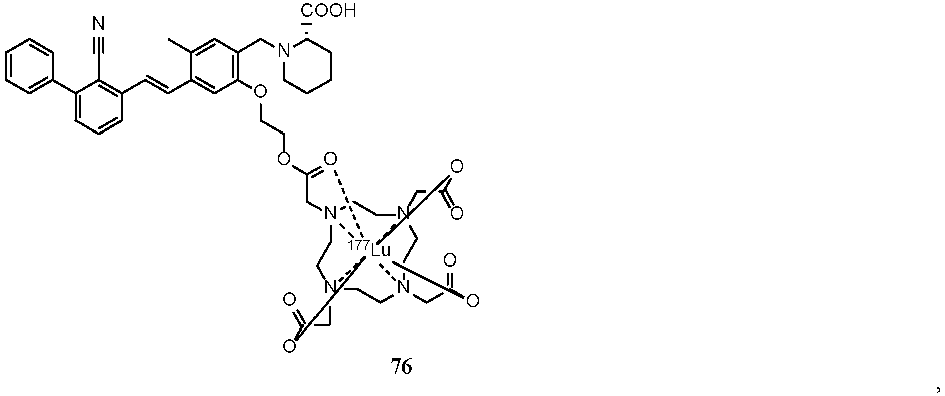 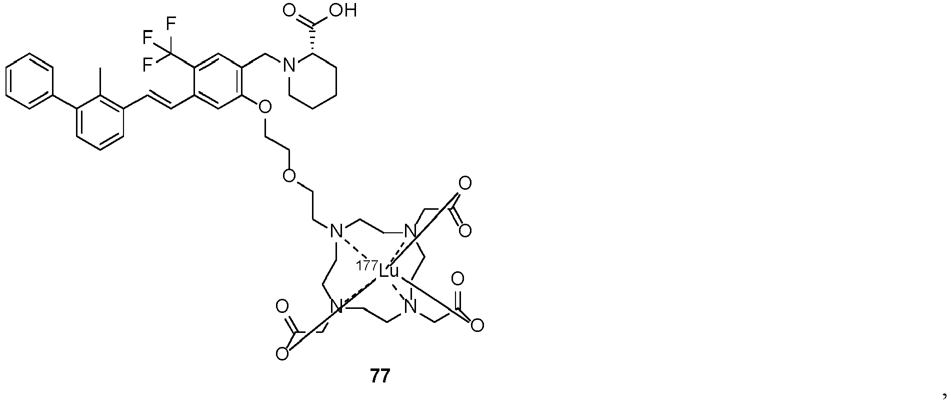 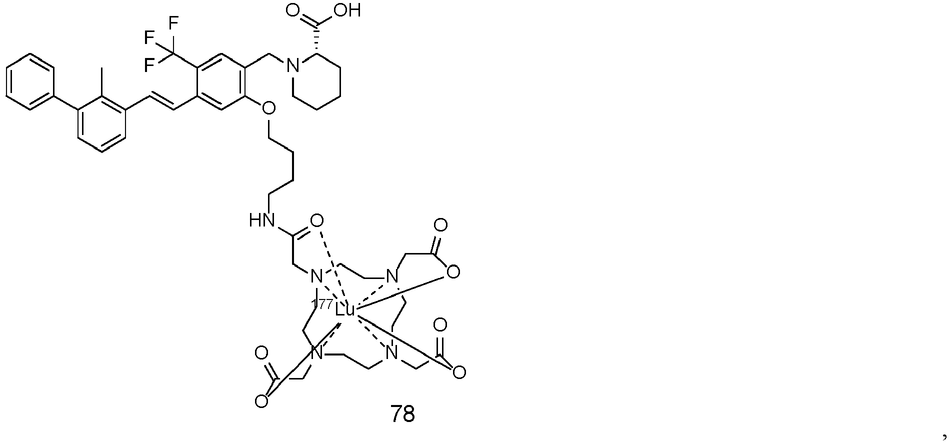 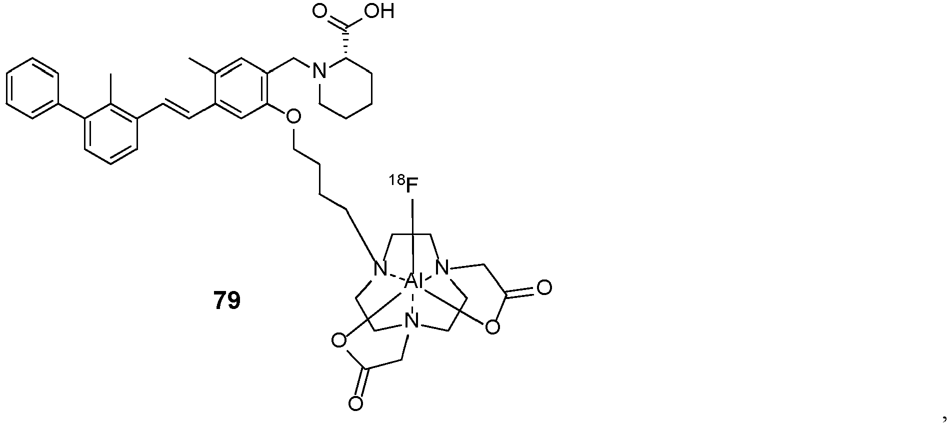 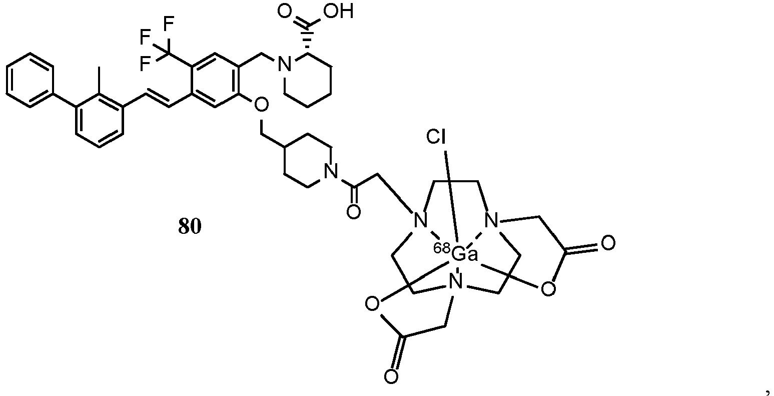 or

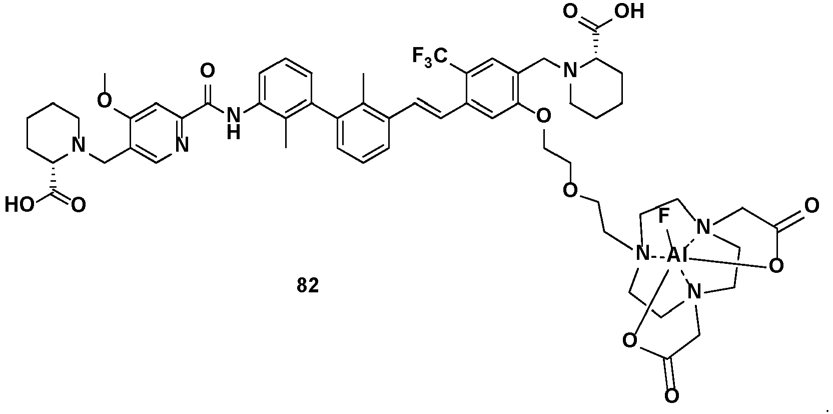

18. A method for preparing the compound represented by the general formula I, which comprises the following steps: in a solvent, reacting the compound represented by the general formula II with M metal halide in the presence or absence of a buffer, to obtain the compound represented by the general formula I; 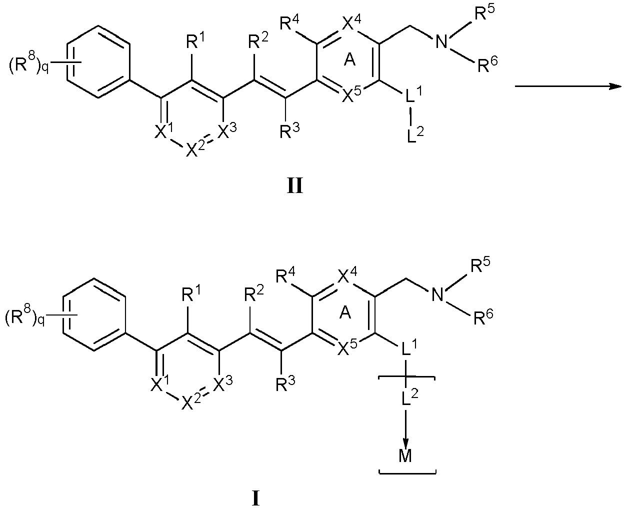 each variable is defined as any one of claims 1-17.

19. A method for preparing the compound represented by the general formula II according to any one of claims 1-17, which comprises the following steps: in a solvent, removing the Boc protective group of the compound represented by the general formula III in the presence of acid, to obtain the compound represented by the general formula II; 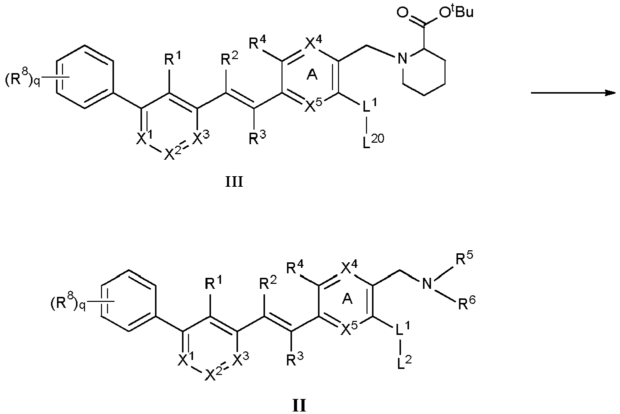
in formula II, L² is R¹¹ or -L⁴-(CH₂)ₛ-R¹¹;
in formula III, L²⁰ is R¹¹⁰ or -L⁴-(CH₂)ₛ-R¹¹⁰;
s is 1, 2, or 3;
L⁴ is 5- to 7-membered carbocyclic ring or 5- to 7-membered heterocyclic ring; in the 5-to 7-membered heterocyclic ring, the number of heteroatoms is 1, 2, 3, or 4, and each heteroatom is independently selected from N, O, and S;
R¹¹ is 8- to 20- membered (for example 8-, 9-, 10-, 11-, 12-, 13-, 14-, 15-, or 16-membered) saturated monocyclic or bridged carbocyclic ring; the 3, 4, 5, or 6 non-adjacent CH₂ of the monocyclic or bridged carbocyclic ring are independently replaced by -Y₅-, and each Y₅ is independently -O-, -NH-, or-N (R^{11a})-;
R¹¹⁰ is 8- to 20-membered (for example 8-, 9-, 10-, 11-, 12-, 13-, 14-, 15-, or 16-membered) saturated monocyclic or bridged carbocyclic ring, and the 3, 4, 5, or 6 non-adjacent CH₂ of the monocyclic or bridged carbocyclic ring are independently replaced by -Y₆-, and each Y₆ is independently -O-, -NH-, or-N (R^{12a})-;
each R^{11a} is independently C₁-C₄ alkyl or C₁-C₄ alkyl substituted by one or more -COOH (for example, -(C₁-C₄ alkylene)-COOH, for example, -CH₂-COOH);
each R^{12a} is independently C₁-C₄ alkyl or C₁-C₄ alkyl substituted by one or more -COO^{t}Bu (for example, -(C₁-C₄ alkylene)-COO^{t}Bu, for example, -CH₂-COO^{t}Bu);
in formula II and formula III, R⁵, R⁶ together with the nitrogen atom to which they are attached form 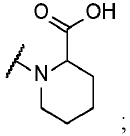
other variables are as defined in any one of claims 1-17.

20. A compound represented by general formula III: 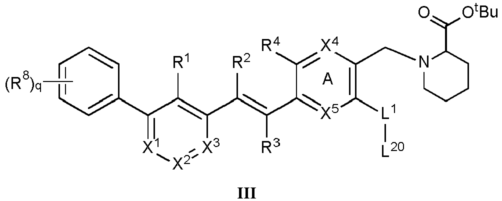 each variable is as defined in claim 19.

21. A compound represented by any one of the following: 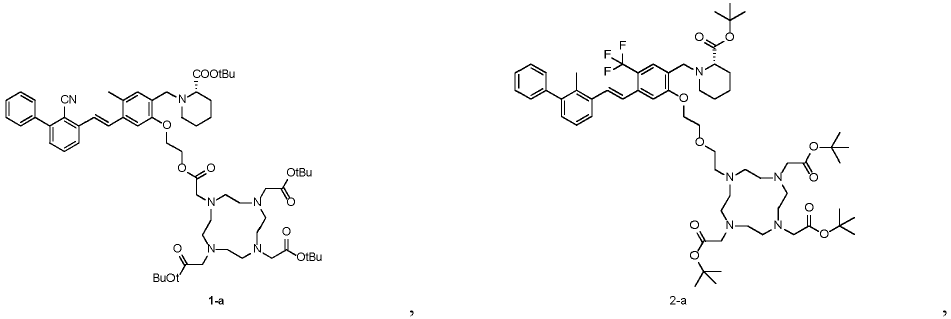 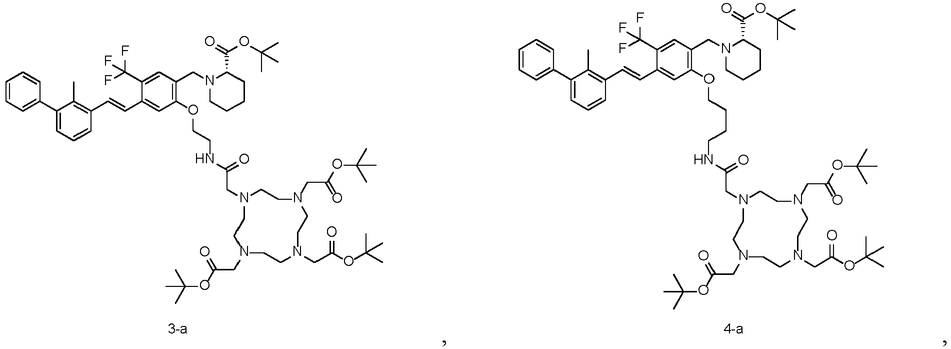 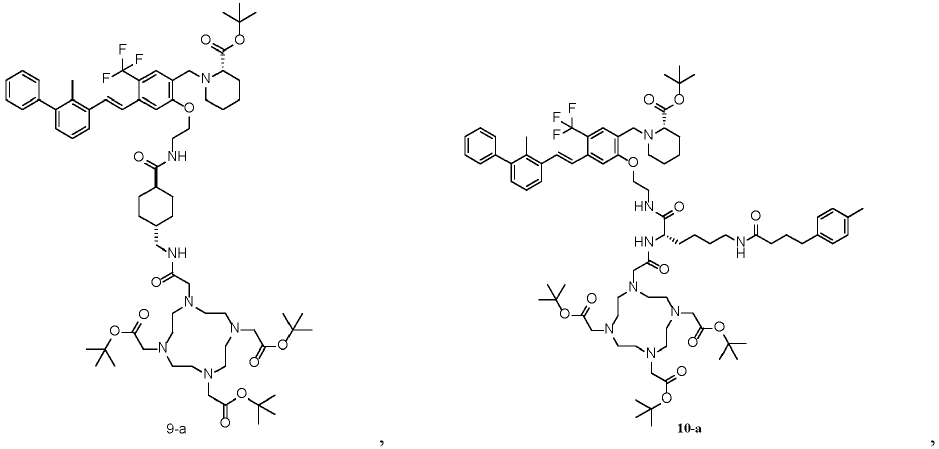 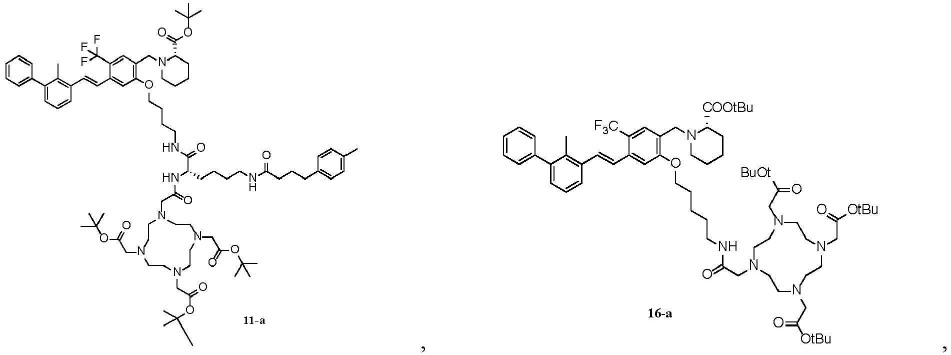 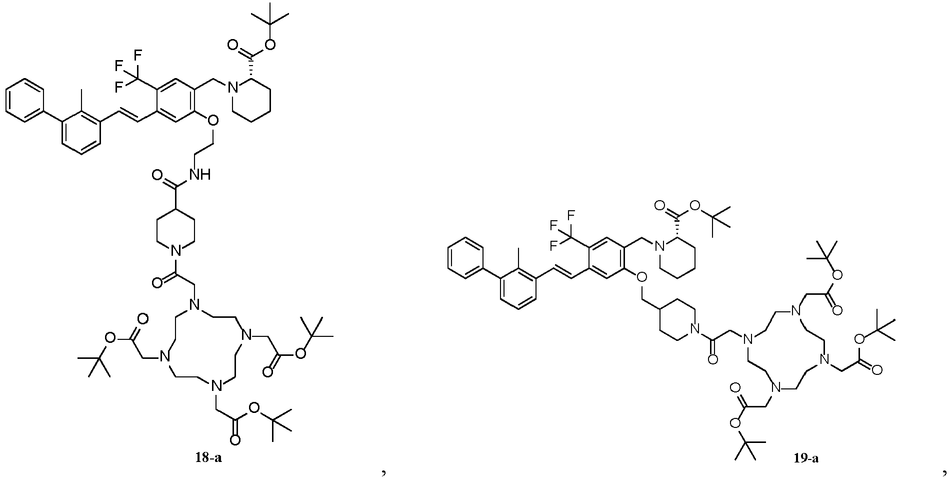 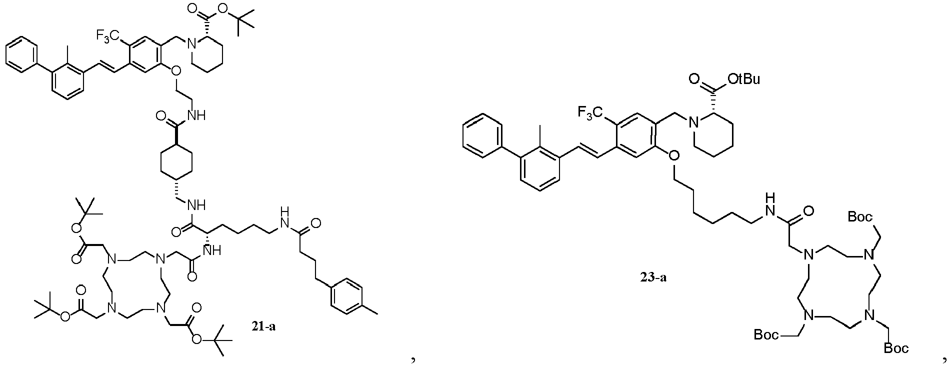 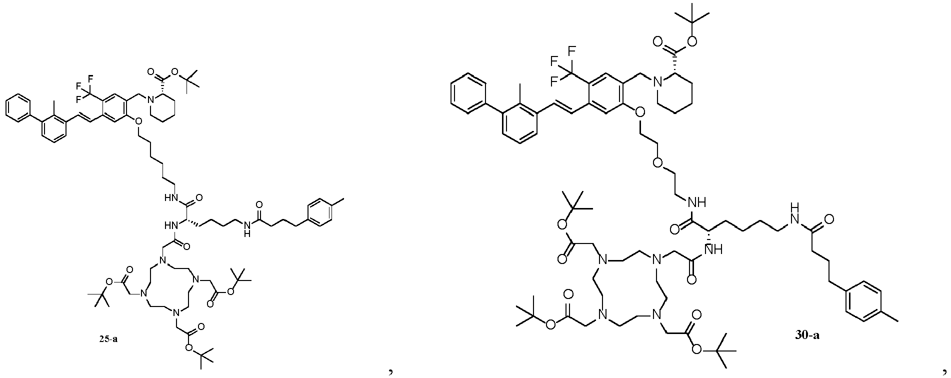 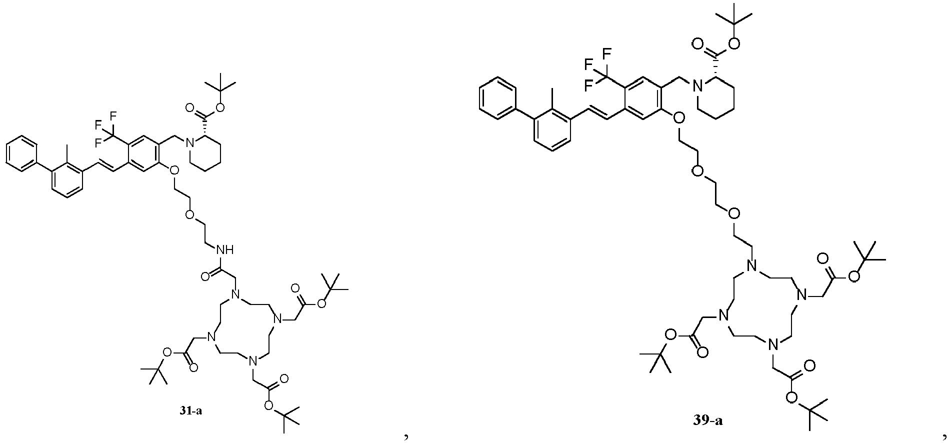 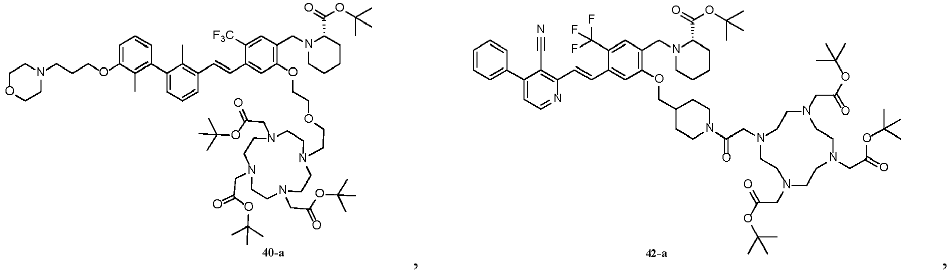 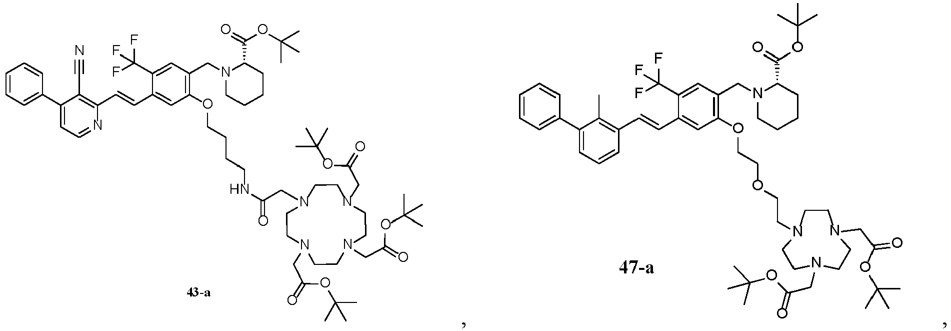 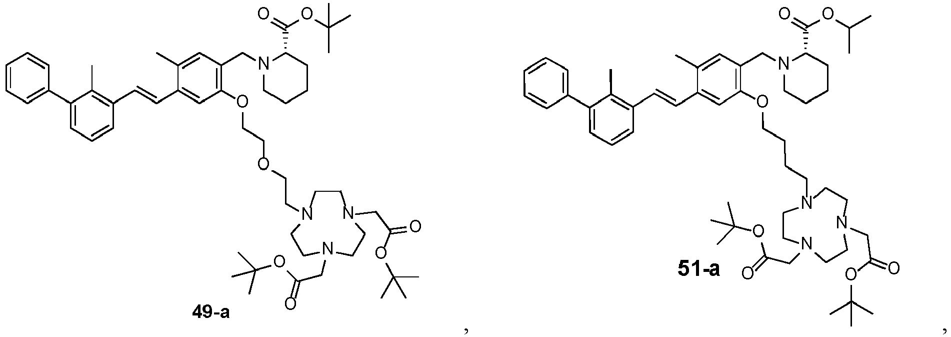 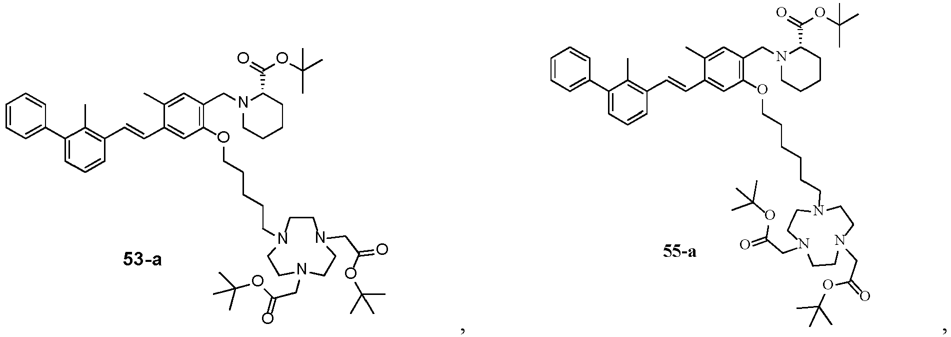 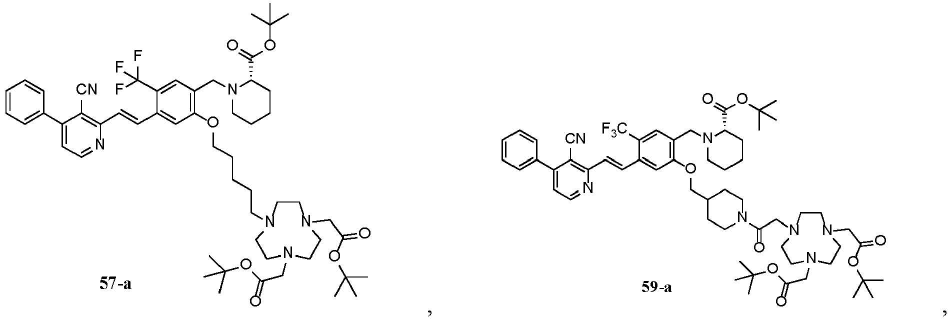 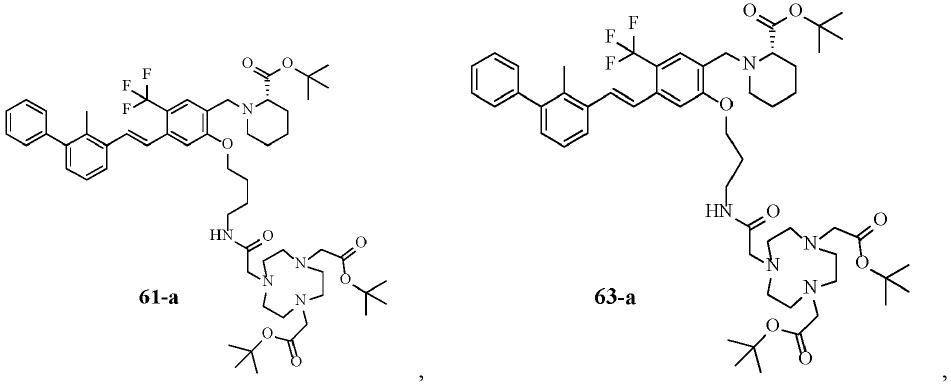 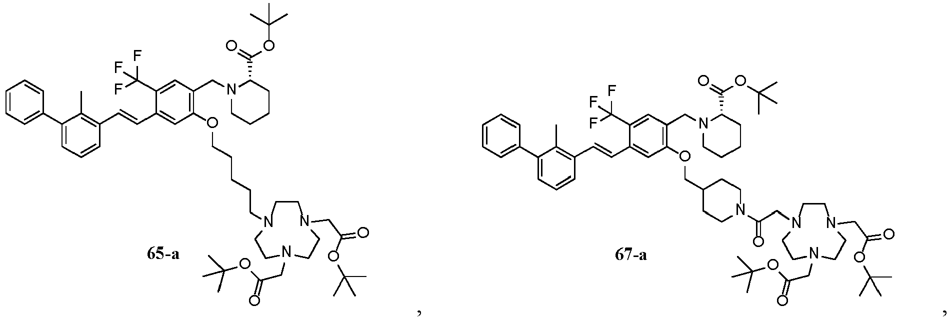 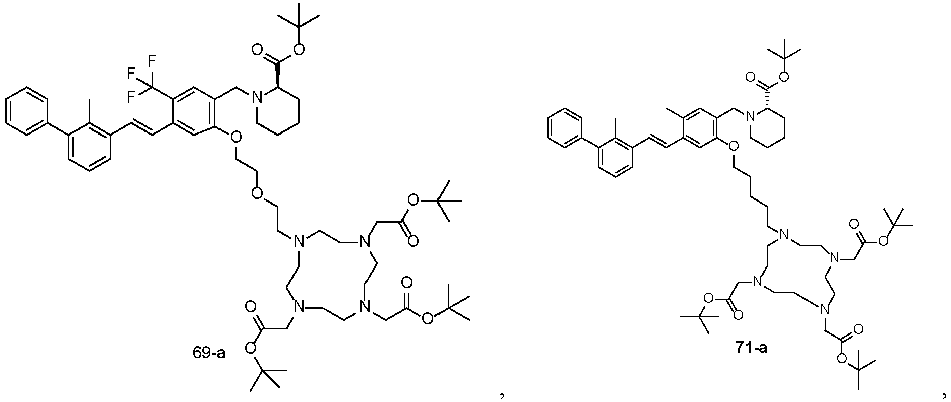 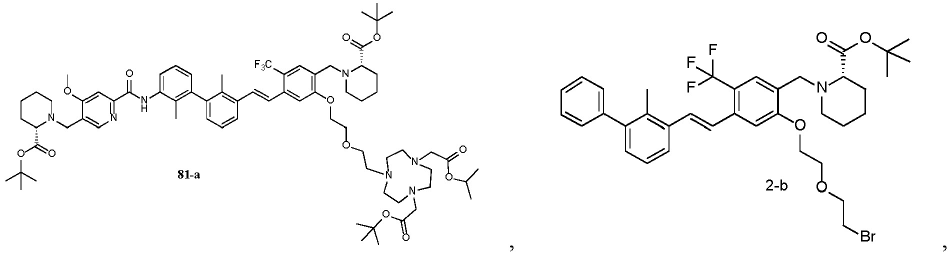 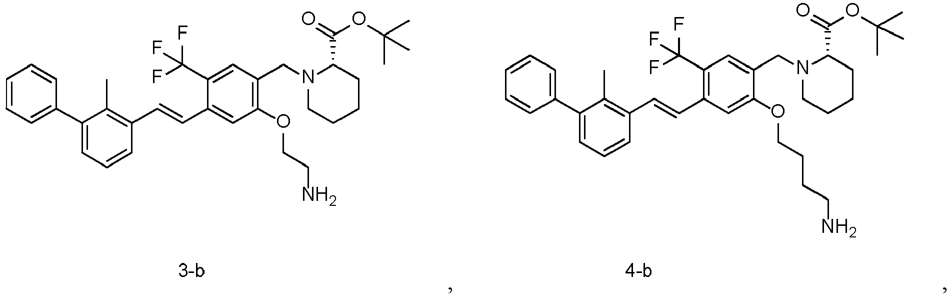 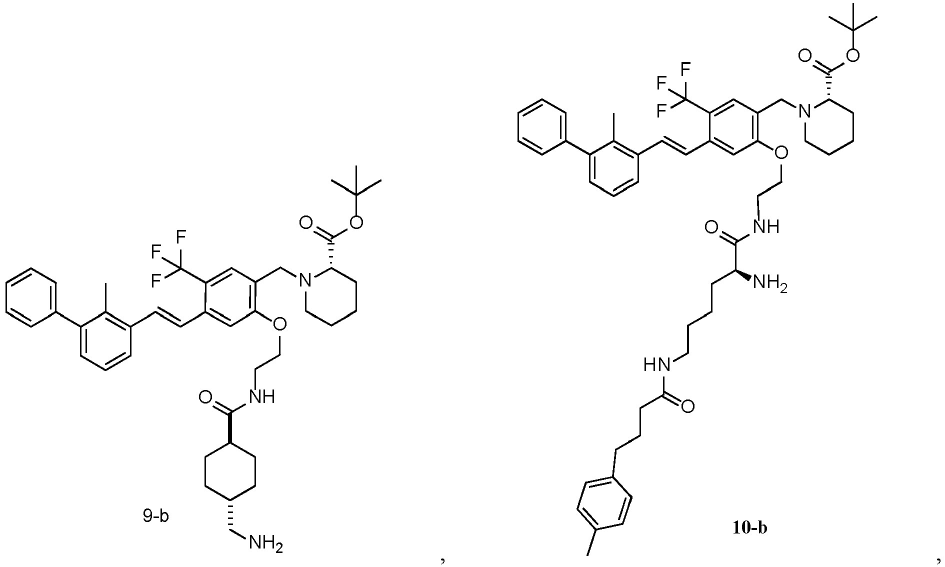 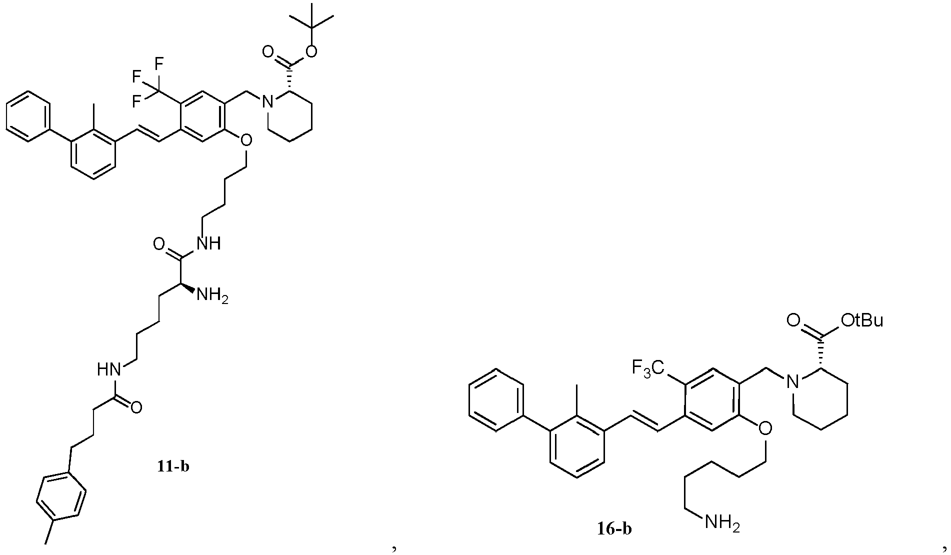 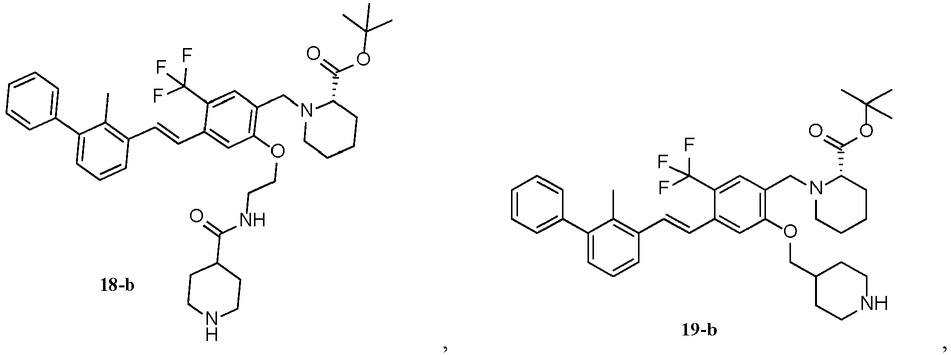 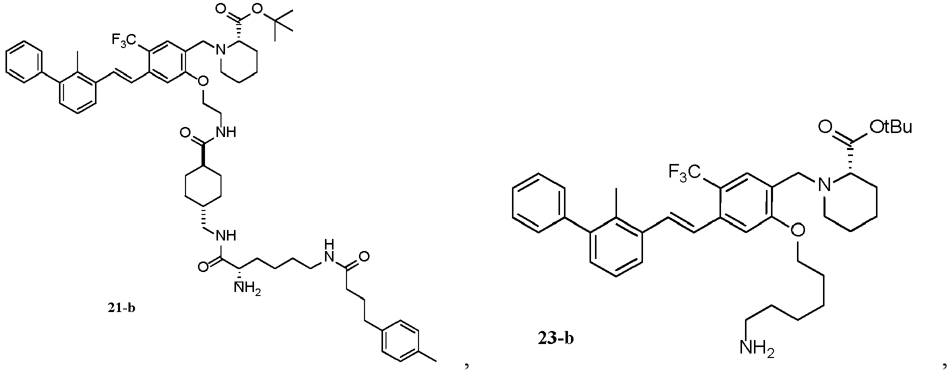 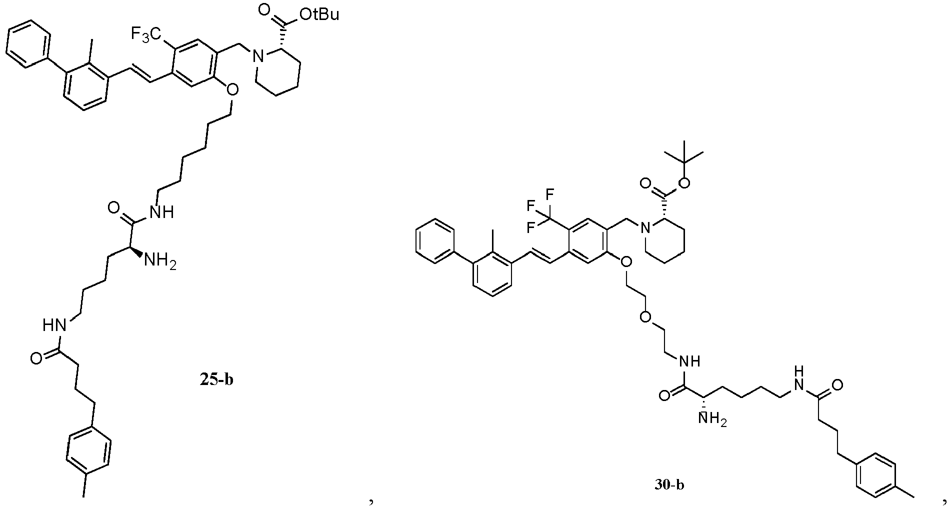 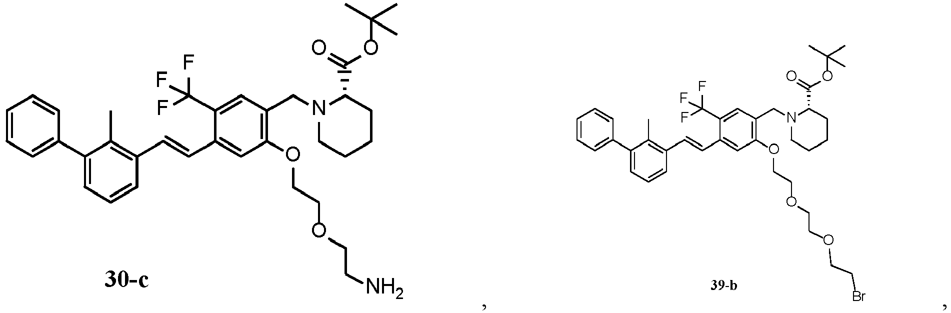 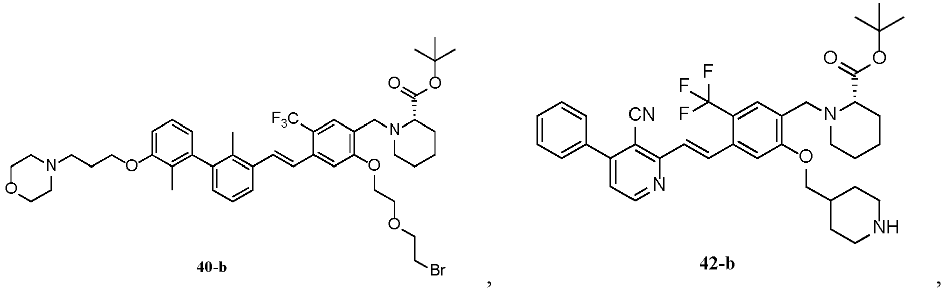 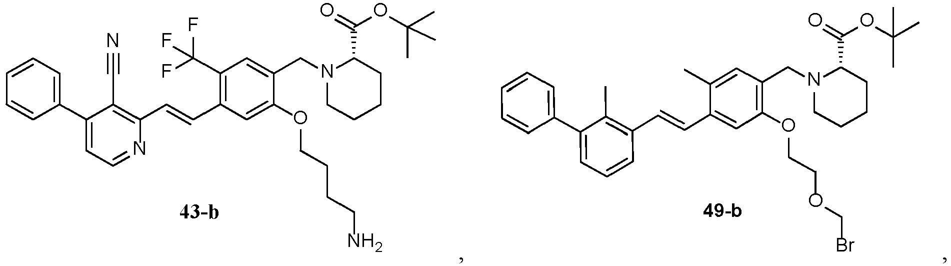 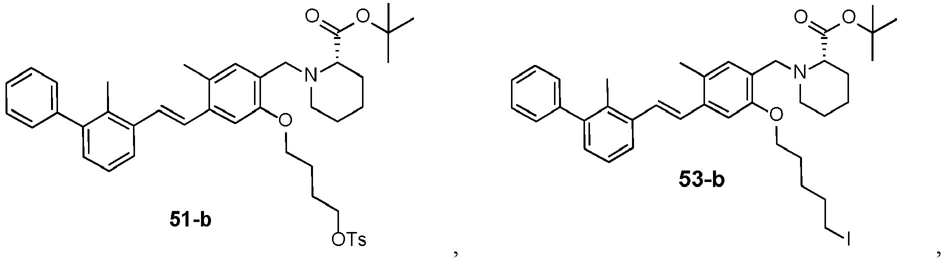 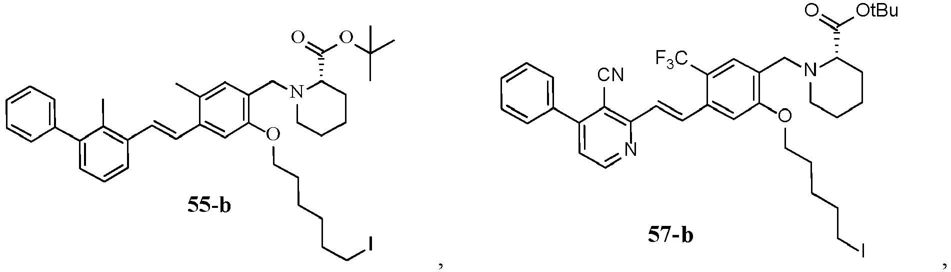 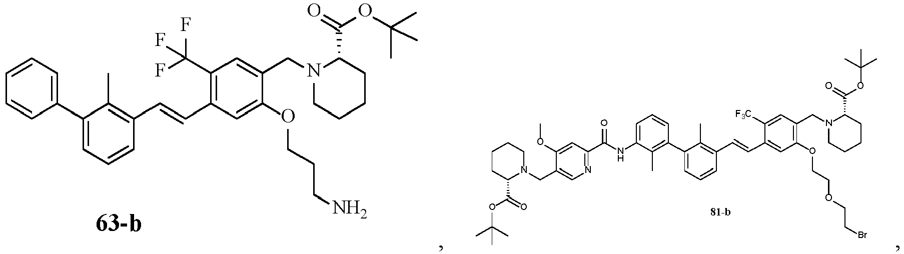 or

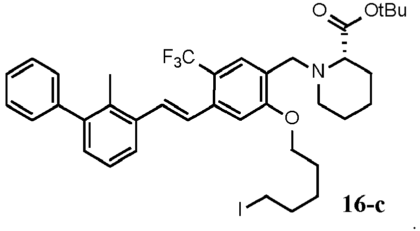

22. A pharmaceutical composition comprising the compound represented by the general formula II or the metal complex thereof, or their tautomers thereof, or their stereoisomers thereof, or their pharmaceutically acceptable salts thereof, or their solvates thereof according to any one of claims 1-17, and at least one pharmaceutical excipient.

23. A use of the compound represented by the general formula II or the metal complex thereof, or their tautomers thereof, or their stereoisomers thereof, or their pharmaceutically acceptable salts thereof, or their solvates thereof according to any one of claims 1-17 in the manufacture of a medicament for the treatment of tumor or a medicament for the radiodiagnosis of tumor; preferably, the metal complex is a complex chelated by the compound represented by general formula II and a radioactive metal ion; the radioactive metal ion is radioactive metal ion for treatment or radioactive metal ion for diagnosis.

24. The use according to claim 23, wherein, the tumor is lung cancer, gastric cancer, colorectal cancer, cervical cancer, ovarian cancer, prostate cancer, breast cancer, pancreatic cancer, liver cancer, bladder cancer, renal cancer, bone cancer, skin cancer, melanoma, glioma, glioblastoma, leukemia, or lymphoma.
